# EUROPEAN PATENT SPECIFICATION

(11) **EP 1 998 751 B1**
(45) Date of publication and mention of the grant of the patent: **19.08.2020**
(21) Application number: 07754184.5
(22) Date of filing: 28.03.2007
(51) Int. Cl.: A61M 5/14, A61M 5/142, A61M 5/00, A61M 5/19, A61M 5/20, A61M 5/24, A61M 5/31, A61M 5/315, A61M 5/32, A61M 15/00, G09F 3/20, G09F 3/10, G09F 27/00, G09F 3/02, G09B 23/28

(54) **SYSTEM FOR MEDICAMENT DELIVERY**
SYSTEM ZUR MEDIKAMENTENABGABE
SYSTÈME POUR ADMINISTRATION D'UN MÉDICAMENT

(30) Priority: 29.03.2006 US 787046 P; 09.01.2007 US 621236; 05.02.2007 US 671025
(43) Date of publication of application: 10.12.2008
(73) Proprietor: Kaleo, Inc., Richmond VA 23219 (US)
(72) Inventor: EDWARDS, Eric, Shawn, Midlothian, Virginia 23112 (US); LICATA, Mark, J., Doswell, VA 23047 (US); EDWARDS, Evan, Thomas, Gordonsville, Virginia 22942 (US); MEYERS, Paul, F., Fishers, Indiana 46037 (US); WEINZEIRL, David, A., Andover, Minnesota 55304 (US); WILLIAMSON, T.Spencer, Richmond, Virginia 23219 (US); WORRELL, Kai R., Richmond, Virginia 23219 (US)
(74) Representative: Gill Jennings & Every LLP
(86) International application number: PCT/US2007/007626
(87) International publication number: WO 2007/126851

(56) References cited:
- WO-A1-2007/087304
- US-A1- 2002 188 259
- US-A1- 2002 188 419
- US-A1- 2004 260 238
- US-A1- 2005 096 588
- US-A1- 2005 168 337
- US-A1- 2005 171 477
- US-A1- 2006 030 819
- US-B1- 6 514 230
- US-B1- 6 544 233
- US-B2- 6 726 661

## Description

The invention relates generally to a medical device, and more particularly to an medicament delivery device for automatically injecting a medicament into a body of a patient.

Exposure to certain substances, such as, for example, peanuts, shellfish, bee venom, certain drugs, toxins, and the like, can cause allergic reactions in some individuals. Such allergic reactions can, at times, lead to anaphylactic shock, which can cause a sharp drop in blood pressure, hives, and/or severe airway constriction. Accordingly, responding rapidly to mitigate the effects from such exposures can prevent injury and/or death. For example, in certain situations, an injection of epinephrine (i.e., adrenaline) can provide substantial and/or complete relief from the allergic reaction. In other situations, for example, an injection of an antidote to a toxin can greatly reduce and/or eliminate the harm potentially caused by the exposure.

Because emergency medical facilities may not available when an individual is suffering from an allergic reaction, some individuals carry an auto-injector to rapidly self-administer a medicament in response to an allergic reaction. Some known auto-injectors are cylindrical in shape and include a spring loaded needle to automatically penetrate the user's skin and inject the medicament. Such known auto-injectors can be bulky and conspicuous, which can make carrying them inconvenient and undesirable. Moreover, some known auto-injectors do not have a retractable needle and, as such, cause a sharps hazard when injection is complete.

Some known auto-injectors include a locking cap at the proximal end of the auto-injector to prevent inadvertent actuation and a needle cover at the distal end of the auto-injector. Such a configuration can, at times, cause a user to become confused as to which end of the auto-injector is the "needle end" (i.e., the distal end) and which end of the auto-injector is the "actuation end" (i.e., the proximal end). As such, in some situations, a user may mistakenly actuate the known auto-injector away from the intended injection site. Such an error can result, for example, in the auto-injector being actuated into the user's thumb and/or finger.

Thus, a need exists for an auto-injector that can be more conveniently carried by a user and does not present a sharps hazard upon completion of the injection. Furthermore, a need exists for an auto-injector that can be actuated from its distal end.

To actuate such a medicament delivery device, however, the user may be required to execute a series of operations. For example, to actuate some known auto-injectors, the user must remove a protective cap, remove a locking device, place the auto-injector in a proper position against the body and then press a button to actuate the auto-injector. Failure to complete these operations properly can result in an incomplete injection and/or injection into an undesired location of the body. In certain instances, for example, users who have become confused in the operation of some known auto-injectors have inadvertently injected the medicament into their thumb by improperly positioning the auto-injector.

The likelihood of improper use of known medicament delivery devices can be compounded by the nature of the user and/or the circumstances under which such devices are used. For example, many users are not trained medical professionals and may have never been trained in the operation of such devices. Moreover, in certain situations, the user may not be the patient, and may therefore have no experience with the medicament delivery device. Similarly, because some known medicament delivery devices are configured to be used relatively infrequently in response to an allergic reaction or the like, even those users familiar with the device and/or who have been trained may not be well practiced at operating the device. Finally, such devices are often used during an emergency situation, during which even experienced and/or trained users may be subject to confusion, panic and/or the physiological effects of the condition requiring treatment.

Some known medicament delivery devices include printed instructions to inform the user of the steps required to properly deliver the medicament. Such printed instructions, however, can be inadequate for the class of users and/or the situations described above. Moreover, because some known medicament delivery devices, such as, for example, auto-injectors, pen injectors, inhalers or the like, can be compact, such printed instructions may be too small to read and comprehend during an emergency situation.

Some known medicament delivery devices include and electronic system to assist the user in setting the proper dosage and/or maintaining a compliance log. Such known medicament delivery devices and the accompanying electronic systems can be large and therefore not conveniently carried by the user. Moreover, such known medicament delivery devices and the accompanying electronic systems can be complicated and/or expensive to manufacture.

Thus, a need exists for a medicament delivery device and/or a medicament container that can be conveniently carried by a user, that provides instructions that can be easily understood by an untrained user in any type of situation, and that can be inexpensively manufactured.

Some known medicament delivery devices are associated with simulated medicament delivery devices (e.g., "trainers") to provide a method for users to practice using the medicament delivery device without being exposed to the medicament or needles typically contained therein. Such simulated medicament delivery devices, however, can also include inadequate use instructions as described above. Moreover, some known simulated medicament delivery devices can be difficult to reset for subsequent use.

Thus, a need exists for a simulated medicament delivery device that provides instructions that can be easily understood by an untrained user in any type of situation. Additionally, a need exists for a simulated medicament delivery device that can be easily reset for subsequent use.

US 2002/0188419 describes an integral data logging system for a jet injector including at least one electrical switch that changes state during an injection procedure.

US 6514230 describes an electronic injection device which can work in an airshot mode and a dose injection mode.

WO 2007/087304 describes a battery powered device having a housing and a battery compartment for accepting at least one battery, the battery compartment comprising a battery carrier and a locking hold down mechanism.

### Summary

Apparatuses and methods for automatic medicament injection are described herein. According to the present invention there is provided an apparatus according to claim 1. Preferred features are recited in the dependent claims. In one example, an apparatus includes a housing, a medicament container disposed within the housing and an actuator. The actuator is configured to be disposed within the housing and to move the medicament container within the housing. The actuator includes a release member and an energy storage member. The energy storage member has a first position and a second position. When in the first position, the energy storage member has a first potential energy. When in the second position the energy storage member has a second potential energy less than the first potential energy. The energy storage member is configured to convert a portion of the first potential energy into kinetic energy when it moves from its first position to its second position to move the medicament container within the housing. The energy storage member has a longitudinal axis offset from a longitudinal axis of the medicament container. The release member is configured to selectively deploy the energy storage member from its first position to its second position.

In some embodiments, an apparatus includes a label configured to be coupled to a medicament delivery device. The label includes a first surface and a second surface. The first surface is configured to be coupled to an outer surface of the medicament delivery device. The second surface includes a textual indicia. The label further includes an electronic circuit system configured to output an electronic signal.

In some embodiments, an apparatus includes a simulated medicament delivery device and an electronic circuit system coupled to the simulated medicament delivery device. The electronic circuit system is configured to output an electronic output associated with a use of the simulated medicament delivery device.

In some embodiments, an apparatus includes a container defining an internal region configured to contain multiple medicament delivery devices, such as, for example, pen injectors, auto-injectors, inhalers or the like. The container includes an electronic circuit system configured to output a first electronic output associated with a first medicament delivery device contained within the internal region when the first medicament delivery device is removed from the internal region of the container. The electronic circuit system is further configured to output a second electronic output associated with a second medicament delivery device contained within the internal region when the second medicament delivery device is removed from the internal region of the container. The second electronic output is different than the first electronic output. At least one of the first electronic output or the second electronic output is associated with a use instruction of the first medicament delivery device and/or the second medicament delivery device.

Because emergency medical facilities may not be available when an individual is suffering from a medical condition, some individuals carry an auto-injector to rapidly self-administer a medicament in response to such medical conditions. Some known auto-injectors are cylindrical in shape and include vial containing a liquid medicament and a spring loaded needle to automatically penetrate the user's skin and inject the medicament. The storage of certain medicaments in a liquid form, however, can result in a shorter shelf life and/or an unstable medicament. Accordingly, some known auto-injectors include a vial containing a first medicament and a medicament stored separately. Such auto-injectors are often referred to as "wet / dry" auto-injectors, because one medicament is often a liquid (e.g., water) and the other medicament is often a solid (e.g., glucagon powder). In use, the first medicament and the second medicament must be mixed prior to injection.

Some known wet / dry auto-injectors, however, require that the user manually actuate a mixing mechanism that must be used prior to injection. Such configurations can, however, result in incomplete mixing and/or injection occurring without mixing. Moreover, such configurations can be complicated, making them difficult for a user to operate during an emergency situation.

Some known wet/dry auto-injectors employ a single mechanism to automatically mix and inject the medicaments contained therein. Because the mixing operation is not independent from the injection operation in such configurations, however, the medicament can be injected prior to the completion of the mixing operation and/or prior to the auto-injector being properly positioned for the injection operation.

Thus, a need exists for an auto-injector that can separately store two or more medicaments and that can automatically mix and inject the medicaments in two distinct operations.

### Brief Description of the Drawings

FIG. 1 is a perspective view of a system according to an embodiment of the invention.
FIG. 2 is a front view of a system according to an embodiment of the invention.
FIG. 3 is a side view of a system according to an embodiment of the invention.
FIG. 4 is a cross-sectional view taken along line A-A of FIG. 3 of a system according to an embodiment of the invention in a first operative position.
FIG. 5 is a cross-sectional view taken along line A-A of FIG. 3 of a system according to an embodiment of the invention in a second operative position.
FIG. 6 is a cross-sectional view taken along line A-A of FIG. 3 of a system according to an embodiment of the invention in a third operative position.
FIG. 7 is a cross-sectional view taken along line A-A of FIG. 3 of a system according to an embodiment of the invention in a fourth operative position.
FIG. 8 is a cross-sectional view taken along line A-A of FIG. 3 of a system according to an embodiment of the invention in a fifth operative position.
FIG. 9 is a cross-sectional view taken along line A-A of FIG. 3 of a system according to an embodiment of the invention in a sixth operative position.
FIG. 10 is a flowchart illustrating a method according to an embodiment of the invention.
FIG. 11 is a perspective view of a system according to an embodiment of the invention.
FIG. 12 is a perspective cross-sectional view the system illustrated in FIG. 11 taken along line B-B of FIG. 11.
FIG. 13 is a perspective view of an apparatus according to an embodiment of the invention.
FIG. 14 is a cross-sectional view of a mechanism according to an embodiment of the invention taken along line A-A of FIG. 3.
FIGS. 15 and 16 are schematic illustrations of an auto-injector according to an embodiment of the invention in a first configuration and a second configuration, respectively.
FIG. 17 is a perspective view of an auto-injector according to an embodiment of the invention.
FIG. 18 is a perspective view of the auto-injector illustrated in FIG. 17 in a first configuration, with at least a portion of the auto-injector illustrated in phantom lines for ease of reference.
FIG. 19 is a front view of the auto-injector illustrated in FIGS. 17 and 18 in a first configuration.
FIG. 20 is a perspective view of the auto-injector illustrated in FIG. 17 showing an assembly according to an embodiment of the invention being removed.
FIG. 21 is a front view of the auto-injector illustrated in FIG. 17 showing a member according to an embodiment of the invention being removed.
FIG. 22 is an exploded perspective view of the a portion of the auto-injector illustrated in FIG. 20.
FIG. 23 is a cross-sectional view of a component illustrated in FIG. 21.
FIG: 24 is a perspective view of a component illustrated in FIG. 21.
FIG. 25 is a perspective view of a member of the auto-injector illustrated in FIG. 21.
FIG. 26 is a perspective view of a portion of the auto-injector illustrated in FIGS. 17 and 21.
FIG. 27 is a perspective view of a portion of the auto-injector illustrated in FIGS. 17 and 26.
FIG. 28 is a partially exploded perspective view of a base of the auto-injector illustrated in FIG. 26.
FIG. 29 is an exploded perspective view of a portion of the auto-injector shown in FIG. 21.
FIG. 30 is a front view of a component of the auto-injector shown in FIG. 29.
FIG. 31 is a front view of the auto-injector illustrated in FIG. 19 in a second configuration.
FIG. 32 is a perspective view of a portion of the auto-injector shown in FIG. 31.
FIGS. 33 and 34 are perspective views of a portion of the auto-injector shown in FIG. 32.
FIG. 35 is a top view of the housing of the auto-injector shown in FIG. 31.
FIG. 36 is a cross-sectional view of the housing taken along line 36-36 in FIG. 35.
FIG. 37 is front view of the auto-injector illustrated in FIGS. 19 and 31 in a third configuration.
FIG. 38 is a front view of the portion of the auto-injector labeled as 38 in FIG. 37.
FIG. 39 is a perspective view of a portion of the auto-injector shown in FIG. 37.
FIG. 40 is a cross-sectional view of a portion of the auto-injector as shown in FIG. 37.
FIG. 41 is a perspective view of a portion of the auto-injector as shown in FIG. 37.
FIG. 42 is an exploded perspective view of a portion the auto-injector as shown in FIG. 37.
FIG. 43 is front view of the auto-injector illustrated in FIGS. 19, 31 and 38 in a fourth configuration.
FIG. 44 is a front view of a portion of the auto-injector illustrated in FIGS. 19, 31, 38 and 43 in a fifth configuration.
FIG. 45 is a front view of the auto-injector illustrated in FIGS. 19, 31, 38, 43 and 44 in a sixth configuration.
FIG. 46 is a perspective view of a medicament delivery device according to an embodiment of the invention.
FIG. 47 is a front cross-sectional view of the medicament delivery device shown in FIG. 46.
FIG. 48 is a schematic illustration of a portion of the medicament delivery device shown in FIG. 46.
FIG. 49 is a schematic illustration of a medicament delivery device according to an embodiment of the invention.
FIG. 50 is a perspective view of an auto-injector according to an embodiment of the invention.
FIG. 51 is a front view of the auto-injector illustrated in FIG. 50, with a portion of the auto-injector illustrated in phantom lines for ease of reference.
FIG. 52 is a partial cut-away front view of a portion of the auto-injector illustrated in FIG. 50.
FIG. 53 is a cross-sectional view of a portion of the auto-injector illustrated in FIG. 50 taken along line 53-53 in FIG. 52.
FIG. 54 is a cross-sectional view of a portion of the auto-injector illustrated in FIG. 50 taken along line 54-54 in FIG. 52.
FIG. 55 is a front view of a portion of the auto-injector illustrated in FIG. 50.
FIG. 56 is a schematic illustration of a portion of the auto-injector illustrated in FIG. 50.
FIG. 57 is a perspective view of a portion of the auto-injector illustrated in FIG. 50 in a second configuration.
FIG. 58 is a perspective view of a portion of the auto-injector illustrated in FIG. 50 in a third configuration.
FIG. 59 is a perspective view of a portion of the auto-injector illustrated in FIG. 50 in a fourth configuration.
FIGS. 60 and 61 are front views of a portion of the auto-injector labeled as region 15 in FIG. 55, in a first configuration and a second configuration, respectively.
FIGS. 62 through 65 are perspective views of a portion of the auto-injector illustrated in FIG 55, in a first configuration, a second configuration, a third configuration and a fourth configuration, respectively.
FIG. 66 is a flow chart of a method according to an embodiment of the invention.
FIG. 67 is a flow chart of a method according to an embodiment of the invention.
FIG. 68 is a flow chart of a method according to an embodiment of the invention.
FIGS. 69 and 70 are perspective views of a medicament delivery device according to an embodiment of the invention.
FIGS. 71 - 73 are schematic illustrations of a medical device according to an embodiment of the invention in a first configuration, a second configuration and a third configuration, respectively.
FIGS. 74 - 76 are schematic illustrations of a medical device according to an embodiment of the invention in a first configuration, a second configuration and a third configuration, respectively.
FIG. 77 is a schematic illustration of a medical device according to an embodiment of the invention.
FIG. 78 is a perspective view of a medical device according to an embodiment of the invention in a first configuration.
FIG. 79 is a perspective view of the medical device shown in FIG. 78 in a second configuration.
FIG. 80 is a perspective view of the medical device shown in FIG. 78 in a third configuration.
FIG. 81 is a schematic illustration of a portion of a medical device according to an embodiment of the invention.
FIGS. 82 - 84 are schematic illustrations of a medical device according to an embodiment of the invention in a first configuration, a second configuration and a third configuration, respectively.
FIG. 85 is a schematic illustration of a simulated medicament delivery device according to an embodiment of the invention.
FIG. 86 is a perspective view of a simulated auto-injector according to an embodiment of the invention.
FIGS. 87 - 91 are front are front views of a simulated auto-injector according to an embodiment of the invention, in a first configuration, second configuration, third configuration, fourth configuration and fifth configuration, respectively.
FIG. 92 is a schematic illustration of a medical device according to an embodiment of the invention.
FIG. 93 is a schematic illustration of a medical device according to an embodiment of the invention.
FIG. 94 is a perspective view of a simulated medicament delivery device according to an embodiment of the invention
FIG. 95 is a front view of a medical device according to an embodiment of the invention.
FIG. 96 is a cross-sectional view of the medical device shown in FIG. 95.
FIG. 97 is a cross-sectional view of a medical device according to an embodiment of the invention.
FIGS. 98 - 101 are schematic illustrations of a medical device according to an embodiment of the invention in a first configuration, a second configuration, a third configuration and a fourth configuration, respectively.
FIG. 102 is a top view of an auto-injector according to an embodiment of the invention.
FIG. 103 is a front view of the auto-injector shown in FIG. 102.
FIG. 104 is a cross-sectional view of the auto-injector shown in FIG. 102 taken along line A-A in FIG. 102.
FIG. 105 is a top view of a portion of the auto-injector shown in FIG. 102.
FIG. 106 is a cross-sectional view of the portion of the auto-injector shown in FIG. 11 taken along line A-A in FIG. 105.
FIG. 107 is a cross-sectional view of the portion of the auto-injector shown in FIG. 11 taken along line B-B in FIG. 105.
FIG. 108 is a perspective view of an auto-injector according to an embodiment of the invention.
FIG. 109 is a perspective exploded view of the auto-injector shown in FIG. 108.
FIG. 110 is a cross-sectional front view of a portion of the auto-injector illustrated in FIG. 108 in a first configuration.
FIG. 111 is a perspective exploded view of a portion of the auto-injector shown in FIG. 108.
FIG. 112 is a perspective view of a member of the auto-injector illustrated in FIG. 111.
FIG. 113 is a perspective view of a member of the auto-injector illustrated in FIG. 111.
FIG. 114 is a perspective exploded view of a portion of the auto-injector shown in FIG. 108.
FIG. 115 is a perspective view of a portion of the auto-injector shown in FIG. 108.
FIG. 116 is a perspective view of a portion of the auto-injector shown in FIG. 108.
FIG. 117 is a perspective exploded view of a portion of the auto-injector shown in FIG. 108.
FIG. 118 is a perspective view of a portion of the auto-injector illustrated in FIG. 109.
FIG. 119 is a perspective view of a member of the auto-injector illustrated in FIG. 118.
FIG. 120 is a perspective view of a member of the auto-injector illustrated in FIG. 108.
FIG. 121 is a perspective view of a member of the auto-injector illustrated in FIG..108.
FIG. 122 is a perspective exploded view of a portion of the auto-injector shown in FIG. 108.
FIG. 123 is a front view of a member of the auto-injector illustrated in FIG. 108.
FIG. 124 is a cross-sectional front view of the portion of the auto-injector labeled as 30 in FIG. 110.
FIG. 125 is a cross-sectional front view of a portion of the auto-injector in FIG. 110.
FIGS. 126 and 127 are perspective views of a member of the auto-injector illustrated in FIG. 108.
FIGS. 128 - 132, are cross-sectional front views of a portion of the auto-injector illustrated in FIG. 108 in a second configuration, a third configuration, a fourth configuration, a fifth configuration and a sixth configuration, respectively.
FIG. 133 is a flow chart of a method according to an embodiment of the invention.
FIGS. 134 - 136 are schematic illustrations of a medical device according to an embodiment of the invention in a first configuration, a second configuration, and a third configuration, respectively.
FIG. 137 is a schematic illustration of a medical device according to an embodiment of the invention.
FIGS. 138 and 139 are cross-sectional front views of a portion of a medical device according to an embodiment of the invention in a first configuration and second configuration, respectively.

### Detailed Description

Apparatuses and methods for automatic medicament injection are described herein. In some embodiments, an apparatus includes a housing, a medicament container disposed within the housing and an actuator. The actuator is configured to be disposed within the housing and to move the medicament container within the housing. The actuator includes a release member and an energy storage member. The energy storage member, which can be, for example, a compressed gas container, has a first position and a second position. When in the first position, the energy storage member has a first potential energy. When in the second position the energy storage member has a second potential energy less than the first potential energy. The energy storage member is configured to convert a portion of the first potential energy into kinetic energy when moved from its first position to its second position to move the medicament container within the housing. The energy storage member has a longitudinal axis offset from a longitudinal axis of the medicament container. The release member is configured to selectively deploy the energy storage member from its first position to its second position.

In some embodiments, an apparatus includes a housing, a needle and an actuator. The needle has a first end and a second end and defines a longitudinal axis. The actuator is configured to be disposed within the housing and to move the needle between a first needle position and a second needle position. When in the first needle position, the second end of the needle is within the housing. When in the second needle position, the second end of the needle is outside the housing. The actuator includes a release member and an energy storage member. The energy storage member has a first position and a second position. When in the first position, the energy storage member has a first potential energy. When in the second position the energy storage member has a second potential energy less than the first potential energy. The energy storage member is configured to convert a portion of the first potential energy into kinetic energy when moved from its first position to its second position to move the needle between the first needle position and the second needle position. The energy storage member has a longitudinal axis offset from the longitudinal axis of the needle. The release member is configured to selectively deploy the energy storage member from its first position to its second position.

In some embodiments, an apparatus includes a housing, a needle, a medicament container and an actuator. The needle has a first end and a second end and defines a longitudinal axis. The actuator is configured to be disposed within the housing and to move the needle between a first needle position and a second needle position. When in the first needle position, the second end of the needle is within the housing. When in the second needle position, the second end of the needle is outside the housing. The actuator is further configured to move the medicament container between a first medicament container position and a second medicament container position. When in the first medicament container position, a lumen defined by the needle is fluidically isolated from the medicament container. When in the second medicament container position, the first end of the needle is disposed within the medicament container such that the lumen is in fluid communication with the medicament container. The actuator includes a release member and an energy storage member. The energy storage member has a first position and a second position. When in the first position, the energy storage member has a first potential energy. When in the second position the energy storage member has a second potential energy less than the first potential energy. The energy storage member is configured to convert a portion of the first potential energy into kinetic energy when moved from its first position to its second position to move the needle between the first needle position and the second needle position. The energy storage member has a longitudinal axis offset from the longitudinal axis of the needle. The release member is configured to selectively deploy the energy storage member from the first position to the second position.

In some embodiments, an apparatus includes an actuator disposable within a housing of an auto-injector. The actuator is configured to move a medicament container relative to the housing, and includes a gas container, a biasing member and a puncturer. The gas container, which is configured to store a compressed gas, is movable between a first position and a second position. The biasing member has a retracted configuration and an expanded configuration. The biasing member is configured to engage the gas container such that when the biasing member moves from the retracted configuration to the expanded configuration the gas container is moved from the first position to the second position. The puncturer is configured to penetrate a portion of the gas container when the gas container moves to the second position to allow a portion of the compressed gas to be released from the gas container into a gas chamber defined within the housing adjacent the medicament container.

In some embodiments, an apparatus includes a housing having a distal end portion and a proximal end portion, a medicament injector, an energy storage member and a retainer. The medicament injector is disposed within the housing and includes a medicament container and a needle. The energy storage member, which can be, for example, a gas container configured to contain a pressurized gas, is configured to produce a force when moved from a first configuration to a second configuration to move the medicament injector between a first position and a second position. The retainer has a first position and a second position. When the retainer is in its first position, the retainer is configured to retain the energy storage member in its first configuration. When the retainer is in its second position, the retainer is configured to allow the energy storage member to be moved from its first configuration to its second configuration. The retainer is configured to be selectively moved from its first position to its second position by manipulating an actuator adjacent the distal end portion of the housing.

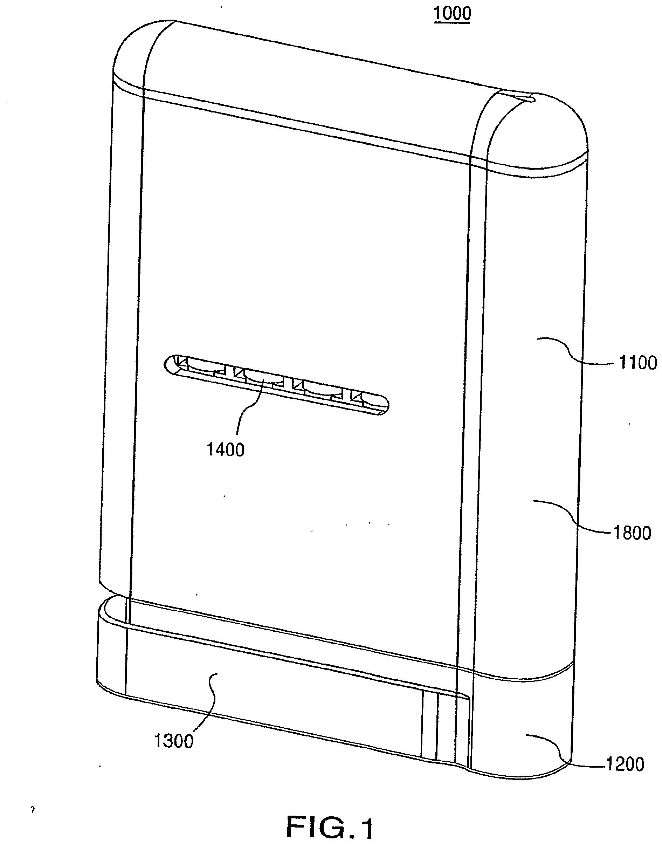

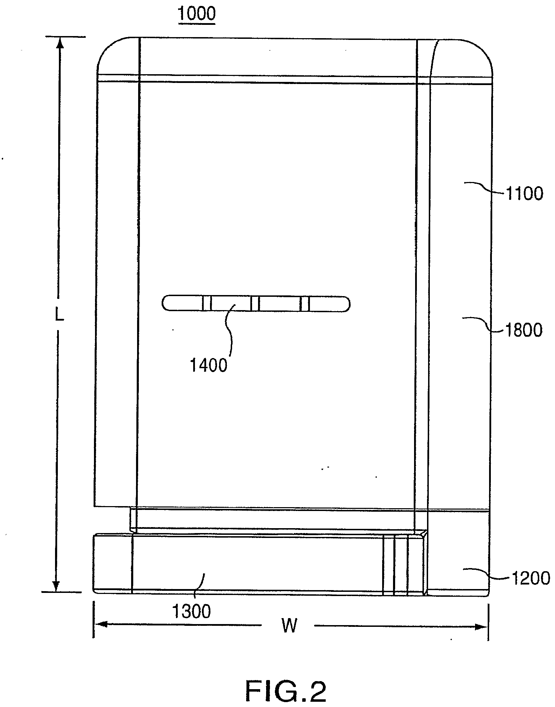

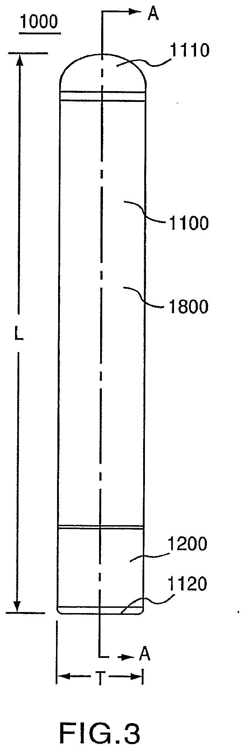

FIG. 1 is a perspective view, FIG. 2 is a front view, and FIG. 3 is a side view, of a system 1000 according to the invention, which can comprise a housing 1100, which, in some embodiments, can comprise a handheld portion 1800 separated via an actuation guard 1200 from an actuation bar 1300. Actuation guard 1200 can prevent accidental activation of system 1000. Housing 1100 can be constructed of a durable material, such as stainless steel, aluminum, polycarbonate, etc., to protect a compressed gas container, medicament, injection apparatus and/or user of system 1000. The injection apparatus can be actuated by a fluid pressure, such as pressure provided by the compressed gas, which upon completion of actuation can escape housing 1100 via gas escape opening, such as via status indicator 1400.
Attorney Docket No. INTJ-003/03WO 306456-2023

A status of a system 1000 can be determined via status indicator 1400, which can provide a view, such as via a UV blocking, photo-sensitive, and/or translucent window, into an interior of housing 1100. Viewable through the window can be a status of medicament carried by housing 1100, a location of a needle and/or injection apparatus for the medicament, and/or an activation status of system 1000. For example, if the medicament has aged to the point of discoloration, which aging might or might not render the medication useless, harmful, etc., status indicator 1400 can allow that situation to be determined. In some embodiments, gas can escape housing 1100 via status indicator 1400 and/or another opening in housing 1100.

Some embodiments of system 1000 can provide a compact medicament delivery mechanism that can efficiently and/or rapidly deliver a prescribed dose. The length (L) and width (W) of system 1000 can be similar to that of a credit card, and the thickness (T) can be less than one inch. Thus, some embodiments of system 1000 can provide a conveniently carried, easy-to-use, easy to activate drug delivery apparatus that can require little to no training to safely carry, use, and/or dispose of.

To assist a user in positioning system 1000 in a correct orientation for injection, system 1000 and/or housing 1100 can provide various tactile clues. For example, a top 1110 of housing 1100 can be rounded, and a bottom 1120 of actuation bar 1300 of housing 1100 can be flat. Other tactile clues are also possible, such as bulges, ribs, grooves, gaps, roughened surfaces, indentations, etc.

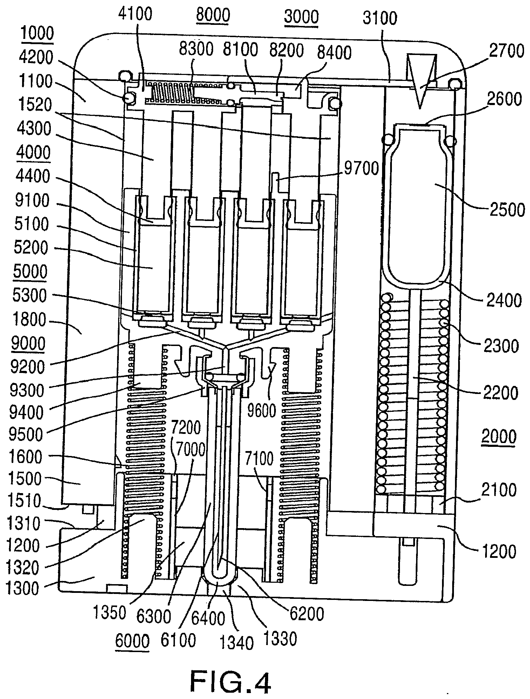

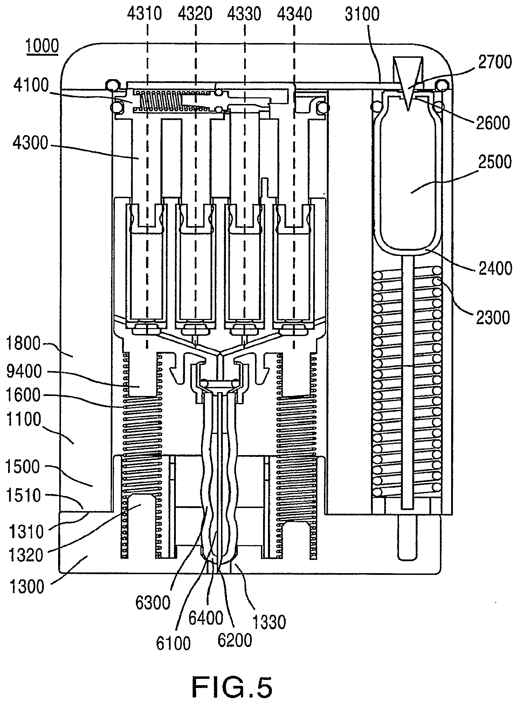

FIG. 4 is a cross-sectional view taken along line A-A of FIG. 3 of an embodiment of a system 1000 in a first operative position. FIGS. 5, 6, 7, 8, and 9 show system 1000 of FIG. 4 in second, third, fourth, fifth, and sixth operative positions, respectively.

System 1000 can comprise a housing 1100, handheld portion 1800, actuation guard 1200, and/or actuation bar 1300. System 1000 can comprise system actuator 2000, gas reservoirs 3000, medicament actuator 4000, medicament storage assembly 5000, medicament carrier 9000, needle assembly 6000, use indicator 7000, and/or gas vent mechanism 8000, etc.

Upon removal, release, rotation, and/or relocation of actuation guard 1200, system actuator 2000 can be adapted to rapidly discharge an actuating portion of a contents of a compress gas container. For example, system actuator 2000 can comprise a compressed gas container 2400, which initially can contain a compressed gas 2500, an actuating portion of which can be released from container 2400 by penetration of a gas port 2600 via a point of a puncturer 2700. Upon removal and/or relocation of actuation guard 1200, actuation bar 1300 can be moved closer to and/or in contact with handheld portion 1800. Upon removal and/or relocation of actuation guard 1200, gas container 2400 can be brought into contact with puncturer 2700 via extension of a pre-compressed spring 2300 and/or movement of an actuation stick 2200. Thus, actuation guard 1200 can prevent accidental activation of system 1000 and/or unintended discharge of an actuating portion of the contents 2500 of gas container 2400.

Once gas port 2600 has been punctured, an actuating portion of compressed gas 2500 can escape from container 2400 and flow via gas reservoirs 3000, such as gas channel 3100. The flowing gas can meet and/or apply gas pressure to medicament actuator 4000, which can comprise a pusher 4100, which can travel within a sleeve 1500 defined by walls 1520. Sleeve 1500 can be constructed of metal, stainless steel, aluminum, plastic, polycarbonate, etc. Seals 4200, such as o-rings, can resist gas leakage, such as past pusher 4100 and/or out of housing 1100. Thus, pusher 4100 can function as a piston traveling within a cylinder, although it is not necessarily required that the cross-sectional shape of sleeve 1500 be round.

Medicament actuator 4000 can interface with medicament storage assembly 5000. For example, medicament actuator 4000 can comprise a plurality of plungers 4300, each of which can be capped with a piston 4400 which can sealingly slide and/or move within a corresponding vial 5100 containing a liquid medicament 5200. For example, in response to pressure applied by an actuating portion of the contents 2500 of compressed gas container 2400, pusher 4100 can cause plungers 4300 and/or pistons 4400 to simultaneously move. The number of corresponding sets of plungers 4300, pistons 4400, and/or vials 5100 can be 2, 3, 4, 5, 6, or more. Pistons 4400 can be constructed of a resilient, durable, and/or sealing material, such as a rubber. Each plunger 4300 from the plurality of plungers can define a longitudinal axis, the longitudinal axes (e.g., axes 4310, 4320, 4330, 4340) of the plurality of plungers can be parallel, non-coaxial, and/or co-planar.

Each vial 5100 from the plurality of vials can be substantially cylindrical with a substantially round and/or substantially elliptical cross-sectional shape. Thus, each vial 5100 can define a longitudinal axis, the longitudinal axes of the plurality of vials can be parallel, non-coaxial, and/or co-planar. The longitudinal axis of each vial can be co-axial with the longitudinal axis of its corresponding plunger.

Each vial can be capped at one end with a frangible seal 5300, which can be burst when piston 4400 generates sufficient pressure upon medicament 5200, thereby allowing at least a portion of medicament 5200 to flow out of vial 5100 and into medicament carrier 9000. Thus, the plurality of vials can be fluidly coupleable to the actuating portion of the contents 2500 of gas container 2400.

Medicament carrier 9000 can hold each of vials 5100 and can travel within sleeve 1500. Medicament carrier 9000 can comprise a plurality of channels 9200 adapted to receive medicament 5200 as it exits its respective vial 5100, and direct medicament 5200 to a common conduit 9300. Medicament carrier 9000 can interface with needle assembly 6000 and/or use indicator 7000.

From common conduit 9300, medicament 5200 can enter needle assembly 6000, such as into a single needle 6100 via which medicament can approach needle tip 6200. As medicament actuator 4000 and/or medicament carrier 9000 are driven toward actuator bar 1300, needle tip 6200 can penetrate an end 6400 of needle sheath 6300 and exit actuator bar 1300 at needle port 1340.

Referring to FIG. 5, upon movement of actuation bar 1300 closer to handheld portion 1800, sheath seat 1330 can come in contact with sheath tip 6400, thereby causing sheath 6300 to buckle and/or crumble. As actuator bar 1300 comes in contact with handheld portion 1800, bar stop 1320 can approach medicament carrier stop 9400, while carrier spring 1600 is compressed.

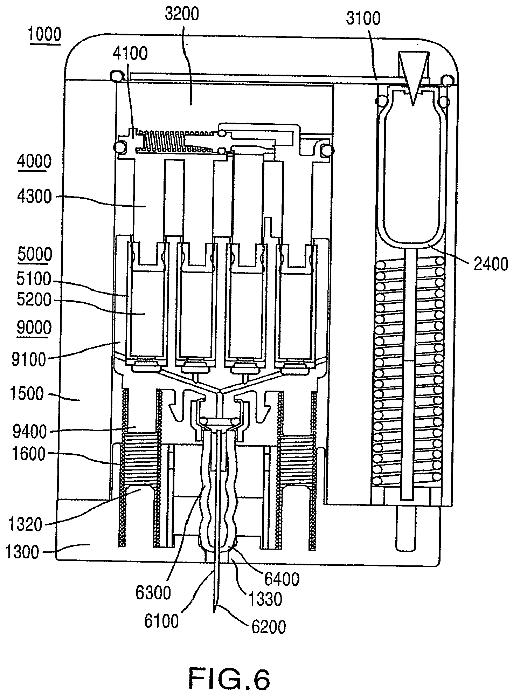

Referring to FIG. 6, as at least a portion of contents 2500 of gas container 2400 escapes, it can flow through channel 3100. The gas, which can still be relatively pressurized, can begin to accumulate behind pusher 4100 to form an expanding gas chamber 3200 and to cause medicament actuator 4000, medicament storage assembly 5000, and medicament carrier 9000 to slide together within sleeve 1500. As medicament actuator 4000, medicament storage assembly 5000, and medicament carrier 9000 slide closer to actuator bar 1300, spring 1600 becomes increasingly compressed between bar stop 1320 and medicament carrier stop 9400. As medicament actuator 4000, medicament storage assembly 5000, and medicament carrier 9000 slide closer to actuator bar 1300, needle tip 6200 can extend further from actuator bar 1300 and sheath 6300 can become further compressed and/or deformed. At its ultimate extension point, needle tip 6200 can extend from housing 1100 from approximately 0.25 millimeters to approximately 20 millimeters, including all values and subranges therebetween, such as up to approximately 2 millimeters, greater than approximately 5 millimeters, from approximately 5.13 millimeters to approximately 9.98 millimeters, etc.

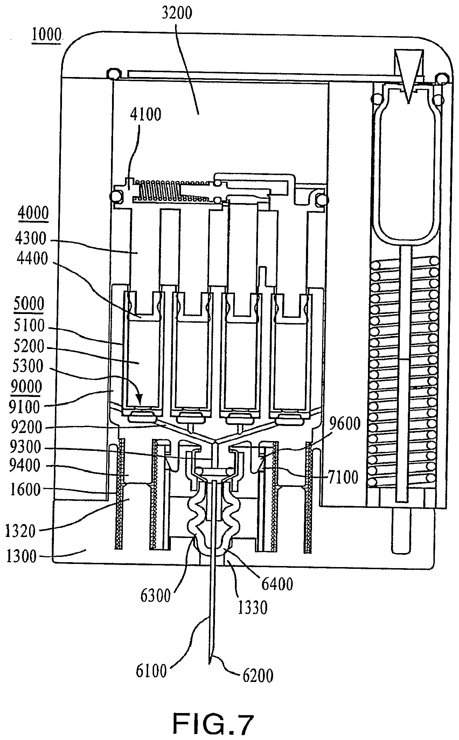

Referring to FIG. 7, as gas chamber 3200 continues to expand, medicament carrier 9000 can be driven until medicament carrier stop 9400 contacts actuator bar stop 1300 thereby resisting further travel of medicament carrier 9000. At that point, additional expansion of gas chamber 3200 can cause medicament actuator 4000, pusher 4100, plungers 4300, and/or pistons 4400 to initiate travel with respect to medicament storage assembly 5000, thereby generating an expulsion pressure in vials 5100, and/or thereby rupturing frangible seals 5300 and allowing medicament 5200 to enter medicament carrier 9000, and begin flowing through medicament channels 9200, medicament conduit 9300, needle 6100, and/or out needle tip 6200 and into a patient. Alternatively, frangible seals 5300 can be replaced and/or augmented by a frangible seal located at or near where medicament conduit 9300 couples to needle 6100. Frangible seals 5300 can be constructed of a thin, taught, resilient, durable, and/or sealing material potentially having a predetermined yield strength, such as a rubber, such as chromo butyl rubber, and/or of a relatively brittle material potentially having a predetermined yield strength, such as ceramic, certain plastics, such as polystyrene, etc.

As medicament carrier stop 9400 contacts actuator bar stop 1320, medicament carrier hooks 9600 can engage with engagement receivers 7100 in use indicator 7000.

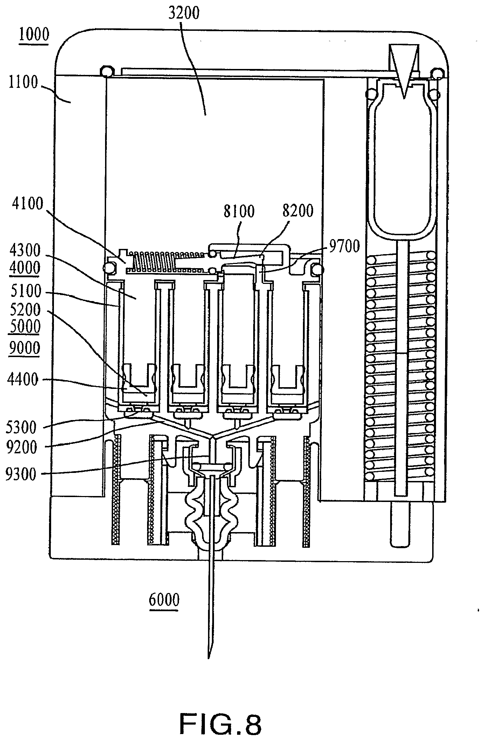

Referring to FIG. 8, as gas chamber 3200 continues to expand, medicament actuator 4000, pusher 4100, plungers 4300, and/or pistons 4400 can continue moving until they complete their travel within medicament storage assembly 5000, thereby expelling a predetermined dose of medicament 5200 from vials 5100, out of needle assembly 6000, external to housing 1100, and/or into the patient. As gas chamber 3200 reaches its maximum size, medicament actuator 4000, pusher 4100, plungers 4300, and/or pistons 4400 can continue moving until they complete their travel with respect to medicament carrier 9000, thereby causing gas release actuator 9700 to engage with gas relief valve 8200. Engagement of gas release actuator 9700 with gas relief valve 8200 can cause gas within gas chamber 3200 to exit gas chamber 3200, discharge away from pistons 4400, and/or exhaust from system 1000 and/or housing 1100, such as via status indicator 1400 and/or a gas escape port located on housing 1100).

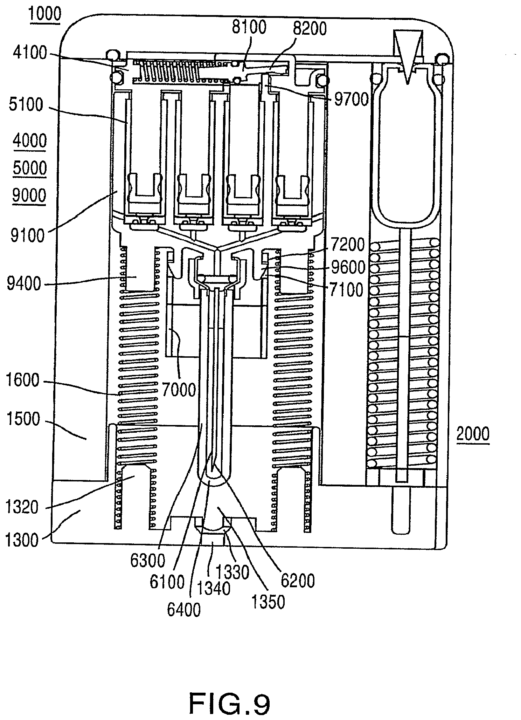

Referring to FIG. 8 and FIG. 9, as sufficient gas is vented from gas chamber 3200, the pressure applied by the gas in gas chamber 3200 can decrease until the force applied by the gas on medicament actuator 4000 is less than the force of compressed spring 1600. Thus, spring(s) 1600 can begin to expand, thereby moving medicament carrier 9000, vial assembly 5000, and medicament actuator 4000 away from actuator bar 1300 and helping to exhaust gas from gas chamber 3200. As medicament carrier 9000 moves, use indicator 7000 can travel with it, due to the engaged relationship of medicament carrier hooks 9600 and engagement receivers 7100 and/or engagement catches 7200 in use indicator 7000. As use indicator 7000 moves away from actuation bar 1300, sheath 6300 can travel with it, thereby creating a gap between sheath tip 6400 and needle port 1340, and thereby exposing a previously non-visible colored portion 1350 of actuation bar 1300 and/or providing an indication that system 1000 has been used (and likely substantially exhausted of its medicament), thereby discouraging any further attempts to use system 1000.

As medicament carrier 9000 moves away from actuator bar 1300, needle 6100 can retract into sheath 6300 which un-buckles and/or un-deforms towards its original shape. Eventually, needle 6100 can retract completely within the boundaries of housing 1100, thereby tending to prevent accidental needle sticks after the initial injection and/or potentially reducing and/or eliminating a sharps hazard.

In some embodiments, system actuator 2000 can comprise a finger triggered, twistable, pivotable, and/or lever-operated mechanism. For example, system actuator 2000 can comprise a twistable handle that can screw into gas port 2600. In some embodiments, system actuator 2000 can be a finger trigger located on a side of the housing.

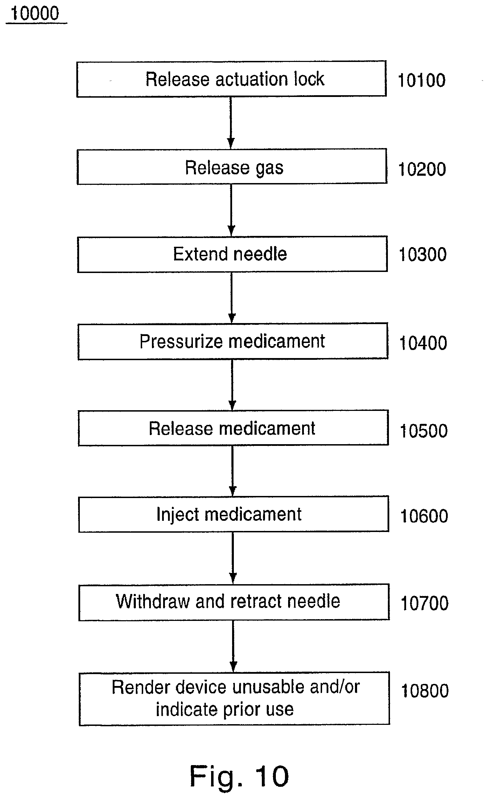

FIG. 10 is a flowchart of an embodiment of a method 10000 for operating a medicament delivery apparatus. At activity 10100, an actuation lock for the apparatus is released. At activity 10200, an actuating portion of the contents of a compressed gas container are released. At activity 10300, via pressure provided by the released gas, a needle is extended from the apparatus. At activity 10400, via pressure provided by the released gas, a piston applies pressure to a medicament stored in one of a plurality of vials. At activity 10500, a frangible seal containing the medicament in the vial is burst. At activity 10600, the medicament flows from the vial, through the needle, and into a patient. At activity 10700, once a predetermined dose is expelled and/or injected, the needle is withdrawn from the patient and/or retracted into the pre-use bounds of the apparatus. At activity 10800, the apparatus is rendered unusable for additional injections and/or indicated as previously utilized.

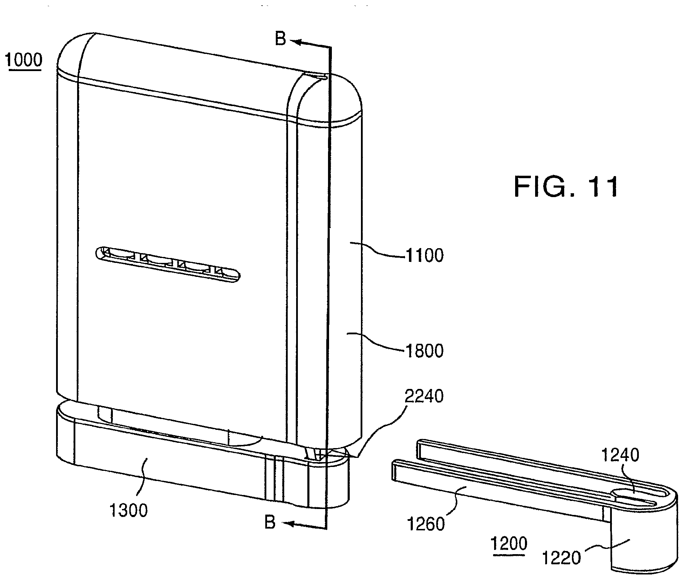

FIG. 11 is a perspective view of an embodiment of system 1000, showing actuation guard 1200 removed from housing 1100, so that actuation guard 1200 no longer separates actuator bar 1300 from handheld portion 1800. Actuation guard 1200 can comprise a grippable portion 1220 that can be gripped by a user to pull actuation guard 1200 away from housing 1100, thereby allowing system 1000 to be activated, such as via slapping actuator bar 1300 against a thigh of the user. Actuation guard 1200 can comprise an actuation stick separator portion 1240, that can keep separate actuation stick prongs 2240 when actuation guard 1200 is installed on housing 1100. Actuation guard 1200 can comprise a guard portion 1260 that can separate actuator bar 1300 from handheld portion 1800 when system 1000 is not in use and/or when system 1000 has not been used.

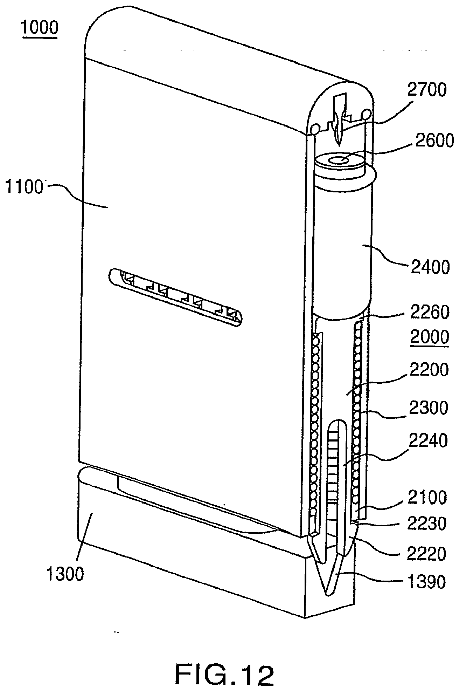

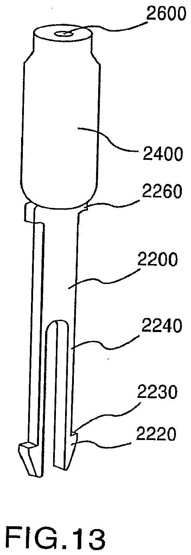

FIG. 12 is a perspective cross-sectional view taken along line B-B of FIG. 11, and FIG. 13 is a perspective view of an embodiment of actuation stick 2200. Referring to FIGS. 12 and 13, system 1000 can comprise housing 1100, actuation bar 1300, and system actuator 2000, which can comprise prong squeezer 1390, actuation stick 2200, prong retainer 2100, spring 2300, upper spring retainer 2260, gas container 2400, gas port 2600, and/or puncturer 2700. When actuation bar 1300 is pressed firmly against a user's body, such as via slapping housing actuation bar against the user's thigh, buttocks, and/or arm, prong squeezer 1390 can urge prong tips 2220 of prongs 2240 of actuation stick 2200 toward one another. Note that prong tips 2200 can have a triangular, wedge, angular, and/or frusto-conical shape. As prongs tips 2220 slide along the angled V-groove of prong squeezer 1390, prong catches 2230 can substantially lose contact with prong retainer 2100. This can allow compressed spring 2300 to rapidly urge actuation stick 2200 and gas container 2400 toward puncturer 2700, which can penetrate gas port 2600, thereby allowing gas to escape from gas container 2400. Although any of many different types of gas containers can be utilized, an example of a suitable gas container can be obtained from Leland Limited, Inc. of South Plainfield, NJ.

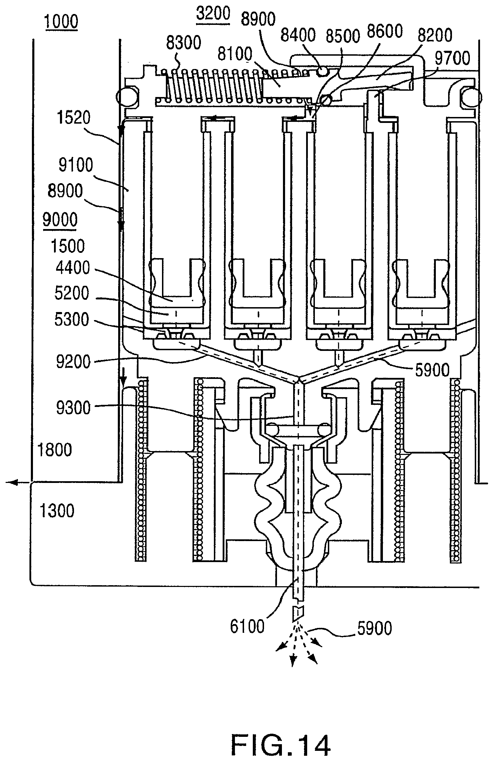

FIG. 14 is a cross-sectional view of an embodiment of gas venting mechanism 8000 of system 1000taken along line A-A of FIG. 3. System 1000 can comprise handheld portion 1800, actuator bar 1300, sleeve 1500. As pistons 4440 near the limit of their travels, medicament 5200 can be expelled along medicament path 5900, which can extend past frangible seal 5300, through medicament channels 9200, medicament conduit 9300, and needle 6100, and into the body of a user, such as subcutaneously, intramuscularly, and/or at a depth of from approximately 0.25 millimeters to approximately 20 millimeters, including all values and subranges therebetween, such as up to 2 millimeters, greater than 5 millimeters, etc.

As pistons 4440 near the limit of their travels, engagement of gas release actuator 9700 with gas relief valve 8200 can cause compressed spring 8300 to move valve arm such that o-ring 8400 is urged away from its seat 8500. This movement can reveal a passage 8600, via which gas can exit gas chamber 3200 along gas exhaust path 8900, which can extend between sleeve inner walls 1520 and outer walls 9100 of medicament carrier 9000. Eventually, gas exhaust path 8900 can extend between handheld portion 1800 and actuator bar 1300. Likewise, an alternative embodiment of valve 8200, made of rubber or any other resilient material, can be placed across seat 8500 to provide a seal that, once gas release actuator 9700 interacts with valve 8200, allows valve 8200 to bend or flap upwards away from seat 8500, causing the gas to escape via passage 8600.

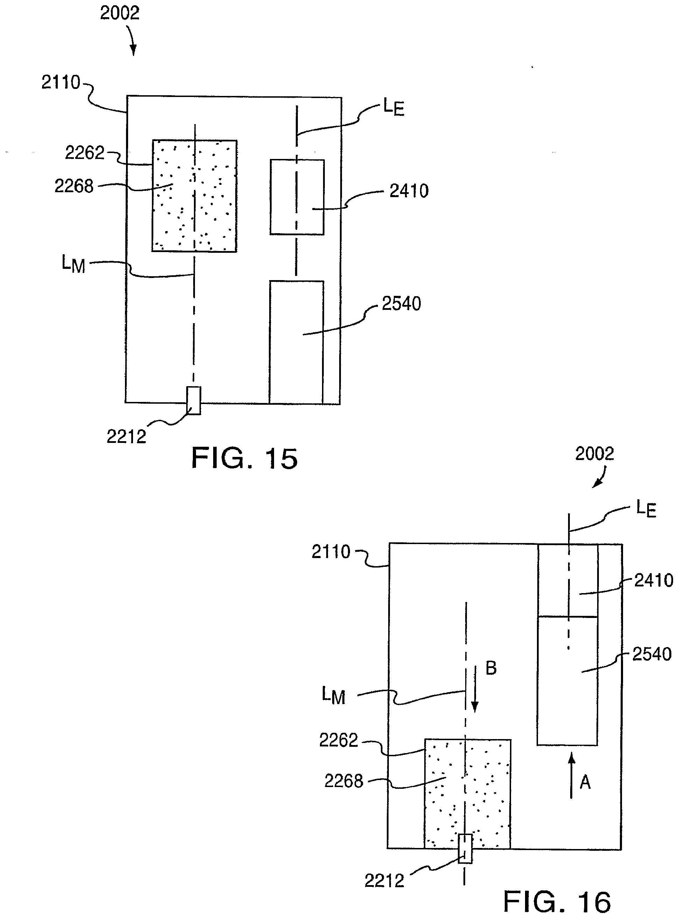

FIGS. 15 and 16 are schematic illustrations of an auto-injector 2002 according to an embodiment of the invention in a first configuration and a second configuration, respectively. The auto-injector 2002 includes a housing 2110 that contains a medicament container 2262, an energy storage member 2410, a release member 2540 and an injection member 2212. The medicament container 2262, which can be, for example, a pre-filled cartridge, a vial, an ampule or the like, is movably disposed within the housing 2110. The medicament container 2262 contains a medicament 2268, such as, for example, epinephrine. As illustrated, the medicament container 2262 can be moved, as indicated by arrow B in FIG. 16, along its longitudinal axis Lm between a first position (FIG. 15) and a second position (FIG. 16). When the medicament container 2262 is in its first (or retracted) position, the medicament container 2262 is spaced apart from the injection member 2212. When the medicament container 2262 is in the second (or advanced) position, the medicament container 2262 is placed in fluid communication with the injection member 2212. In this manner, when the medicament container 2262 is in the second (or advanced) position, the medicament 2268 can be conveyed via the injection member 2212 from the medicament container 2262 into a body of a patient. The injection member 2212 can be, for example, a needle, a nozzle or the like.

The energy storage member 2410, which can be any suitable device for storing energy, such as, for example, a spring, a battery, a compressed gas cylinder or the like, is also movably disposed within the housing 2110. As shown, the energy storage member 2410 defines a longitudinal axis Le that is offset from the longitudinal axis Lm of the medicament container 2262. The energy storage member 2410 can be moved, as indicated by arrow A in FIG. 16, within the housing 2110 along its longitudinal axis Le between a first position (FIG. 15) and a second position (FIG. 16). When the energy storage member 2410 is in its first position, the energy storage member 2410 has a first potential energy. When the energy storage member 2410 is in its second position, the energy storage member 2410 has a second potential energy that is less than the first potential energy. When the energy storage member 2410 moves from its first position to its second position, it converts at least a portion of its first potential energy into kinetic energy to move the medicament container 2262 between its first position and its second position.

Said another way, the movement of the energy storage member 2410 from its first position to its second position results in the production of a force that acts upon the medicament container 2262 to move the medicament container 2262 between its first position and its second position. The non-coaxial relationship between the longitudinal axis Lm of the medicament container 2262 and the longitudinal axis Le of the energy storage member 2410 allows the medicament container 2262 and the energy storage member 2410 to be arranged within the housing 2110 in any number of different configurations. In this manner, the auto-injector 2002 can have any number of different sizes and shapes, such as, for example, a substantially rectangular shape.

The release member 2540 is disposed within the housing 2110 and is configured to selectively deploy the energy storage member 2410 from its first position to its second position. The release member 2540 can be any suitable mechanism for moving the energy storage member 2410, such as, for example, a mechanical linkage, a spring-loaded rod or the like. In this manner, a user can actuate the auto-injector by manipulating a portion of the release member 2540.

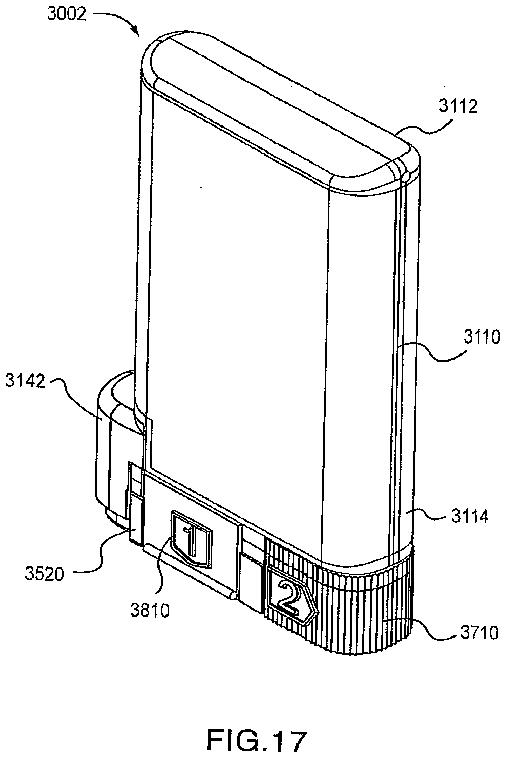

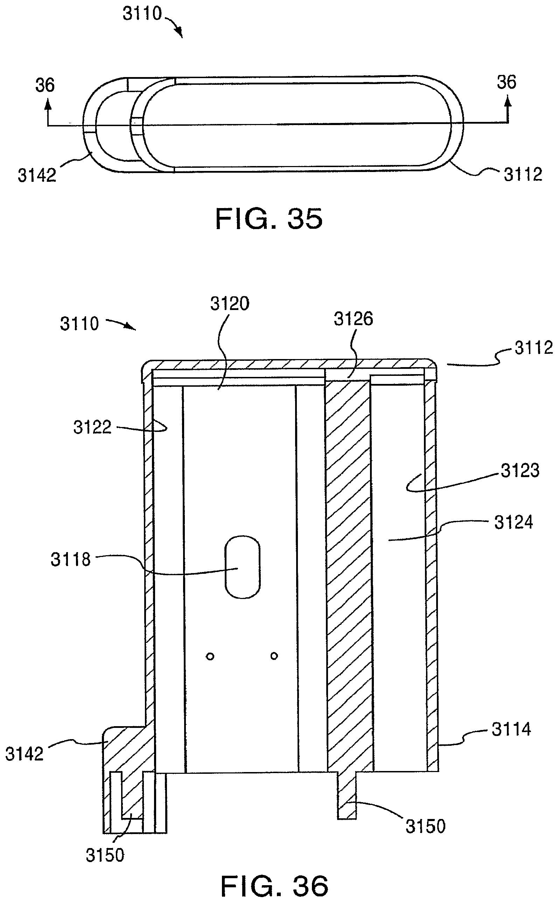

FIG. 17 is a perspective view of an auto-injector 3002 according to an embodiment of the invention in a first configuration. The auto-injector 3002 includes a housing 3110 having a proximal end portion 3112 and a distal end portion 3114. The distal end portion 3114 of the housing 3110 includes a protrusion 3142 to help a user grasp and retain the housing 3110 when using the auto-injector 3002. Said another way, the protrusion 3142 is configured to prevent the auto-injector 3002 from slipping from the user's grasp during use. A base 3520 is movably coupled to the distal end portion 3114 of the housing 3110. A needle guard assembly 3810 is removably coupled to the base 3520. Similarly, a safety lock 3710 is removably coupled to the base 3520. To inject a medicament into the body, the distal end portion 3114 of the housing is oriented towards the user such that the base 3520 is in contact with the portion of the body where the injection is to be made. The base 3520 is then moved towards the proximal end 3112 of the housing 3110 to actuate the auto-injector 3002. The housing 3110 also includes a transparent status window 3118 (see FIG. 36) to allow a user to determine the status of the auto-injector 3002 or the medicament contained therein.

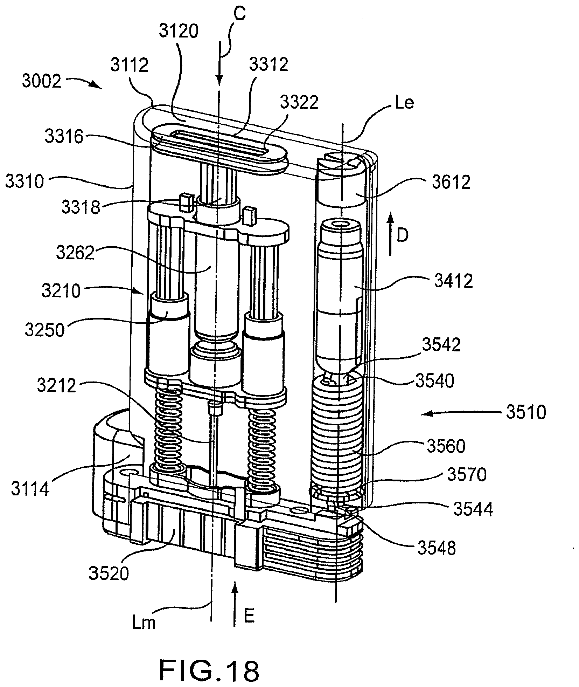

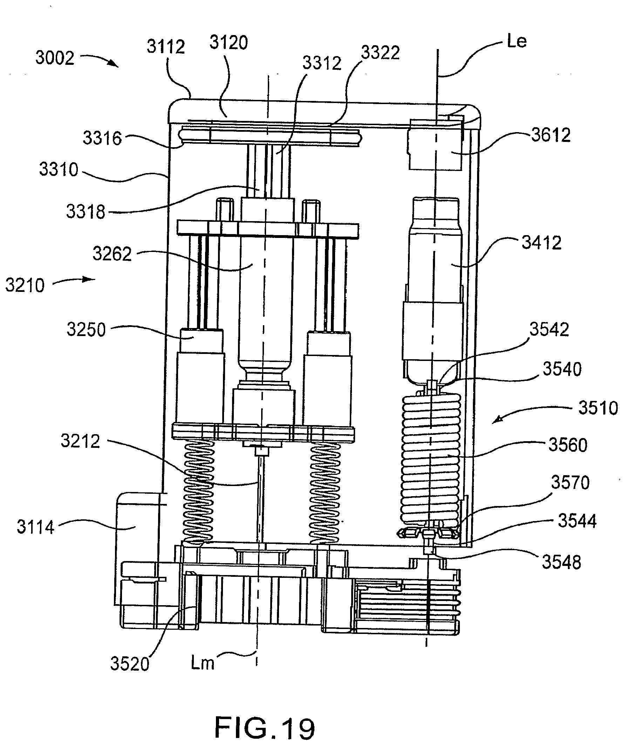

FIG. 18 is a perspective view of the auto-injector 3002 showing the housing 3110 in phantom lines so that the components contained within the housing 3110 can be more clearly seen. For clarity, FIG. 18 shows the auto-injector 3002 without the needle guard assembly 3810 and the safety lock 3710. Similarly, FIG. 19 is a front view of the auto-injector 3002 showing the housing 3110 in phantom lines. The auto-injector 3002 includes a medicament injector 3210 and a movable member 3312 engaged with the medicament injector 3210, each of which are disposed within the housing 3110. The auto-injector 3002 also includes a system actuator 3510, a compressed gas container 3412 and a gas release mechanism 3612.

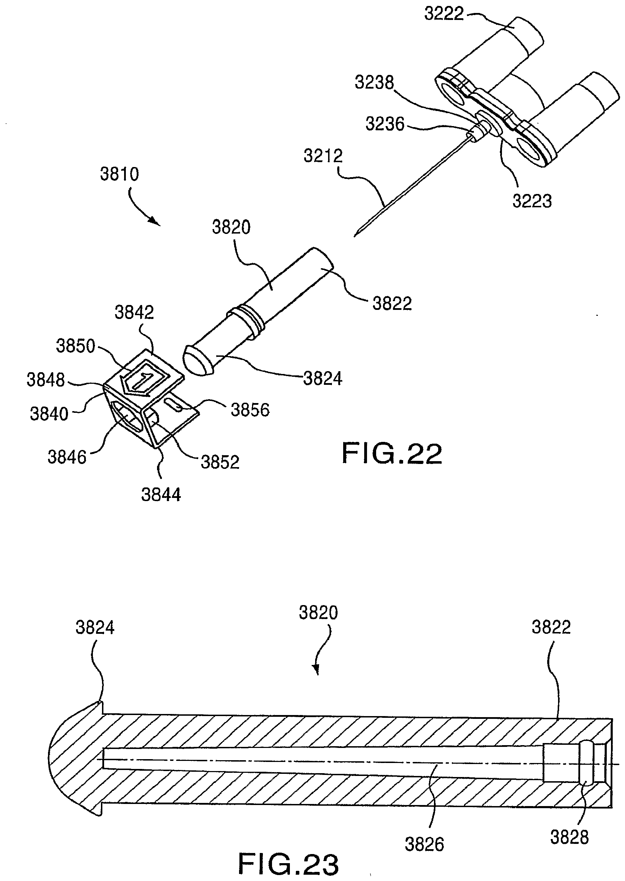

The medicament injector 3210 includes a carrier 3250 that is movable within the housing 3110, a medicament container 3262 and a needle 3212. The medicament container 3262 is coupled to the carrier 3250. The needle 3212 is disposed within a needle hub portion 3223 (see FIG. 22) of the carrier to allow the needle 3212 to be placed in fluid communication with the medicament container 3262 during an injection event.

The movable member 3312 includes a proximal end portion 3316 and a distal end portion 3318. The proximal end portion 3316 includes a surface 3322 that, together with the housing 3110, defines a gas chamber 3120. Said another way, the surface 3322 defines a portion of a boundary of the gas chamber 3120. The distal end portion 3318 is disposed within the medicament container 3262. In use, the movable member 3312 moves towards the distal end portion 3114 of the housing 3110, as indicated by arrow C, in response to a force produced by a pressurized gas on the surface 3322 of the movable member 3312. As a result, the movable member 3312 and the medicament injector 3250 are moved towards the distal end portion 3114 of the housing 3110, thereby exposing the needle 3212 from the housing 3110. The movable member 3312 then continues to move within the medicament container 3262 to expel a medicament from the medicament container 3262 through the needle 3212.

The auto-injector 3002 is actuated by the system actuator 3510, which is configured to move the compressed gas container 3412 into contact with the gas release mechanism 3612. The gas release mechanism 3612 punctures a portion of the compressed gas container 3412 to release the pressurized gas contained therein into the gas chamber 3120 defined by the housing 3110.

The system actuator 3510 includes a rod 3540, a spring 3560 and a spring retainer 3570. The rod 3540 has a proximal end portion 3542 and a distal end portion 3544. The proximal end portion 3542 of the rod 3540 is coupled to the compressed gas container 3412. The distal end portion 3544 of the rod 3540 is coupled to the spring retainer 3570 by two projections 3548, which can be moved inwardly towards each other to decouple the rod 3540 from the spring retainer 3570, as discussed below.

The spring 3560 is disposed about the rod 3540 in a compressed state such that the spring 3560 is retained by the proximal end portion 3542 of the rod 3540 and the spring retainer 3570. In this manner, the rod 3540 is spring-loaded such that when the distal end portion 3544 of the rod 3540 is decoupled from the spring retainer 3570, the force of the spring 3560 causes the rod 3540, and therefore the compressed gas container 3412, to move proximally as indicated by arrow D and into contact with the gas release mechanism 3612.

The base 3520 defines an opening 3522 configured to receive a portion of the projections 3548 when the base is moved towards the proximal end 3112 of the housing 3110, as indicated by arrow E. When the projections 3548 are received within the opening 3522, they are moved together causing the distal end portion 3544 of the rod 3540 to be released from the spring retainer 3570.

As shown in FIGS. 18 and 19, the medicament injector 3210 defines a longitudinal axis Lm that is non-coaxial with the longitudinal axis Le defined by the compressed gas container 3412. Accordingly, the medicament injector 3210, the compressed gas container 3412 and the system actuator 3510 are arranged within the housing 3110 such that the housing has a substantially rectangular shape. Moreover, the non-coaxial relationship between the medicament injector 3210 and the compressed gas container 3412 allows the auto-injector 3002 to be actuated by manipulating the base 3520, which is located at the distal end portion 3114 of the housing 3110.

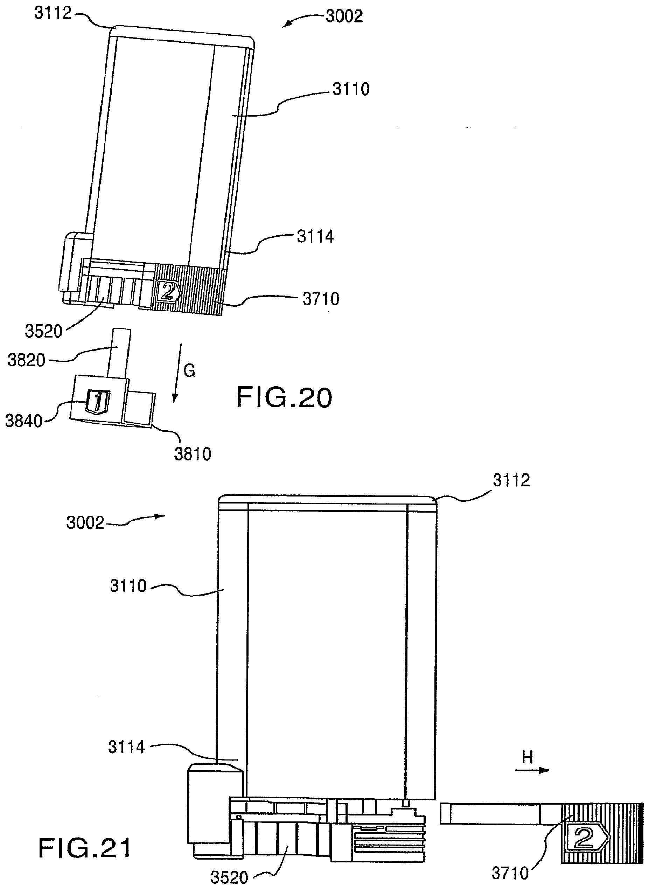

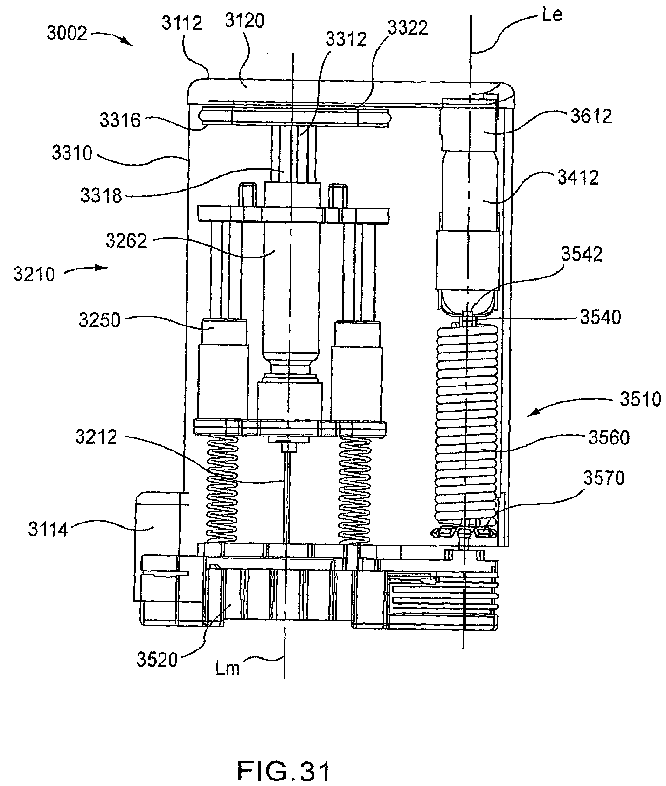

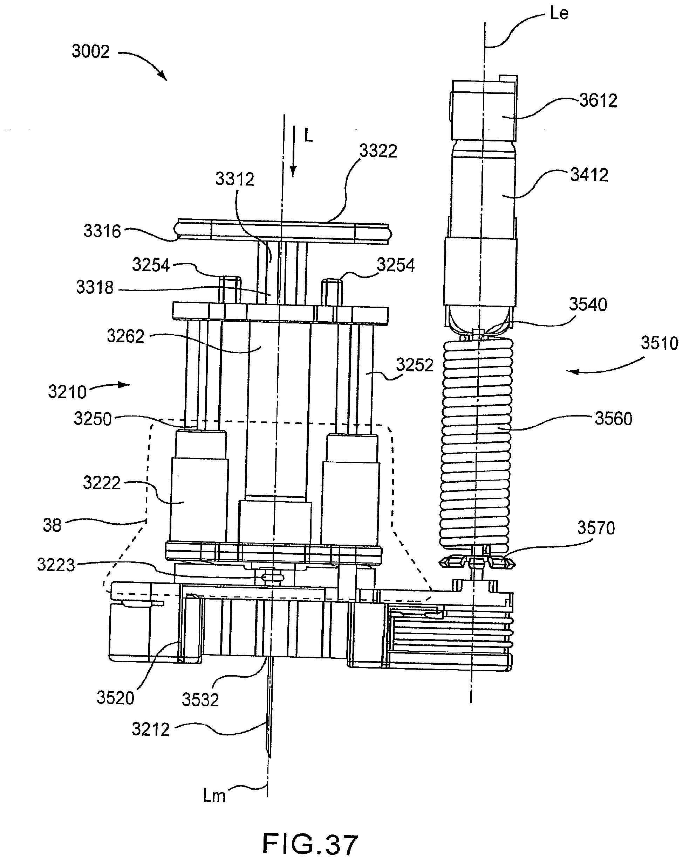

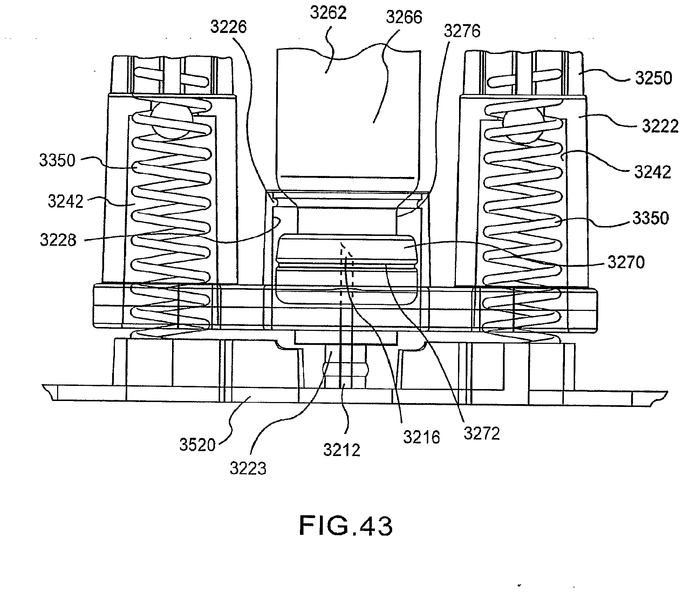

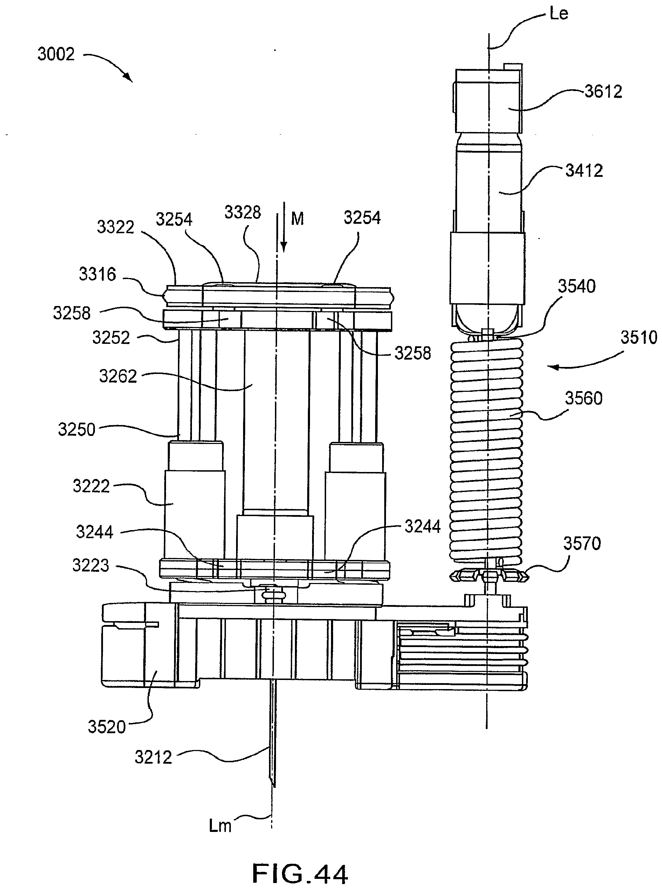

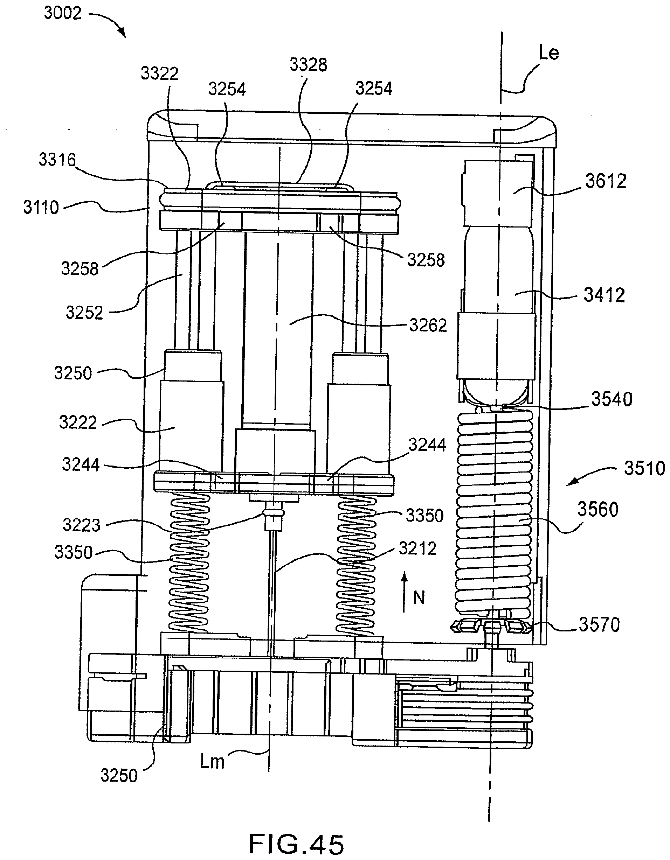

As discussed above, the use and actuation of the auto-injector 3002 includes several discrete operations. First, the auto-injector 3002 is enabled by removing the needle guard 3810 and the safety lock 3710 (see FIGS. 20 and 21). Second, the auto-injector 3002 is actuated by moving the base 3520 proximally towards the housing 3110. Third, when actuated, the compressed gas container 3412 engages the gas release mechanism 3612, which causes the pressurized gas to be released into the gas chamber 3120 (see FIG. 31). Fourth, the pressurized gas produces a force that causes the movable member 3312 and the medicament injector 3210 to move distally within the housing 3110 (see FIG. 37). The movement of the medicament injector 3210 causes the needle 3212 to extend from distal end portion 3114 of the housing 3110 and the base 3520. This operation can be referred to as the "needle insertion" operation. Fifth, when the medicament injector 3210 has completed its movement (i.e., the needle insertion operation is complete), the movable member 3312 continues to move the medicament container 3262 distally within the carrier 3250. The continued movement of the medicament container 3262 places the needle 3212 in fluid communication with the medicament container 3262, thereby allowing the medicament to be injected (see FIG. 43). Sixth, the force from the pressurized gas causes the movable member 3312 to move within the medicament container 3262, thereby expelling the medicament through the needle 3212 (see FIG. 44). This operation can be referred to as the "injection operation." Seventh, upon completion of the injection, the pressurized gas is released from the gas chamber 3120, thereby allowing the medicament injector 3210 and the movable member 3312 to be moved proximally within the housing. This operation can be referred to as the "retraction operation" (see FIG. 45). A detailed description of the components contained in the auto-injector 3002 and how they cooperate to perform each of these operations is discussed below.

Prior to use, the auto-injector 3002 must first be enabled by first removing the needle guard 3810 and then removing the safety lock 3710. As illustrated by arrow G in FIG. 20, the needle guard 3810 is removed by pulling it distally. Similarly, as illustrated by arrow H in FIG. 21, the safety lock 3710 is removed by pulling it substantially normal to the longitudinal axis Le of the compressed gas container 3412. Said another way, the safety lock 3710 is removed by moving it in a direction substantially normal to the direction that the needle guard 3810 is moved. As described in more detail herein, the needle guard 3810 and the safety lock 3710 are cooperatively arranged to prevent the safety lock 3710 from being removed before the needle guard 3810 has been removed. Such an arrangement prevents the auto-injector 3002 from being actuated while the needle guard 3810 is in place.

As illustrated in FIG. 22, the needle guard 3810 includes a sheath 3820 and a sheath retainer 3840. The sheath 3820 has a proximal end portion 3822 and a distal end portion 3824 and defines an opening 3826 configured to receive a portion of the needle 3212 when the needle guard 3810 is in a first (or installed) position. The sheath 3820 further defines a recessed portion 3828 within the opening 3826 that engages a corresponding protrusion 3238 defined by an outer surface 3236 of the needle hub 3223. In this manner, when the needle guard 3810 is in its first position, the sheath 3820 is removably coupled to the needle hub 3223. In some embodiments, the recessed portion 3828 and the protrusion 3238 form a seal that is resistant to microbial penetration.

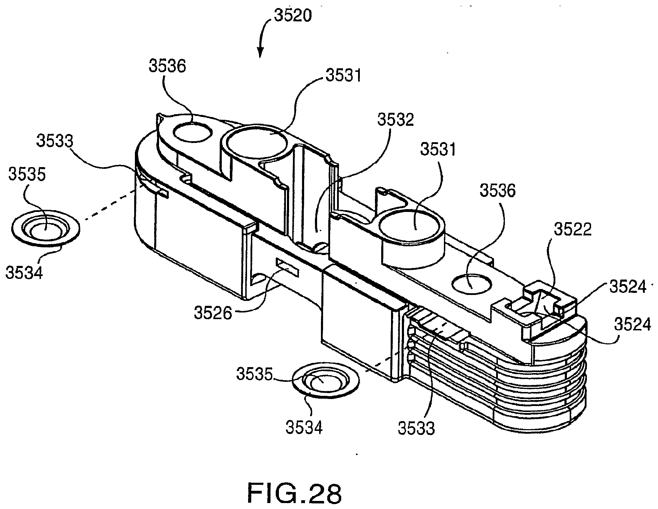

The sheath retainer 3840 has a proximal portion 3842 and a distal portion 3844. The proximal portion 3842 of the sheath retainer 3840 includes a protrusion 3856 that engages a corresponding recess 3526 in the base 3520 (see FIG. 28) to removably couple the sheath retainer 3840 to the base 3520. The distal portion 3844 of the sheath retainer 3840 defines an opening 3846 through which the distal end portion 3824 of the sheath 3820 is disposed. The distal portion 3844 of the sheath retainer 3840 includes a series of retaining tabs 3852 that engage the distal end portion 3824 of the sheath 3820 to couple the sheath 3820 to the sheath retainer 3840. In this manner, when the sheath retainer 3840 is moved distally away from the base 3520 into a second (or removed) position, as shown in FIG. 20, the sheath 3820 is removed from the needle 3412. Moreover, this arrangement allows the sheath 3820 to be disposed about the needle 3412 independently from when the sheath retainer 3840 is coupled to the sheath 3820. As such, the two-piece construction of the needle guard provides flexibility during manufacturing. The distal portion 3844 of the sheath retainer 3840 also includes a protrusion 3848 to aid the user when grasping the needle guard 3810.

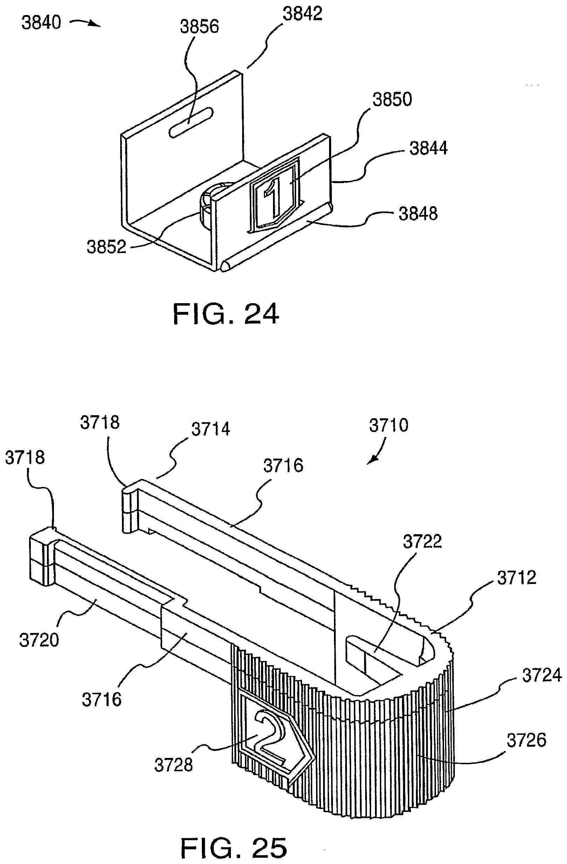

When the needle guard 3810 is in its first position, the sheath retainer 3840 is disposed within a recess 3720 defined by one of the extended portions 3716 of the safety lock 3710 (see FIG. 25). This arrangement prevents the safety lock 3710 from being removed when the needle guard 3810 is in its first position, which in turn, prevents the auto-injector 3002 from being actuated when the needle guard 3810 is in its first position.

The outer surface of the sheath retainer 3840 includes an indicia 3850 to instruct the user in operating the auto-injector 3002. As shown in FIG. 21, the indicia 3850 includes a numeral to indicate the order of operation and an arrow to indicate the direction in which the needle guard 3810 should be moved. In some embodiments, the indicia 3850 can include different colors, detailed instructions or any other suitable indicia to instruct the user. In other embodiments, the indicia 3850 can protrude from the sheath retainer 3840 to aid the user when grasping the needle guard 3810.

In some embodiments, the sheath 3820 can be constructed from any suitable material, such as, for example polypropylene, rubber or any other elastomer. In some embodiments, the sheath 3820 can be constructed from a rigid material to reduce the likelihood of needle sticks during the manufacturing process. In other embodiments, the sheath 3820 can be constructed from a flexible material.

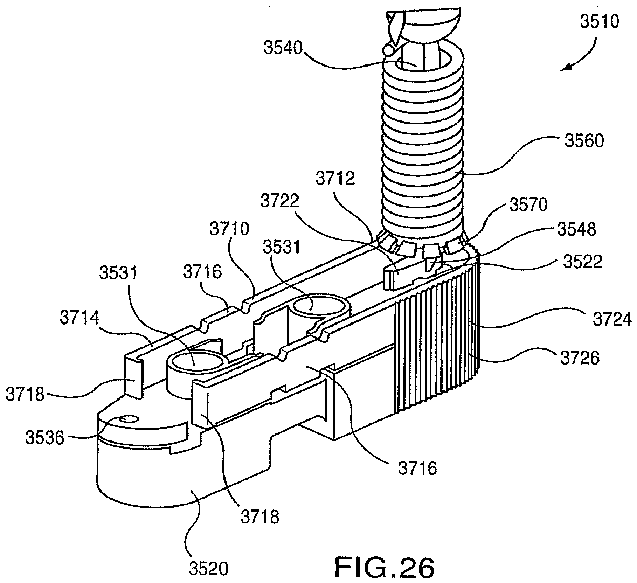

After the needle guard 3810 is removed, the user must then remove the safety lock 3710, as indicated in FIG. 21. As shown in FIG. 25, the safety lock 3710 is a U-shaped member having a first end 3712 and a second end 3714. The second end 3714 of the safety lock 3710 includes two extended portions 3716, each of which includes an inwardly facing protrusion 3718. When the safety lock 3710 is in its first (or locked) position, the extended portions 3716 extend around a portion of the base 3520 to space the base 3520 apart from the distal end portion 3114 of the housing 3110. As shown in FIG. 26, the protrusions 3718 are configured engage a portion of the base 3520 to removably couple the safety lock 3710 in its first position.

One of the extended portions 3716 defines a recess 3720 that receives the sheath retainer 3840 when the needle guard 3810 is in its first position, as discussed above. Although only one extended portion 3716 is shown as including a recess 3720, in some embodiments both extended portions 3716 can include a recess 3720 to receive the sheath retainer 3840. In other embodiments, the safety lock 3710 can be engaged with the needle guard 3810 to prevent movement of the safety lock 3710 when the needle guard 3810 is in place in any suitable manner. For example, in some embodiments, the sheath retainer can include protrusions that are received within corresponding openings defined by the safety lock. In other embodiments, the safety lock can include protrusions that are received within corresponding openings defined by the sheath retainer.

The first end 3712 of the safety lock 3710 includes a locking protrusion 3722 that extends inwardly. As shown in FIG. 26, when the safety lock 3710 is in its first position, the locking protrusion 3722 extends between the projections 3548 of the rod 3540 and obstructs the opening 3522 of the base 3520. In this manner, when the safety lock 3710 is in its first position, the base 3520 cannot be moved proximally to allow the projections 3548 to be received within the opening 3522. The arrangement of the locking protrusion 3722 also prevents the projections 3548 from being moved inwardly towards each other. Accordingly, when the safety lock 3710 is in its first position, the auto-injector 3002 cannot be actuated.

The outer surface 3724 of the first end 3712 of the safety lock 3710 includes a series of ridges 3726 to allow the user to more easily grip the safety lock 3710. The outer surface 3724 of the first end 3712 of the safety lock 3710 also includes an indicia 3728 to instruct the user in operating the auto-injector 3002. As shown in FIG. 25, the indicia 3728 includes a numeral to indicate the order of operation and an arrow to indicate the direction in which the safety lock 3710 should be moved. In some embodiments, the indicia 3728 can include different colors, detailed instructions or any other suitable indicia to instruct the user. In other embodiments, the indicia 3728 can protrude from the safety lock 3710 to aid the user when grasping the safety lock 3710.

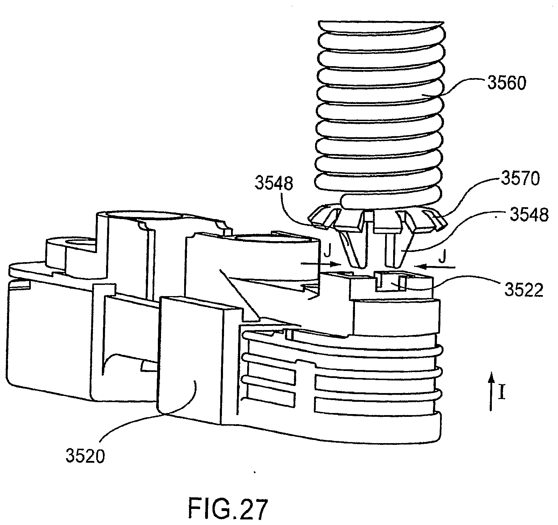

After being enabled, the auto-injector 3002 can then be actuated by moving the base 3520 proximally towards the housing 3110, as indicated by arrow I in FIG. 27. As shown in FIGS. 28 and 36, the base 3520 defines two openings 3536 that receive corresponding attachment protrusions 3150 disposed on the distal end portion 3114 of the housing 3110. In this manner, the movement and/or alignment of the base 3520 relative to the housing 3110 is guided by the attachment protrusions 3150 and the openings 3536 (see FIG. 36).

Each attachment protrusion 3150 is secured within its corresponding opening 3536 by a lock washer 3534. The lock washers 3534 each define an opening 3535 that receives a portion of the attachment protrusion 3150. The lock washers 3534 are disposed within slots 3533 defined by the base 3520 so that the openings 3535 are aligned with the attachment protrusions 3150. The openings 3535 are configured to allow the lock washers 3534 to move proximally relative to the attachment protrusions 3150, but to prevent movement of the lock washers 3534 distally relative to the attachment protrusions 3150. In this manner, when the attachment protrusions 3150 are disposed within the openings 3535 of the lock washers 3534, the base 3520 becomes fixedly coupled to the housing 3110. Moreover, after the base 3520 is moved proximally relative to the housing 3110, the lock washers 3534 prevent the base 3520 from returning to its initial position. Said another way, the arrangement of the lock washers 3534 prevents the base 3520 from being "kicked back" after the auto-injector 3002 has been actuated.

The base 3520 also defines a needle opening 3532, a recess 3526 and two retraction spring pockets 3531. The needle opening 3532 receives a portion of the needle guard 3810 when the needle guard is in its first position. Additionally, when the auto-injector is in its third configuration (see FIG. 37), the needle 3212 extends through the needle opening 3532. As described above, the recess 3526 receives the corresponding protrusion 3856 on the sheath retainer 3840 to removably couple the needle guard 3810 to the base 3520. As will be described in more detail herein, the retraction spring pockets 3531 receive a portion of the retraction springs 3350.

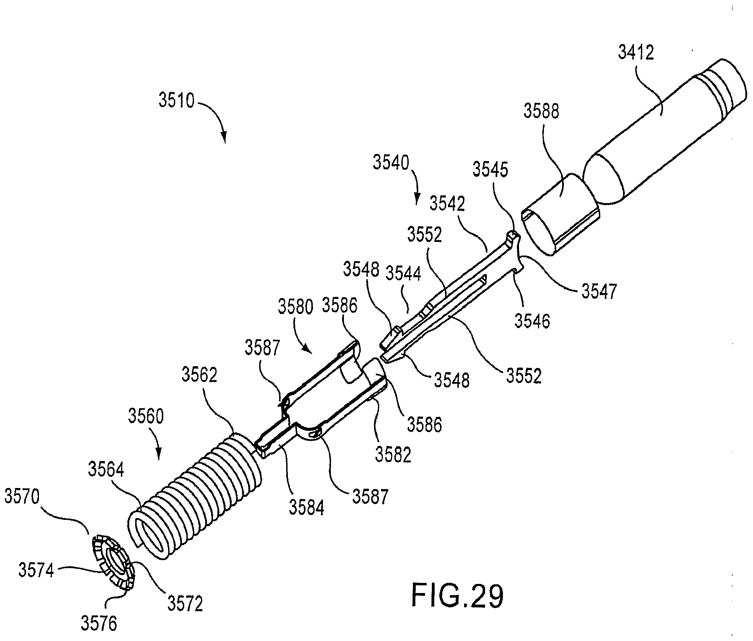

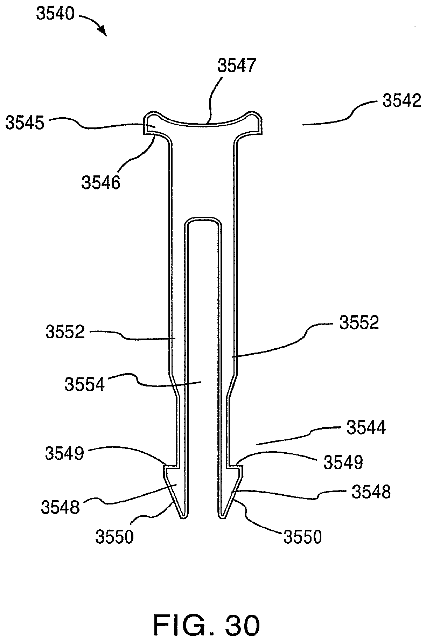

As shown in FIG. 28, the base 3520 includes two opposing tapered surfaces 3524 that define an opening 3522 configured to receive a corresponding tapered surface 3550 of the projections 3548 when the base is moved proximally towards the housing 3110. When the projections 3548 are received within the tapered opening 3522, they are moved together as indicated by arrows J in FIG. 27. The inward movement of the projections 3548 causes the rod 3540 to become disengaged from the spring retainer 3570, thereby allowing the rod 3540 to be moved proximally along its longitudinal axis as the spring 3560 expands. A more detailed description of the components included in the system actuator 3510 is provided below with reference to FIGS. 29 and 30.

The system actuator 3510 includes a rod 3540, a spring 3560 disposed about the rod 3540 and a spring retainer 3570. As described in more detail herein, the spring retainer 3570 retains both the spring 3560 and the rod 3540. The spring retainer 3570 includes a first surface 3572, a second surface 3574 and a series of outwardly extending engagement tabs 3576. The spring retainer 3570 is disposed within the gas container opening 3124 defined by the housing 3110 (see FIG. 36) such that the engagement tabs 3576 engage the interior surface 3123 of the housing 3110 to produce an interference fit. In this manner, the spring retainer 3570 is fixedly disposed within the housing 3110.

The rod 3540 has a proximal end portion 3542 and a distal end portion 3544. The distal end portion 3544 of the rod 3540 includes two extensions 3552 disposed apart from each other to define an opening 3554 therebetween. Each extension 3552 includes a projection 3548 having a tapered surface 3550 and an engagement surface 3549. When the rod 3540 is in its first (or engaged) position, the engagement surfaces 3549 engage the second surface 3574 of the spring retainer 3570 to prevent the rod 3540 from moving proximally along its longitudinal axis. As described above, when the base 3520 is moved proximally towards the housing 3110, the tapered surfaces 3550 of the projections 3548 cooperate with the corresponding tapered surfaces 3524 of the base 3520 to move the extensions 3552 inwardly towards each other. The inward motion of the extensions 3552 causes the engagement surfaces 3549 to become disengaged from the second surface 3574 of the spring retainer 3570, thereby allowing the rod 3540 to move between its first position to a second (or actuated) position.

The proximal end portion 3542 of the rod 3540 includes a retention portion 3545 having a first surface 3547 and a second surface 3546. The first surface 3547 of the retention portion 3545 engages the distal portion 3416 of the compressed gas container 3412. The second surface 3546 of the retention portion 3545 engages a proximal end 3562 of the spring 3560. Similarly, the first surface 3572 of the spring retainer 3570 engages a distal end 3564 of the spring 3560. In this manner, when the rod 3540 is in its first position, the spring 3560 can be compressed between the spring retainer 3570 and the retention portion 3545 of the rod 3540. Accordingly, when the rod 3540 is disengaged from the spring retainer 3570, the force imparted by the spring 3560 on the retention portion 3545 of the rod 3540 causes the rod 3540 to move proximally into its second position.

The proximal end portion 3542 of the rod 3540 is coupled to the compressed gas container 3412 by a connector 3580, which is secured to the distal end portion 3416 of the compressed gas container 3412 by a securing member 3588. The connector 3580 includes a proximal end portion 3582 and a distal end portion 3584. The distal end portion 3584 of the connector 3580 is disposed within the opening 3554 defined between the extensions 3552. In this manner, the connector 3580 is retained by the proximal end portion 3542 of the rod 3540. As will be described in more detail, the distal end portion 3584 of the connector 3580 includes locking tabs 3587.

The proximal end portion 3582 of the connector 3580 includes engagement portions 3586 that engage the distal end portion 3416 of the compressed gas container 3412. The engagement portions 3586 are coupled to the compressed gas container 3412 by the securing member 3588, which can be, for example, a shrink wrap, an elastic band or the like. In other embodiments, the engagement portions 3586 can produce an interference fit with the compressed gas container 3412, thereby eliminating the need for a securing member 3588.

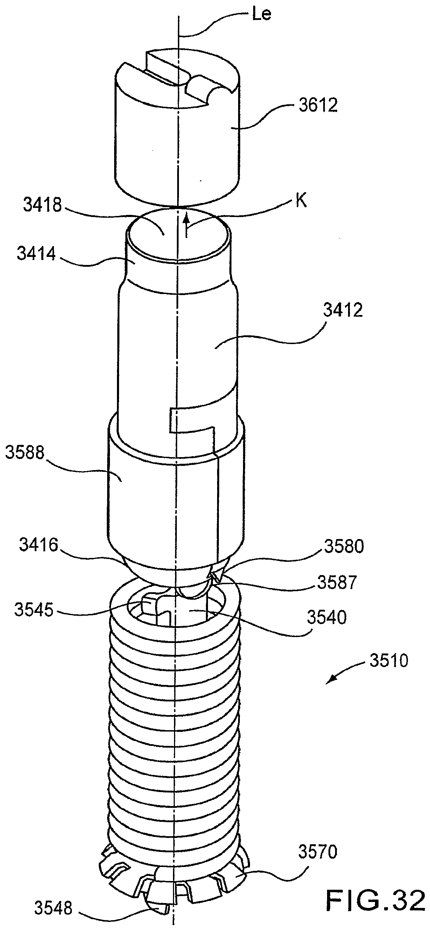

Because the rod 3540 is coupled to the compressed gas container 3412, when the rod 3540 is moved from its first (engaged) position to its second (actuated) position, the compressed gas container 3412 is moved proximally within the housing 3110 into engagement with the gas release mechanism 3612. FIG. 31 shows the auto-injector in a second configuration, in which the compressed gas container 3412 is engaged with the gas release mechanism 3612. When in the second configuration, the compressed gas contained within the compressed gas container 3412 is released to actuate the medicament injector 3210. A more detailed description of the gas release process is provided below with reference to FIGS. 32 through 36.

FIG. 32 shows an exploded view of the system actuator 3510, the compressed gas container 3412 and the gas release mechanism 3612, each of which are disposed within the gas container opening 3124 defined by the housing 3110 (see FIG. 36). As shown, the compressed gas container 3412, the system actuator 3510 and the gas release mechanism 3612 are arranged substantially coaxial with each other. As previously discussed, when the auto-injector 3002 is actuated, the compressed gas container 3412 is moved proximally within the gas container opening 3124 defined by the housing 3110, as indicated by the arrow K in FIG. 32, until the proximal end 3414 of the compressed gas container 3412 engages the gas release mechanism 3612.

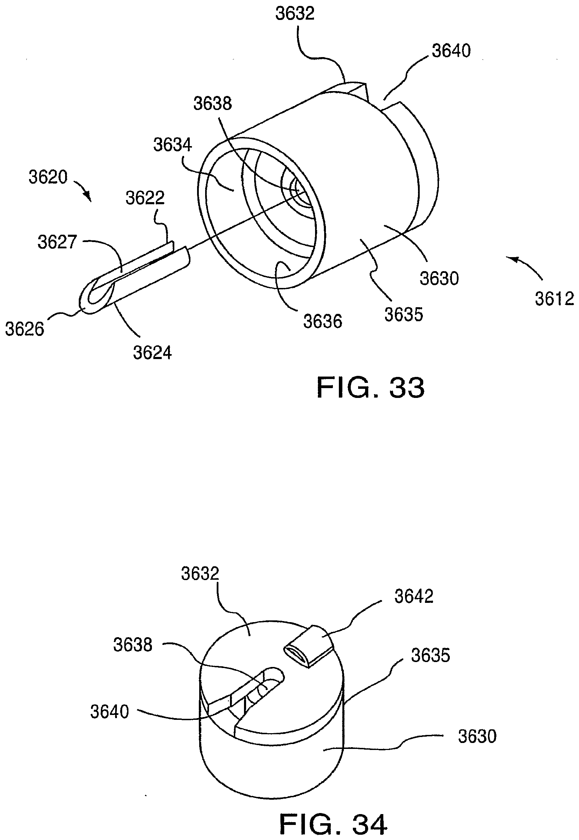

As shown in FIGS. 33 and 34, the gas release mechanism 3612 includes a cap 3630 and a puncturing element 3620 coupled to and disposed within the cap 3630. The puncturing element has a proximal end 3622 and a distal end 3624. The distal end 3624 of the puncturing element 3620 defines a sharp point 3626 configured to puncture the proximal end 3414 of the compressed gas container 3412. The puncturing element 3620 defines an opening 3627 extending from its distal end 3624 to its proximal end 3622.

The cap 3630 has a proximal end 3632, an outer surface 3635 and an inner surface 3636. The inner surface 3636 of the cap 3630 defines an opening 3634 that receives the proximal end 3414 of the compressed gas container 3412 when the auto-injector 3002 is in its second configuration. The proximal end 3632 of the cap 3630 defines an opening 3638 therethrough and a channel 3640 in fluid communication with the opening 3638. The opening 3638 receives the proximal end 3622 of the puncturing element 3620 to couple the puncturing element 3620 to the cap 3630. The puncturing element 3620 is disposed within the cap 3630 such that when the compressed gas container 3412 is moved into the opening 3634, the distal end 3624 of the puncturing element 3620 punctures the proximal end 3414 of the compressed gas container 3412.

The cap 3630 is disposed within the gas container opening 3124 such that the outer surface 3635 of the cap 3630 engages the inner surface 3123 of the housing 3110. In some embodiments, the outer surface 3635 of the cap 3630 can be sized to produce an interference fit with the inner surface 3123 of the housing 3110. In other embodiments, the cap 3630 can be fixedly coupled within the gas container opening 3124 using an adhesive or any other suitable attachment mechanism.

The cap 3630 is oriented within the gas container opening 3124 so that the channel 3640 is aligned with and in fluid communication with the gas passageway 3126 defined by the housing 3110. Moreover, when oriented in this manner, the protrusion 3642 on the proximal end 3632 of the cap 3630 obstructs a portion of the gas passageway 3126, which can be manufactured as a through-hole, to fluidically isolate the gas passageway 3126 from an area outside of the housing 3110. After the proximal end 3414 of the compressed gas container 3412 has been punctured, pressurized gas flows from the compressed gas container 3412 into the gas passageway 3126 through the opening 3627 defined by the puncturing element 3620 and the channel 3640 defined by the proximal end 3632 of the cap 3630.

The inner surface 3636 of the cap 3630 is configured to hermetically seal the proximal end 3414 of the compressed gas container 3412 within the opening 3638. This arrangement prevents pressurized gas from leaking around the compressed gas container 3412 to an area outside of the housing 3110 after the proximal end 3414 of the compressed gas container 3412 has been punctured. In some embodiments, the inner surface 3636 is sized to produce an interference fit with the compressed gas container 3412. In other embodiments, the cap 3630 includes a separate sealing member, such as, for example, an o-ring, to seal the proximal end 3414 of the compressed gas container 3412 within the opening 3638.

After the compressed gas container 3412 is moved into engagement with the gas release mechanism 3612, the position of the compressed gas container 3412 within the gas container opening 3124 is maintained by the locking tabs 3587 on the connector 3580. As shown in FIG. 29, each locking tab 3587 includes a pointed portion that is angled outwardly from the connector 3580. This arrangement allows the connector 3580 to move proximally within the gas container opening 3124 of the housing 3110, but prevents the connector 3580 from moving distally within the gas container opening 3124 of the housing 3110. Said another way, the arrangement of the locking tabs 3587 prevents the compressed gas container 3412 from being "kicked back" when exposed to the force produced by the pressurized gas as the pressurized gas is released.

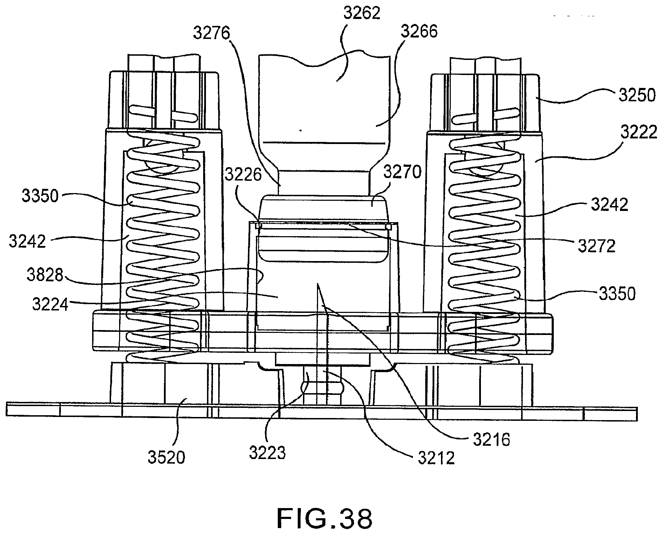

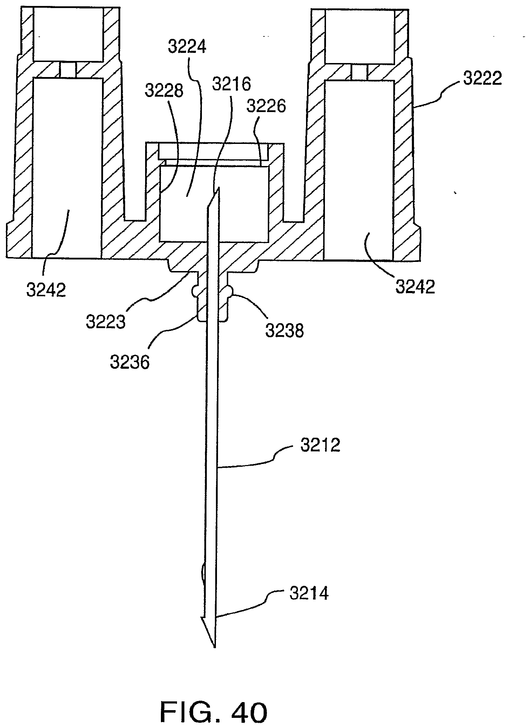

As previously discussed, the pressurized gas released from the compressed gas container 3412 produces a force on the boundary of the gas chamber 3120, including the surface 3322 of the movable member 3312. This force causes the movable member 3312 and the medicament injector 3210 move together distally within the housing 3110, as shown by arrow L, placing the auto-injector 3002 in a third configuration, as shown in FIG. 37. When in the third configuration, the distal end 3214 of the needle 3212 is disposed through the opening 3532 defined by the base 3520 to an area outside of the auto-injector 3002. Moreover, as shown in FIG. 38, when the auto-injector 3002 is in the third configuration, the proximal end 3216 of the needle 3212 remains spaced apart from the distal end 3266 of the medicament container 3210, ensuring that the needle 3212 remains fluidically isolated from the medicament container 3210. In this manner, the needle 3212 can be inserted into a patient as the auto-injector 3002 moves between its second configuration (FIG. 31) and its third configuration (FIG. 37) without injecting the medicament until after insertion is completed. A more detailed description of the medicament injector 3210 and the movable member 3312 is provided below with reference to FIGS. 37 through 42.

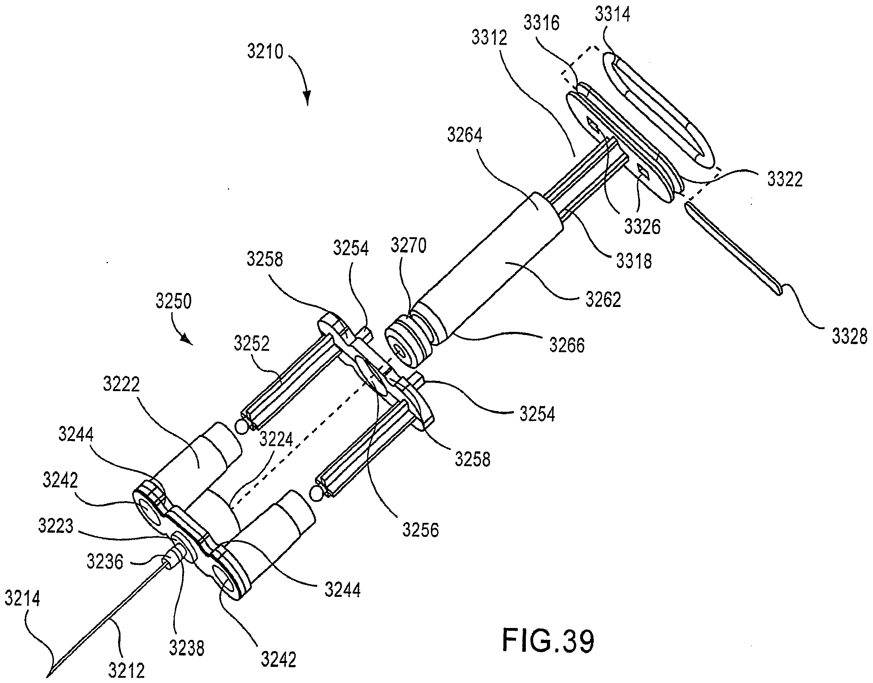

As previously described, the medicament injector 3210 includes a carrier 3250, a medicament container 3262 and a needle 3212. The carrier 3250 has a lower portion 3222 and an upper portion 3252. The lower portion 3222 of the carrier 3250 includes a needle hub 3223, which contains the needle 3212. The lower portion 3222 of the carrier 3250 also defines an opening 3224 configured to receive a distal portion 3266 the medicament container 3262. As shown in FIG. 39, the needle 3212 is coupled to the needle hub 3223 such that the proximal end 3216 of the needle 3212 is disposed within the opening 3224 and the distal end 3214 of the needle 3212 extends distally outside of the needle hub 3223.

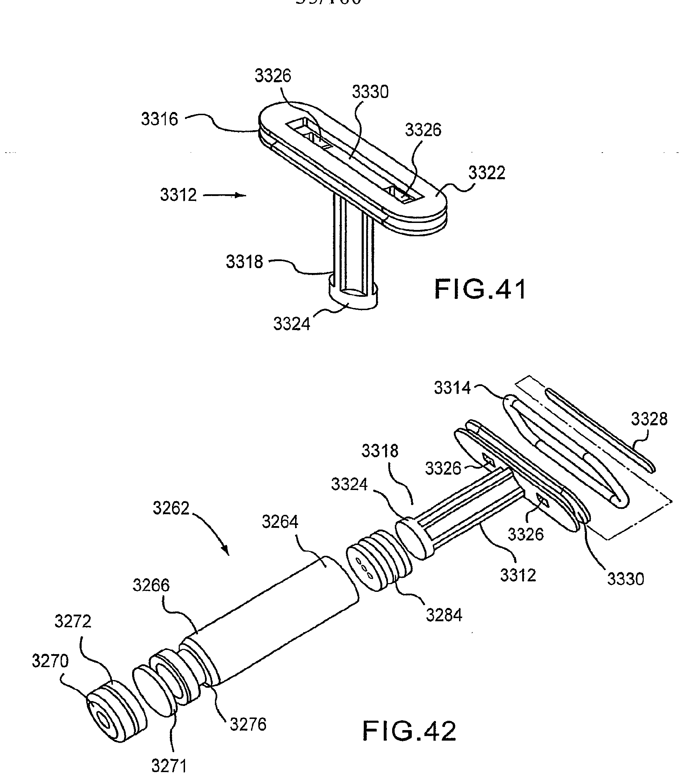

The inner surface 3228 of the lower portion 3222 defining the opening 3224 includes a protrusion 3226. The protrusion 3226 is configured to engage a corresponding recess 3272 defined by a sealing cap 3270 disposed at the distal portion 3266 of the medicament container 3262 (see FIG. 42) to secure the medicament container 3262 within the opening 3224 such that the proximal end 3216 of the needle 3212 is spaced apart from the distal end 3266 of the medicament container 3210. The protrusion 3226 and the recess 3272 are configured such that the protrusion 3226 will become disengaged from the recess 3272 when the force applied exceeds a predetermined value. Said another way, the protrusion 3226 and the recess 3272 collectively form a removable snap-fit that allows the medicament container 3262 to be moved within the opening 3224 when the force applied to the medicament container 3262 exceeds a predetermined value. This arrangement ensures that the needle 3212 remains fluidically isolated from the medicament container 3262 during the insertion operation.

The outer surface 3236 of the lower portion 3222 includes a protrusion 3238. As previously described, the protrusion 3238 is configured to engage a corresponding recess portion 3828 within the opening 3826 of the sheath 3820 (see FIG. 23) to removably couple the sheath 3820 to the needle hub 3223.

The lower portion 3222 of the carrier 3250 also defines two retraction spring pockets 3242 each receiving the proximal end 3352 of a retraction spring 3350. As previously discussed, the distal end 3354 of each retraction spring 3350 is retained within the retraction spring pockets 3531 defined by the base 3520. As shown in FIG. 38, when the carrier 3250 moves distally within the housing 3110, the retraction springs 3350 are compressed and therefore bias the carrier 3250 towards the proximal portion 3112 of the housing 3110.

The upper portion 3252 of the carrier 3250 defines an opening 3256 configured to receive a proximal portion 3264 of the medicament container 3262 and includes two valve actuators 3254. As described in more detail herein, the valve actuators 3254 are configured to engage a gas relief valve 3328 to allow the pressurized gas contained within the gas chamber 3120 to escape when the injection event is complete.

The upper portion 3252 of the carrier 3250 defines four gas relief passageways 3258. Similarly, the lower portion 3222 of the carrier 3250 defines four gas relief passageways 3244. When the pressurized gas is released from the gas chamber 3120, the gas relief passageways 3258, 3244 provide a fluid path to allow the pressurized gas to flow from the gas chamber 3120 to an area outside of the housing 3110.

As described above, the movable member 3312 includes a proximal end portion 3316 and a distal end portion 3318. The distal end portion 3318 includes a piston 3324 disposed within the proximal portion 3264 of the medicament container 3262, such that the piston engages a plunger 3284 contained within the medicament container 3262, as shown in FIG. 42.

The proximal end portion 3316 includes a surface 3322 that defines a portion of a boundary of the gas chamber 3120. As shown in FIG. 41, the proximal end portion 3316 defines two openings 3326 therethrough, each of which are in fluid communication between the gas chamber 3120 and the interior of the housing 3110 outside the gas chamber 3120. The proximal end portion 3316 further defines a slot 3330 that receives a gas relief valve 3328, which can be, for example, a flexible rubber member. The gas relief valve 3328 is positioned within the slot 3330 and adjacent the openings 3326 to selectively allow fluid communication between the gas chamber 3120 and the area outside the gas chamber 3120 through the openings 3326. The operation of the gas relief valve 3328 is discussed in more detail herein.

The proximal end portion 3316 of the movable member 3312 also includes a seal 3314 that engages a portion the inner surface 3122 of the housing 3110 (see FIG. 36) to fluidically isolate the gas chamber 3120. Although the seal 3314 is shown as being an o-ring seal, in some embodiments, the seal need not be a separate component, but can rather be a portion of the proximal end portion 3316 of the movable member 3312.

When the needle insertion operation is completed, the lower portion 3222 of the carrier 3250 engages the base 3520, preventing further distal movement of the carrier 3250 within the housing. Because the distal motion of the carrier 3250 is opposed, the force exerted by the pressurized gas on the surface 3322 of the movable member 3312 increases until the protrusion 3226 of the lower portion 3222 of the carrier 3250 and the recess 3272 defined by sealing cap 3270 of the medicament container 3262 become disengaged. Accordingly, the medicament container 3262 to moves distally relative to the carrier 3250, placing the auto-injector 3002 in a fourth configuration, as shown in FIG. 43. When moving between the third configuration (FIG. 38) and the fourth configuration (FIG. 43), the proximal end 3216 of the needle 3212 pierces the sealing cap 3270 and the liner 3271 disposed at the distal portion 3266 of the medicament container 3262. As such, when in the fourth configuration, the proximal end 3216 of the needle 3212 is in fluid communication with the medicament container 3262, thereby allowing the medicament to be injected.

Once the needle 3212 is in fluid communication with the medicament container 3262, the force from the pressurized gas causes the piston 3324 of the movable member 3312 to move the plunger 3284 within the medicament container 3262, as shown by arrow M, thereby expelling the medicament through the needle 3212. The piston 3324 and the plunger 3284 move a predetermined distance within the medicament container 3262, placing the auto-injector 3002 in a fifth configuration, as shown in FIG. 44. When the auto-injector 3002 is in the fifth configuration, the injection of medicament is complete.

When the auto-injector 3002 is in its fifth configuration, proximal portion 3316 of the movable member 3312 is in contact with the upper portion 3252 of the carrier 3250, thereby preventing further movement of the piston 3324 within the medicament container 3262. In this manner, the distance through which the piston 3324 travels, and therefore the amount of medicament injected, can be controlled.

Additionally, when the auto-injector 3002 is in its fifth configuration, the valve actuators 3254 are disposed within the openings 3326 such that the valve actuators 3254 displace the gas relief valve 3328. Accordingly, the pressurized gas contained within the gas chamber 3120 can flow from the gas chamber 3120 to the area within the housing 3310 outside of the gas chamber 3310. As previously discussed, the gas relief passageways 3258, 3244 provide a fluid path to allow the pressurized gas to flow from the gas chamber 3120, through the opening 3532 defined by the base 3520 and to an area outside of the housing 3110.

When the pressurized gas flows out of the gas chamber 3120, the pressure exerted on the surface 3322 of the movable member 3312 decreases. Accordingly, the force exerted by the retraction springs 3350 is sufficient to move the medicament injector 3210 and the movable member 3312 proximally within the housing 3110, as shown by arrow N, into a sixth (or retracted) configuration as shown in FIG. 45. Because the medicament injector 3210 and the movable member 3312 move together, the valve actuators 3254 remain disposed within the openings 3326 as the auto-injector 3002 moves into the sixth configuration. In this manner, the gas relief valve 3328 remains displaced and the openings 3326 remain in fluid communication with the gas chamber 3120 and the area within the housing 3310 outside of the gas chamber 3310 independent of the position of the movable member 3312. Such an arrangement ensures that all of the pressurized gas flows out of the gas chamber 3120, thereby ensuring that the medicament injector 3210 and the movable member 3312 return to the sixth configuration and do not oscillate between the sixth configuration and the fifth configuration, which could lead to the needle 3212 not being fully retracted into the housing 3110.

Although the auto-injector 3002 has been shown and described having a housing 3110 having a substantially rectangular shape, in some embodiments, an auto-injector can have a housing having any shape. In some embodiments, for example, an auto-injector can have a substantially cylindrical shape. In other embodiments, for example, the auto-injector can have an irregular and/or asymmetrical shape.

Although the auto-injector 3002 has been shown and described as including a protrusion 3142 disposed at the distal end portion 3114 of the housing 3110 to help a user grasp and retain the housing 3110, in some embodiments, a protrusion can be disposed anywhere along the housing. In other embodiments, a protrusion can symmetrically surround the distal portion of the housing. In yet other embodiments, the housing of an auto-injector can include a gripping portion configured to help a user grasp and retain the housing. The gripping portion can include, for example, a textured surface, a contoured surface, a surface having an adhesive that forms a tacky surface to adhere to the user's hand or the like.

Certain components of the auto-injector 3002 are shown and described as being coupled together via protrusions and mating recesses. The protrusions and/or recesses can be disposed on any of the components to be coupled together and need not be limited to only a certain component. For example, the base 3520 is shown as defining two openings 3536 that receive corresponding attachment protrusions 3150 on the distal end portion 3114 of the housing 3110. In some embodiments, however, the protrusions can be disposed on the base and the mating recesses can be defined by the distal end portion of the housing. In other embodiments, two or more components can be coupled together in any suitable way, which need not include protrusions and mating recesses. For example, in some embodiments, two or more components can be coupled together via mating shoulders, clips, adhesive and the like.

Similarly, although certain components of the auto-injector 3002 are shown and described as being constructed from multiple separate components, in some embodiments, such components can be monolithically constructed. For example, the carrier 3250 is shown and described as including an upper portion 3252 and a lower portion 3222 that are constructed separately and then coupled together. In other embodiments, a carrier can be constructed monolithically.

Although the base 3520 of the auto-injector 3002 has been shown and described covering almost the entire distal end portion 3114 of the housing 3110, in some embodiments, a base configured to actuate the auto-injector can be disposed about only a portion of the distal end of the housing. For example, in some embodiments, an auto-injector can include a button extending from the distal end portion of the housing configured to engage and release the system actuator.

Although the rod 3540 is shown and described as being an elongated member that is released by being elastically deformed, in some embodiments, a rod can be of any suitable shape and in any suitable orientation within the housing. Moreover, in some embodiments, a rod can be released by being plastically deformed. For example, in some embodiments, a rod can be disposed along an axis that is offset from the longitudinal axis of the energy storage member. In some embodiments, the rod can be configured to break upon actuation.

Although the gas release mechanism 3612 is shown and described as including a puncturing element 3620 to puncture a portion of the compressed gas container 3262, the gas release mechanism 3612 need not include a puncturing element 3620. For example, in some embodiments, the gas release mechanism can include an actuator configured to actuate a valve that controls the flow of gas out of the compressed gas container. For example, in some embodiments, a compressed gas container can include a spring loaded check ball and the gas release mechanism can include an actuator configured to engage and depress the check ball to release pressurized gas from the compressed gas container.

Although the distance through which the piston 3324 travels, and therefore the amount of medicament injected, is shown and described as being controlled by configuring the movable member 3312 such that it is in contact with the upper portion 3252 of the carrier 3250 when the auto-injector 3002 is in its fifth configuration, in other embodiments, any suitable method of controlling the piston travel can be employed. For example, in some embodiments, piston travel can be limited by including a protrusion within the medicament container, such as a necked portion, that limits the motion of the piston within the medicament container. In other embodiments, the housing can include a protrusion to limit the motion of the movable member. In yet other embodiments, the valve actuator can be configured to actuate the gas relief valve when the piston has moved a predetermined distance within the medicament container. In yet other embodiments, a combination of each of the above methods for controlling the piston travel can be employed.

Although the auto-injector 3002 is shown and described as having six different configurations that are different from each other, in some embodiments, certain configuration of an auto-injector can be the same as another configuration. For example, in some embodiments, a "pre-actuation configuration can be the same as a "retracted" configuration. In other embodiments, any of the functions described above can be accomplished when an auto-injector is moved between any number of different configurations.

Although the auto-injector 3002 is shown and described as including a compressed gas cylinder 3412, in other embodiments an auto-injector can include any suitable energy storage member. For example, in some embodiments, an auto-injector can include a mechanical energy storage member, such as a spring, an electrical energy storage member, such as a battery or a capacitor, a chemical energy storage member, such as a container containing two substances that can react to produce energy, a magnetic energy storage member or the like. Similarly, although the auto-injector 3002 is shown and described as including a gas release mechanism 3612, in other embodiments an auto-injector can include any suitable energy release mechanism. Such energy release mechanism can include, for example, an electrical circuit, a mechanical spring retainer, a fluid control valve or the like.

In some embodiments, an apparatus includes a label configured to be coupled to a medicament delivery device and/or a simulated medicament delivery device. The label includes a first surface and a second surface. The first surface is configured to be coupled to an outer surface of the medicament delivery device and/or the simulated medicament delivery device. In some embodiments, for example, the first surface can include an adhesive. The second surface includes a textual indicia, such as, for example, a description of the medicament delivery device, a mark indicating the manufacturer or distributor of the medicament delivery device and/or an instruction associated with the use of the medicament delivery device. The label further includes an electronic circuit system configured to output an electronic signal. In some embodiments, the electronic signal can include an instruction associated with the use of the medicament delivery device and/or the simulated medicament delivery device.

In some embodiments, an apparatus includes a printed circuit board configured to be coupled to a medicament delivery device and/or a simulated medicament delivery device. The printed circuit board includes a substrate and an electrical conductor disposed on the substrate. The substrate includes an actuation portion configured to receive an actuator. The actuator is configured to deform the actuation portion of the substrate, thereby separating the electrical conductor.

In some embodiments, an apparatus includes a printed circuit board configured to be coupled to a medicament delivery device and/or a simulated medicament delivery device. The printed circuit board includes a substrate and an electrical conductor disposed on the substrate. The substrate includes an actuation portion configured to receive an actuator. The actuation portion of the substrate defines an opening adjacent the electrical conductor, the opening being configured to receive the actuator. The actuator is configured to move substantially parallel to a plane defined by a surface of the actuation portion of the substrate to produce a tear in the actuation portion of the substrate, thereby severing the electrical conductor. In some embodiments, the opening can be configured to propagate the tear in a predetermined direction.

In some embodiments, an apparatus includes a medicament delivery device configured to deliver a medicament into a body. The medicament delivery device, which can be, for example, a pen injector, an auto-injector, an inhaler or a transdermal delivery device, includes an electronic circuit system and a locking member. The electronic circuit system is configured to output an electronic signal associated with a use of the medicament delivery device. In some embodiments, the electronic signal can be, for example, associated with recorded speech. The locking member is configured to prevent the medicament from being delivered into the body. The locking member includes an actuator configured to actuate the electronic circuit system.

In some embodiments, an apparatus includes a medicament delivery device configured to deliver a medicament into a body. The medicament delivery device includes an electronic circuit system and a locking member. The electronic circuit system includes a switch and is configured to output a signal when the switch is moved from a first state to a second state. The locking member is configured to prevent the medicament from being delivered into the body when in a first position and to allow the medicament to be delivered into the body when in a second position. A portion of the locking member is configured to move the switch from the first state to the second state when the locking member is moved from the first position to the second position.

In some embodiments, an apparatus includes a housing configured to contain a medicament, a flexible printed circuit board, an energy storage member and a label. The flexible printed circuit board is disposed on an outer surface of the housing and includes a first electrical contact portion and a second electrical contact portion. The label is coupled to the flexible printed circuit board and the housing and is configured to maintain a first surface of the energy storage member in electrical communication with the first electrical contact portion and maintain a second surface of the energy storage member in electrical communication with the second electrical contact portion. The energy storage member, can be, for example, a battery.

In some embodiments, a method includes assembling a medicament delivery device and/or a simulated medicament delivery device, such as, for example, an auto-injector or an auto-injector simulator. An electronic circuit system is then placed against an outer surface of the medicament delivery device and/or the simulated medicament delivery device. A label is then coupled to the medicament delivery device and/or the simulated medicament delivery device such that the label is disposed about a portion of the electronic circuit system.

In some embodiments, an apparatus includes a container defining an internal region configured to contain multiple medicament delivery devices, such as, for example, pen injectors, auto-injectors, inhalers or the like. The container includes an electronic circuit system configured to output a first electronic output associated with a first medicament delivery device contained within the internal region when the first medicament delivery device is removed from the internal region of the container. The electronic circuit system is further configured to output a second electronic output associated with a second medicament delivery device contained within the internal region when the second medicament delivery device is removed from the internal region of the container. The second electronic output is different than the first electronic output. At least one of the first electronic output or the second electronic output is associated with a use instruction of the first medicament delivery device and/or the second medicament delivery device.

In some embodiments, an apparatus includes a container defining an internal region configured to contain multiple medicament delivery devices. The container includes an electronic circuit system configured to output a first electronic output associated with a first medicament delivery device contained within the internal region when the first medicament delivery device is removed from the internal region of the container. The first medicament delivery device includes a label configured to output a signal associated with at least one of a contents of the first medicament delivery device, an expiration date of the first medicament delivery device, a dosage of the first medicament delivery device or a use instruction associated with the first medicament delivery device. In this manner, the first electronic output can be associated with the signal received by the electronic circuit system. The electronic circuit system is further configured to output a second electronic output associated with a second medicament delivery device contained within the internal region when the second medicament delivery device is removed from the internal region of the container. The second electronic output is different than the first electronic output. At least one of the first electronic output or the second electronic output is associated with a use instruction of the first medicament delivery device and/or the second medicament delivery device.

In some embodiments, a kit includes a medicament delivery device and a container. The container defines an internal region configured to contain the medicament delivery device. The container includes a movable portion, an electronic circuit system, a first switch and a second switch. The movable portion has a first position, in which the movable portion covers the internal region of the container, and a second position, in which the internal region of the container is exposed to an area outside the container. The first switch is configured to move between a first state and a second state when the movable portion moves between its first position and its second position. The first switch is operatively coupled to the electronic circuit system such that the electronic circuit system is configured to output a first electronic output when the first switch is moved from its first state to its second state. The first electronic output can be, for example, a visual output, an audible output or a haptic output. The second switch is configured to move between a first state and a second state when the medicament delivery device is removed from the internal region of the container. The second switch is operatively coupled to the electronic circuit system such that the electronic circuit system is configured to output a second electronic output when the second switch is moved from its first state to its second state. The second electronic output, which includes an instruction for using the medicament delivery device, can be, for example, a visual output (e.g. a video showing the proper use of the medicament delivery device), an audible output (e.g., a voice recording providing instructions for use) or a haptic output (e.g., a vibration indicating the location of a particular item).

In some embodiments, an apparatus includes a container, a retainer and an electronic circuit system. The_container defines an internal region configured to contain at least a portion of a medicament delivery device, such as, for example a pen injector. The retainer is configured to be movably coupled to the container and to retain the portion of the medicament delivery device within the internal region defined by the container. The electronic circuit system is configured to output a first electronic output when the retainer is moved relative to the container and a second electronic output when the medicament delivery device is removed from the internal region. At least one of the first electronic output or the second electronic output is associated with an instruction for using the medicament delivery device.

In some embodiments, an apparatus includes a container, a retainer, an electronic circuit system and a label. The container defines an internal region configured to contain at least a portion of a medicament delivery device, such as, for example a pen injector. The retainer is configured to be movably coupled to the container and to retain the portion of the medicament delivery device within the internal region defined by the container. The label is configured to be coupled to the medicament delivery device and contain information associated with the medicament delivery device in a machine-readable format. The electronic circuit system is configured to output a first electronic output when the retainer is moved relative to the container and a second electronic output when the medicament delivery device is removed from the internal region. The electronic circuit system is further configured to receive the information contained on the label include at least a portion of the information in the first electronic output and/or the second electronic output. At least one of the first electronic output or the second electronic output is associated with an instruction for using the medicament delivery device.

In some embodiments, an apparatus includes a simulated medicament delivery device and an electronic circuit system coupled to the simulated medicament delivery device. The simulated medicament delivery device can be configured, for example, to simulate the look, feel and/or functionality associated with a pen injector, an auto-injector, an inhaler and/or a transdermal delivery device. The electronic circuit system is configured to output an electronic output associated with a use of the simulated medicament delivery device. The electronic output can include, for example, a signal associated with a visual output, an audible output, a haptic output, an olfactory output and/or a taste output. Moreover, the electronic output can include, for example, an instruction for using the simulated medicament delivery device and/or a medicament delivery device

In some embodiments, an apparatus includes a housing associated with a medicament delivery device and an electronic circuit system. The electronic circuit system is coupled to the housing. The housing and the electronic circuit system are configured to cooperatively simulate the medicament delivery device. The electronic circuit system is configured to output an electronic output to simulate a tactile sensation, an audible sensation, a visual sensation, an olfactory sensation and/or a taste sensation associated with a use of the medicament delivery device.

In some embodiments, a kit includes a medicament delivery device and a simulated medicament delivery device. The simulated medicament delivery device includes an electronic circuit system configured to output an electronic output associated with a use of the simulated medicament delivery device and/or the medicament delivery device.

In some embodiments, a kit includes a medicament delivery device, a simulated medicament delivery device and a container. The container is configured to contain the medicament delivery device and the simulated medicament delivery device. The simulated medicament delivery device includes an electronic circuit system configured to output a first electronic output associated with a use of at least one of the simulated medicament delivery device or the medicament delivery device. The container includes an electronic circuit system. The electronic circuit system of the container and the electronic circuit system of the simulated medicament delivery device are configured to cooperatively output a second electronic output associated with a use of at least one of the simulated medicament delivery device or the medicament delivery device.

In some embodiments, an apparatus includes a label configured to be coupled to a simulated medicament delivery device. The label includes a first surface, a second surface and an electronic circuit system. The first surface is configured to be coupled to a housing of the simulated medicament delivery device. The second surface includes a textual indicia. The electronic circuit system configured to output an electronic signal.

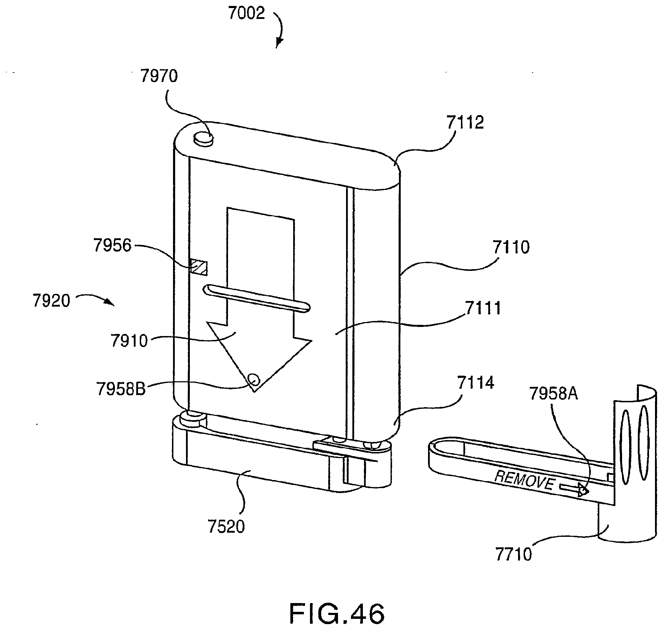

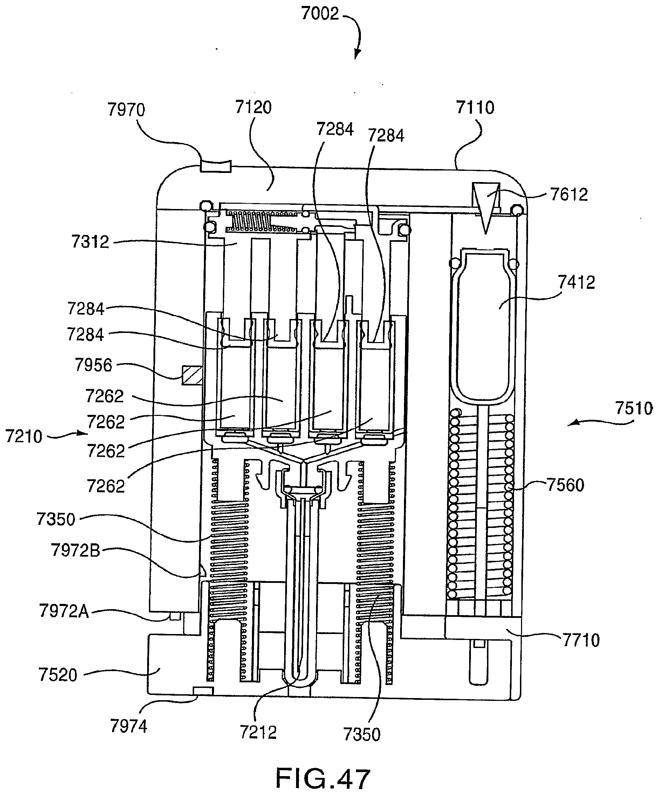

FIGS. 46 and 47 are a perspective view and a partial cutaway front view, respectively, of an auto-injector 7002 according to an embodiment of the invention. The auto-injector 7002 is similar to the auto-injectors described in U.S. Patent Application Serial Number 11/562,061, entitled "Devices, Systems and Methods for Medicament Delivery," filed November 21, 2006, which is incorporated herein by reference in its entirety. Accordingly, only an overview of the mechanical components and related operation of the auto-injector 7002 is included below.

The auto-injector 7002 includes a housing 7110 that defines a gas chamber 7120. The housing 7110 has a proximal end portion 7112 and a distal end portion 7114. A base 7520 is movably coupled to the distal end portion 7114 of the housing 7110. A safety lock 7710 is removably coupled to the base 7520. As discussed in more detail herein, when the safety lock 7710 is coupled to the base 7520, the auto-injector 7002 cannot be actuated. When the safety lock 7710 is removed from the base 7520, the base 7520 can be moved relative to the housing 7110, thereby actuating the auto-injector 7002. Accordingly, to inject a medicament into the body, the distal end portion 7114 of the housing 7110 is oriented towards the user such that the base 7520 is in contact with the portion of the body where the injection is to be made. The base 7520 is then moved towards the proximal end 7112 of the housing 7110 to actuate the auto-injector 7002.

The auto-injector 7002 includes a medicament injector 7210 and a system actuator 7510 disposed non-coaxially within the housing 7110. The medicament injector 7210 includes multiple medicament vials 7262, a plunger 7284 movably disposed within each medicament vial 7262, a movable member 7312 engaged with each plunger 7284 and a needle 7212. Retraction springs 7350 located within a portion of the base 7520 and the housing 7110 can push the needle 7212 back within the housing 7110 after injection. The system actuator 7510 includes a compressed spring 7560, a compressed gas cylinder 7412, and a puncturing mechanism 7612 to dispel the contents of the compressed gas cylinder 7412.

In use, when the auto-injector 7002 is actuated, the puncturing mechanism 7612 punctures the compressed gas cylinder 7412 allowing a pressurized gas to flow into the gas chamber 7120. In response to a force produced by the pressurized gas on the movable member 7312, the movable member 7312 moves distally within the housing 7110. As a result, the needle 7212 is extended through the housing 7110. The movement of the movable member 7312 also causes the plungers 7284 to move within the vials 7262, thereby expelling a medicament from the vials 7262.

The auto-injector 7002 includes an electronic circuit system 7920 to provide a predetermined sequence of electronic outputs during the use of the auto-injector 7002. The electronic circuit system 7920 is powered by a battery (not shown in FIGS. 46 and 47) and includes a processor (not shown in FIGS. 46 and 47), a start button 7970, two switches 7972A and 7972B, a proximity sensor 7974, two visual output devices 7958A and 7958B and an audio output device 7956. The components of the electronic circuit system 7920 are operatively coupled by any suitable mechanism, such as, for example, a printed circuit board (not shown in FIGS. 46 and 47) having conductive traces.

The start button 7970 is disposed on the proximal end of the housing 7110 and can be manually actuated by the user to begin the sequence of electronic outputs. The first switch 7972A is disposed on the distal portion 7114 of the housing 7110 adjacent the base 7520 and the locking member 7710. The locking member 7710 is configured to engage the first switch 7972A such that when the locking member 7710 is removed, as shown in FIG. 46, the first switch 7972A changes states. In this manner, removal of the locking member 7710 can trigger the processor to output a predetermined electronic output.

Similarly, the second switch 7972B is disposed on the housing 7110 adjacent the medicament injector 7210. The medicament injector 7210 is configured to engage the second switch 7972B such that when the medicament injector 7210 is moved distally within the housing 7110 the second switch 7972B changes states. In this manner, the processor can be prompted to output a predetermined electronic output based on the position of the medicament injector 7210.

The proximity sensor 7974 is disposed on the base 7520 and is configured to produce an output when the base 7520 engages the body. The proximity sensor can be, for example, a temperature sensor, an optical sensor or the like. In this manner, the processor can be prompted to output a predetermined electronic output when the base 7520 is positioned against the body.

The first visual output device 7958A is disposed on the locking member 7710. Similarly, the second visual output device 7958B is disposed on the outer surface 7111 of the housing 7110. The visual output devices 7958A and 7958B are in electronic communication with the processor and are configured to produce an output in response to an electronic signal output by the processor. The visual output devices 7958A and 7958B can be any suitable visual indicia, such as, light-emitting diodes (LEDs), liquid-crystal display (LCD) screens, optical polymers, fiber optic components or the like. In some embodiments, the visual output devices 7958A and 7958B can be coupled to the housing 7110 and/or the locking member 7710 by a label 7910.

The audio output device 7956 is disposed within the housing 7110 such that it can project sound outside of the housing 7110. The audio output device 7956 can be any suitable device for producing sound, such as a micro-speaker a piezo-electric transducer or the like. Such sound output can include, for example, an alarm, a series of beeps, recorded speech or the like. The audio output device 7956 is in electronic communication with the processor and is configured to produce an output in response to an electronic signal output by the processor.

In use, the user activates the electronic circuit system by pushing the start button 7970 to activate the processor, thereby causing the processor to output a predetermined sequence of electronic outputs. In some embodiments, the start button 7970 can activate the processor by providing an input to the processor. In other embodiments, the start button 7970 can activate the processor by placing the battery (not shown in FIGS. 46 and 47) in electronic communication with the processor.

In some embodiments, upon activation, the processor can output an electronic signal to the audio output device 7956 thereby producing a first electronic output instructing the user in how to use the auto-injector 7002. Such a message can state, for example, "please remove the safety tab." Additionally, the first visual output device 7958A can produce a flashing light to further indicate to the user where the locking member 7710 is located. The processor can be configured to repeat the first audible instruction if the locking member 7710 is not removed within a predetermined time period.

When the user removes the locking member 7710, the first switch 7972A changes states thereby triggering the processor to output an electronic output providing a second instruction to the user. The second instruction can be, for example, an audible speech output instructing the user to "please place the base of the device on the outer portion of your thigh." The first visual output device 7958A can produce a lighted output during this audible instruction, thereby visually indicating where the base 7520 is located and/or what portion of the base 7520 should be placed on the thigh.

When the user places the base 7520 against the body, the proximity sensor 1974 provides an input to the processor, thereby triggering the processor to output an electronic output providing a third instruction to the user. The third instruction can be, for example, an audible speech output instructing the user to "push down on the top of the device to activate the injector."

When the injection is completed, the medicament injector 7210 is configured to engage the second switch 7972B, thereby triggering the processor to output an electronic output providing a fourth instruction to the user. Such a post-use instruction can be, for example, an audible speech output instructing the user to seek further medical attention, providing instructions for the safe disposal of the auto-injector 7002 or the like.

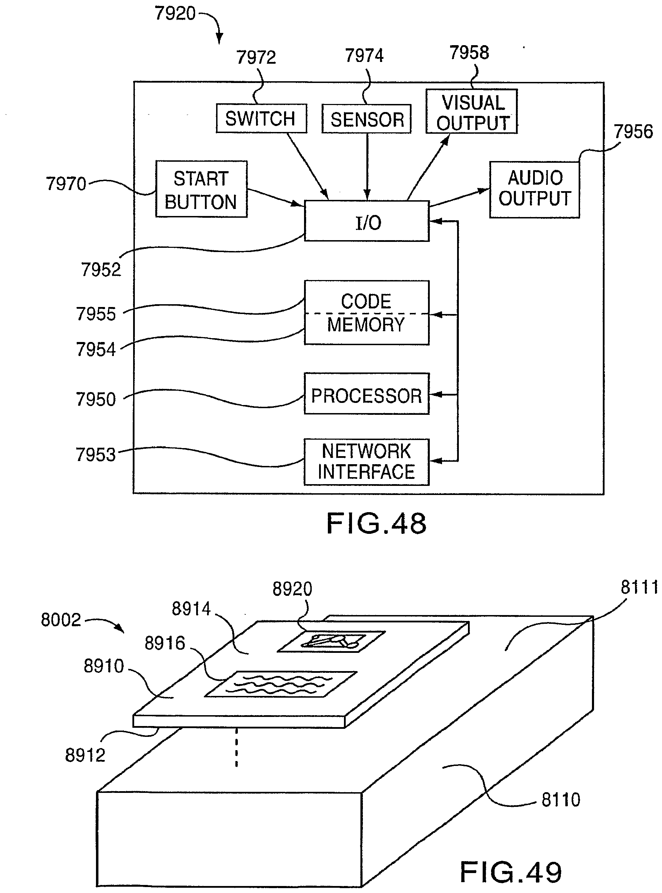

FIG. 48 is a schematic illustration of the electronic circuit system 7920 of the auto-injector 7002. The electronic circuit system 7920 includes a processor 7950 operatively coupled to a memory device 7954. The memory device 7954 can be configured to store processor-readable code 7955 instructing the processor 7950 to perform the functions described above. In some embodiments, the processor-readable code 7955 can be modified and/or updated as circumstances dictate. The electronic circuit system 1920 includes an input/output device 7952 configured to receive electronic inputs from the switches 7972A and 7972B, the proximity sensor 7974 and/or the start button 7970. The input/output device 7952 is also configured to provide electronic signals to the various output devices, such as the visual output devices 7958A and 7958B and the audio output device 7956.

The electronic circuit system 7920 also includes a network interface 7953 configured to couple the electronic circuit system 7920 to a communications network. Such an arrangement can be used, for example, to download replacement processor-readable code 7955 from a central network (not shown) to the memory device 7954. The network interface 7953 can also be configured to transmit information from the electronic circuit system 7920 to a central network, the user's home computer, the user's cell phone or the like.

FIG. 49 is a schematic illustration of a medical device 8002 according to an embodiment of the invention. The medical device 8002, which can be, for example, a medicament delivery device such as an auto-injector, a pen injector, an inhaler, a transdermal delivery system or the like, includes a housing 8110 and a label 8910. The label 8910 is coupled to an outer surface 8111 of the housing 8110. The label 8910 includes a first surface 8912, a second surface 8914 and an electronic circuit system 8920. The first surface 8912 is configured to engage the outer surface 8111 of the housing 8110 to couple the label 8910 to the housing 8110. In some embodiments, the first surface 8912 can include an adhesive to fixedly couple the label 8910 to the housing 8110. The second surface 8914 includes a textual indicia 8916. The textual indicia 8916 can include, for example, a description of the medicament delivery device, a source of the medicament delivery device and/or an instruction associated with the use of the medicament delivery device. Although the first surface 8912 is shown as being opposite the second surface 8914, in other embodiments, the first surface 8912 and the second surface 8914 can be adjacent each other and/or co-planar.

The electronic circuit system 8920 is configured to output an electronic signal. As discussed in more detail herein, the electronic circuit system 8920 can include many components, such as, for example, a processor, a switch, a visual output device and/or an audio output device. The electronic signal can be, for example, an electronic signal communicated to an output device, such as, for example, a visual output device, an audio output device, a haptic output device or the like. In some embodiments, the electronic signal can be associated with an aspect of the medical device 8002, such as an instruction associated with an initial use of the medical device 8002. For example, in some embodiments, the electronic circuit system 8920 can output a text message to a display screen (not shown) disposed on the medical device 8002 instructing the user in the use of the medical device 8002. In other embodiments, the electronic circuit system 8920 can produce an audio output, such as recorded speech, instructing the user in the use of the medical device 8002.

Although the electronic circuit system 8920 is shown as being disposed on the second surface 8914 of the label 8910, in other embodiments, the electronic circuit system can be disposed on the first surface 8912 of the label 8910. In yet other embodiments, the electronic circuit system 8920 can be disposed between the first surface 8912 and the second surface 8914 of the label 8910. In yet other embodiments, the label 8910 can include multiple discrete layers coupled together, within which portions of the electronic circuit system can be disposed.

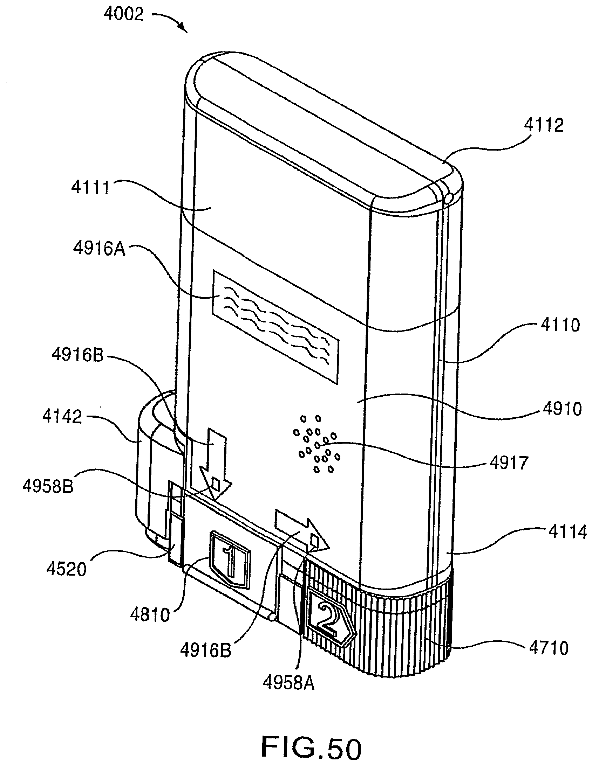

FIG. 50 is a perspective view of an auto-injector 4002 according to an embodiment of the invention. The auto-injector 4002 is similar to the auto-injectors described in U.S. Patent Application Serial Number 11/562,061, entitled "Devices, Systems and Methods for Medicament Delivery," filed November 21, 2006. Accordingly, the mechanical components and operation of the auto-injector 4002 are not described in detail herein.

The auto-injector 4002 includes a housing 4110 having a proximal end portion 4112 and a distal end portion 4114. The distal end portion 4114 of the housing 4110 includes a protrusion 4142 to help a user grasp and retain the housing 4110 when using the auto-injector 4002. Said another way, the protrusion 4142 is configured to prevent the auto-injector 4002 from slipping from the user's grasp during use. A base 4520 is movably coupled to the distal end portion 4114 of the housing 4110. A needle guard assembly 4810 is removably coupled to the base 4520. Similarly, a safety lock 4710 is removably coupled to the base 4520. To inject a medicament into the body, the distal end portion 4114 of the housing is oriented towards the user such that the base 4520 is in contact with the portion of the body where the injection is to be made. The base 4520 is then moved towards the proximal end 4112 of the housing 4110 to actuate the auto-injector 4002.

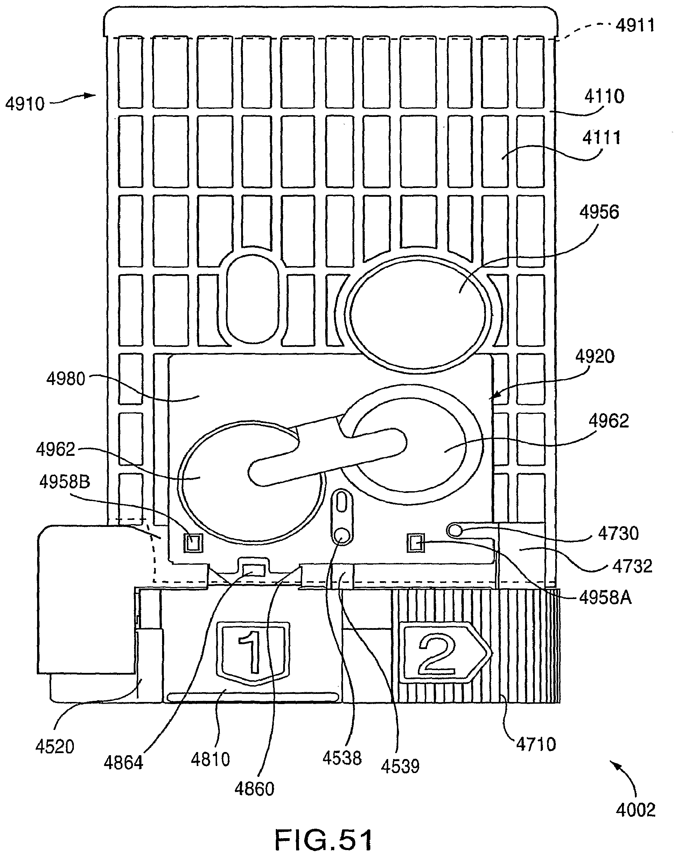

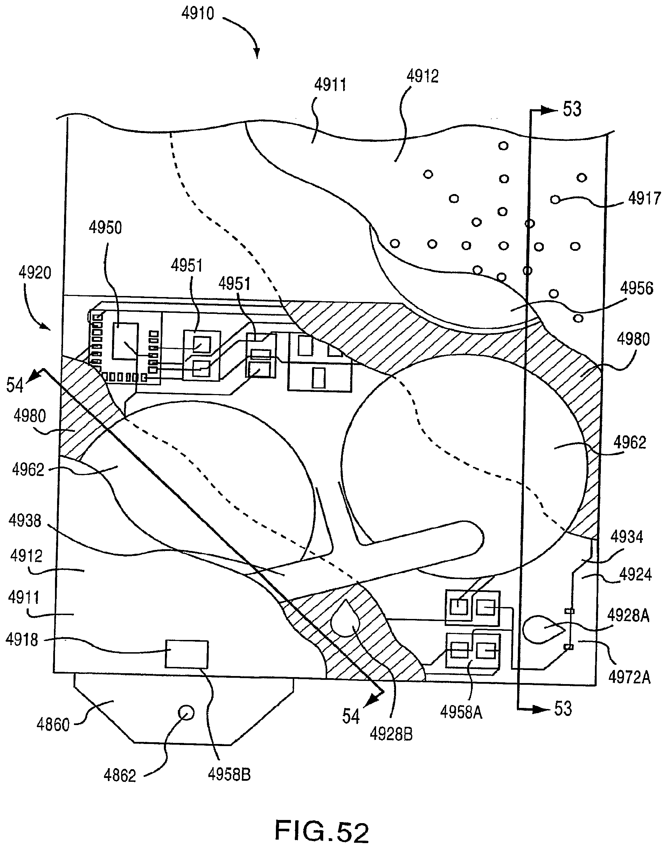

The auto-injector 4002 includes a label 4910 coupled to an outer surface 4111 of the housing 4110. The label 4910 includes an outer layer 4911, an intermediate layer 4980 and an electronic circuit system 4920 (see FIGS. 52 - 54). FIG. 51 is a front view of the auto-injector 4002 showing the outer layer 4911 of the label 4910 in phantom lines so that the intermediate layer 4980 and an electronic circuit system 4920 can be more clearly seen. As shown in FIGS. 52 - 54, the outer layer 4911, which, in some embodiments, can be constructed from paper, has a first surface 4912 and a second surface 4914 opposite the first surface 4912. Multiple indicia 4916 are disposed on the first surface 4912. The indicia 4916 include a textual indicia 4916A and two symbolic indicia 4916B. The textual indicia 4916B can be written text describing the medicament delivery device, indicating a source of the medicament delivery device and/or instructing a user in the use of the medicament delivery device. The symbolic indicia 4916B can include, for example, arrows, pointers, trademarks, symbols describing the use of the medicament delivery device or the like. The label 4910 is coupled to the outer surface 4111 of the housing 4110 such that the portion of the first surface 4912 including the indicia 4916 is visible.

A portion of the second surface 4914 of the outer layer 4911 can be coupled to the outer surface 4111 of the housing 4110 by any suitable method. For example, in some embodiments, the second surface 4914 of the outer layer 4911 includes an adhesive configured to bond the outer layer 4911 to the outer surface 4111 of the housing 4110. Other portions of the second surface 4914 of the outer layer 4911 are adjacent the intermediate layer 4980 and portions of the electronic circuit system 4920. In this manner, the outer layer 4911 of the label 4910 retains the intermediate, or spacer, layer 4980 and the electronic circuit system 4920 in a predetermined position against the outer surface 4111 of the housing 4110.

The outer layer 4911 of the label 4910 includes multiple openings 4917 adjacent the audio output device 4956. In this manner, sound waves produced by the audio output device 4956 can be transmitted to an area outside of the housing 4110. Similarly, the outer layer 4911 of the label 4910 includes openings 4918 adjacent the light emitting diodes (LEDs) 4958A and 4958B to allow the user to see the visual output. In some embodiments, the outer layer 4911 of the label 4910 can include a transparent portion adjacent the LEDs 4958A and 4958B to allow the user to see the visual output.

The electronic circuit system 4920 includes a printed circuit board 4922 upon which a microprocessor 4950, two LEDs 4958A and 4958B, two switches 4972A and 4972B and various electronic components 4951, such as, for example, resistors, capacitors and diodes, are mounted. The electronic circuit system 4920 also includes an audio output device 4956, such as, for example, a micro-speaker, coupled to the outer surface 4111 of the housing 4110 adjacent the printed circuit board 4922. The printed circuit board 4922 includes a substrate 4924 upon which a series of electrical conductors 4934, such as for example, copper traces, are etched. The substrate 4924 can be constructed from any material having suitable electrical properties, mechanical properties and flexibility, such as, for example Mylar®, Kapton® or impregnated paper.

A mask layer (not shown) is disposed over the substrate 4924 to electrically isolate selected portions of the electrical conductors 4934 from adjacent components. The electrical conductors 4934 operatively couple the above-mentioned circuit components in a predetermined arrangement. In this manner, the electronic circuit system 4920 can be configured to output, via the LEDs 4958A and 4958B and/or the audio output device 4956, a predetermined sequence of electronic outputs during the use of the auto-injector 4002.

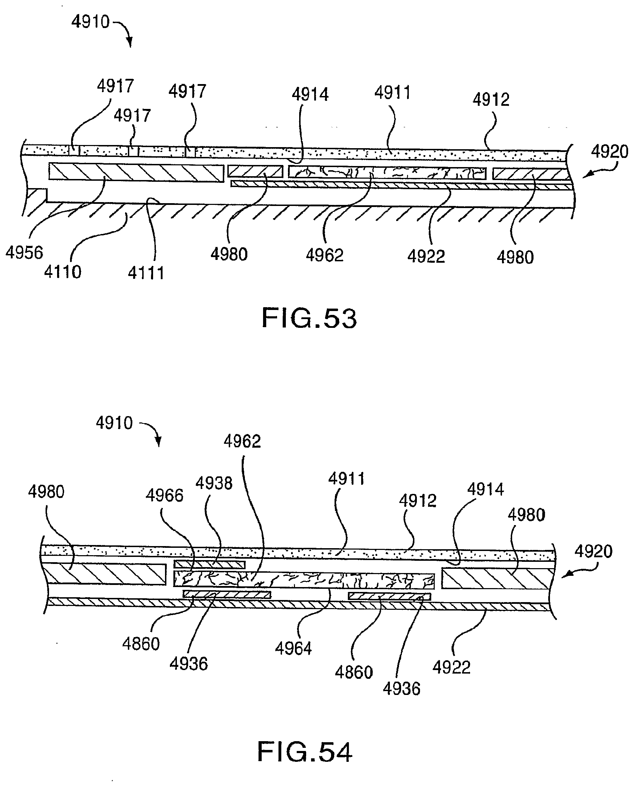

Power is supplied to the electronic circuit system 4920 by two batteries 4962 connected in series. The batteries can be, for example, three volt, "watch-style" lithium batteries. As shown in FIG. 54, each of the batteries 4962 has a first surface 4964 and a second surface 4966 opposite the first surface. The first surface 4964 can be, for example, an electrically negative terminal. Similarly, the second surface 4966 can be an electrically positive terminal. As discussed in more detail herein, the batteries 4962 are positioned such that a first electrical contact portion 4936 of the printed circuit board 4922 can be placed in contact with the first surface 4964 of the battery 4962 and a second electrical contact portion 4938 of the printed circuit board 4922 can be placed in contact with the second surface 4966 of the battery 4962. In this manner, the batteries 4962 can be operatively coupled to the electronic circuit system 4920.

As shown in FIGS. 52 and 54, a battery isolation tab 4860 is movably disposed between the first electrical contact portion 4936 of the printed circuit board 4922 and the first surface 4964 of one of the batteries 4962. The battery isolation tab 4860 can be constructed from any electrically isolative material, such as, for example, Mylar®. As discussed in more detail herein, in this manner, the batteries 4962 can be selectively placed in electronic communication with the electronic circuit system 4920.

The intermediate, or spacer, layer 4980 is disposed between the outer layer 4911 and the electronic circuit system 4920. The intermediate layer 4980 includes openings (not shown) within which various components of the electronic circuit system, such as, for example, the batteries 4962 are disposed. The intermediate layer 4980 is sized to maintain a predetermined spacing between the various components included in the label 4910. The intermediate layer can be constructed from any suitable material, such as, for example, flexible foam having an adhesive surface, polycarbonate or the like.

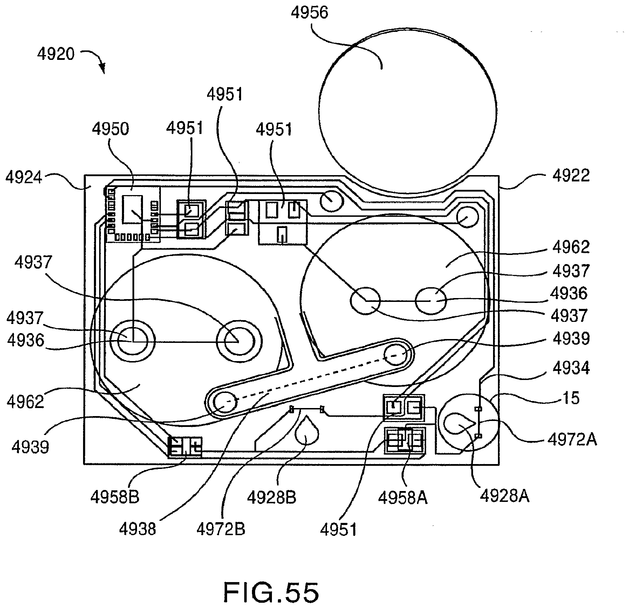

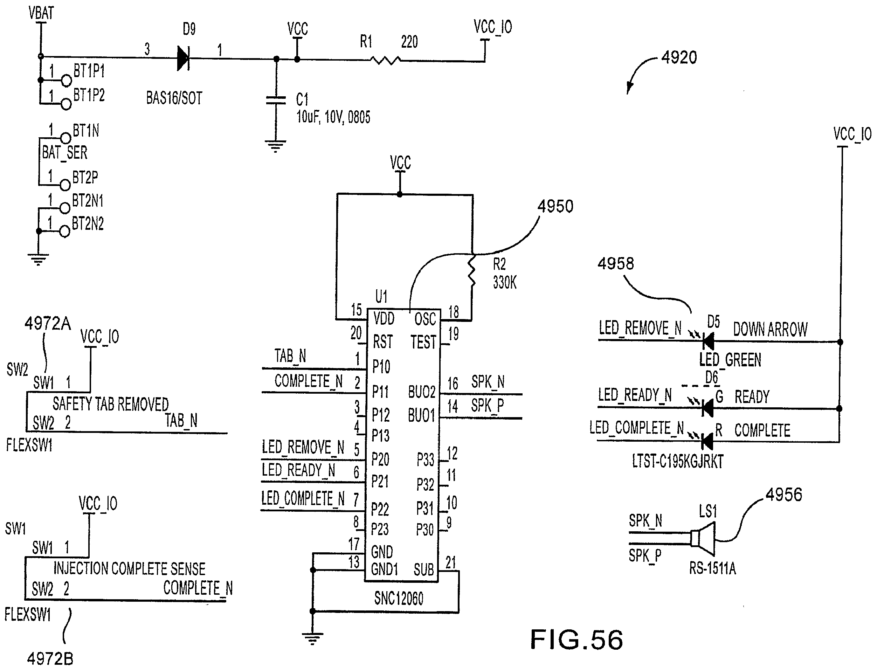

FIG. 55 is a front view of the electronic circuit system 4920 showing the arrangement of the various components (i.e., the microprocessor 4950, LEDs 4958A and 4958B, switches 4972A and 4972B, audio output device 4956 or the like). FIG. 56 is a schematic illustration of the electronic circuit system 4920.

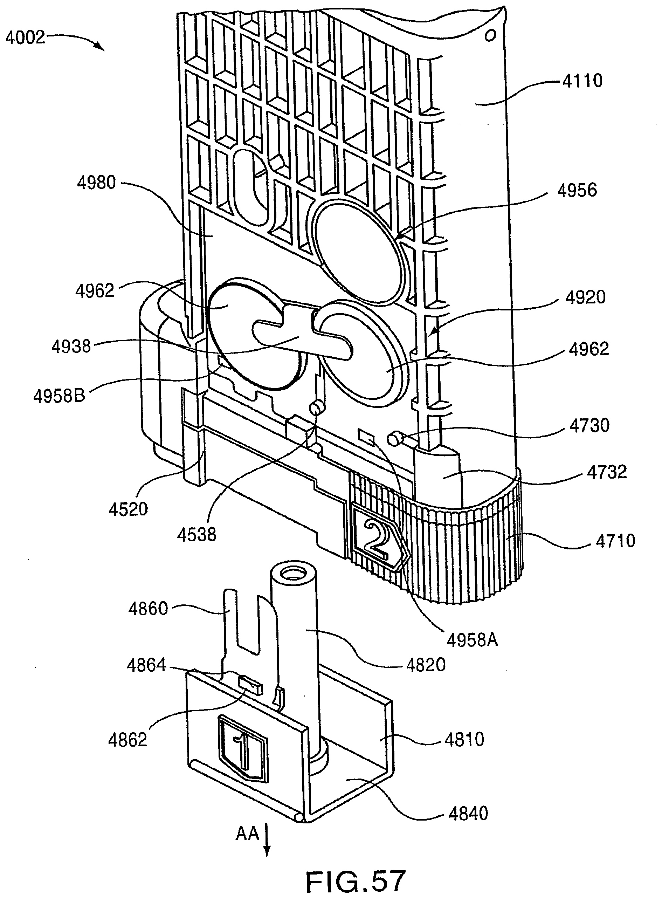

The operation of the auto-injector 4002 and the electronic circuit system 4920 is now discussed with reference to FIGS. 57 - 59. The actuation of the electronic circuit system 4920 includes several operations that are incorporated into the standard procedures for using the auto-injector 4002. In this manner, the user can actuate the electronic circuit system 4920 without completing any additional operations.

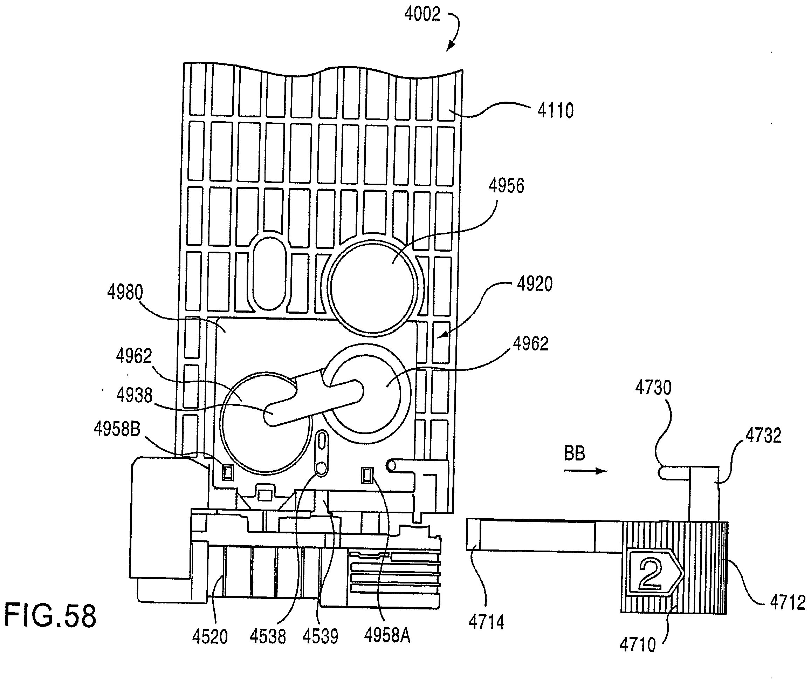

Prior to use, the auto-injector 4002 is first enabled by removing the needle guard 4810 and the safety lock 4710 (see FIGS. 57 and 58). As illustrated by arrow AA in FIG. 57, the needle guard 4810 is removed by moving it distally. The needle guard 4810 includes a sheath retainer 4840 and a sheath 4820. The sheath 4820 is configured to receive a portion of the needle (not shown) when the needle guard 4810 is in a first (or installed) position. The sheath retainer 4840 is coupled to the sheath 4820 such that when the sheath retainer 4840 is moved distally away from the base 4520 into a second (or removed) position, the sheath 4820 is removed from the needle.

The sheath retainer 4840 includes an actuator 4864 that is received by an opening 4862 in the isolation tab 4860. Accordingly, when the sheath retainer 4840 is moved distally away from the base 4520, the isolation tab 4860 is removed from the area between the first electrical contact portion 4936 of the printed circuit board 4922 and the first surface 4964 of one of the batteries 4962. In this manner, the batteries 4962 can be operatively coupled to the electronic circuit system 4920 when the needle guard 4810 is removed, thereby actuating the electronic circuit system 4920.

When actuated, the electronic circuit system 4920 can output one or more predetermined electronic outputs. For example, in some embodiments, the processor 4950 can output an electronic signal associated with recorded speech to the audible output device 4956. Such an electronic signal can be, for example, associated with a .WAV file that contains a recorded instruction instructing the user in the operation of the auto-injector 4002. Such an instruction can state, for example, "remove the blue safety tab near the base of the auto-injector." The processor can simultaneously output an electronic signal to the first LED 4958A, thereby causing the first LED 4958A, which is located near the safety lock 4710, to flash a particular color. In this manner, the electronic circuit system 4920 can provide both audible and visual instructions to assist the user in the initial operation of the auto-injector 4002.

In other embodiments, the electronic circuit system 4920 can output an electronic output associated with a description and/or status of the auto-injector 4002 and/or the medicament contained therein. For example, in some embodiments, electronic circuit system 4920 can output an audible message indicating the type of medicament contained in the auto-injector, the expiration date of the medicament, the dosage of the medicament or the like.

As illustrated by arrow BB in FIG. 58, the safety lock 4710 is removed by moving it substantially normal to the longitudinal axis of the housing 4110. The safety lock 4710 has a first end 4712 and a second end 4714. When the safety lock 4710 is in its first (or locked) position, the second end 4714 extends around a portion of the base 4520 to space the base 4520 apart from the distal end portion 4114 of the housing 4110. Additionally, the first end 4714 includes a locking protrusion (not shown) that obstructs portions of the system actuator (not shown) further preventing the base 4520 from being moved proximally towards the housing 4110. Accordingly, when the safety lock 4710 is in its first position, the auto-injector 4002 cannot be actuated.

In some embodiments, the safety lock 4710 includes an actuator 4732 that actuates the electronic circuit 4920 to trigger a predetermined output or sequence of outputs when the safety lock 4710 is moved from the first position to a second (or unlocked) position, as shown in FIG. 58. More particularly, as shown in FIGS. 55, 60 and 61, the actuator 4732 includes a protrusion 4730 that is received within a first opening 4928A defined by an actuation portion 4926 of the substrate 4924 when the safety lock 4710 is in the first position. The boundary 4929 of the first opening 4928A has a discontinuous shape, such as, for example, a teardrop shape, that includes a stress concentration riser 4930. The discontinuity and/or the stress concentration riser 4930 of the boundary 4929 can be of any suitable shape to cause the substrate 4924 to deform in a predetermined direction when the protrusion 4730 is moved relative to the first opening 4928A.

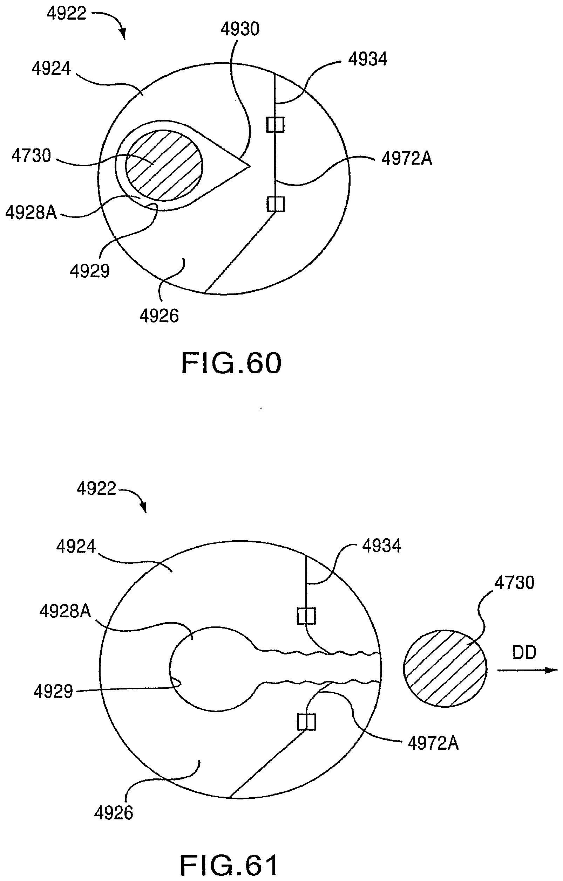

As shown in FIGS. 60 and 61, the first opening 4928A is defined adjacent an electrical conductor 4934 that, as discussed above, electronically couples the components included in the electronic circuit system 4920. The electrical conductor 4934 includes a first switch 4972A, which can be, for example a frangible portion of the electrical conductor 4934. In use, when the safety lock 4710 is moved from the first position to the second position, the actuator 4732 moves in a direction substantially parallel to a plane defined by a surface of the actuation portion 4926 of the substrate 4924. The movement of the actuator 4732 causes the protrusion 4730 to move within the first opening 4928A, as indicated by the arrow DD in FIG. 61. The movement of the protrusion 4730 tears the actuation portion 4926 of the substrate 4924, thereby separating the portion of the electrical conductor 4934 including the first switch 4972A. Said another way, when the safety lock 4710 is moved to the second position, the actuator 4732 moves irreversibly the first switch 4972A from a first state (e.g., a state of electrical continuity) to a second state (e.g., a state of electrical discontinuity).

When the actuator 4732 actuates the electronic circuit system 4920 as described above, the electronic circuit system 4920 can output one or more predetermined electronic outputs. For example, in some embodiments, the processor 4950 can output an electronic signal associated with recorded speech to the audible output device 4956. Such an electronic signal can be, for example, associated with a recorded message notifying the user of the status of the auto-injector 4002. Such a status message can state, for example, "The auto-injector is now enabled." The processor can also simultaneously output an electronic signal to the first LED 4958A, thereby causing the first LED 4958A to stop flashing, change color or the like.

In some embodiments, the electronic circuit system 4920 can be configured to output the status message for a predetermined time period, such as, for example, five seconds. After the predetermined time period has elapsed, the electronic circuit system 4920 can output an audible message further instructing the user in the operation of the auto-injector 4002. Such an instruction can state, for example, "Place the base of the auto-injector against the patient's thigh. To complete the injection, press the base firmly against the patient's thigh." In some embodiments, the processor can simultaneously output an electronic signal to the second LED 4958B, thereby causing the second LED 4958B, which is located near the base 4520, to flash a particular color. In this manner, the electronic circuit system 4920 can provide both audible and visual instructions to assist the user in the placement and actuation of the auto-injector 4002. In some embodiments, the electronic circuit system 4920 can be configured to repeat the instructions after a predetermined time period has elapsed.

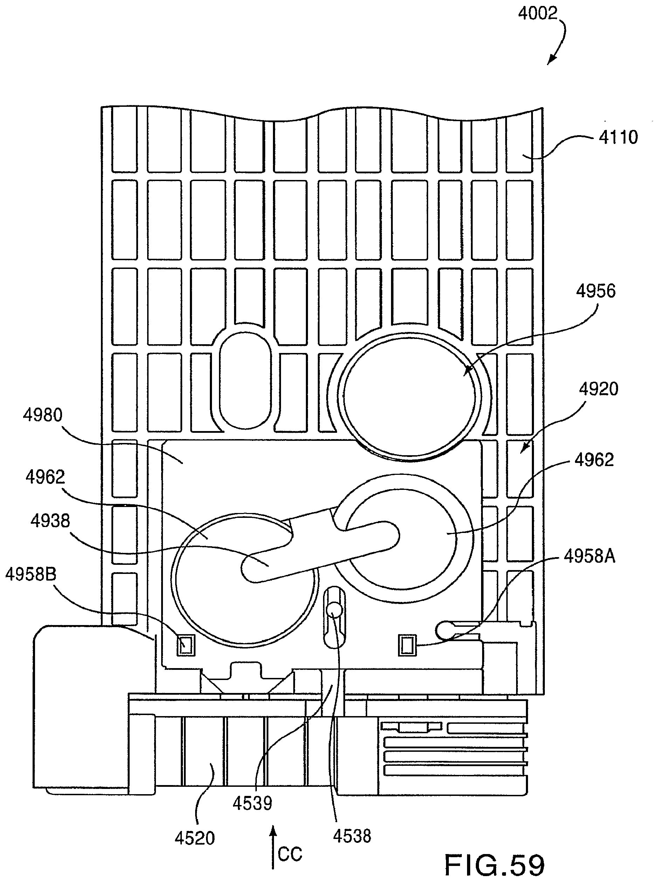

After the auto-injector 4002 is enabled and placed against the body of the patient, the auto-injector 4002 is actuated by moving the base 4520 proximally towards the housing 4110, as illustrated by arrow CC in FIG. 59. The base 4520 includes an actuator 4538 that actuates the electronic circuit 4920 to trigger a predetermined output or sequence of outputs when the base 4520 is moved from a first position to a second position, as shown in FIG. 58. The actuator 4538 includes a protrusion 4539 that is received within a second opening 4928B (see FIG. 55) defined by the substrate 4924 when the base 4520 is in the first position. The configuration and operation of the protrusion 4539, the second opening 4928B and the second switch 4972B are similar to the configuration and operation of the protrusion 4730, the first opening 4928A and the first switch 4972A, and are therefore not described in detail.

When the actuator 4538 actuates the electronic circuit system 4920, the electronic circuit system 4920 can output one or more predetermined electronic outputs. For example, in some embodiments, the processor 4950 can output an electronic signal associated with recorded speech to the audible output device 4956. Such an electronic signal can be, for example, associated with a recorded message notifying the user that the injection is complete, instructing the user on post-injection disposal and safety procedures, instructing the user on post-injection medical treatment or the like. Such a status message can state, for example, "The injection is now complete. Please seek further medical attention from a doctor." The processor can also simultaneously output an electronic signal to the first LED 4958A, thereby causing the first LED 4958A to stop flashing, change color or the like, to provide a visual indication that the injection is complete.

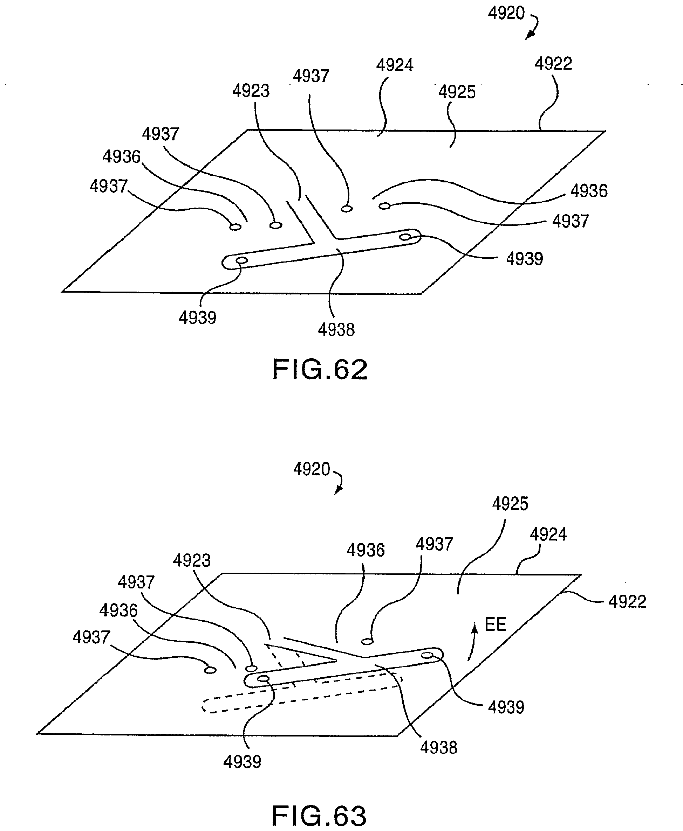

As described above, the batteries 4962 are positioned such that the first electrical contact portions 4936 of the printed circuit board 4922 can be placed in contact with the first surface 4964 of each battery 4962 and the second electrical contact portion 4938 of the printed circuit board 4922 can be placed in contact with the second surface 4966 of each battery 4962. As shown in FIGS. 55 and 62, the first electrical contact portions 4936 each include a pair of electrical contacts 4937 that are operatively coupled to the electronic circuit system 4920. Similarly, the second electrical contact portion 4938 includes a pair of electrical contacts 4939 that is operatively coupled to the electronic circuit system 4920.

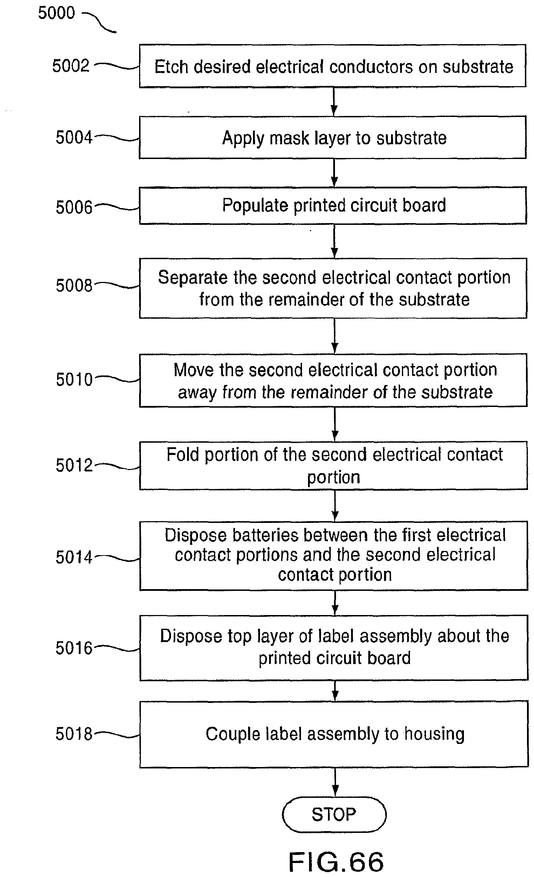

The first electrical contact portions 4936 and the second electrical contact portion 4938 are monolithically constructed from the printed circuit board 4922. FIGS. 62 - 65 are perspective views showing the printed circuit board 4922 in various stages of manufacture. FIG. 66 is a flow chart illustrating a method 5000 for manufacturing a flexible printed circuit board according to an embodiment of the invention. The illustrated method includes disposing a copper layer on the top surface 4925 of the flexible substrate 4924 and etching the desired series of electrical conductors (not shown in FIGS. 62-65) at 5002. A mask layer (not shown) is disposed on portions of the top layer 4925 of the substrate 4924 to electrically isolate selected portions of the electrical conductors from adjacent components at 5004. During this operation, the electrical contacts 4937, 4939 are constructed.

The printed circuit board 4922 is then populated with the microprocessor, switches, output devices and/or other electronic components to form the electronic circuit system 4920 at 5006. For clarity, the circuit components are not shown in FIGS. 62 - 65. After the printed circuit board 4922 is populated, the portion of the flexible substrate 4924 forming the second electrical contact portion 4938 is separated from the remainder of the substrate 4924 at 5008. As shown in FIG. 62, during this operation, a portion 4923 of the boundary between the second electrical contact portion 4938 and the remainder of the substrate 4924 is left intact.

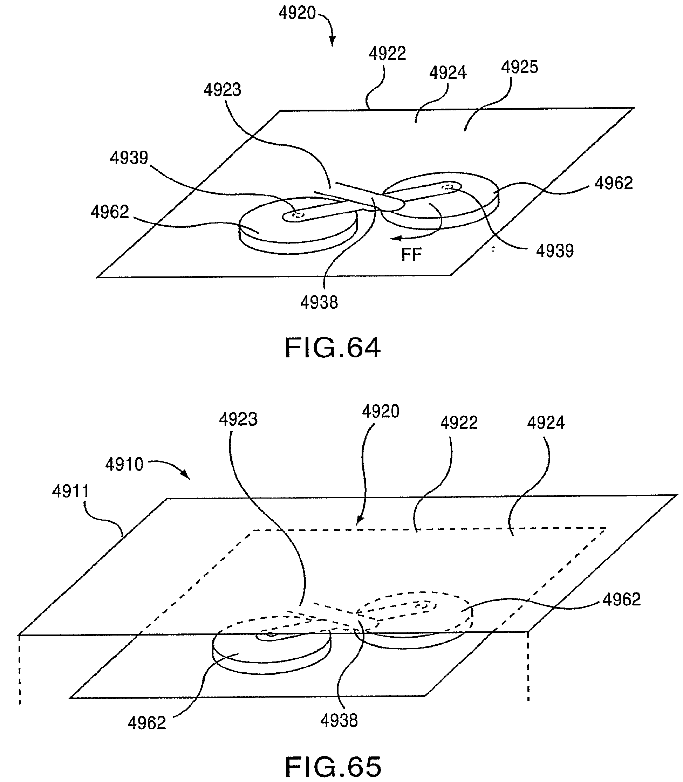

As shown by the arrow EE in FIG. 63, the second electrical contact portion 4938 is then moved upwardly away from the remainder of the substrate 4924 at 5010. In this manner, the second electrical contact portion 4938 is spaced apart from the first electrical contact portions 4936. As shown by the arrow FF in FIG. 64, the portion of the second electrical contact portion 4938 containing the electrical contacts 4939 is then folded so that the electrical contacts 4939 on the second electrical contact portion 4938 are facing the electrical contacts 4937 on the first electrical contact portions 4936, at 5012. In this manner, opposing electrical contacts 4937, 4939 are constructed on the printed circuit board 4922 without disposing electrical conductors on and/or etching multiple surfaces of the printed circuit board 4922.

The batteries 4962 are then disposed between the first electrical contact portions 4936 and the second electrical contact portion 4938 at 5014. Although not shown in FIG. 64, in some embodiments, a battery isolation tab of the type discussed above can be disposed between one of the batteries and the printed circuit board 4922. Once the batteries 4962 are in place, the top layer 4911 of the label 4910 is disposed about the printed circuit board 4922 (see FIG. 65) to maintain the position of the batteries 4962 within the printed circuit board 4922, at 5016. The label assembly 4910 is then coupled to the outer surface of the housing (not shown) at 5018. The label 4910 is coupled to the housing with sufficient tension and/or stretch to maintain the electrical contacts 4937 in electrical communication with the first surface 4964 of each battery 4962 and to maintain the electrical contacts 4939 in electrical communication with the second surface 4966 of each battery 4962. In this manner, the batteries 4962 can be held in place in a printed circuit board 4922 devoid of springs, clips or other rigid members.

As described above, the audio output device 4956, can include, for example, a micro-speaker. In some embodiments, for example, the audio output device 4956 can include an RS-1511A micro-speaker manufactured by Regal Electronics, Inc.

Similarly, the microprocessor 4950 can be a commercially-available processing device dedicated to performing one or more specific tasks. For example, in some embodiments, the microprocessor 4950 can be a commercially-available microprocessor, such as the Sonix SNC 12060 voice synthesizer. Alternatively, the microprocessor 4950 can be an application-specific integrated circuit (ASIC) or a combination of ASICs, which are designed to perform one or more specific functions. In yet other embodiments, the microprocessor 4950 can be an analog or digital circuit, or a combination of multiple circuits.

The microprocessor 4950 can include a memory device (not shown) configured to receive and store information, such as a series of instructions, processor-readable code, a digitized signal, or the like. The memory device can include one or more types of memory. For example, the memory device can include a read only memory (ROM) component and a random access memory (RAM) component. The memory device can also include other types of memory suitable for storing data in a form retrievable by the microprocessor 4950, for example, electronically-programmable read only memory (EPROM), erasable electronically-programmable read only memory (EEPROM), or flash memory.

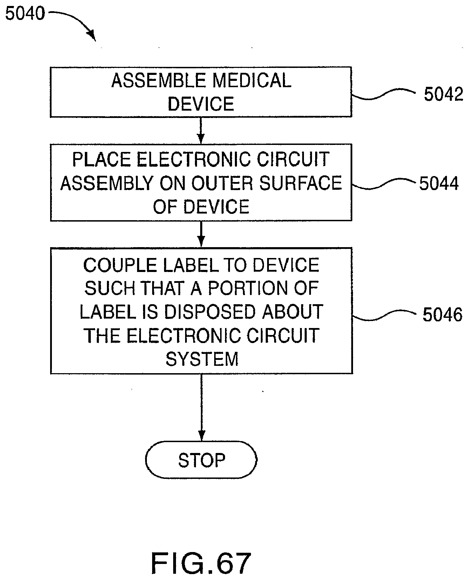

FIG. 67 is a flow chart illustrating a method 5040 for manufacturing a medical device according to an embodiment of the invention. The medical device can be any medicament delivery device of the type discussed above, such as, for example, an auto-injector, a pen injector, an inhaler, or a transdermal delivery device. The medical device can also be a medicament container, such as, for example, a pill bottle, a blister pack an intravenous solution bag or the like. The illustrated method includes assembling the medical device, 5042. After the medical device is assembled, an electronic circuit system is placed on an outer surface of the medicament delivery device, 5044. The electronic circuit system can be any electronic circuit system of the type shown and described above. In some embodiments, the electronic circuit system is placed on the outer surface of the medical device in a predetermined orientation. For example, in some embodiments, the electronic circuit system can include openings, such as openings 4928 that are aligned with mating portions of the medical device, such as, for example, protrusions 4730, 4538. In other embodiments, however, the electronic circuit system can be placed on the outer surface of the medical device in any orientation.

After the electronic circuit system is placed on an outer surface of the medical device, a label is coupled to the medical device, 5046. The label, which can be, for example, a label containing a textual indicia, is coupled to the medical device such that a portion of the label is disposed about the electronic circuit system. In this manner, the coupling of the label to the medical device also serves to maintain the electronic circuit system in its position against the outer surface of the medicament delivery device.

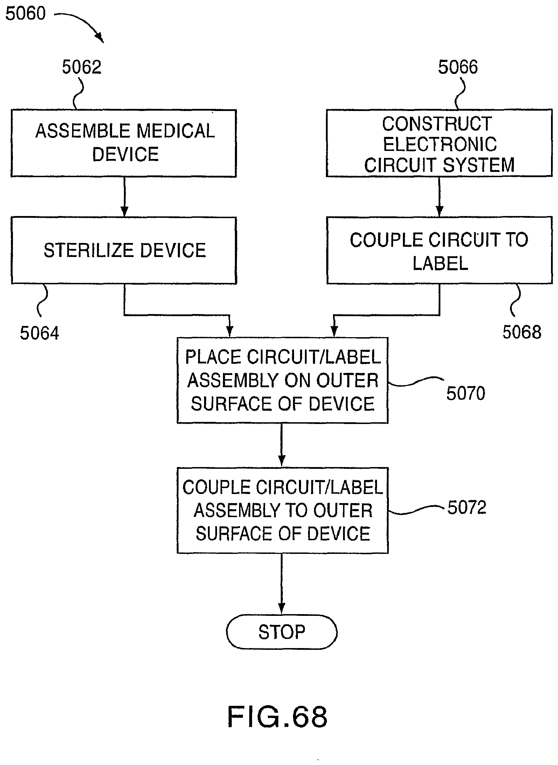

FIG. 68 is a flow chart illustrating a method 5060 for manufacturing a medical device according to an embodiment of the invention. The medical device can be any medicament delivery device of the type discussed above, such as, for example, an auto-injector, a pen injector, an inhaler, or a transdermal delivery device. The medical device can also be a medicament container, such as, for example, a pill bottle, a blister pack, an intravenous (IV) bag or the like. The illustrated method includes assembling the medical device, 5062. The medical device is then sterilized using any suitable sterilization process, 5064. In some embodiments, for example, such as those embodiments in which the medicament is epinephrine, the medical device can be sterilized by exposure to ethylene oxide (EtO) gas. In other embodiments, the medical device can be sterilized by exposure to gamma radiation. In yet other embodiments, the medical device can be sterilized by exposure to heat, such as for example, by placing the medicament delivery device into an autoclave.

In parallel with the manufacture of the medical device, the illustrated method includes constructing an electronic circuit system of the type shown and described above, 5066. The electronic circuit system is then coupled to a label, 5068, to form a label assembly. Because the circuit construction is done apart from the manufacture of the medicament delivery device, it is not subjected the sterilization process, which, in some instances, may damage the circuit components.

The illustrated method then includes placing the label assembly on the outer surface of the medical device, 5070. The label assembly is then coupled to the outer surface of the medical device, 5072. In some embodiments, the label assembly can be coupled to the medicament delivery device by an adhesive, an elastic fastener, a shrink wrap or any other suitable method.

While various embodiments of the invention are described herein, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above.

For example, although the first surface 4912 of the top layer 4911 of the label 4910 is shown and described as being opposite the second surface 4914 of the top layer 4911 of the label 4910, in other embodiments, the first surface 4912 and the second surface 4914 can be adjacent each other and/or co-planar. Similarly, although the top layer 4911 of the label 4910 is shown and described as covering substantially all of the housing 4110, in some embodiments, the top layer 4911 of the label 4910 can cover only a portion of the housing.

Although the label 4910 is shown and described as including a top layer 4911, an intermediate layer 4980 and a printed circuit board 4922, in some embodiments, the layers comprising the label 4910 can be arranged in any suitable order. For example, in some embodiments, a multi-layered label can include a printed circuit board as an intermediate layer. In other embodiments, a multi-layered label can include a printed circuit board as the outer layer. Moreover, in yet other embodiments, the label need not include multiple layers. For example, in some embodiments, a label can include a single layer that includes an electronic circuit system and textual indicia.

Although the indicia 4916 are shown and described as being visible (e.g., textual indicia and/or symbolic indicia), in some embodiments, a label can include indicia that are haptic. For example, in some embodiments a label can include Braille. In other embodiments, a label can include indicia having a distinct feel, such as for example, a particularly rough or smooth surface.

Although the electronic circuit system 4920 is shown and described as including a printed circuit board 4922 having a flexible substrate 4924, in other embodiments, an electronic circuit system can include a rigid printed circuit board. In yet other embodiments, an electronic circuit system can include a printed circuit board having a substrate having at least a rigid portion.

Moreover, in some embodiments, an electronic circuit system need not include a printed circuit board. For example, in some embodiments, an electronic circuit system can include electronic components operatively coupled by any suitable method other than by a printed circuit board.

Similarly, although the components included in the electronic circuit system 4920 (e.g., the microprocessor 4950, the LEDs 4958A and 4958B or the like) are shown and described as being operatively coupled by electrical conductors 4934, in other embodiments, the components can be operatively coupled without being physically connected. For example, in some embodiments, at least a portion of the components included in an electronic circuit system can be inductively coupled. In other embodiments, at least a portion of the components included in an electronic circuit system can be evanescently coupled.

Although the switches 4972A and 4972B are shown and described as being "tear-through" switches that are monolithically formed from the electrical conductors 4934, in other embodiments, a switch can be formed separately from the electrical conductors 4934. For example, in some embodiments, an electrical circuit system can include a series of first electrical conductors having a first set of characteristics (e.g., the width, height, material from which the conductor is fabricated or the like) and a switch constructed from a second electrical conductor having a second set of characteristics different than the first set of characteristics. In other embodiments, a switch can be a separate component, such as, for example, a microswitch, that is mounted to the printed circuit board. In yet other embodiments, an electrical circuit system can include a "pop-out" switch that includes a biasing member to bias the switch in a predetermined state. In yet other embodiments, an electrical circuit system can include a switch that is disposed at a location other than on a printed circuit board.

Similarly, although the switches 4972A and 4972B are shown and described as being irreversibly movable from a first state to a second state, in other embodiments, a switch can be reversibly movable between a first state and a second state. Moreover, in yet other embodiments, a switch can have more than two distinct states.

Although the actuators 4732, 4539 are shown and described as being configured to move in a direction substantially parallel to the surface of the substrate 4924, in other embodiments, an actuator can be configured to actuate an electronic circuit system by moving in any direction. For example, in some embodiments a circuit actuator can be moved in a direction substantially normal to a portion of an electronic circuit system.

Similarly, although the actuators 4732, 4539 are shown and described as actuating the switches 4972A and 4972B by tearing and/or deforming a portion of the substrate 4924, in other embodiments, a switch can be moved from a first state to a second state without deforming the substrate. For example, in some embodiments, an electronic circuit system can include a printed circuit board having a substrate and a frangible switch tab disposed on the substrate. An electrical conductor and/or a switch can be disposed on the frangible switch tab, such that when the switch tab is removed from the substrate the switch is moved from a first state to a second state. In this manner, the switch can be actuated without tearing and/or deforming a portion of the substrate.

Although the actuators 4732, 4539 are shown and described as being included on the safety lock 4710 and the base 4520, respectively, in other embodiments, the actuators can be included on any component of a medicament delivery device. For example, in some embodiments, an auto-injector can include a start button having an actuator configured to actuate an electronic circuit system. In other embodiments, an auto-injector can include a movable member configured to move a medicament container and/or a needle within a housing of the auto-injector, the movable member including an actuator configured to actuate an electronic circuit system.

Although the safety lock 4710 is shown and described as being removed from the housing 4110 of the auto-injector 4002 when in its second position, in other embodiments, a safety lock can remain coupled to the housing of an auto-injector when in its second position. For example, in some embodiments, a safety lock can be moved from its first position to its second position by rotating a portion of the safety lock.

Certain components of the auto-injector 4002 are shown and described as being coupled together via protrusions and mating openings. The protrusions and/or openings can be disposed on any of the components to be coupled together and need not be limited to only a certain component. For example, the safety lock 4710 is shown and described as including an actuator 4732 having a protrusion 4730 configured to be received within an opening 4928A defined by the substrate 4924. In some embodiments, however, the protrusions can be disposed on the substrate 4924 and the mating openings can be defined by the actuator 4732. In other embodiments, such components can be coupled together in any suitable way, which need not include protrusions and mating openings. For example, in some embodiments, an actuator can be operatively coupled to an actuation portion of a substrate via mating shoulders, clips, adhesive or the like.

Similarly, although certain components of the auto-injector 4002 are shown and described as being constructed from multiple separate components, in some embodiments, such components can be monolithically constructed. For example, the needle guard 4810 and the battery isolation tab 4860 are shown and described as being constructed separately and then coupled together. In other embodiments, a needle guard and a battery isolation tab can be constructed monolithically.

Although the electronic circuit systems are shown and described herein as including a proximity sensor, in other embodiments, an electronic circuit system can include any suitable sensor for providing feedback to the electronic circuit system. For example, in some embodiments, the electronic circuit system can include a pressure sensor configured to sense the internal gas pressure within a gas-powered auto-injector. In this manner, the electronic circuit system can output an instruction and/or a status message when the internal gas pressure crosses a predetermined threshold. For example, in some embodiments, when the internal gas pressure rapidly increases, the electronic circuit system can output a message, such as, for example, "Internal gas chamber has been successfully punctured - injection is in process."

Similarly, in some embodiments, the electronic circuit system can include a temperature sensor configured to sense the temperature of the medicament contained within the medicament delivery device. In this manner, the electronic circuit system can output an instruction and/or a status message when the medicament is too cold for effective delivery. For example, in some embodiments, when the medicament is too cold for effective delivery (this may occur, for example, if the medicament delivery device has been left outside overnight), the electronic circuit system can output a message, such as, for example, "Medicament is too cold - please briskly rub the auto-injector between your hands."

Although the batteries 4962 are shown and described as having a first surface 4964 (an electrically negative terminal) and a second surface 4966 (an electrically positive terminal) opposite the first surface, in other embodiments the batteries can include a first surface and a second surface that are adjacent each other and/or co-planar. In other embodiments, an electronic circuit system can be powered by a battery having any shape and/or any number of surfaces. In yet other embodiments, an electronic circuit system can be powered by any suitable energy storage device, such as, for example, a capacitor, solar cell, spring actuated generator, or the like.

Although the medicament delivery devices have been shown and described above as being primarily single-use medical injectors, in some embodiments a medicament delivery device can include any suitable device for delivering one or more doses of a medicament into a patient's body. For example, in some embodiments, a medicament delivery device can be a pen injector containing multiple doses of a chronic-care medicament, such as, for example, insulin. In such embodiments, an electronic circuit system can output instructions associated with not only an initial use of the medicament delivery device, but also associated with repeated uses, dosage monitoring or the like. In other embodiments, a medicament delivery device can include a transdermal medicament delivery device, an inhaler or a nasal medicament delivery device.

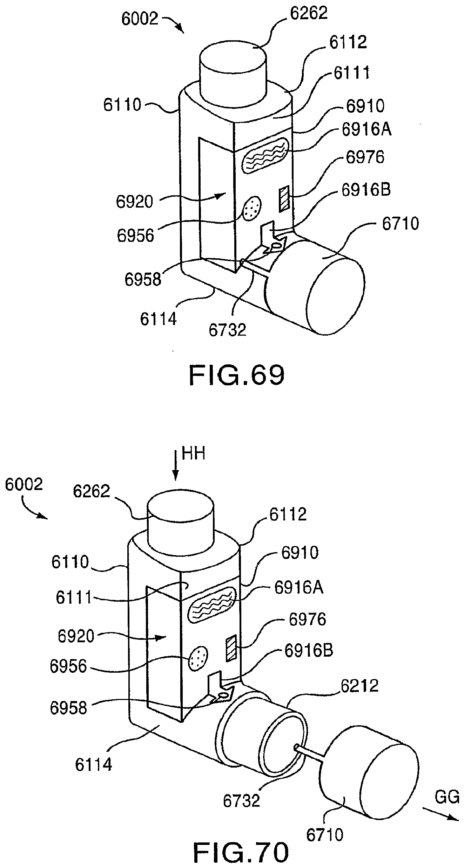

FIGS. 69 and 70 show an inhaler 6002 according to an embodiment of the invention. The inhaler 6002 includes a housing 6110 and a medicament container 6262 movably disposed within the housing 6110. The medicament container 6262 includes a metering mechanism (not shown in FIGS. 69 and 70) configured to discharge a predetermined volume of medicament when the inhaler 6002 is actuated.

The housing 6110 has a proximal end portion 6112 and a distal end portion 6114. An label 6910, which includes at least a portion of an electronic circuit system 6920, is disposed on an outer surface 6111 of the housing 6110. As described above, a portion of the label 6910 can include a textual indicia 6916. Similar to the electronic circuit systems shown and described above, the electronic circuit system 6920 is configured to output at least one electronic signal associated with the user of the inhaler 6002. The electronic circuit system 6920 includes a microprocessor (not shown), a microspeaker 6956 and an LED 6958. The electronic circuit system 6920 also includes a motion sensor 6976, the function of which is discussed in more detail below.

The distal end portion 6114 of the housing 6110 includes a mouthpiece 6212 about which a protective cap 6710 is disposed. Prior to use, the inhaler 6002 is first enabled by removing the protective cap 6710, as shown by the arrow GG in FIG. 70. The protective cap 6710 includes an actuator 6732 that actuates the electronic circuit system 6920 to trigger a predetermined output or sequence of outputs when the protective cap 6710 is removed. In some embodiments, the actuator 6732 can include a protrusion that is received by an actuation portion of the electronic circuit system 6920, in a similar manner as described above. In other embodiments, the actuator 6732 can be configured to engage a microswitch that can be repeatedly moved between a first state and a second state.

When actuated, the electronic circuit system 6920 can output one or more predetermined electronic outputs. For example, in some embodiments, the electronic circuit system 6920 can output an audible message via the microspeaker 6956 instructing the user to "vigorously shake the inhaler for five seconds." The processor can simultaneously enable the motion sensor 6976.

Upon receiving a predetermined input from the motion sensor 6976, which can be any sensor suitable for detecting the rapid motion of the inhaler 6002, the processor can then send an electronic signal to produce a second audible message. Such a message can state, for example, "the inhaler is now sufficiently shaken and is ready for use." In some embodiments, the electronic circuit system 6920 can also output an instruction associated with the correct placement of the inhaler 6002. For example, the electronic circuit system 6920 can output an audible message stating "please place the mouthpiece in your mouth and firmly press down on the medicament container." The electronic circuit system 6920 can also simultaneously output a signal to the LED 6958 to provide a visual indication of where the mouthpiece 6212 is located.

After the inhaler 6002 is enabled and placed within the mouth of the patient, the inhaler 6002 is actuated by moving the medicament container 6262 distally within housing 6110, as illustrated by arrow HH in FIG. 70. In some embodiments, the medicament container 6262 can include an actuator (not shown) that actuates the electronic circuit 6920, in a manner similar to those described above, to trigger a predetermined output or sequence of outputs. For example, in some embodiments, the processor can output an electronic signal associated with recorded speech to the microspeaker 6956. Such an electronic signal can be, for example, associated with a recorded message notifying the user that the injection is complete, instructing the user on post-injection procedures, instructing the user on post-injection medical treatment or the like. Such a status message can state, for example, "The injection is now complete."

In other embodiments, a medicament delivery device can include a transdermal medicament delivery device, such as for example, a medicament patch. In such embodiments, an electronic circuit system can be configured, for example, to output instructions associated with the enablement, placement and/or removal of the transdermal medicament delivery device. For example, in some embodiments, the electronic circuit system can be actuated by removing a protective barrier that seals the portion of the device that contacts the skin.

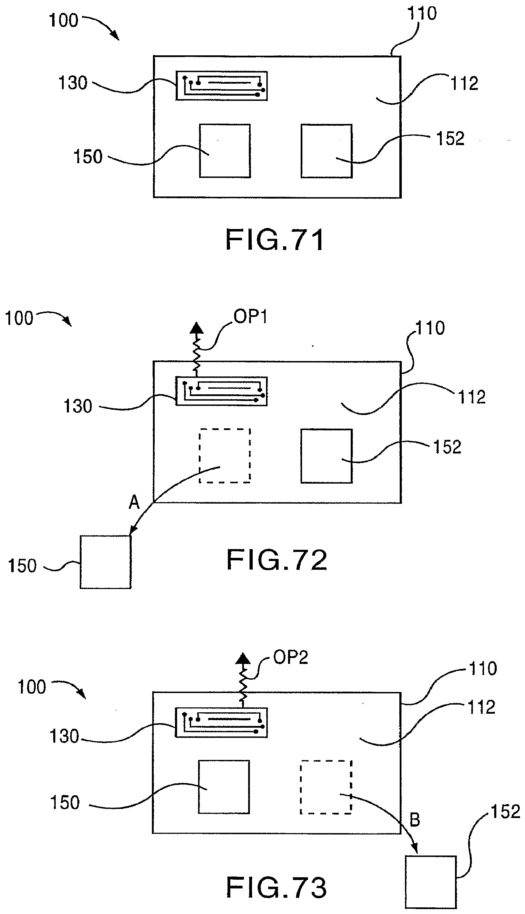

Although the medical devices are shown and described above as being medicament delivery devices, such as, for example, medical injectors, inhalers or the like, in other embodiments, a medical device can include a medicament container, such as, for example, a pill bottle, a blister pack or the like. In yet other embodiments, a medical device can include a container configured to contain one or more medicament delivery devices. For example, FIGS. 71 - 73 are schematic illustrations of a medical device 100 according to an embodiment of the invention in a first configuration, a second configuration and a third configuration, respectively. The medical device 100 includes a container 110 including an electronic circuit system 130 and defining an internal region 112. The internal region 112 is configured to contain one or more medicament delivery devices. Disposed within the internal region 112 are at least a first medicament delivery device 150 and a second medicament delivery device 152. The first medicament delivery device 150 and/or the second medicament delivery device 152 can be any suitable medicament delivery device, such as, for example, an auto-injector, a pen injector, an inhaler or the like.

As shown in FIG. 72, the electronic circuit system 130 is configured to output a first electronic output OP1 when the first medicament delivery device 150 is removed from the internal region 112 of the container 110, as indicated by the arrow A. As discussed in more detail herein, the first electronic output OP1 can be associated with an identification of the first medicament delivery device 150, an identification of a physical condition and/or an instruction for using the first medicament delivery device 150. Moreover, the first electronic output OP1 can include a visual output, an audible output and/or a haptic output. For example, in some embodiments, the first electronic output OP1 can be associated with an audible message instructing a user in the use of the first medicament delivery device 150. Such an audible message can state, for example, "You have removed an auto-injector containing Epinephrine. To actuate the auto-injector, first remove the red safety tab located at the end of the auto-injector." In other embodiments, for example, the first electronic output OP1 can be associated with a visual text message instructing to perform a series of tests on and/or observe the symptoms exhibited by a patient to determine whether the patient is suffering from a certain physical condition (e.g., anaphylactic shock).

Similarly, as shown in FIG. 73, the electronic circuit system 130 is configured to output a second electronic output OP2 when the second medicament delivery device 152 is removed from the internal region 112 of the container 110, as indicated by the arrow B. The second electronic output OP2, which is different than the first electronic output OP1, can include a visual output, an audible output and/or a haptic output. Moreover, as with the first electronic output OP1, the second electronic output OP2 can be associated with at least one of an identification of the second medicament delivery device 152, an identification of a physical condition and/or an instruction for using the second medicament delivery device 152. In this manner, the electronic circuit system 130 can provide the user with information about the particular medicament delivery device that has been removed from the container 110.

Although the second electronic output OP2 is described as being different than the first electronic output OP1, in some embodiments, the second electronic output OP2 can be the same as the first electronic output OP1. In some embodiments, for example, the second electronic output OP2 can include the same information as previously output via the first electronic output OP1 along with additional information. In this manner, the second electronic output OP2 can confirm the instructions and/or information provided by the first electronic output OP1.

The container 110 can be any container suitable for containing a plurality of medicament delivery devices. For example, in some embodiments, the container 110 can be a box-like structure that includes a lid or cover that can be repeatedly opened and closed to selectively expose the internal region 112 of the container 110 to an area outside the container 110. In other embodiments, the container 110 can include a frangible portion that can be irreversibly moved to expose the internal region 112 of the container 110 to allow access to the first medicament delivery device 150 and/or the second medicament delivery device 152.

The container 110 can be either portable or permanently installed at a particular location. For example, in some embodiments, the container 110 can be configured to be moved by the user. For example, in such embodiments, a user may carry the container 110 to events, such as picnics, field trips, children's camps or the like, where the likelihood of use increases. In other embodiments, the container 110 can be removably coupled to a mounting area within a building, such as a restaurant, airport and/or shopping mall. In this manner, when a user recognizes an emergency situation, the user can locate the container 110 and move it to the area in which the emergency situation is occurring. In yet other embodiments, the container 110 can be permanently coupled to a wall of a building.

The container 110 can be constructed from any suitable material, such as, for example, plastic, metal alloys, insulative foam, fabric or any combination thereof. In some embodiments, for example, the container 110 can include a hard, plastic outer casing and an insulative, shock-absorbing inner liner. In some embodiments, the container 110 can be constructed from a waterproof material and/or can be configured to float. In some embodiments, the container 110 can be constructed from a material configured to prevent light from reaching the interior region 112 of the container. In this manner, the container can prevent the medicaments contained therein from being exposed to light that can impact the chemical structure and/or stability of the medicament.

Although the container 110 is shown and described above as containing a first medicament delivery device 150 and a second medicament delivery device 152 having similar sizes and/or shapes, in some embodiments, a container can be configured to include medicament delivery devices of different sizes and/or shapes. For example, in some embodiments, a container can be configured to include a medical injector having a long, narrow shape and an inhaler having a wider shape.

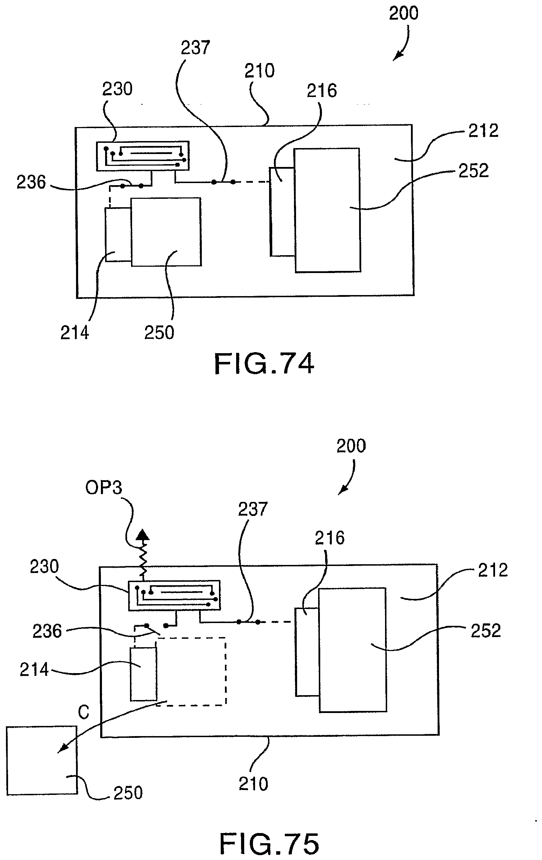

FIGS. 74 - 76 are schematic illustrations of a medical device 200 according to one such embodiment of the invention in a first configuration, a second configuration and a third configuration, respectively. The medical device 200 includes a container 210 including an electronic circuit system 230 and defining an internal region 212. The internal region 212 includes a first retainer 214 and a second retainer 216. The first retainer 214 retains a first medicament delivery device 250 within the internal region 212 of the container. Similarly, the second retainer 216 retains a second medicament delivery device 252 within the internal region 212 of the container.

The electronic circuit system 230 includes a first switch 236 associated with the first retainer 214 and a second switch 237 associated with the second retainer 216. The first switch 236 is configured move between a first state (e.g., closed) and a second state (e.g., opened) when the first medicament delivery device 250 is removed from the first retainer 214. Similarly, the second switch 237 is configured move between a first state and a second state when the second medicament delivery device 252 is removed from the second retainer 216. In this manner, the electronic circuit system 230 can output electronic outputs based on the state of the first switch 236 and/or the second switch 237.

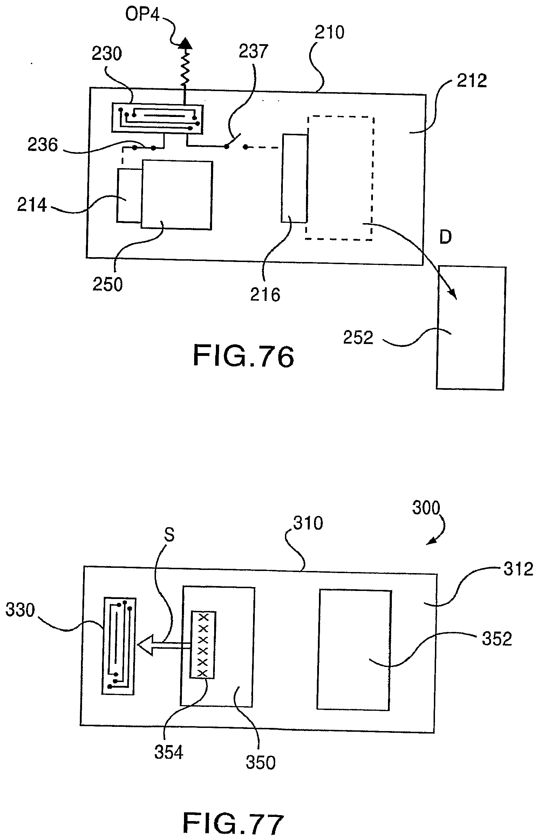

More particularly, as shown in FIG. 75, the electronic circuit system 230 is configured to output a first electronic output OP3, of the type described above, when the first medicament delivery device 250 is removed from the first retainer 214, as indicated by the arrow C. Similarly, as shown in FIG. 76, the electronic circuit system 230 is configured to output a second electronic output OP4, of the type described above, when the second medicament delivery device 252 is removed from the second retainer 216, as indicated by the arrow D. Said another way, the electronic circuit system 230 is configured to output the first electronic output OP3 in response to the first switch 236 moving between its first state and its second state. Similarly, the electronic circuit system 230 is configured to output the second electronic output OP4 in response to the second switch 237 moving between its first state and its second state.

The first retainer 214 can be any structure that cooperates with the first medicament delivery device 250 to retain the first medicament delivery device 250 within the internal region 212 of the container 210. Similarly, the second retainer 216 can be any structure that cooperates with the second medicament delivery device 252 to retain the second medicament delivery device 252 within the internal region 212 of the container 210. In some embodiments, for example, the first retainer 214 can be a recessed portion (not shown in FIGS. 74-76) of the internal region 212 having a shape to receive at least a portion of the first medicament delivery device 250. Such a recess can include, for example, an edge, a contour or a ridge that forms an interference fit with a portion of the first medicament delivery device 250 when the first medicament delivery device 250 is received within the first retainer 214. In other embodiments, for example, the first retainer 214 and/or the second retainer 216 can be a clip configured to engage a portion of the first medicament delivery device 250 and/or the second medicament delivery device 252, respectively, to retain the first medicament delivery device 250 and/or the second medicament delivery device 252 in the internal region 212. In yet other embodiments, the first retainer 214 and/or the second retainer 216 can be an elastic member, such as an elastic band configured to engage a portion of the first medicament delivery device 250 and/or the second medicament delivery device 252. In yet other embodiments, the first retainer 214 and/or the second retainer 216 can include a frangible member, such as a removable plastic covering configured to retain the first medicament delivery device 250 and/or the second medicament delivery device 252 in the internal region 212.

In some embodiments, the first retainer 214 can be uniquely associated with the first medicament delivery device 250 and/or the second retainer 214 can be uniquely associated with the second medicament delivery device 252. In this manner, the first medicament delivery device 250 can only be associated with the first switch 236 and the second medicament delivery device 252 can only be associated with the second switch 237. Said another way, such an arrangement prevents second medicament delivery 252 from inadvertently being retained by the first retainer 214, which could result in the electronic circuit system 230 outputting the first electronic output OP3 when the second medicament delivery device 252 is removed from the container 210 or vice-versa. Moreover, by using the first retainer 214 and the second retainer 216, the internal region 212 can be adapted to contain a variety of different medicament delivery devices having different sizes, shapes and/or characteristics. For example, in those embodiments in which the first retainer 214 is a recessed portion of the internal region 212, the shape of the recess can be uniquely associated with a shape of the first medicament delivery device 250, thereby preventing the second medicament delivery device 252 from being received within the first retainer 214. Similarly, in some embodiments, the second retainer 216 can be a recessed portion of the internal region 212, of the type described above, having a shape to receive at least a portion of the second medicament delivery device 252.

Although the retainers are described above as cooperating with the medicament delivery devices to retain the medicament delivery devices within the internal region 212 of the container 210, in some embodiments, the first retainer 214 and/or the second retainer 216 can perform additional functions. For example, in some embodiments, the first retainer 214 can electronically couple an electronic circuit system (not shown in FIGS. 74-76) disposed on the first medicament delivery device 250 to the electronic circuit system 230. The electronic circuit system included in the first medicament delivery device 250 can be of the type shown and described above with reference FIGS. 46-70. Similarly, the second retainer 216 can electronically couple an electronic circuit system (not shown in FIGS. 74-76) disposed on the second medicament delivery device 252 to the electronic circuit system 230. In this manner, the first retainer 214 and/or the second retainer 216 can be used as a battery charging port, a data exchange port or the like.

FIG. 77 is a schematic illustration of a medical device 300 according to an embodiment of the invention. Because the medical device 300 is similar in many respects to the medical devices shown and described above, the medical device 300 is shown in only one configuration. The medical device 300 includes a container 310 including an electronic circuit system 330 and defining an internal region 312. The internal region 312 includes a first medicament delivery device 350 and a second medicament delivery device 352 of the types described above.

The electronic circuit system 330 is configured to output a first electronic output (not shown in FIG. 77), of the type described above, when the first medicament delivery device 350 is removed from the internal region 312 of the container 310. Similarly the electronic circuit system 330 is configured to output a second electronic output (not shown in FIG. 77), of the type described above, when the second medicament delivery device 352 is removed from the internal region 312 of the container 310.

Moreover, as shown in FIG. 77, the first medicament delivery device 350 includes a label 354, such as, for example, a radio frequency identification ("RFID") tag, configured to output a signal S that can be received by the electronic circuit system 330. In some embodiments, the signal S can indicate the position of the first medicament delivery device 350 (e.g., whether the first medicament delivery device 350 is outside the internal region 312). In other embodiments, the signal S can include information characterizing the first medicament delivery device 350. For example, in some embodiments, the signal S can be associated with the contents of the first medicament delivery device 350 (e.g., the amount and type of medicament contained therein), an expiration date of the first medicament delivery device 350, a dosage of the first medicament delivery device 350 and/or a use instruction associated with the first medicament delivery device 350. In this manner, the electronic circuit system 330 can receive the signal S and produce the first electronic output (not shown in FIG. 77) to include information contained within the signal S. Said another way, this arrangement allows the electronic circuit system 330 to produce an electronic output that is unique to the medicament delivery devices contained within the container 310.

In some embodiments, for example, the first electronic output can be associated with an audible message including information contained from the signal S, such as for example, the expiration date of the medicament contained within the first medicament delivery device 350. Such an audible message can state, for example, "You have removed an auto-injector containing DOSE mg of Epinephrine. The expiration date of this device is EXPIRATION DATE. If the current date is later than EXPIRATION DATE please select another auto-injector from within the container." In other embodiments, for example, the first electronic output can be a message providing the user with use instructions or other information contained within the signal S that is uniquely associated with the first medicament delivery device 350. For example, such a message can prompt a user to call a phone number unique to the manufacturer of the first medicament delivery device 350 for assistance before, during or after the use of the first medicament delivery device 350. In yet other embodiments, as described in more detail herein, the electronic circuit system 330 can automatically call such a phone number when the first medicament delivery device 350 is removed from the internal region 312 of the container 310.

The label 354 can be any device suitable for outputting the signal S that includes information associated with the first medicament delivery device 350 and that can be received by the electronic circuit system 330. For example, in some embodiments, the label 354 can include a passive RFID tag. In other embodiments, the label can include an active RFID tag.

In some embodiments, label 354 can include its own electronic circuit system, similar to the electronic circuit systems described above with reference to FIGS. 46-70. In such embodiments, the label 354 can produce multiple signals associated with the first medicament delivery device 350 based on the ongoing status of the medicament delivery device 350 as determined by the electronic circuit system included within the label 354. For example, in some embodiments, the first medicament delivery device 350 can include its own electronic circuit system having various switches, sensors or the like, such that when the user completes certain operations (e.g., removing the needle guard, removing the safety tab, etc.), a signal S can be transmitted to the electronic circuit system 330 of the container 310. The electronic circuit system 330 of the container 310 can then output one or more electronic outputs of the type described above to provide information to the user that is unique to the status of the first medicament delivery device 350.

Although the label 354 is shown and described as outputting a signal S that can be received by the electronic circuit system 330, in other embodiments, the label 354 can be a passive device that does not output an electronic signal, but rather, contains information associated with the medicament delivery device 350 in a machine-readable format. For example, in such embodiments, the label 354 can include a bar code portion containing information associated with the medicament delivery device 350. In other embodiments, the label 354 can include a magnetic strip containing information associated with the medicament delivery device 350.

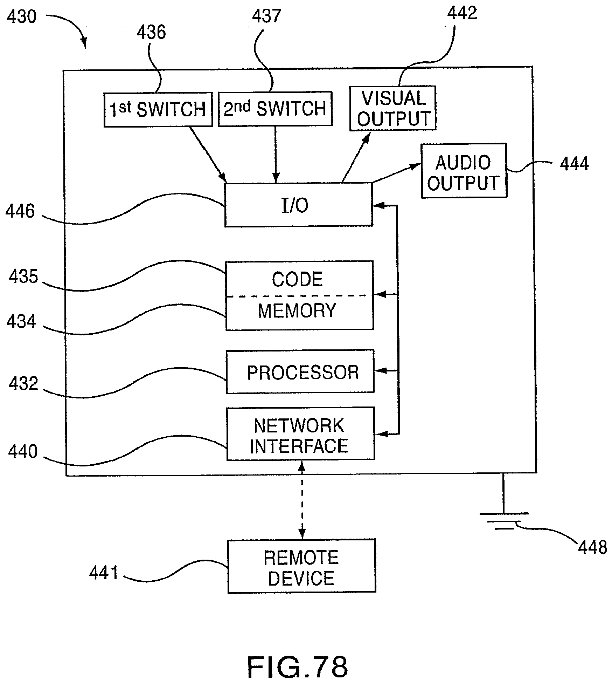

The electronic circuit systems shown and described above can include many components operatively coupled to perform the functions described herein For example, FIG. 78 is a schematic illustration of an electronic circuit system 430 according to an embodiment of the invention. The electronic circuit system 430 includes a processor 432 operatively coupled to a memory device 434. The memory device 434 can be configured to store processor-readable code 435 instructing the processor 432 to perform the functions described herein. In some embodiments, the processor-readable code 435 can be modified and/or updated as circumstances dictate. The electronic circuit system 430 includes an input/output device 446 configured to receive electronic inputs from a first switch 436 and/or a second switch 437. In some embodiments, the input/output device 446 can receive inputs from an RFID tag (as described above), the user's voice (e.g., through a microphone), a keyboard, a touch screen, a proximity sensor and/or any other suitable device. The input/output device 446 is also configured to provide electronic signals to various output devices, such as, for example, a visual output device 442, an audio output device 444, a haptic output device (not shown in FIG. 78) a wireless receiver (e.g., an RFID tag, a cellular phone system or the like) and/or a wired receiver (e.g., a wired network).

The visual output device 442 can be any suitable device for producing visual indicia, such as, light-emitting diodes (LEDs), liquid-crystal display (LCD) screens, optical polymers, fiber optic components or the like. Similarly, the audio output device 444 can be any suitable device for producing sound, such as a micro-speaker a piezo-electric transducer or the like. Such sound output can include, for example, an alarm, a series of beeps, recorded speech or the like.

In some embodiments, the electronic circuit system 430 includes a network interface 440 configured to operatively couple the electronic circuit system 430 to a remote device 441, either via a wired connection or via a wireless connection. The remote device 441 can be, for example, a remote communications network, a computer, a cell phone, a personal digital assistant (PDA) or the like. Such an arrangement can be used, for example, to download replacement processor-readable code 435 from a central network to the memory device 434. In some embodiments, the electronic circuit system 430 can download information associated with a medicament delivery device, such as an expiration date, a recall notice, updated use instructions or the like.

The network interface 440 can also be configured to transmit information from the electronic circuit system 430 to a central network, such as, for example, an emergency response network. In some embodiments, for example, the electronic circuit system 430 can notify an emergency responder when a medicament delivery device is removed and/or actuated. In other embodiments, the electronic circuit system 430 can transmit information to a third party, such as a physician, an emergency contact and/or the manufacturer of a medicament device, when the medicament delivery device is removed and/or actuated. Such information can include, for example, the location of use, the date and/or time of use or the like.

As shown in FIG. 78, power is supplied to the electronic circuit system 430 by a power source 448. The power source 448 can be any suitable power source, such as, for example, a DC power source and/or an AC power source. In some embodiments, for example, power can be provided to the electronic circuit system 430 by an AC circuit within the building in which the medical device is located. In other embodiments, power can be provided to the electronic circuit system 430 by one or more batteries. In yet other embodiments, power can be provided to the electronic circuit system 430 by both an AC circuit (e.g. as the primary source of power) and by batteries (e.g. as the secondary source of power). In yet other embodiments, the electronic circuit system 430 can be powered by any suitable energy storage device, such as, for example, a capacitor, solar cell or the like.

The processor 432 can be a commercially-available processing device dedicated to performing one or more specific tasks. For example, in some embodiments, the microprocessor 432 can be a commercially-available microprocessor, such as the Sonix SNC 12060 voice synthesizer. Alternatively, the processor 432 can be an application-specific integrated circuit (ASIC) or a combination of ASICs, which are designed to perform one or more specific functions. In yet other embodiments, the processor 132 can be an analog or digital circuit, or a combination of multiple circuits.

The memory device 434 can include one or more types of memory. For example, in some embodiments, the memory device 434 can include a read only memory (ROM) component and a random access memory (RAM) component. The memory device 432 can also include other types of memory suitable for storing data in a form retrievable by the processor 432, for example, electronically-programmable read only memory (EPROM), erasable electronically-programmable read only memory (EEPROM) and/or flash memory.

Although the medical devices shown and described herein include one electronic circuit system, in some embodiments, a medical device can include multiple electronic circuit systems configured to perform the functions described herein.

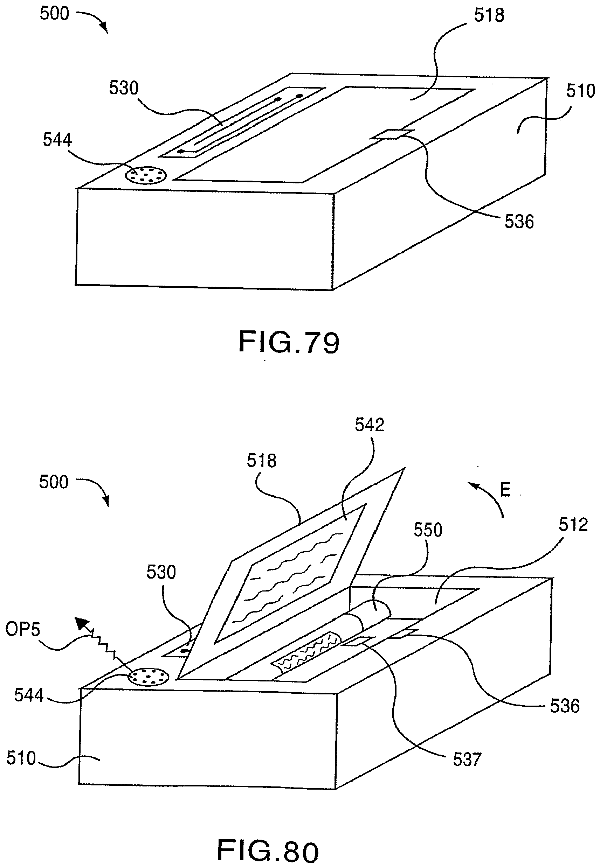

Although the containers shown and described above include multiple medicament delivery devices, in some embodiments, a container can include only one medicament delivery device. For example, FIGS. 79 - 81 show a medical device 500 including a container 510 that contains a medicament delivery device 550, such as, for example a pen injector or an auto-injector. As described above, the container 510 defines an internal region 512 in which the medicament delivery device 550 is contained. The container includes an electronic circuit system 530 configured to produce one or more electronic outputs of the type described above. More particularly, the electronic circuit system 530 includes a speaker 544 and an LCD screen 542.

The container 510 also includes a movable portion 518, such as, for example, a hinged lid, that has a first position (see FIG. 79) and a second position (see FIGS. 80 -81). When the movable portion 518 is in the first position, the movable portion 518 covers the internal region 512 of the container 510. Conversely, when the movable portion 518 is in the second position, at least a portion of the internal region 512 of the container 510 is exposed. Said another way, when the movable portion 518 is in the second position, the medicament delivery device 550 can be removed from the internal region 512 of the container 510.

The electronic circuit system 530 is operatively coupled to a first switch 536 and a second switch 537. The first switch 536 is configured to move between a first state (e.g., closed) and a second state (e.g., opened) when the movable portion 518 moves between its first position and its second position, as indicated by arrow E in FIG. 80. The electronic circuit system 530 is configured to output a first output OP5 via the speaker 544 when the first switch 536 is moved from its first state to its second state. The first output OP5 can be a recorded speech output associated with an identification of the medicament delivery device 550, an identification of patient symptoms (e.g., instructions for assessing the physical condition of the patient) and/or an instruction for using the medicament delivery device 550. For example, in some embodiments the first output OP5 can state "You have activated the allergic reaction response kit. This kit includes an auto-injector containing DOSE mg of Epinephrine. Before using this auto-injector, please ensure that the patient is exhibiting the following symptoms ..." Although described as an audible output, in other embodiments, the first output OP5 can be any type of electronic output as described above.

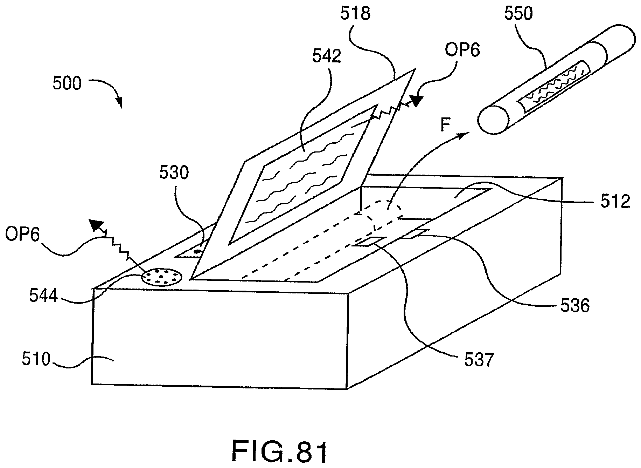

The second switch 537 is configured to move between a first state (e.g., closed) and a second state (e.g., opened) when the medicament delivery device 550 is removed from the internal region 512 of the container 510, as indicated by the arrow F in FIG. 81. The electronic circuit system 530 is configured to output a second output OP6 via the speaker 544 and/or the LCD screen 542 when the second switch 537 is moved from its first state to its second state. The second output OP6 can be, for example, a recorded speech output and/or a video output associated with an identification of the medicament delivery device 550, an identification of patient symptoms (e.g., instructions for assessing the physical condition of the patient) and/or an instruction for using the medicament delivery device 550. For example, in some embodiments the second output OP6 can be an audio-visual output via both the speaker 544 and the LCD screen 542 providing step-by-step instructions for using the medicament delivery device 550.

Although the movable member 518 is shown and described as being a hinged lid, in some embodiments, the movable member can be coupled to the container in any suitable fashion. For example, in some embodiments, the movable member 518 can be a removable cover that is slidingly coupled to the container. In other embodiments, the movable member 518 can be a removable cover that is threadedly coupled to the container (i.e., a removable cap). In yet other embodiments, the movable member 518 can be a removable cover that is coupled to the container via an interference fit. In yet other embodiments, the movable member 518 can be a frangible cover that is irreversibly removed from the container during use of the medical device. For example, in some embodiments the movable member 518 can be a frangible cover that provides a tamperproof seal, a sanitary seal, or the like.

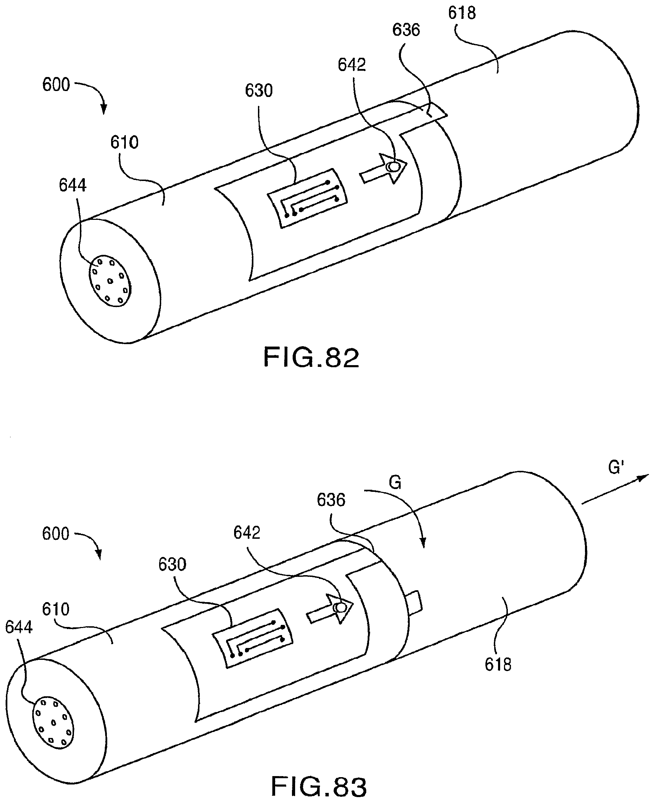

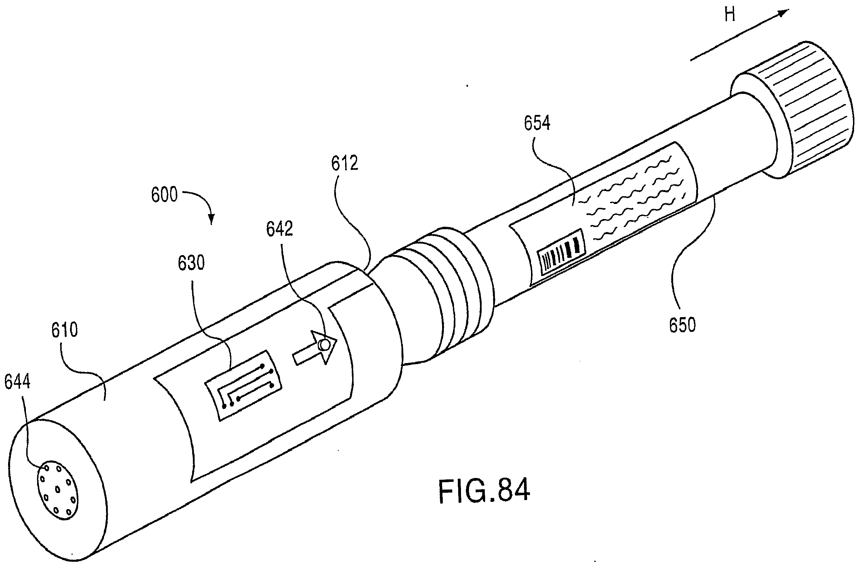

Although the containers are shown and described above as being rigid, box-like containers, in other embodiments, a container can have any suitable shape and/or flexibility. For example, in some embodiments, a container can be a flexible, pouch-like container. Such a container can be more easily carried in certain circumstances, such as, for example at outdoor events (e.g., children's camps, concerts, picnics or the like). In other embodiments, a container can be a tube configured to contain all or a portion of a medicament delivery device. For example, FIGS. 82 - 84 show a medical device 600 including a tube-shaped container 610 and a retainer 618. The container 610 defines an internal region 612 (see FIG. 84) in which at least a portion of a medicament delivery device 650 can be contained.

The retainer 618, which can be, for example, a mating tube-shaped lid, is movably coupled to the container 610 to retain the medicament delivery device 650 within the internal region 612. Said another way, the retainer 618 has a first position (FIG. 82) and a second position (FIG. 58). When the retainer 618 is in the first position, the retainer 618 prevents the medicament delivery device 650 from being removed from the internal region 612 of the container 610. When the retainer 618 is in the second position, the medicament delivery device 650 can be removed from the internal region 612 of the container 610.

The medical device 600 includes an electronic circuit system 630 coupled to the container 610. The electronic circuit system 630 includes a speaker 644 and a light emitting diode (LED) 642 for providing an electronic output associated with the use of the medicament delivery device 650, as described herein. In some embodiments, the electronic circuit system can be, for example, a flexible circuit included in a label coupled to an outer surface of the container 610, similar to the electronic circuit systems described above with reference to FIGS. 46 - 70.

The electronic circuit system 630 is configured to output a first electronic output via the LED 642 and/or the speaker 644 when the retainer 618 is moved relative to the container 610, as indicated by arrows G and/or G' in FIG. 83. As described above, the first electronic output can be associated with an identification of the medicament delivery device 650, an identification of a physical condition and/or an instruction for using the medicament delivery device 650. For example, in some embodiments, the first electronic output can be associated with an audible message instructing a user in the use of the medicament delivery device 650. Such an audible message can state, for example, "You have activated an interactive auto-injector containing DOSE mg of Epinephrine. Please remove the top of the container by twisting and pulling as indicated by the flashing arrow. After removing the top of the container, please remove the auto-injector from the container by firmly pulling on the exposed end of the auto-injector."

The electronic circuit system 630 can be prompted to output the first electronic output by a switch 636 configured to change states when the retainer 618 is moved relative to the container 610. The switch 636 can be any suitable electronic switch having at least two states. For example, in some embodiments, the switch 636 can be a single-use "tear-through" switch, as described above with reference to FIGS. 46 - 70. In other embodiments, a switch can be a multi-use switch, such as a microswitch.

Similarly, the electronic circuit system 630 is configured to output a second electronic output via the LED 642 and/or the speaker 644 when the medicament delivery device 650 is removed from the internal region 612 defined by the container 610, as shown by arrow H in FIG. 84. The second electronic output can be associated with an identification of the medicament delivery device 650, an identification of a physical condition and/or an instruction for using the medicament delivery device 650. For example, in some embodiments, the second electronic output can be an audible message stating, "To activate the auto-injector, first remove the needle guard. The needle guard is at the bottom of the auto-injector and contains the number one inside of an arrow pointing downward. Remove the needle guard by pulling in the direction of the arrow."

As shown in FIG. 84, the medicament delivery device 650 includes a label 654 containing information associated with the medicament delivery device 650 arranged in a machine-readable format. The electronic circuit system 630 is configured to receive (e.g., "read") the information contained in the label 654 and include at least a portion of the information in the first electronic output and/or the second electronic output. In this manner, the electronic circuit system 630 can be configured to produce an electronic output that is unique to the medicament delivery device 650 contained within the container 610. This arrangement allows the container 610 to be reused with any number of different medicament delivery devices 650. Moreover, this arrangement allows the container 610 to track the usage of a chronic-care medicament delivery devices. For example, in some embodiments, the electronic circuit system 630 can track each use of the medicament delivery device 650 and log such information on the label 654.

The label 654 can be any device suitable for containing information associated with the medicament delivery device 650 in a machine-readable format. For example, in some embodiments, the label 654 can include a bar code portion containing information associated with the medicament delivery device 650. In other embodiments, the label 654 can include a magnetic strip containing information associated with the medicament delivery device 650. In yet other embodiments, the label 654 can include a passive RFID tag containing information associated with the medicament delivery device 650. In yet other embodiments, the label 654 can include an active RFID tag containing information associated with the medicament delivery device 650.

Although the retainer 618 is shown as covering the internal region 612 defined by the container 610, in some embodiments, the retainer 618 can allow access to the internal region 612 while still retaining the medicament delivery device 650 within the internal region 612. For example, in some embodiments, the retainer 618 can be a clip, a strap or the like.

Although the medicament delivery device 650 is shown in FIGS. 82 - 83 as being disposed entirely within the container 610 and the retainer 618, in some embodiments, only a portion of the medicament delivery device 650 is disposed within the container 610 and/or the retainer 618. For example, in some embodiments, a container can be a sleeve configured to be disposed about a portion of a medicament delivery device, such as a chronic care pen injector. The retainer can function to retain the pen injector within the sleeve and/or to prevent the pen injector from being actuated (e.g., the retainer can act as a locking member). In use, the user can activate the electronic circuit system by depressing a start button disposed on the container. Alternatively, in some embodiments, the electronic circuit system can be activated by removing the retainer from the pen injector and/or the container. In yet other embodiments, the electronic circuit system can be activated by moving the pen injector relative to the container (i.e., twisting the pen injector within the container). Upon activation, the electronic circuit system then "reads" a label and outputs a first electronic output and/or a second electronic output, as described above.

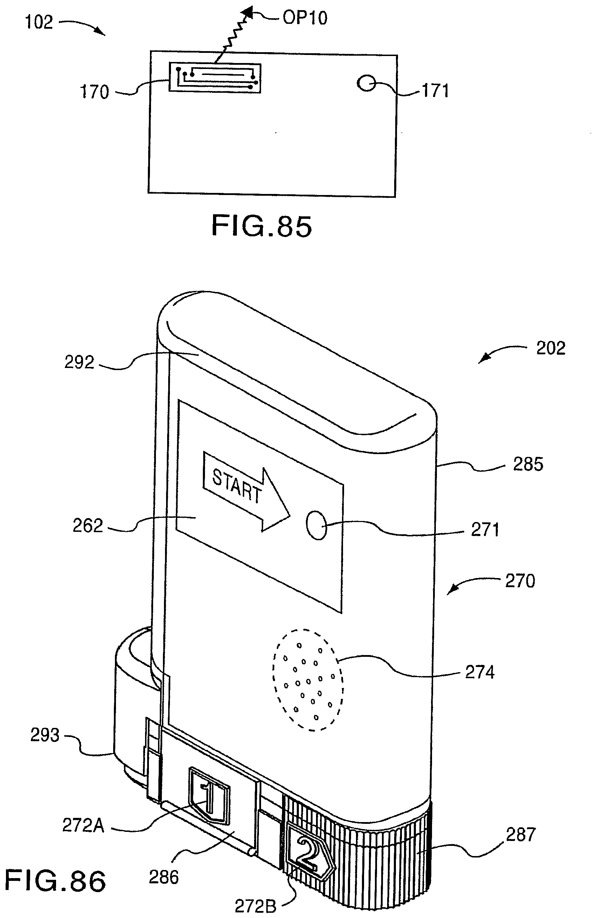

Although the medical devices are shown and described above as including medicament delivery devices, such as, for example, medical injectors, inhalers or the like, in other embodiments, a medical device can include a simulated medicament delivery device. FIG. 85 is a schematic illustration of a simulated medicament delivery device 102 according to an embodiment of the invention. In some embodiments, the simulated medicament delivery device 102 can correspond to an actual medicament delivery device (i.e., a device actually configured to deliver a medicament, not shown in FIG. 85) and can be used, for example, to train a user in the operation of the corresponding actual medicament delivery device.

The simulated medicament delivery device 102 includes an electronic circuit system 170 configured to output an electronic output OP10 associated with the use of the simulated medicament delivery device 102. As described herein, in some embodiments, for example, the electronic output OP10 can be associated with an identification of the simulated medicament delivery device 102, an identification of certain components of the simulated medicament delivery device 102 (e.g., a top portion, a safety lock, or the like), an identification of a physical condition for which a patient may require the medicament delivery device (not shown in FIG. 85) and/or an instruction for using the simulated medicament delivery device 102 and/or the corresponding actual medicament delivery device (not shown in FIG. 85).

Moreover, the electronic output OP10 can include any type of electronic output and/or signal discussed herein, such as, for example, a visual output, an audible output and/or a haptic output. For example, in some embodiments, the electronic output OPIO can be a signal associated with an audible message (e.g., recorded speech) identifying the simulated medicament delivery device 102. Such an audible message can state, for example, "You have removed an auto-injector trainer that will teach you how to use an actual auto-injector. This trainer does not contain any medicament. If this an actual emergency, please dial 911 or locate an actual auto-injector." In some embodiments, an audible output can instruct a user in the use of the simulated medicament delivery device 102. Such an audible message can state, for example, "The first step in using an actual auto-injector is to identify the key features of the auto-injector. The key features of the auto-injector are the safety lock and the actuator button..." In other embodiments, the electronic output OP10 can be associated with a visual indicator that identifies one or more components of the simulated medicament delivery device 102.

In some embodiments, the user can activate the electronic circuit system 170 by pushing the start button 171, which prompts the electronic circuit system 170 to output at least the electronic output OP10. In some embodiments, for example, when the start button 171 is actuated, the electronic circuit system 170 can output a predetermined sequence of electronic outputs. As described above, in some embodiments, the start button 171 can activate the electronic circuit system 170 by providing an input to a processor (not shown in FIG. 85). In other embodiments, the start button 171 can activate the electronic circuit system 170 by placing a battery (not shown in FIG. 85) in electronic communication with a portion of the electronic circuit system 170.

The simulated medicament delivery device 102 can simulate the actual medicament delivery device in any number of ways. For example, in some embodiments, the simulated medicament delivery device 102 can have a shape corresponding to a shape of the actual medicament delivery device, a size corresponding to a size of the actual medicament delivery device and/or a weight corresponding to a weight of the actual medicament delivery device. Moreover, in some embodiments, the simulated medicament delivery device 102 can include components that correspond to the components of the actual medicament delivery device. In this manner, the simulated medicament delivery device 102 can simulate the look, feel and sounds of the actual medicament delivery device. For example, in some embodiments, the simulated medicament delivery device 102 can include external components (e.g., a housing, a needle guard, a sterile cover, a safety lock or the like) that correspond to external components of the actual medicament delivery device. In some embodiments, the simulated medicament delivery device 102 can include internal components (e.g., an actuation mechanism, a spring, a compressed gas source, a medicament container or the like) that correspond to internal components of the actual medicament delivery device.

In some embodiments, however, the simulated medicament delivery device 102 can be devoid of a medicament and/or those components that cause the medicament to be delivered (e.g., a needle, a nozzle or the like). In this manner, the simulated medicament delivery device 102 can be used to train a user in the use of the actual medicament delivery device without exposing the user to a needle and/or a medicament. Moreover, the simulated medicament delivery device 102 can have features to identify it as a training device to prevent a user from mistakenly believing that the simulated medicament delivery device 102 can be used to deliver a medicament. For example, in some embodiments, the simulated medicament delivery device 102 can be of a different color than a corresponding actual medicament delivery device. Similarly, in some embodiments, the simulated medicament delivery device 102 can include a label clearly identifying it as a training device.

The simulated medicament delivery device 102 can simulate any number of medicament delivery devices. For example, in some embodiments, the simulated medicament delivery device 102 can simulate a medical injector, such as an auto-injector, a pen injector or the like. In other embodiments, the simulated medicament delivery device 102 can simulate an inhaler. In yet other embodiments, the simulated medicament delivery device 102 can simulate a transdermal delivery device.

In some embodiments, the simulated medicament delivery device 102 can repeatedly simulate the actual medicament delivery device. For example, in some embodiments, after the simulation is complete the electronic circuit system can be reset, for example, by pushing the start button 171. In this manner, the simulated medicament delivery device 102 can be configured to repeat the electronic output OP10 or predetermined sequence of electronic outputs during subsequent simulations.

FIG. 86 is a perspective view of a simulated auto-injector 202 according to an embodiment of the invention. The simulated auto-injector 202 is configured to simulate an auto-injector (not shown in FIG. 86) similar to the auto-injectors described herein and in U.S. Patent Application Serial Number 11/562,061, entitled "Devices, Systems and Methods for Medicament Delivery," filed November 21, 2006, which is incorporated herein by reference in its entirety.

The simulated auto-injector 202 includes a housing 285 having a proximal end portion 292 and a distal end portion 293. A simulated needle guard assembly 286 is removably coupled to the distal end portion 293 of the housing 285. The simulated needle guard assembly 286 is configured to simulate an actual needle guard assembly (e.g., needle guard assembly 4810 shown and described above with reference to FIGS. 50, 57-59). Similarly, a simulated safety lock 287 is removably coupled to the distal end portion 293 of the housing 285. The simulated safety lock 287 is configured to simulate an actual safety lock (e.g., safety lock 4710 shown and described above with reference to FIGS. 50, 57-59).

The simulated auto-injector 202 includes an electronic circuit system 270 and a label 262. The label 262 can be any suitable label of the type described herein. In some embodiments, for example, the label 262 can include at least a portion of the electronic circuit system 270 (i.e., portions of an electronic conductors, portions of a printed circuit board, a battery, an LED or the like). In other embodiments, the label 262 can be devoid of any portion of the electronic circuit system 270.

The electronic circuit system 270 includes a start button 271, a speaker 274 and two LEDs 272A, 272B. The electronic circuit system 270 can be any electronic circuit system of the type shown and described herein. For example, in some embodiments, the electronic circuit system 270 can include a flexible printed circuit board to electronically coupled the components contained therein. Moreover, the electronic circuit system 270 can be disposed in any suitable manner relative to the housing 285. In some embodiments, for example, the electronic circuit system 270 can be integrated with the simulated medicament delivery device 202. Said another way, in some embodiments, the electronic circuit system 270 can be contained within the housing 285 and/or the electronic circuit system 270 can be assembled concurrently and/or using common processes with the simulated medicament delivery device 202. In other embodiments, the electronic circuit system 270 can be partially-integrated with the simulated medicament delivery device 202. Said another way, in some embodiments, at least a portion of the electronic circuit system 270 can be contained within the housing 285 and/or at least a portion of the electronic circuit system 270 can be assembled concurrently and/or using common processes with the simulated medicament delivery device 202. In yet other embodiments, the electronic circuit system 270 can be disposed entirely on an outer surface of the housing 285 and/or the electronic circuit system 270 can be assembled using separate processes from those used to manufacture the simulated medicament delivery device 202. In some embodiments, for example, the electronic circuit system can be included in the label 262. In other embodiments, the label 262 can be used to secure the electronic circuit system to an outer portion of the housing 285.

To activate the electronic circuit system 270, the user first pushes the start button 271. As described above, when actuated, the electronic circuit system 270 can output one or more electronic outputs. For example, in some embodiments, an electronic output can be associated with an audio and/or a visual output used to describe the features of and/or identify component of the simulated medicament delivery device 202. For example, in some embodiments, the first LED 272A, the output of which is shaped as the numeral "1," can output a flashing light of a first color while the speaker 274 simultaneously outputs a recorded voice message stating "the simulated needle guard is identified by the FIRST COLOR flashing light shaped as the numeral one." Similarly, the second LED 272BA, the output of which is shaped as the numeral "2," can output a flashing light of a second color different than the first color while the speaker 274 simultaneously outputs a recorded voice message stating "the simulated safety lock is identified by the SECOND COLOR flashing light shaped as the numeral two." In this manner, the electronic circuit system 270 can provide both audible and visual instructions to assist the user in the operation of the simulated medicament delivery device 202.

In some embodiments, the electronic circuit system 270 can output at least one electronic output in response to a switch (not shown in FIG. 86) being moved between a first state and a second state. For example, similar to the needle guard assembly 4810 shown and described above with reference to FIG. 57, the simulated needle guard assembly 286 can include an actuator configured to actuate a switch contained within the electronic circuit system 270. The switch can be any suitable switch of the types shown and described above. For example, in some embodiments, a switch can be a "tear-through" switch configured to move irreversibly from a first state to a second state. In other embodiments, a switch can be a microswitch configured to repeatedly move between a first state and a second state. In this manner, the electronic circuit system 270 can output instructions when the user moves the simulated needle guard assembly 286 relative to the housing 285. Such instructions can state, for example, "You have now removed the needle guard assembly. The next step is to remove the safety lock. Please pull the safety lock as indicated by the flashing arrow." In a similar manner, the simulated safety lock 287 can include an actuator configured to actuate a switch contained within the electronic circuit system.

Although the simulated medicament delivery device 202 is shown as including a start button 271 to activate the electronic circuit system (not shown in FIG. 86), in other embodiments, an electronic circuit system 270 can be activated by any suitable means. For example, in some embodiments, the electronic circuit system can be activated by removing the simulated needle guard assembly 286, as described above with reference to FIG. 57. In other embodiments, the electronic circuit system 270 can be activated by removing the simulated safety lock 287, as described above with reference to FIG. 58. In yet other embodiments, the electronic circuit system 270 can be activated by removing the simulated medicament delivery device 202 from a container (not shown in FIG. 86), as shown and described above with reference to FIGS. 71 - 84.

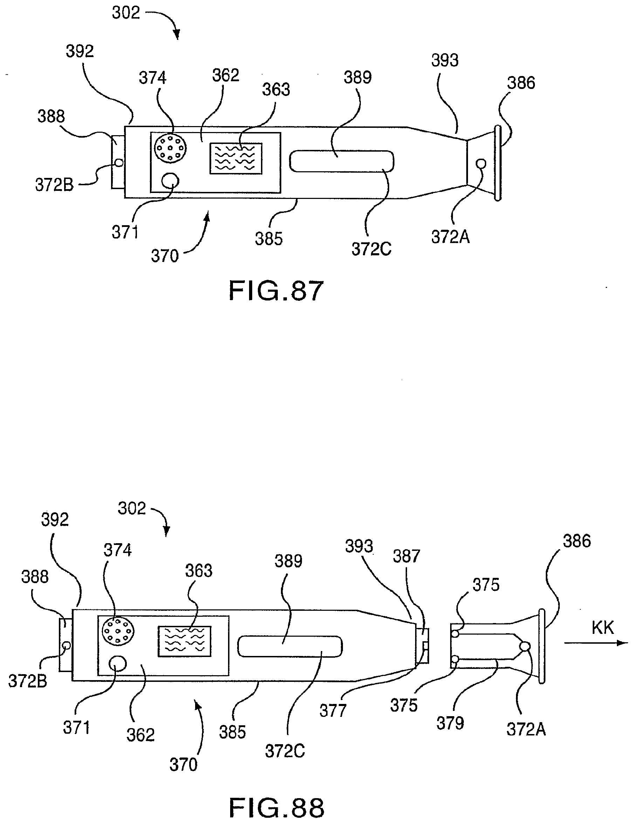

FIGS. 87 - 91 are front views of a simulated auto-injector 302 according to an embodiment of the invention. The simulated auto-injector 302 includes a housing 385 having a proximal end portion 392 and a distal end portion 393. The housing defines a window 389, which can, for example, simulate a status window of a corresponding actual auto-injector (not shown in FIGS. 87 - 91), as described below. A simulated needle guard assembly 386 is removably coupled to the distal end portion 393 of the housing 385. Similarly, a simulated safety lock 387 is disposed at the distal end portion 393 of the housing (see FIG. 88). The proximal end portion 392 of the housing 385 includes a simulated injector actuation button 388. The simulated injector actuation button 388 is configured to simulate an actuation button of the corresponding auto-injector.

The simulated auto-injector 302 includes an electronic circuit system 370 and a label 362. The label 362 can include a textual indicia 363 and can be any suitable label of the type described herein. In some embodiments, for example, the label 362 can include at least a portion of the electronic circuit system 370 (i.e., portions of an electronic conductor, portions of a printed circuit board, a battery, an LED or the like). In other embodiments, the label 362 can be devoid of any portion of the electronic circuit system 370.

The electronic circuit system 370 includes a start button 371, a speaker 374 and three visual output devices 372A, 372B and 372C. The visual output devices 372A, 372B and 372C can be, for example, LEDs, LCDs, organic polymer devices and/or fiber optic devices. The electronic circuit system 370 also includes a force sensor 377 (shown in FIG. 88) and a position sensor (not shown in FIGS. 87 - 91). The above described components can be electronically coupled together by any suitable mechanism, such as, for example a printed circuit board of the types shown and described herein.

As described above, to activate the electronic circuit system 370, the user pushes the start button 371. When actuated, the electronic circuit system 370 can output one or more electronic outputs. For example, in some embodiments, the first visual output device 372A can output a flashing light while the speaker 374 simultaneously outputs a recorded voice message stating "Please remove the simulated needle guard, which is at the end of the injector as indicated by the flashing light."

As illustrated by arrow KK in FIG. 88, the simulated needle guard 386 is removed by moving it along the longitudinal axis of the housing 385. When the simulated needle guard 386 is removed, the portion of the electronic circuit system 370 that includes the first visual output device 372A is no longer electronically coupled to the remainder of the electronic circuit system 370. Accordingly, the first visual output device 372A becomes deactivated when the simulated needle guard 386 is removed. Moreover, the terminals 375 of the electronic conductors 379 can form a portion of a switch, such that when the simulated needle guard 386 is removed, the switch changes from a first state to a second state, thereby prompting the electronic circuit system 370 to output an additional electronic output. For example, in some embodiments, the speaker 374 can output a recorded voice message stating "Please place the simulated auto-injector against your thigh. Do not tilt the simulated auto-injector. When in the proper position, please press firmly against the thigh before actuating the auto-injector."

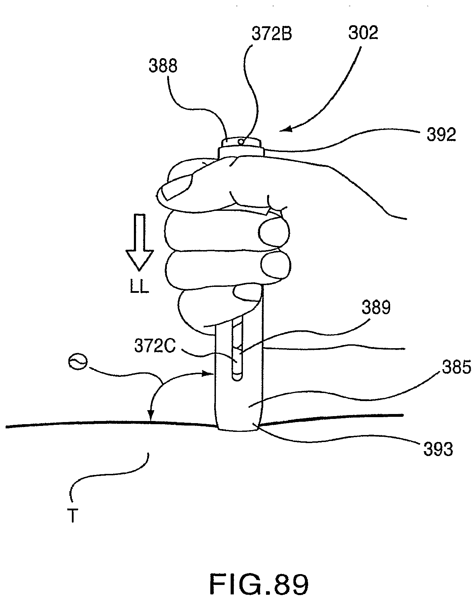

In addition to prompting the electronic circuit system 370 to output additional visual and/or audible outputs, the removal of the simulated needle guard 386 can also activate the position sensor (not shown in FIGS. 87 - 91). The position sensor can be any suitable sensor for sensing a position, location and/or orientation of the simulated auto-injector 302. For example, in some embodiments, the position sensor can be configured to sense the angle Θ between the longitudinal axis of the housing 385 and the surface of the target T (see FIG. 89). In other embodiments, the position sensor can be configured to sense the absolute angle of the longitudinal axis of the housing based on gravity. In yet other embodiments, the position sensor can be capacitance sensor, a temperature sensor, an optical sensor or any other suitable sensor for determining when the distal end 393 of the simulated medicament delivery device 302 is in contact with the target T. In this manner, the position sensor can provide feedback to the user to ensure that the simulated medicament delivery device 302 is correctly positioned relative to the target T.

Similarly, when the user presses the simulated medicament delivery device 302 against the target T, as shown by the arrow LL in FIG. 89, the force sensor 377 can sense the force and/or pressure between the target T and the simulated safety lock 387. In this manner, the force sensor 377 can provide feedback to the user to ensure that the simulated medicament delivery device 302 is pressed against the target T with sufficient force to move the safety lock of an actual medicament delivery device (not shown in FIGS. 87 -91). The force sensor 377 can also provide feedback to the user to ensure that the simulated medicament delivery device 302 is not pressed too firmly against the target T. The force sensor 377 can be any sensor suitable for sensing a force and/or pressure, such as for example, a strain-gauge load sensor, a piezo-electric sensor or the like.

In some embodiments, after the simulated medicament delivery device 302 is correctly positioned with sufficient force against the target T, the force sensor 377 can prompt the electronic circuit system 370 to output an additional electronic output or sequence of electronic outputs. For example, in some embodiments, the second visual output device 372B can output a flashing light while the speaker 374 simultaneously outputs a recorded voice message stating "The simulated auto-injector is now correctly positioned against your body. Please press the injector actuation button at the top of the auto-injector as indicated by the flashing light."

In some embodiments, the electronic circuit system 370 can include a timer (not shown in FIGS. 87 - 91) to determine the duration of any of the various operations discussed herein. In this manner, the electronic circuit system 370 can repeat a previous electronic output if no action has been sensed within a predetermined amount of time. For example, in some embodiments, the electronic circuit system 370 can repeat the electronic output prompting the user to remove the simulated needle guard 386 if a predetermined time period has elapsed after the start button 371 is pushed and before the simulated needle guard 386 is removed. In some embodiments, the electronic circuit system 370 can augment the electronic output prompting the user to remove the simulated needle guard 386 if a predetermined time period has elapsed after the start button 371 is pushed and before the simulated needle guard 386 is removed. The electronic output can be augmented, for example, by automatically increasing the volume of the audible output, changing the characteristics (e.g., the color, flash rate or the like) of the visual outputs or the like.

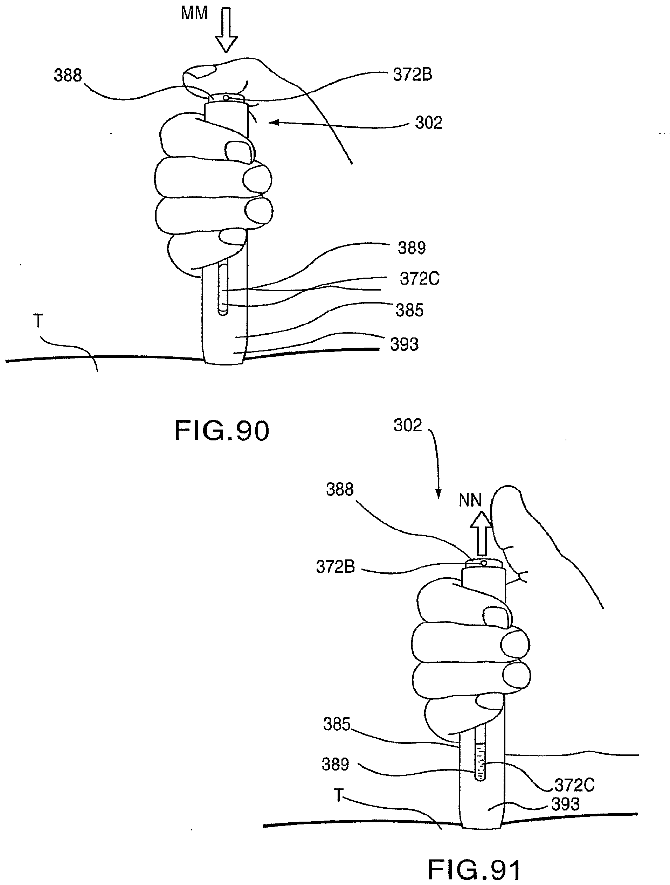

In other embodiments, the electronic circuit system 370 can output an electronic output to instruct the user to move to the next operation after a predetermined amount of time has elapsed. For example, in some embodiments, the speaker 374 can output a recorded voice message stating "Release the actuation button. Do not continue to hold the actuation button down" when the duration between when the user presses the simulated injector actuation button 388 (as shown by arrow MM in FIG. 90) and when the user releases the simulated injector actuation button 388 (as shown by arrow NN in FIG. 91) exceeds a predetermined duration. In this manner, the electronic circuit system 370 can provide feedback to the user to ensure that the simulated medicament delivery device 302 is used properly.

As shown in FIG. 91, the third visual output device 372C is visible through the window 389 defined by the housing 385. In some embodiments, the third visual output device 372C and the window 389 can collectively simulate a status window of the actual medicament delivery device (not shown in FIG. 91). For example, in some embodiments, the third visual output device 372C can gradually change color to simulate an associated color change of a status window that alerts a user when an actual injection is complete.

Although the simulated medicament delivery devices are shown and described as including external components and/or internal components to simulate actual medicament delivery devices, in some embodiments, a simulated medicament delivery device can be devoid of certain components, such as, for example, springs, actuation mechanisms or the like. For example, in some embodiments, a simulated medicament delivery device can include an electronic circuit system configured to output an electronic output to simulate any one of a tactile sensation, an audible sensation, a visual sensation, an olfactory sensation and/or a taste sensation associated with a use of the medicament delivery device. In this manner, the simulated medicament delivery device can simulate a medicament delivery device without mechanical components and/or medicament, which can be make the simulated medicament delivery device expensive, unsafe to use, difficult to use, difficult to reset for repeated use or the like.

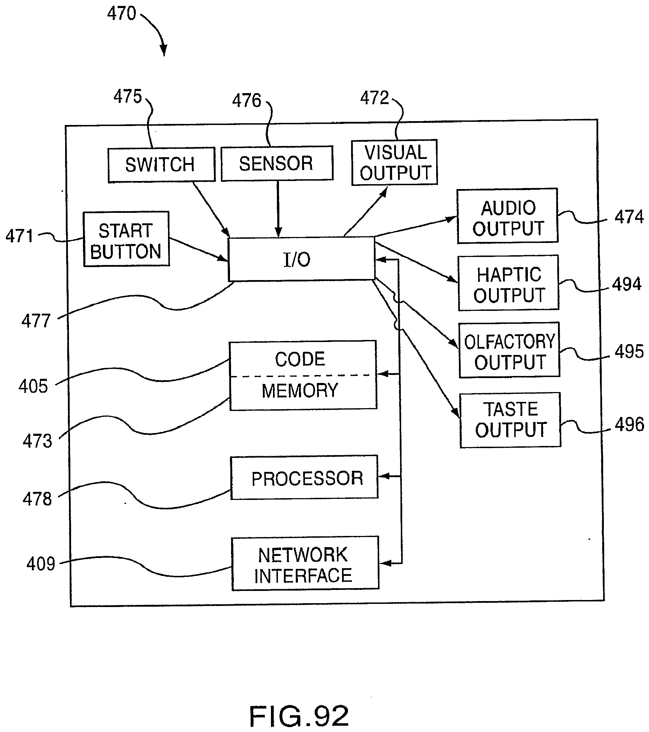

FIG. 92 is a schematic illustration of an electronic circuit system 470 according to an embodiment of the invention configured to cooperate with a housing (not shown in FIG. 92) to simulate a medicament delivery device (not shown in FIG. 92). The electronic circuit system 470 includes a processor 478 operatively coupled to a memory device 473, of the types shown and described above with reference to FIGS. 48 and 78. The memory device 473 can be configured to store processor-readable code 405 instructing the processor 478 to perform the functions described herein. In some embodiments, the processor-readable code 405 can be modified and/or updated as circumstances dictate.

The electronic circuit system 470 includes an input/output device 477 configured to receive electronic inputs from a switch 475 and/or a sensor 476, as described above. In some embodiments, the input/output device 477 can receive inputs from any suitable device, such as an RFID tag (as described above), the user's voice (e.g., through a microphone), a start button 471 or the like. The input/output device 477 is also configured to output electronic signals to various output devices, such as, for example, a visual output device 472, an audio output device 474, a haptic output device 494, an olfactory output device 495, a taste output device 496, a wireless receiver (e.g., an RFID tag, a cellular phone system or the like) and/or a wired receiver (e.g., a wired network).

The visual output device 472 can be any suitable device for producing visual indicia, such as, light-emitting diodes (LEDs), liquid-crystal display (LCD) screens, optical polymers, fiber optic components or the like. In this manner, the electronic circuit system 470 can simulate a particular visual feature of a medicament delivery device, such as, for example, a change in the color of a status window.

Similarly, the audio output device 474 can be any suitable device for producing sound, such as a micro-speaker, a piezo-electric transducer or the like. Such audible output can include, for example, an alarm, a series of beeps, recorded speech or the like. In this manner, the electronic circuit system 470 can simulate a particular audible feature of a medicament delivery device, such as, for example, a series of clicks associated with the actuation of the medicament delivery device and/or the delivery of the medicament.

The haptic output device 494 can be any suitable device for producing a haptic output, such as a vibrator, a piezo-electric device, a heater, a cooler or the like. In this manner, the electronic circuit system 470 can simulate a particular feel of a medicament delivery device. For example, in some embodiments, a simulated medicament delivery device can be configured to simulate a transdermal medicament delivery device by simulating the thermal feel of a medicament delivery area against the skin. In other embodiments, a simulated medicament delivery device can be configured to simulate an auto-injector by simulating the vibration associated with the actuation of the auto-injector.

The olfactory output device 495 can be any suitable device for producing a scent output. In this manner, the electronic circuit system 470 can simulate a particular smell associated with a medicament delivery device. For example, in some embodiments, a simulated medicament delivery device can be configured to simulate an inhaler by simulating the smell of a medicament as it is being delivered orally.

Similarly, the taste output device 495 can be any suitable device for producing a simulated taste. In this manner, the electronic circuit system 470 can simulate a particular taste associated with a medicament delivery device. For example, in some embodiments, a simulated medicament delivery device can be configured to simulate an inhaler by simulating the taste of a medicament as it is being delivered orally.

In some embodiments, the electronic circuit system 470 can include a network interface 409 configured to operatively couple the electronic circuit system 470 to a remote device (not shown in FIG. 92), as described above. The network interface 409 can also be configured to transmit information from the electronic circuit system 470 to a central network, such as, for example, a doctor's office, as described above.

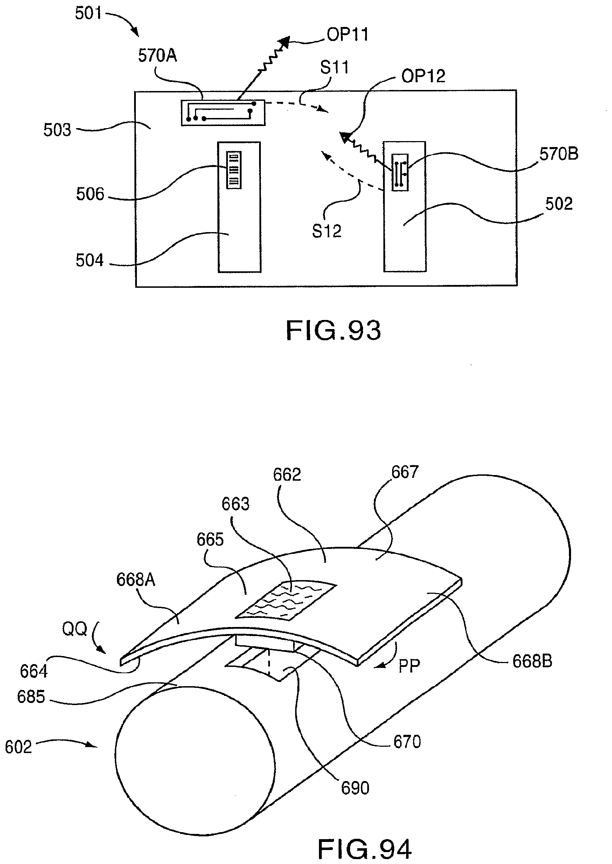

In some embodiments, a simulated medicament delivery device can be included in a kit. FIG. 93 is a schematic illustration of a medical device 501 according to an embodiment of the invention. The medical device 501 includes a container 503, a medicament delivery device 504 and a simulated medicament delivery device 502. The container 503, which includes a first electronic circuit system 570A, can be similar to the containers shown and described above with reference to FIGS. 71 - 84. The medicament delivery device 502, which includes a label 506 can be similar to the medicament delivery devices shown and described herein. Similarly, the simulated medicament delivery device 502, which includes a second electronic circuit system 570B, can be similar to the simulated medicament delivery devices shown and described above with reference to FIGS. 85 - 92.

The first electronic circuit system 570A can output an electronic output OP11, of the type described above, when a user presses a start button (not shown in FIG. 93), when the container 503 is opened, when the medicament delivery device 504 is removed from the container and/or when the simulated medicament delivery device 502 is removed from the container. Moreover, the label 506 can contain information associated with the medicament delivery device 504 in a machine-readable format. Accordingly, the first electronic circuit system 570A can receive (e.g., "read") the information contained in the label 506 and include at least a portion of the information in the electronic output OP11. In this manner, as described above, the first electronic circuit system 570A can be configured to produce an electronic output OP11 that is unique to the medicament delivery device 504 contained within the container 503. For example, in some embodiments, the electronic output OP11 can notify a user when the medicament delivery device 504 has been removed from the container 503 and alert the user to the presence of the simulated medicament delivery device 502.

Similarly, the second electronic circuit system 570B can output an electronic output OP12, of the type described above, when a user presses a start button (not shown in FIG. 93), when the simulated medicament delivery device 502 is removed from the container 503 or the like. Moreover, similar to the function of the first electronic circuit system 570A, the second electronic circuit system 570B can also receive (e.g., "read") the information contained in the label 506 and include at least a portion of the information in the electronic output OP12. For example, in some embodiments, the electronic output OP11 can notify a user of a status (e.g., the dosage, expiration date or the like) of the medicament delivery device 504.

In some embodiments, the second electronic circuit system 570B can output a signal S12 that can be received by the first electronic circuit system 570A. In some embodiments, the signal S12 can indicate that the simulated medicament delivery device 502 has been removed from the container 503. In other embodiments, the signal S12 can include information associated with the use of the simulated medicament delivery device 502 and/or the medicament delivery device 504. For example, in some embodiments, the signal S12 can be associated with an identification of the simulated medicament delivery device 502, an identification of certain components of the simulated medicament delivery device 502 and/or a status of the simulated medicament delivery device 502, as described above. In this manner, the first electronic circuit system 570A can receive the signal S12 and produce the electronic output OP11 to include information contained within the signal S12. Said another way, this arrangement allows the first electronic circuit system 570A and the second electronic circuit system 570B to cooperatively output the electronic output OP11. For example, in some embodiments, the simulated medicament delivery device 502 can output a signal S12 that prompts the first electronic circuit system 570A to augment the electronic output OP12 (e.g., by displaying an output on a larger LCD screen or the like) previously output by the simulated medicament delivery device 502.

Similarly, in some embodiments, the first electronic circuit system 570A can output a signal S11 that can be received by the second electronic circuit system 570B. In some embodiments, the signal S11 can include, for example, updated use instructions that have been received by the first electronic circuit system 570A (e.g., via a wireless network). As described above, the second electronic circuit system 570B can receive the signal S11 and produce the electronic output OP12 to include information contained within the signal S11. This arrangement allows the first electronic circuit system 570A and the second electronic circuit system 570B to cooperatively to produce the electronic output OP12.

Although the first electronic circuit system 570A and the second electronic circuit system 570B are each shown and described as being configured to output at least an electronic output (e.g., OP11 and OP12, respectively) and a signal (e.g., S11 and S12, respectively), the use of separate terms is made for clarity. Accordingly, there is no distinction between signals and electronic outputs.

Although the medicament delivery device 504 is shown and described as including a label 506 containing information associated with the medicament delivery device 504 in a machine-readable format, in some embodiments, the medicament delivery device can include its own electronic circuit system. In such embodiments, the electronic circuit system of the medicament delivery device can cooperate with the first electronic circuit system 570A and/or the second electronic circuit system 570B to produce various electronic outputs, as described above.

In some embodiments the medical device 501 can include a simulated target (not shown in FIG. 93) to simulate a portion of a body for use with the simulated medicament delivery device 502. In some embodiments, the simulated target can be a skin pad that simulates a portion of a thigh or an arm. In other embodiments, the simulated target can be a strap or band that is placed around a portion of the user's body to provide a target for use with the simulated medicament delivery device 502 and/or the medicament delivery device 504. In some embodiments, a simulated target can include its own electronic circuit system. In such embodiments, for example, the simulated target can include one or more LEDs to provide a visual indication of a location for receiving a medicament, a force sensor to sense the force and/or pressure between the simulated target and the simulated medicament delivery device 502, or the like.

Although the labels are shown and described above as including a portion of an electronic circuit system and/or securing an electronic circuit system to an outer portion of a simulated medicament delivery device, in some embodiments, a label and a housing of a simulated medicament delivery device can cooperatively contain an electronic circuit system. For example, FIG. 94 is a perspective view of a simulated medicament delivery device 602 according to an embodiment of the invention. The simulated medicament delivery device 602 includes a housing 685, an electronic circuit system 670 and a label 662.

The label 662 includes a first surface 664 and a second surface 665. The first surface 664 is configured to be coupled to the housing 685 of the simulated medicament delivery device 602. In some embodiments, for example, the first surface 664 can include an adhesive to secure the label 662 to the housing 685. The second surface 665 includes an indicia 663, which can be for example, a textual indicia (e.g., a name of the device, use instructions or the like) or a symbolic indicia (e.g., an arrow pointing to a start button). Although the first surface 664 is shown as being opposite the second surface 665, in other embodiments, the first surface 664 and the second surface 665 can be adjacent each other and/or co-planar.

The label 662 also includes a rigid portion 667 disposed between two flexible portions 668A and 668B. The flexible portions 668A and 668B are configured to conform to the surface of the housing 685, as shown by the arrows PP and QQ in FIG. 94. The rigid portion 667 includes the electronic circuit system 670. The rigid portion 667 can be constructed from any suitable material, such as, for example, plastic, that can protect the electronic circuit system 670. Conversely, the flexible portions 668A and 668B can be constructed from any suitable flexible material, such as, for example, paper, flexible foam, Mylar®, Kapton® or the like. This arrangement allows the label 662 to be wrapped around the housing 685 to securely couple the electronic circuit system 670 within an opening 690 defined by the housing 685. Said another way, the label 662 and the housing 685 cooperatively define an enclosed region 690 within which at least a portion of the electronic circuit system 670 can be disposed.

Although the electronic circuit systems are shown and described above as outputting a single electronic output in response to an input (e.g., the movement of a safety lock, pressing a start button, the removal of a medicament delivery device, the change in position of a hinged lid, etc.), in some embodiments, an electronic circuit system can output a sequence of electronic outputs in response to such an input. In some embodiments, for example, when a medicament delivery device is removed from a container, an electronic circuit system disposed on the medicament delivery device and/or container can output a predetermined sequence of use instructions over a predetermined time period. For example, upon removing the medicament delivery device, the first instruction can be an audible output indicating the type of medicament delivery device removed. After a predetermined time period, the electronic circuit system can then output a second instruction, which can be a visual output instructing the user in how to diagnose the patient and/or prepare the patient for the medicament. In a similar manner, the electronic circuit system can provide additional outputs to instruct the user in the use of the medicament delivery device. Moreover, in some embodiments, the electronic circuit system can output an electronic output instructing the user in post-use procedures, such as for example, the disposal of the medicament delivery device, instructions for follow-up treatment or the like.

Although the electronic circuit systems are shown and described above as outputting recorded speech in English, in other embodiments, the electronic circuit system can output recorded speech in any language. In yet other embodiments, the electronic circuit system can output recorded speech in multiple languages. In yet other embodiments, the user can select the language in which the recorded speech is to be output.

For example, although electronic circuit systems are shown and described above as outputting one or more outputs directed towards a single, immediate user, in some embodiments, an electronic circuit system can output multiple outputs directed towards multiple different classes of users. For example, in some embodiments, an electronic circuit system can output a first electronic output to the immediate user and second electronic output to a remotely located emergency response team. In such embodiments, the second electronic output can be, for example, a phone call, a page, an e-mail or the like. For example, in some embodiments, the second electronic output can be an e-mail to the parents and/or caregivers of a child. Moreover, such a second electronic output can be transmitted either wirelessly or through a wired network.

Although the electronic circuit systems are shown and described above as outputting one or more outputs in response to one or more switches, in other embodiments an electronic circuit system can output an electronic output in response to any number of different inputs. For example, in some embodiments, an electronic circuit system can output an electronic output based on input from the user provided via a keyboard, a touch screen, a microphone or any other suitable input device. In this manner, the electronic outputs can be produced in response to direct feedback from the user.

Some embodiments of the invention relate to a computer storage product with a computer-readable medium having instructions or computer code thereon for performing various computer-implemented operations. The media and computer code may be those specially designed and constructed for the purposes of the invention, or they may be of the kind well known and available to those having skill in the computer software arts. Examples of computer-readable media include, but are not limited to: magnetic storage media such as hard disks, floppy disks, and magnetic tape; optical storage media such as Compact Disc/Digital Video Discs ("CD/DVDs"), Compact Disc-Read Only Memories ("CD-ROMs"), and holographic devices; magneto-optical storage media such as floptical disks; carrier wave signals; and hardware devices that are specially configured to store and execute program code, such as Application-Specific Integrated Circuits ("ASICs"), Programmable Logic Devices ("PLDs"), and ROM and RAM devices. Examples of computer code include, but are not limited to, micro-code or micro-instructions, machine instructions, such as produced by a compiler, and files containing higher-level instructions that are executed by a computer using an interpreter. For example, an embodiment of the invention may be implemented using Java, C++, or other object-oriented programming language and development tools. Additional examples of computer code include, but are not limited to, control signals, encrypted code, and compressed code.

Although various embodiments have been described as having particular features and/or combinations of components, other embodiments are possible having a combination of any features and/or components from any of embodiments where appropriate. For example, in some embodiments a medical device can include a container including an electronic circuit system, two or more medicament delivery devices and a movable portion. In such embodiments, each of the medicament delivery devices can be associated with a switch. Moreover, the movable portion can also be associated with a switch. In this manner, the electronic circuit system can be configured to output a first electronic output when the movable portion is moved, a second electronic output when the first medicament delivery device is removed from the container and a third electronic output when the second medicament delivery device is removed from the container

Although the simulated medicament delivery devices and the actual medicament delivery devices are shown and described above as being separate, in some embodiments a single device can contain certain features and perform certain functions of both the a simulated medicament delivery device and an actual medicament delivery device. For example, in some embodiments, a medicament delivery device can be moved between a simulation configuration and a medicament delivery configuration. For example, in some embodiments, a simulated medicament delivery device can be configured to receive an actual medicament delivery device to subsequently move from a simulation configuration to a medicament delivery configuration.

In some embodiments, an apparatus includes a housing and a medicament container disposed within the housing. The medicament container includes a first plunger and a second plunger, each disposed therein. The medicament container has a first configuration and a second configuration. In the first configuration, the first plunger is disposed in a first position within the medicament container and the second plunger is disposed in a second position spaced apart from the first position by a first distance. Accordingly, a first medicament containing portion is defined between the first plunger and the second plunger, which can contain, for example, a liquid medicament. A second medicament containing portion is defined between the second plunger and a distal end of the medicament container, which can contain, for example, a solid medicament. In the second configuration, the first plunger is disposed in the first position within the medicament container and the second plunger is disposed in a third position spaced apart from the first position by a second distance. The second distance is less than the first distance. A volume of the second medicament containing portion is greater when the medicament container is in the second configuration than when the medicament container is in the first configuration.

In some embodiments, an apparatus includes a housing, a medicament container disposed within the housing, a first movable member and a second movable member. The medicament container has a first plunger disposed within a proximal end portion of the medicament container and a second plunger disposed therein spaced apart from the first plunger. The first movable member is configured to move the first plunger within the medicament container toward a distal end of the medicament container. The second movable member is configured to move the second plunger within the medicament container toward the proximal end portion of the medicament container. In some embodiments, for example, the second movable member can be configured to move the second plunger without moving the first plunger.

In some embodiments, an apparatus includes a housing, a medicament container disposed within the housing, a first energy storage member and a second energy storage member. The medicament container has a first plunger disposed within a proximal end portion of the medicament container and a second plunger disposed therein spaced apart from the first plunger. The first energy storage member, which can be, for example, a compressed gas container, is configured to produce a force when moved from a first configuration to a second configuration to move the first plunger within the medicament container. The second energy storage member, which is different from the first energy storage member and can be, for example, as spring, is configured to produce a force when moved from a first configuration to a second configuration to move the second plunger within the medicament container.

In some embodiments, an apparatus includes a housing, a medicament container disposed within the housing, and a movable member. The medicament container has a first plunger disposed within a proximal end portion of the medicament container and a second plunger disposed therein such that the medicament container is divided into a first medicament containing portion and a second medicament containing portion. The movable member is configured to move the second plunger within the medicament container to mix a medicament contained in the first medicament containing portion with a medicament contained in the second medicament containing portion. The movable member is offset from a longitudinal axis of the medicament container.

In some embodiments, a method includes moving a mixing plunger within a medicament container toward a proximal end of the medicament container. The mixing plunger is disposed within the medicament container between a distal end of the medicament container and an injection plunger disposed at the proximal end of the medicament container. The injection plunger is moved within the medicament container toward a distal end of the medicament container to expel a medicament contained within the medicament container.

As used in this specification and the appended claims, the term "medicament" includes any constituent of a therapeutic substance. A medicament can include such constituents regardless of their state of matter (e.g., solid, liquid or gas). Moreover, a medicament can include the multiple constituents that may be included in a therapeutic substance in a mixed state, in an unmixed state and/or in a partially mixed state. A medicament can include both the active constituents and inert constituents of a therapeutic substance. Accordingly, as used herein, a medicament can include non-active constituents such as, water, colorant or the like.

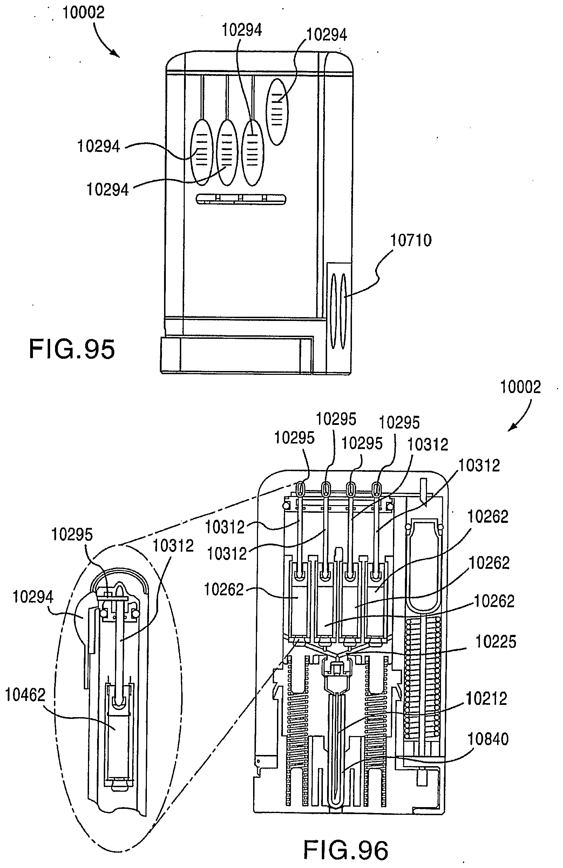

FIGS. 95 and 96 show a compact auto-injector 10002 that includes a plurality of vials 10262, allowing for multiple medicaments to be injected at one time or at different times. The auto-injector 10002 also can comprise a needle protection system 10840.

The auto-injector 10002 can include medicament selectors 10294 in order to allow the user to select which medicament to inject. The user can select the medicaments by sliding one or more selectors 10294 upward into their final position. An audible click or some other indicator may occur to alert the user to this final position. Moving the selector 10294 or multiple selectors 10294 upwards can allow a pin 10295 to snap into the plunger rod 10312 and/or into the pusher bar (not shown in FIG. 96), which can create an entire portion that can push the vial system downwards and can inject the medication through the vial 10262, the reservoir 10225 and/or needle 10212. This method can also be used with a needleless injector method as shown and described earlier in U.S. Patent Application Serial No. 10/572,148, entitled "Devices, Systems and Methods for Medicament Delivery," filed March 16, 2006. Methods such as this embodiment could be extremely useful in applications for anti-nerve agents or pain therapies. The device can also include a resilient material, such as rubber, to seal the selector openings and that can also slide within the housing once the selector is pushed upward. Once the aforementioned pins 10295 are in place, the device 10002 can function and activate as described in U.S. Patent Application Serial No. 11/562,061, entitled "Devices, Systems and Methods for Medicament Delivery," filed November 21, 2006. In some embodiments, a safety mechanism 10710 can be modified to eliminate the sliding selectors from being prematurely pushed upwards.

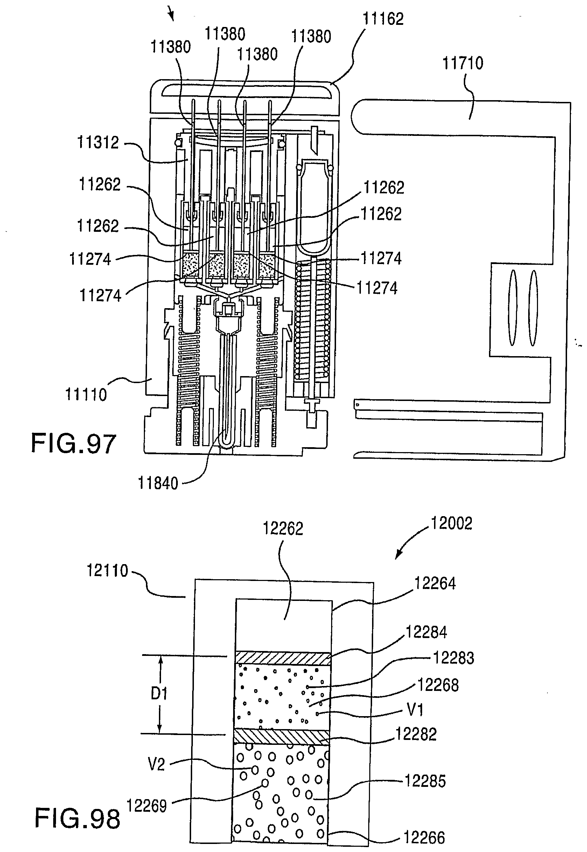

FIG. 97 depicts an apparatus and method for injecting lyophilized medications, and/or powdered biologics that could need to be reconstituted pre-injection. FIG. 97 shows an auto-injector 11002 including a mechanism to mix and/or create an injectable medicament from two or more separate aforementioned substances. The auto-injector 11002 includes multiple vials 11262 that could have two substances in each vial 11262 separated by a pierceable membrane 11274 and/or other frangible piece. The vials 11262 in this embodiment can have one wet substance (such as sterilized water) and one dry substance (such as glucagon powder).

The user can take off the safety tab 11710 which can prevent the user from accidental injection and/or pre-mature activation of the device. Once the safety tab 11710 is removed, the user can twist and/or rotate the twisting portion 11162 at the top of the housing 11110. By rotating this top portion 11162, the rods 11380 attached to this portion (which can be threaded rods) can move downward. The rods 11380 can be located in the vials 11262 and/or through the pusher bar 11312. The rods 11380 can have a sharp piercing portion on the distal end which can aid in puncturing the aforementioned pierceable membrane 11274 that can separate the substances in the vial 11262. Once the piercing rod 11380 punctures the frangible seal and/or pierceable membrane 11274, the substances can mix together to form one medicament. The user can also shake the entire housing 11110 in order to aid in this mixing process. Accordingly, this embodiment can comprise a compact auto-injector 11002 that can have the ability to mix two or more medicaments in either a liquid or powder form to create one injectable medicament. The device also can comprise a needle protection system 11840.

An exemplary delivery system can comprise a housing, plurality of vials, a plunger for each vial, a mixing activation mechanism, an activation chamber or vial, single needle or needle cannula, and/or a medicament or medicaments stored within each vial. Pre-injection, two or more medicaments can be stored separately in a vial and/or storage compartment and can communicate with each other once the mixing activation mechanism is initialized. The mixing activation mechanism could comprise a button, trigger, threaded rod, and or some other member that removes a piece or portion and/or punctures a piece or portion that is preventing each medicament to communicate with each other. The mixing activation mechanism may comprise a membrane, piece, and/or portion that may be removed pre-injection by the user in order to allow the separate vials and/or storage containers to communicate with each other. The mixing activation mechanism can be a piece that is manipulated in some way by the user in order to cause the contents of each compartment to mix with each other. This communication may occur by shaking the device and/or may occur automatically with the mixing activation mechanism. For instance, the mixing activation mechanism may cause each medicament to be released into an activation chamber, which may itself be a separate vial. This mixed medicament can be the medicament that will be injected into the patient.

FIGS. 98 - 101 are schematic illustrations of a medical device 12002 according to an embodiment of the invention in a first configuration, a second configuration, a third configuration and a fourth configuration, respectively. The medical device 12002 can be any suitable device for delivering a medicament into a body, such as for example, a syringe, a medical injector, an auto-injector or the like. The medical device 12002 includes a housing 12110 that contains a medicament container 12262. The medicament container 12262 has a proximal end portion 12264 and a distal end portion 12266. The medicament container 12262 includes a first plunger 12284 and a second plunger 12282.

As shown in FIG. 98, when the medical device 12002 is in the first configuration, the first plunger 12284 is disposed in a first position within the medicament container 12262. The second plunger 12282 is disposed in a second position within the medicament container 12262. The second position is spaced apart from the first position by a first distance D1. In this manner, a first medicament containing portion 12283 having a volume V1 is defined between the first plunger 12284 and the second plunger 12282. Similarly, a second medicament containing portion 12285 having a volume V2 is defined between the second plunger 12282 and the distal end portion 12266 of the medicament container 12262. In some embodiments, the medicament container 12262 can be a cartridge, a vial, an ampule, or the like that is filled with one or more medicaments. For example, in some embodiments, the first medicament containing portion 12283 can include a liquid medicament 12268, such as a water, and the second medicament containing portion 12285 can include a second medicament 12269, such as a lyophilized powder. Similarly, in some embodiments, the first medicament containing portion 12283 can include a liquid medicament 12268 that is devoid of a gas (e.g., stored in a vacuum) and/or the second medicament containing portion 12285 can include a solid medicament 12269 that is devoid of a gas (e.g., stored in a vacuum).

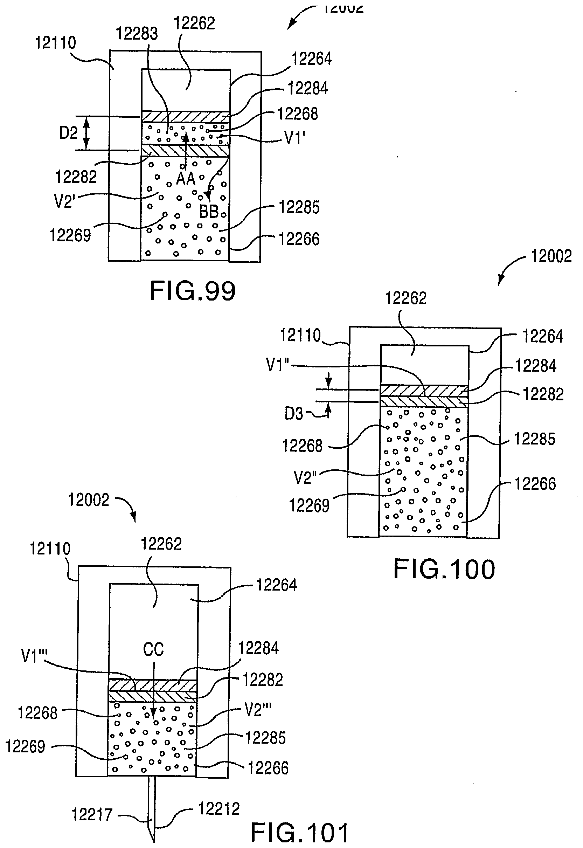

As shown in FIG. 99, when the medical device 12002 is in the second configuration, the first plunger 12284 remains disposed in the first position within the medicament container 12262. The second plunger 12282 is disposed in a third position within the medicament container 12262. The third position is spaced apart from the first position by a second distance D2 that is less than the first distance D1. Said another way, when the medical device 12002 is in the second configuration, the volume V2' of the second medicament containing portion 12285 is greater than the volume V2 when the medicament container 12262 is in the first configuration. Similarly, because the first plunger 12284 remains disposed in the first position, the total volume of the medicament container 12262 when the medical device 12002 is in the first configuration (V1 + V2) is the same as the total volume of the medicament container 12262 when the medical device 12002 is in the second configuration (V1' + V2'). Said another way, the area between the first plunger 12284 and the distal end portion 12266 defines a constant volume when the second plunger 12282 moves from its first position to its second position

In some embodiments, when the medical device 12002 is moved from the first configuration to the second configuration, as indicated by the arrow AA in FIG. 99, a portion of the first medicament 12268 contained in the first medicament containing portion 12283 can be conveyed to the second medicament containing portion 12285, as indicated by the arrow BB in FIG. 99. Said another way, when the second plunger 12282 moves towards the proximal end 12264 of the medicament container 12262, a portion of the contents of the first medicament containing portion 12283 can be conveyed to the second medicament containing portion 12285. In this manner, the medical device 12002 can combine and/or mix the first medicament 12268 with the second medicament 12269 contained in the second medicament containing portion 12285 to produce a mixture suitable for delivery via the medical device 12002.

In some embodiments, the first medicament containing portion 12283 can be fluidically isolated from the second medicament containing portion 12285 when the medical device 12002 is in the first configuration. The first medicament containing portion 12283 can be in fluid communication with the second medicament containing portion 12285 when the medical device 12002 is moving from the first configuration to the second configuration. In some embodiments, for example, the second plunger 12282 can form a fluid-tight seal within the medicament container 12262 when the medicament container 12262 is in the first configuration. The second plunger 12282 and/or the medicament container 12262 can further be configured to allow fluid communication between the first medicament containing portion 12283 and the second medicament containing portion 12285 when the second plunger 12282 is moving from its first position to its second position. In this manner, the medical device 12002 can be configured to store the first medicament 12268 (e.g., a liquid medicament) separately from the second medicament 12269 (e.g., a lyophilized powder) until such time as the first medicament 12268 and the second medicament 12269 are combined and/or mixed in preparation for delivery into the body.

As shown in FIG. 100, when the medical device 12002 is in the third configuration, the first plunger 12284 remains disposed in the first position within the medicament container 12262. The second plunger 12282 is disposed in a fourth position within the medicament container 12262. The fourth position is spaced apart from the first position by a third distance D3 that is less than the second distance D2. Moreover, when the second plunger 12282 is in the fourth position, a portion of the second plunger 12282 is in contact with a portion of the first plunger 12284. In some embodiments, when the second plunger 12282 is in the fourth position, it engages the first plunger 12284 such that there is no space between the first plunger 12284 and the second plunger 12282 (i.e., D3 is zero). Said another way, when the medical device 12002 is in the third configuration, the volume VI" of the first medicament containing portion 12283 is substantially zero. Moreover, the volume V2" of the second medicament containing portion 12285 is substantially equal to the total volume of the medicament container 12262 when the medical device 12002 is in the first configuration (V1 + V2). In this manner, when the medical device 12002 is in the third configuration, substantially all of the first medicament 12268 and substantially all of the second medicament 12269 are contained within the second medicament containing portion 12285. The first medicament 12268 and the second medicament 12269 can be contained within the second medicament containing portion 12285 in any suitable form. For example, in some embodiments, when the medical device 12002 is in the third configuration, the first medicament 12268 and the second medicament 12269 can form a non-homogenous mixture, a homogeneous mixture, a solution, a suspension and/or a combination.

As shown in FIG. 101, when the medical device 12002 is in the fourth configuration, the first plunger 12284 is disposed in a fifth position within the medicament container 12262. The second plunger 12282 is disposed in a sixth position within the medicament container 12262, such that a portion of the second plunger 12282 is in contact with a portion of the first plunger 12284. Moreover, when the medical device 12002 is in the fourth configuration, the volume V1''' of the first medicament containing portion 12283 is substantially zero and the volume V2''' of the second medicament containing portion 12285 is less than the total volume of the medicament container 12262 when the medical device 12002 is in the first configuration (V1+ V2). Said another way, when the medical device 12002 is in the fourth configuration, the first plunger 12284 and the second plunger 12282 are collectively moved distally within the medicament container 12262 as indicated by the arrow CC in FIG. 101. In this manner, the first medicament 12268 and the second medicament 12269 can be collectively expelled from the distal end portion 12266 of the medicament container 12262.

As shown in FIG. 101, in some embodiments, the medical device 12002 includes a needle 12212 that can be disposed at the distal end portion 12266 of the medicament container. When the medical device 12002 is in the first configuration, the second configuration and/or the third configuration, a lumen 12217 defined by the needle 12212 is fluidically isolated from the first medicament containing portion 12283 and/or the second medicament containing portion 12285. When the medical device 12002 is in the fourth configuration, the a lumen 12217 defined by the needle 12212 is in fluid communication with the first medicament containing portion 12283 and/or the second medicament containing portion. In this manner, when the medical device 12002 is moving between the first configuration, the second configuration and the third configuration, the first medicament 12268 and the second medicament 12269 can be combined and/or mixed without the first medicament 12268 and/or the second medicament 12269 being expelled from the medicament container. Similarly, when the medical device 12002 is in the fourth configuration, the first medicament 12268 and the second medicament 12269 can be collectively injected into a body via the needle 12212.

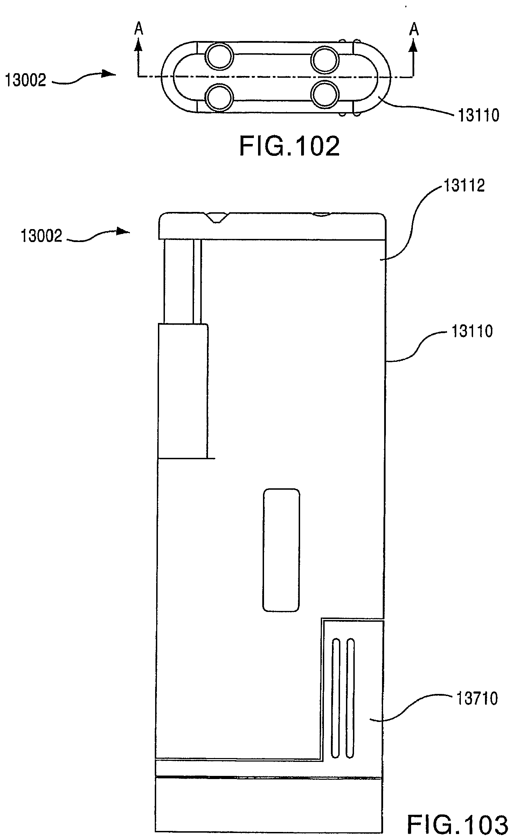

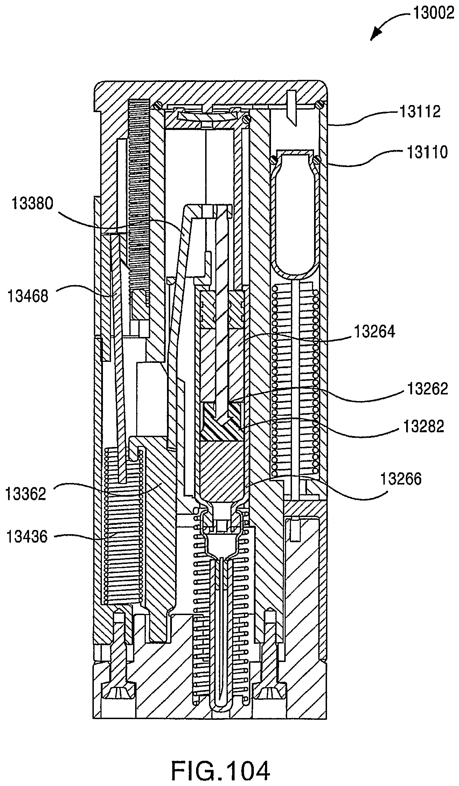

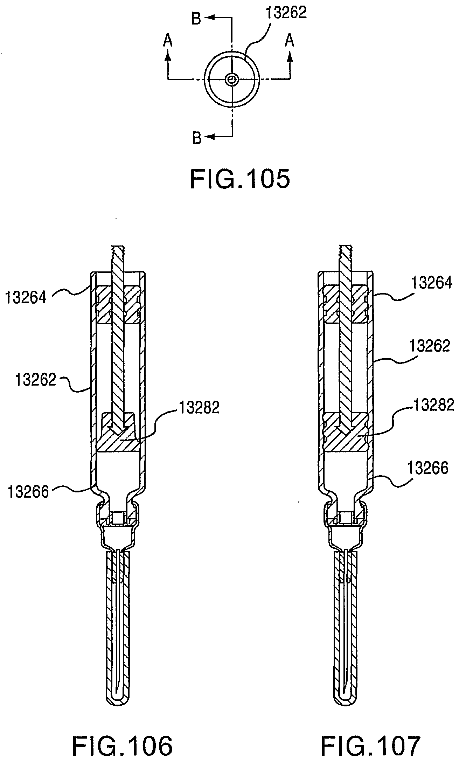

FIGS. 102 - 104 depict an apparatus for injecting lyophilized medications, and/or powdered biologics that could need to be reconstituted pre-injection. FIGS. 102 -104 show an auto-injector 13002 to mix and/or create an injectable medicament from two or more separate aforementioned substances. FIGS. 105 - 107 depict a single vial 13262 that could have two substances in the vial 13262 separated by a plunger 13282 and/or other frangible piece that may be located in the auto-injector 13002 shown in FIGS. 102 -104. The vial 13262 in this embodiment can contain one wet substance (such as sterilized water) and one dry substance (such as glucagon powder).

To mix the substances, the user can push a button or trigger 13468 at the side of the housing 13110 to initiate the reconstitution and mixing of the two substances. By pushing this button or trigger 13468 , a spring 13436 can be activated and forced toward the proximal end 13112 of the housing 13110. This spring 13436 can be attached to a solid member 13362 which can be attached to a member 13380 connected to the plunger 13282 or membrane in the vial 13262. As the spring 13436 is forced upward, the solid member 13362 and therefore plunger/membrane 13282 can be forced upward toward the proximal end 13264 of the vial 13262. This force can cause the wet substance to bypass the plunger/membrane 13282, moving down toward the distal end 13266 of the vial 13262 and can thereby cause the wet substance to mix with the dry substance forming the new injectable medicament. The user can shake the entire housing 13110 in order to aid in this mixing process. The user can then take off the safety tab 13710 located at the base of the auto-injector 13002 and operate the auto-injector 13002.

Some embodiments can include a method for reconstitution with an auto-injector. Some embodiments can comprise a compact auto-injector that can have the ability to mix two or more medicaments in either a liquid or powder form to create one injectable medicament. Some embodiments can use a secondary activation mechanism in order to effectively mix the two substances to form one injectable medicament. The device can comprise a needle protection system.

An exemplary delivery system can comprise a housing, vial or plurality of vials, plunger for each vial, a mixing activation mechanism, an activation chamber or vial, single needle or needle cannula, and/or a medicament or medicaments stored within each vial. Pre-injection, two or more medicaments can be stored separately in a vial and/or storage compartment and can communicate with each other once the mixing activation mechanism is initialized.

The mixing activation mechanism could comprise a button, trigger, plunger, spring and or some other member or combination of members that, once activated, can cause the two or more medicaments to interact with each other to form one injectable medicament.

The mixing mechanism can comprise a membrane, piece, plunger and/or portion that can be manipulated pre-injection by the user using the aforementioned mixing activation mechanism in order to allow the separate substances to fluidly communicate with each other. For instance, the mixing activation mechanism can activate a spring attached to solid member that is attached to a plunger in one vial. This plunger can be separating two substances (one wet and one dry) within one vial by being placed in between the two substances. The activated spring can push the solid member and plunger upwards toward the proximal end of the aforementioned vial, which can force the wet part past the plunger to interact with the dry part, forming the newly mixed injectable medicament.

The delivery system can further encompass the mixed medicament vial or plurality of mixed medicament vials in fluid communication with a reservoir that can contain a single needle or needle cannula at the distal end. The needle can be protected by some sheath/ shield.

The housing can further comprise a passage that is in fluid communication with the proximal end of the plunger such that when the spring(s) is activated from the distal or proximal end, a force can be applied through the passage on the plunger at the proximal end allowing for the plunger(s), vial(s), reservoir, and/or needle to travel towards the distal end of the housing. The force provided can be caused by a spring, bar, contents from a gas cylinder, and/or other force mechanism. The plunger can slideably travel through the vial towards the distal end to allow for the appropriate dose of medicament to be delivered. Upon exit of the desired contents of the vial, the entire needle, reservoir, vial, and/or plunger assembly can retract towards the proximal end of housing by some means such as a wire, spring, o-ring, and/or rubber membrane and/or a needle protection portion slides over the needle following delivery of the medicament.

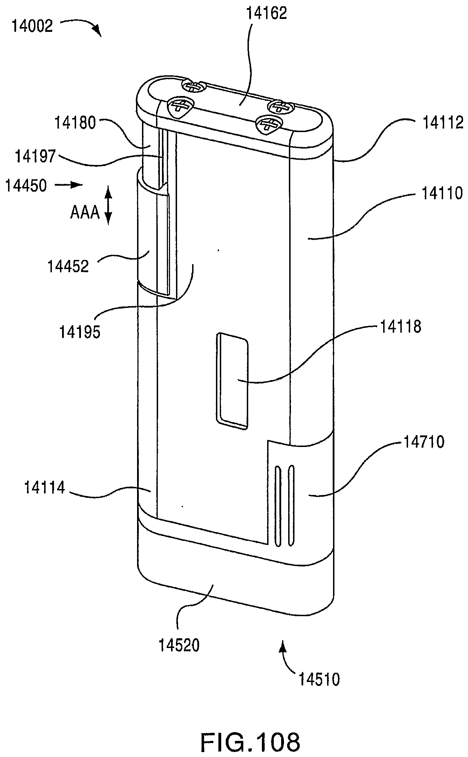

FIG. 108 is a perspective view of an auto-injector 14002 according to an embodiment of the invention in a first configuration. The auto-injector 14002 includes a housing 14110 having a proximal end portion 14112 and a distal end portion 14114. A proximal cover 14162 is disposed at the proximal end portion 14112 of the housing 14110. A mixing actuator 14450 is disposed adjacent the proximal end portion 14112 at a side portion 14195 of the housing 14110. A safety cover 14452 is slidably coupled to the side portion 14195 of the housing 14110. Similarly, an injection actuator 14510, which includes a base 14520, is disposed at the distal end portion 14114 of the housing 14110. An actuation safety lock 14710 is removably coupled adjacent the distal end portion 14114 of the housing 14110. The housing 14110 also includes a transparent status window 14118 to allow a user to determine the status of the auto-injector 14002 and/or the medicament contained therein.

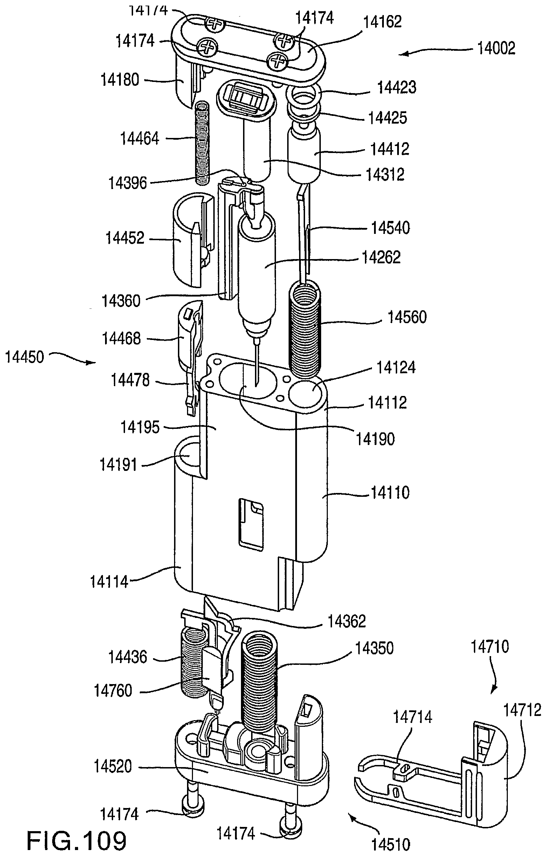

To inject a medicament into the body, the safety cover 14452 is moved towards the proximal end portion 14112 of the housing 14110 to uncover the mixing actuator button 14468 (see FIG. 109). The mixing actuator button 14468 is moved inwardly to actuate the medicament mixer 14360 (see FIG. 109), which can combine and/or mix different medicaments contained within the auto-injector 14002 to produce a mixture suitable for delivery via the auto-injector 14002.

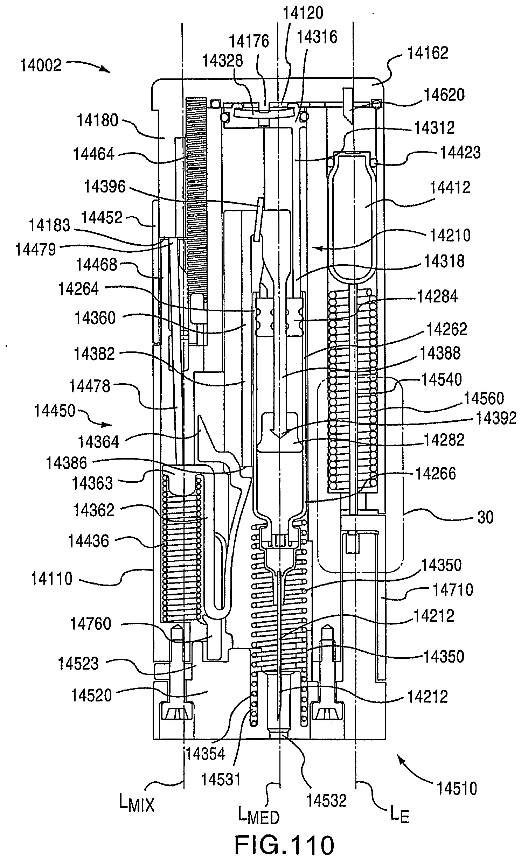

After the medicament is suitably mixed, the distal end portion 14114 of the housing is oriented towards the user such that the base 14520 is in contact with the portion of the body where the injection is to be made. The base 14520 is then moved (after the removal of the actuation safety lock 14710) towards the proximal end 14112 of the housing 14110 to actuate the auto-injector 14002. Upon actuation, the medicament is injected into the body through a needle 14212 (see FIG. 110). The needle 14212 is automatically retracted when the injection is complete. The use of the auto-injector 14002 includes several discrete operations and involves many different components. Accordingly, a detailed description of the components contained in the auto-injector 14002 is presented below, followed by a step-by-step description of operation of the auto-injector 14002.

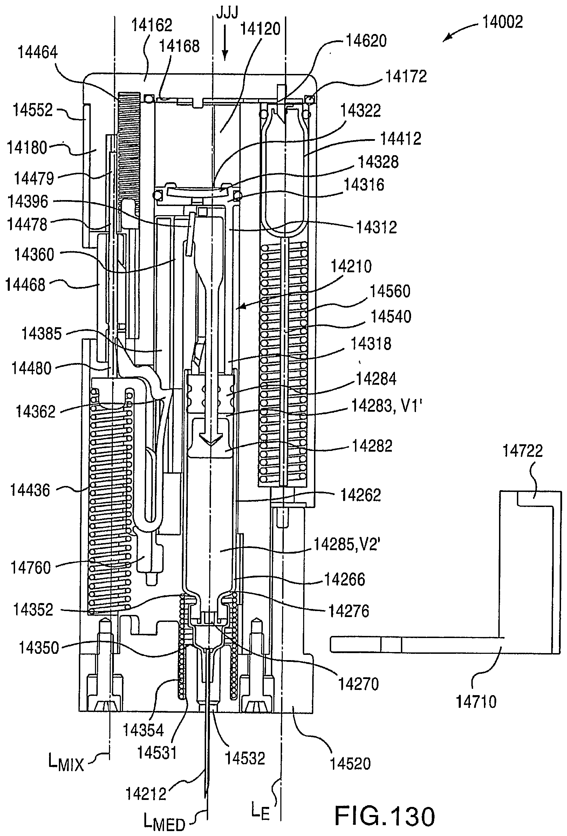

FIG. 109 is a perspective exploded view of the auto-injector 14002 showing the arrangement of the components therein. FIG. 110 is a cross-sectional front view of the auto-injector 14002 in a first configuration (i.e., the initial configuration). The auto-injector 14002 includes a medicament mixer 14360 and a medicament injector 14210, each of which are disposed within the housing 14110. The auto-injector 14002 also includes a mixing actuator 14450 and an injection actuator 14510. As described in more detail herein, the mixing actuator 14450 is configured to release a spring 14436 which is coupled to and causes the medicament mixer 14360 to move within the housing 14110 to mix the contents of the medicament container 14262. The injection actuator 14510 is configured to move a compressed gas container 14412 into engagement with a puncturing element 14620 that is coupled to the proximal cover 14162. In this manner, a compressed gas can be released into a gas chamber 14120 (see FIG. 130) to produce a force necessary to cause the medicament injector 14210 to inject the medicament.

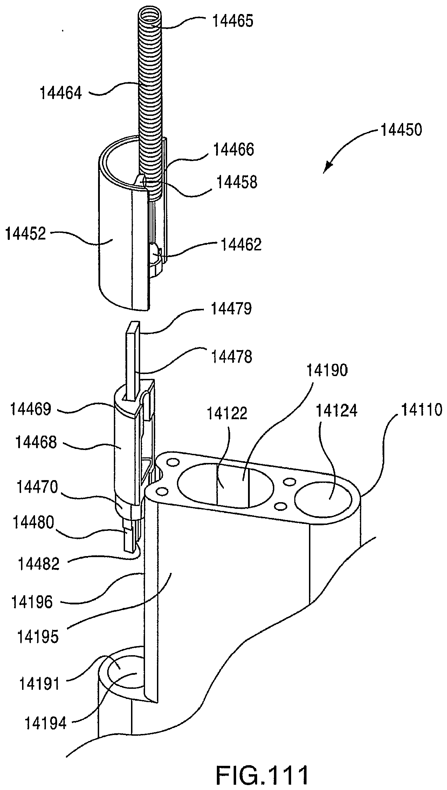

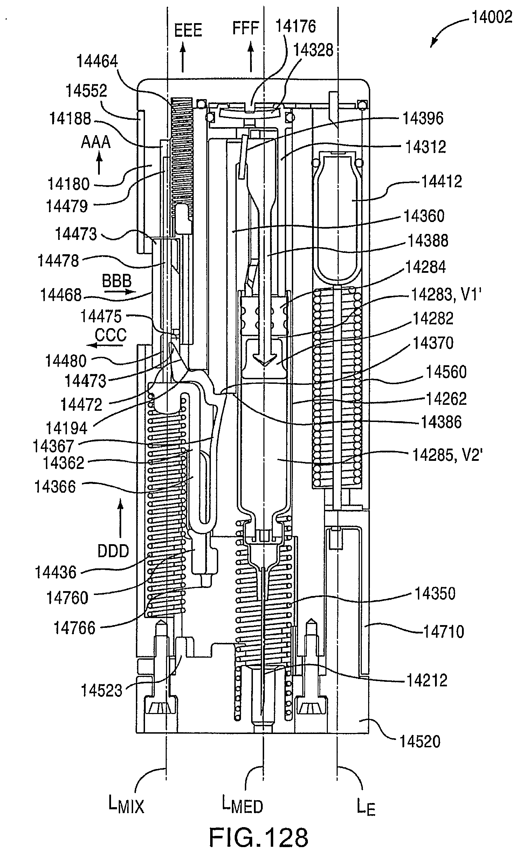

As shown in FIGS. 111 - 114, the mixing actuator 14450 includes a safety cover 14452, a mixing actuator button 14468 and a retaining rod 14478. The safety cover 14452 is slidably disposed within a pair of grooves 14197 (see FIG. 108) defined by an inner surface 14185 of a slide track 14180 of the proximal cover 14162 and a side surface 14196 of the housing 14110. The safety cover 14452 has a proximal end portion 14453 and a distal end portion 14454. An outer surface 14455 of the safety cover 14452 has a curved shape corresponding to the shape of the housing 14110. An inner surface 14456 of the safety cover 14452 defines an opening 14459 that can receive at least a portion of the slide track 14180. The inner surface 14456 of the safety cover 14452 has a shape that corresponds to a shape of an outer surface 14184 of the slide track 14180 and/or a shape of an outer surface 14472 of the mixing actuator button 14468. The inner surface 14456 of the safety cover 14452 also defines two elongated protrusions 14458 that are received within the grooves 14197 and engage the inner surface 14185 of the slide track 14180 and the side surface 14196 of the housing 14110 to allow the safety cover 14452 to slide longitudinally relative to the slide track 14180, as shown by the arrow AAA in FIGS. 108 and 128, while remaining coupled to the housing 14110.

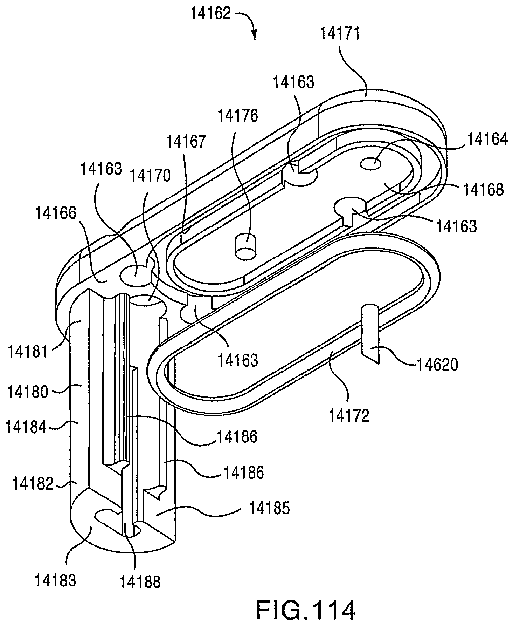

The distal end portion 14454 of the safety cover 14452 includes a protrusion 14462 that is received within a distal end 14466 of a safety cover spring 14464. The proximal end 14465 of the safety cover spring 14464 is received within a spring pocket 14170 defined by an interior surface 14166 of the proximal cover 14162, as shown in FIG. 114. In this manner, the safety cover 14452 is biased in a first position (i.e., towards the distal end portion 14114 of the housing 14110) by the safety cover spring 14464, as shown in FIG. 108. When the safety cover 14452 is in its first position, the mixing actuator button 14468 is received within the opening 14459 defined by the inner surface 14456 of the safety cover 14452. In this manner, the mixing actuator button 14468 is covered by the safety cover 14452.

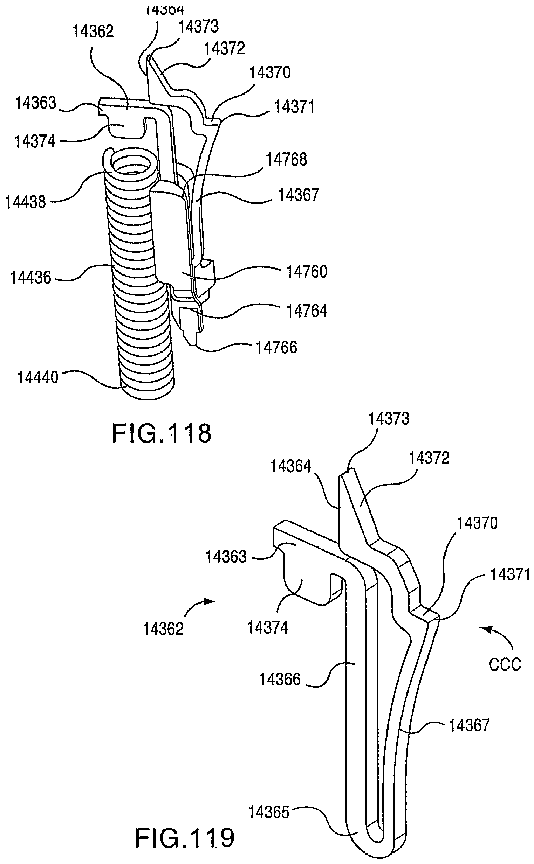

As shown in FIG. 111, the retaining rod 14478 has a proximal end portion 14479 and a distal end portion 14480. The distal end portion 14480 of the retaining rod 14478 defines a slot 14482 that receives a first end portion 14363 of a spring clip 14362 (see FIG. 119). When the auto-injector 14002 is in the first (i.e., the initial) configuration, the proximal end portion 14479 of the retaining rod 14478 is in contact with a distal surface 14183 of the slide track 14180 (see FIGS. 110 and 114). Accordingly, the retaining rod 14478 is maintained in a first (i.e., distal) position, in which the retaining rod 14458 retains the mixing spring 14436 in a compressed configuration. As shown in FIG. 110, when the auto-injector 14002 is in the first configuration, the retaining rod 14478 is angularly offset from a longitudinal axis L_{MIX} of the mixing spring 14436.

The mixing actuator button 14468 has a proximal end portion 14469 and a distal end portion 14470. The distal end portion 14470 of the mixing actuator button 14468 is received within the mixing spring opening 14191 defined by the housing 14110, as shown in FIG. 111. The mixing actuator button 14468 defines two openings 14474 that receive the rod 14478, as shown in FIG. 111. In this manner, when the mixing actuator button 14468 is moved inwardly as shown by arrow BBB in FIG. 128, the retaining rod 14478 moves with the mixing actuator button 14468. Accordingly, as described in more detail herein, when the mixing actuator button 14468 is moved inwardly, the proximal end portion 14479 of the retaining rod can be aligned with groove 14188 defined in the slide track 14180 (see FIG. 114), thereby allowing the mixing spring 14436 to move from its compressed configuration to its expanded configuration along a longitudinal axis L_{MIX} of the mixing spring 14436.

As described in more detail herein, the distal end portion 14470 of the mixing actuator button 14468 defines an opening 14475 that receives a portion of the spring clip 14362 when the mixing actuator 14450 is actuated (see e.g., FIG. 128). Similarly, the proximal end portion 14469 of the mixing actuator button 14468 has a proximal end surface 14473 that engages a distal end surface 14457 of the safety cover 14452 when the mixing actuator 14450 is actuated.

As shown in FIG. 114, the proximal cover 14162 includes a top portion 14171 and the slide track 14180. As previously described, an outer surface 14184 of the slide track 14180 has a shape that corresponds to a shape of the safety cover 14452. An inner surface 14185 of the slide track 14180, together with the side surface 14196 of the housing 14110 define the grooves 14197 within which the safety cover 14452 is slidably disposed. The inner surface 14185 of the slide track 14180 also defines two elongated protrusions 14186 that extend distally from the spring pocket 14170 defined by the interior surface 14166 of the top portion 14171 of the proximal cover 14162. Accordingly, the elongated protrusions 14186 partially enclose the safety cover spring 14464.

The slide track 14180 has a proximal end portion 14181 and a distal end portion 14182. The slide track 14180 defines a longitudinal groove 14188 that extends from the proximal end portion 14181 to the distal end portion 14182. The groove 14188 has a shape corresponding to and slightly larger than the cross-sectional shape of the retaining rod 14478. In this manner, the proximal end portion 14479 of the retaining rod 14478 can be received within the groove 14188.

The distal end portion 14182 of the slide track 14180 also includes a distal surface 14183 adjacent the groove 14188. As described above, when the auto-injector 14002 is in the first configuration, the proximal end portion 14479 of the retaining rod 14478 is in contact with the distal surface 14183 of the slide track 14180.

The top portion 14171 of the proximal cover 14162 includes an interior surface 14166 defining an opening 14164 that receives a puncturing element 14620. The opening 14164 is positioned such that the puncturing element 14620 is aligned with the compressed gas container 14412. In some embodiments, for example, a longitudinal center line of the opening 14164 is coaxial with a longitudinal axis L_{E} defined by the compressed gas container 14412. Although the opening 14164 is shown as being a blind hole, in other embodiments, the opening 14164 can be a through hole.

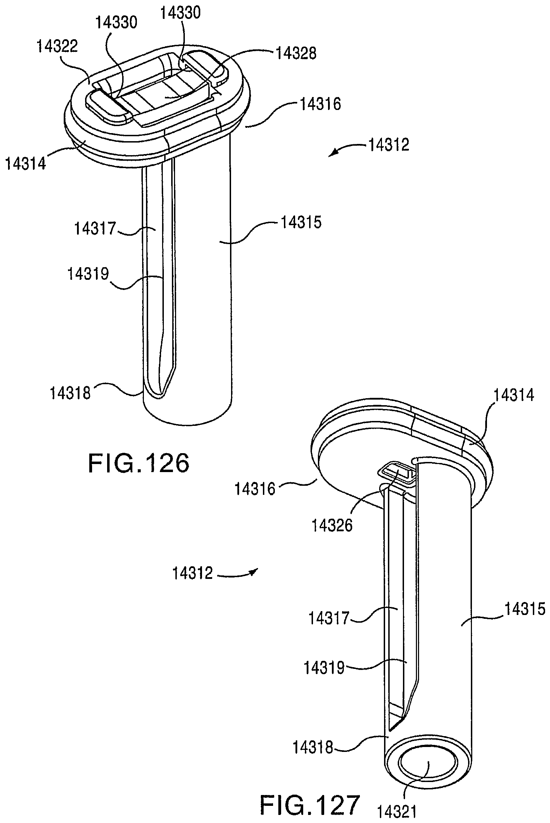

The interior surface 14166 also defines a recess 14168. As described in more detail herein, the recess 14168 of the top portion 14171, the surface 14122 defining the injector opening 14190 (see FIG. 111) and the proximal end surface 14322 of the movable member 14312 (see FIG. 126) collectively define a gas chamber 14120. Said another way, the recess 14168 of the proximal cover 14162 defines a portion of a boundary of the gas chamber 14120.

A protrusion 14176 is disposed within the recess 14168. As shown in FIG. 110, when the auto-injector 14002 is in the first configuration, the protrusion 14176 engages a gas release valve 14328 to maintain the gas release valve 14328 in a closed position. Moreover, when the auto-injector 14002 is in the first configuration, the protrusion 14176 prevents the movable member 14312 from moving proximally within the housing 14110.

The interior surface 14166 of the proximal cover 14162 also defines a spring pocket 14170 and an o-ring groove 14167. As described above, the spring pocket 14170 receives the proximal end 14465 of the safety cover spring 14464. An o-ring 14172 is disposed within the o-ring groove 14167 to hermetically seal the gas chamber 14120. The proximal cover 14162 is coupled to the housing 14110 by four mounting screws 14174, as shown in FIG. 109. The top portion 14171 of the proximal cover 14162 defines four mounting holes 14163 that receive the mounting screws 14174.

Although shown and described as being monolithically formed, in some embodiments, the top portion 14171 and the slide track 14180 can be formed separately and joined together to form the proximal cover 14162. Similarly, in some embodiments the proximal cover 14162 and the housing 14110 can be joined together by any suitable means, such as, for example, sonic welding, chemical bonding, laser welding or the like.

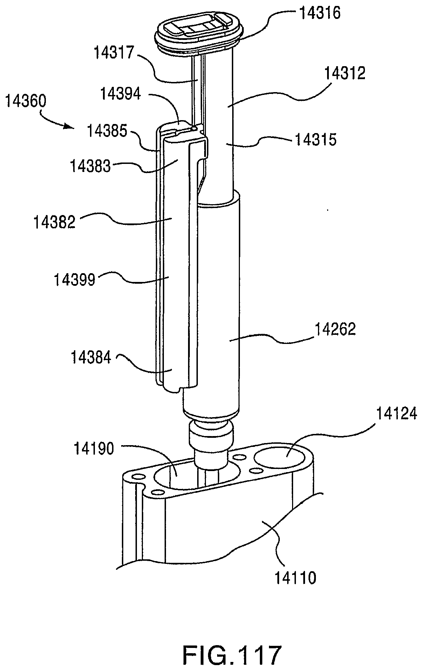

As shown in FIGS. 109 and 117, the medicament mixer 14360 is disposed, along with the medicament container 14262 and the movable member 14312, within the medicament injector opening 14190 defined by the housing 14110. As shown in FIGS. 110, 115 and 116, the medicament mixer 14360 includes a spring engagement portion 14382, a plunger engagement portion 14388 and a junction portion 14394. The junction portion 14394 is disposed between the spring engagement portion 14382 and the plunger engagement portion 14388. The spring engagement portion 14382 is disposed outside of and adjacent to the medicament container 14262. Said another way, the spring engagement portion 14382, and therefore the medicament mixer 14260, is offset from a longitudinal axis L_{MED} of the medicament container 14262. Similarly, the medicament mixer 14360 is offset from the longitudinal axis L_{MIX} of the mixing spring 14436.

An inner surface 14387 of the spring engagement portion 14382 has a curved shape that corresponds to the shape of the medicament container 14262. Similarly, an outer surface 14399 of the spring engagement portion 14382 has a curved shape that corresponds to the shape of the injector opening 14190 defined by the housing 14110. In this manner, the medicament mixer 14360 can move longitudinally within the injector opening 14190 while maintaining a constant orientation within the injector opening 14190 (e.g., without tilting and/or binding within the injector opening 14190).

The outer surface 14399 of the spring engagement portion 14382 defines an opening 14385 that extends longitudinally from a proximal end portion 14383 of the spring engagement portion 14382 to a distal end portion 14384 of the spring engagement portion 14382. As described in more detail herein, the opening 14385 is configured to receive a portion of the spring clip 14362 when the movable member 14312 of the medicament injector 14210 is moving distally within the housing 14110 (e.g., during the needle insertion and/or medicament injection process). The distal end portion 14384 of the spring engagement portion 14382 includes an engagement surface 14386 adjacent the opening 14385. As described in more detail herein, the engagement surface 14386 is configured to releasably engage a corresponding engagement surface 14370 of the spring clip 14362.

The spring engagement portion 14382 of the medicament mixer 14360 is releasably coupled to the mixing spring 14436 by the spring clip 14362. As shown in FIGS. 118 and 119, the spring clip 14362 includes a first end 14363, a second end 14364 and a U-shaped bend 14365 disposed between the first end 14363 and the second end 14364. Accordingly, the spring clip 14362 includes an outer portion 14366 disposed between the first end 14363 and the U-shaped bend 14365 and an inner portion 14367 disposed between the second end 14364 and the U-shaped bend 14365. The spring clip 14362 is constructed from a resilient material, such as, for example, brass. Accordingly, in use, the inner portion 14367 can elastically deform relative to the outer portion 14366, as indicated by the arrow CCC in FIGS. 119 and 128.

The outer portion 14366 of the spring clip 14362 includes a protrusion 14374 that is received within the proximal end portion 14438 of the mixing spring 14436. As described above, the protrusion 14374 is secured within proximal end portion 14438 of the mixing spring 14436 by the retaining rod 14478. A portion of the outer portion 14366 is received within a retaining groove 14768 defined by a mixing safety lock 14760. The details of the mixing safety lock 14760 are described in more detail herein.

The inner portion 14367 of the spring clip 14362 includes a point 14373 at the second end 14364 thereof. The inner portion 14367 of the spring clip 14362 also includes an angled surface 14372 extending distally from the point 14373, an engagement surface 14370 and a curved surface 14371. In use, the angled surface 14372 engages the surface 14194 of the-housing 14110 that defines the mixing spring opening 14191 (see FIG. 111), thereby causing the inner portion 14367 to bend outwardly (as indicated by the arrow CCC in FIGS. 118 and 128). In this manner, the engagement surface 14370 of the spring clip 14362 can be disengaged from the corresponding engagement surface 14386 of the medicament mixer 14360, thereby decoupling the medicament mixer 14360 from the mixing spring 14436.

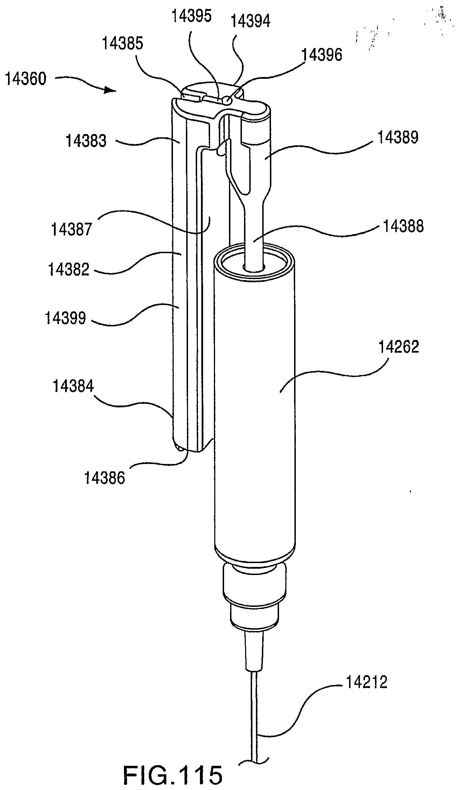

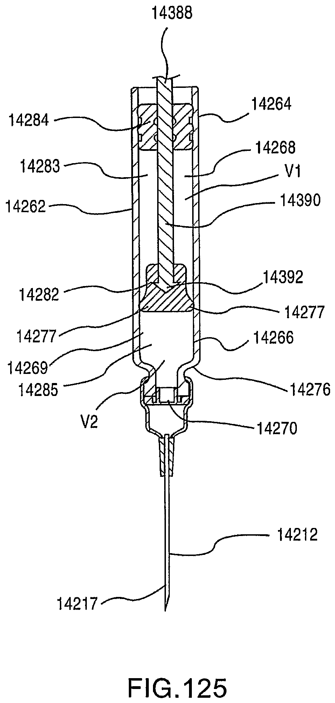

The plunger engagement portion 14388 of the medicament mixer 14360 has a proximal end portion 14389 and a distal end portion 14390. As shown in FIGS. 115, 116 and 126, the proximal end portion 14389 of the plunger engagement portion 14388 is disposed through an opening 14317 defined by a side wall 14315 of the movable member 14312 and within a lumen 14319 defined by the side wall 14315 of the movable member 14312. The plunger engagement portion 14388 is also disposed through an opening 14321 defined at the distal end portion 14318 of the movable member 14312 such that the distal end portion 14390 of the plunger engagement portion 14388 is disposed through an opening defined by the first plunger 14284 and into the medicament container 14262. As shown in FIG. 125, the second plunger 14282 is coupled to the distal end portion 14390 of the plunger engagement portion 14388 by a plunger coupling 14392.

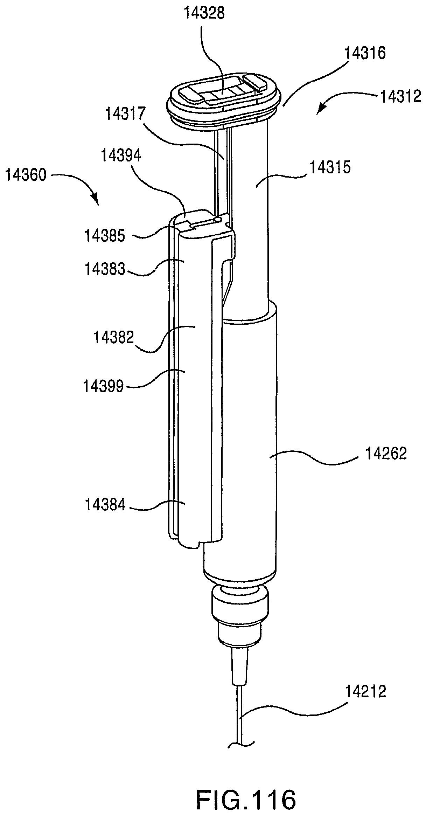

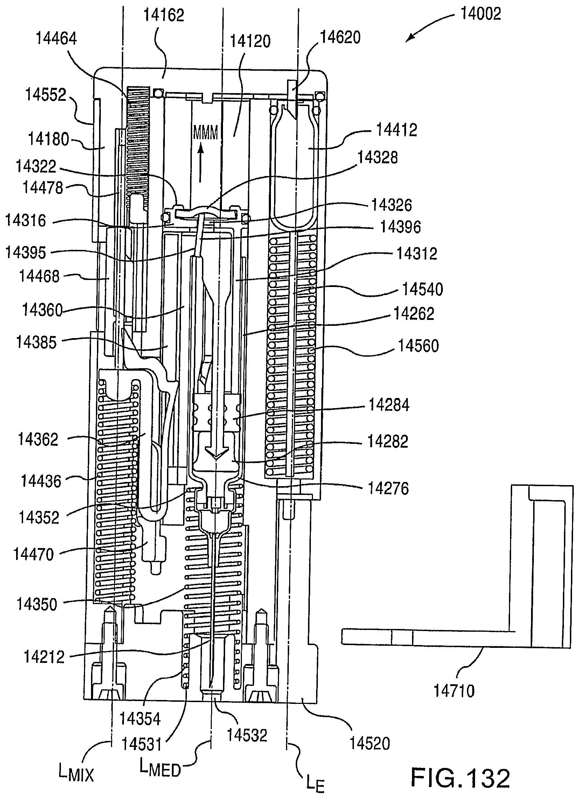

The junction portion 14394 of the medicament mixer 14360 is disposed between the proximal end portion 14383 of the spring engagement portion 14382 and the proximal end portion 14389 of the plunger engagement portion 14388. The junction portion 14394 defines an opening 14395 within which a pin 14396 is movably disposed. As described in more detail herein, when the injection operation is completed, the pin 14396 actuates a gas release valve 14328 (see e.g., FIGS. 116 and 132) to allow the pressurized gas within the gas chamber 14120 to escape.

This arrangement of the medicament mixer 14360 and the movable member 14312 allows the medicament mixer 14360 to move with the movable member 14312, relative to the movable member 14312 and/or independently from the movable member 14312. This arrangement of the medicament mixer 14360 and the movable member 14312 also allows the medicament mixer 14360 to move in a first direction (e.g., proximally) when the movable member 14312 is moving in a second direction opposite the first direction (e.g., distally). In this manner, as described in more detail herein, the medicament mixer 14360 can combine and/or mix the different medicaments contained in the medicament container 14262 to produce a mixture suitable for delivery via the auto-injector 14002.

When the medicament and/or medicaments contained within the medicament container 14262 are suitably mixed, the auto-injector 14002 can be actuated by the system actuator 14510, which is configured to move the compressed gas container 14412 into engagement with a puncturing element 14620. As shown in FIGS. 109 and 120 - 124, the injection actuator 14510 include a base 14520, a retaining rod 14540, a spring 14560 and an actuation safety lock 14710.

Prior to actuation, the spring 14560 is disposed about the retaining rod 14540 in a compressed configuration, such that the spring 14560 is retained by a proximal end portion 14542 of the retaining rod 14540 and a retention shoulder 14570 defined within the gas container opening 14124 of the housing 14110. In this manner, the retaining rod 14540 is spring-loaded such that when a distal end portion 14544 of the rod 14540 is decoupled from the retention shoulder 14570, the force of the spring 14560 causes the retaining rod 14540, and therefore the compressed gas container 14412, to move proximally within the housing 14110 along the longitudinal axis L_{E} of the compressed gas container 14412.

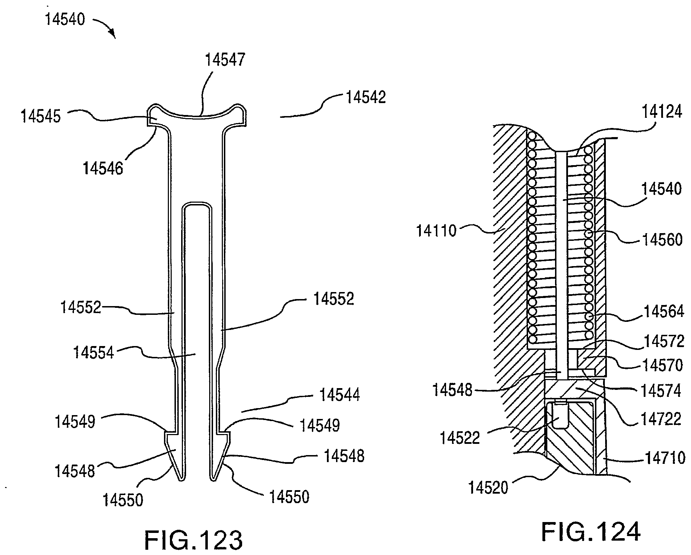

The distal end portion 14544 of the retaining rod 14540 includes two extensions 14552 disposed apart from each other to define an opening 14554 therebetween. Each extension 14552 includes a projection 14548 having a tapered surface 14550 and an engagement surface 14549. As shown in FIG. 124, when the retaining rod 14540 is in its first (or engaged) position, the engagement surfaces 14549 engage a distal surface 14574 of a retention shoulder 14570 defined within the gas container opening 14124 defined by the housing 14110. Accordingly, when the retaining rod 14540 is in its first position, the retaining rod 14540 is prevented from moving proximally along the longitudinal axis L_{E}. As described in more detail herein, when the base 14520 is moved proximally towards the housing 14110 to actuate the auto-injector 14002, the tapered surfaces 14550 of the projections 14548 cooperate with corresponding tapered surfaces 14524 defined by the base 14520 to move the extensions 14552 inwardly towards each other. The inward motion of the extensions 14552 causes the engagement surfaces 14549 to become disengaged from the distal surface 14574 of the retention shoulder 14570, thereby allowing the retaining rod 14540 to move between its first position (FIG. 110) and a second (or actuated) position (FIG. 130).

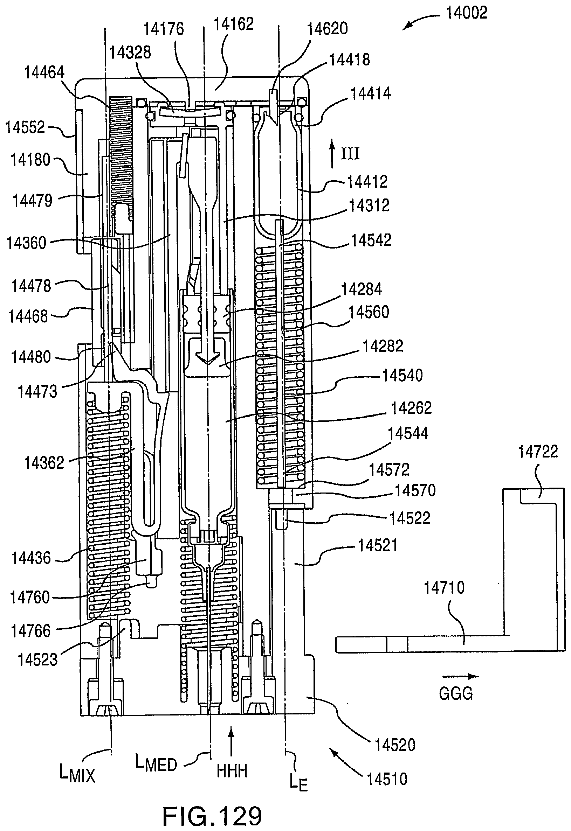

The proximal end portion 14542 of the retaining rod 14540 includes a retention portion 14545 having a first surface 14547 and a second surface 14546. The first surface 14547 of the retention portion 14545 engages the distal portion 14416 of the compressed gas container 14412. The second surface 14546 of the retention portion 14545 engages a proximal end 14562 of the spring 14560. A distal end 14564 of the spring 14560 engages a proximal surface 14572 of the retention shoulder 14570. In this manner, when the retaining rod 14540 is in its first position, the spring 14560 is compressed between the retention shoulder 14570 and the retention portion 14545 of the retaining rod 14540. Accordingly, when the retaining rod 14540 is disengaged from the retention shoulder 14570, the force imparted by the spring 14560 on the retention portion 14545 of the retaining rod 14540 causes the retaining rod 14540 to move proximally along the longitudinal axis L_{E} into its second position (see e.g., FIG. 124 and 129).

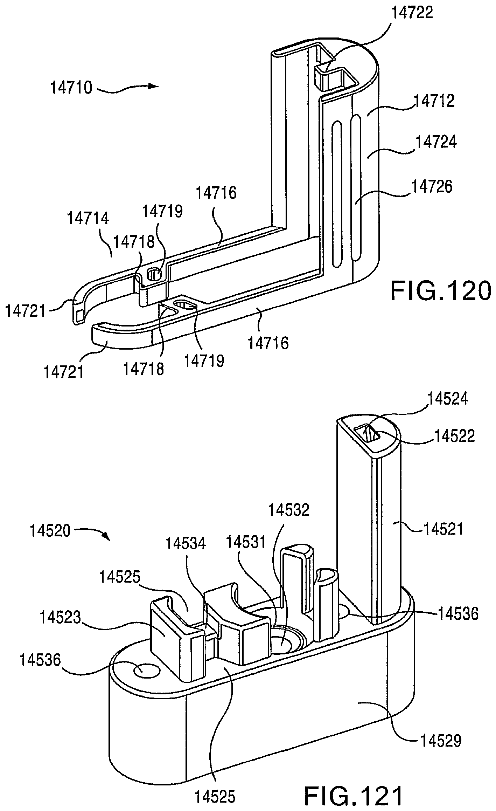

As shown in FIG. 121, the base 14520 includes a proximal portion 14521 and a distal portion 14529. The distal portion 14529 of the base 14520 is movably coupled to the distal end portion 14114 of the housing 14110 by two mounting screws 14174, as shown in FIG. 109. The distal portion 14529 of the base 14520 defines two openings 14536 that receive the mounting screws 14174. In this manner, the movement and/or alignment of the base 14520 relative to the housing 14110 is guided by the mounting screws 14174 and the openings 14536.

The distal portion 14529 of the base 14520 also defines a needle opening 14532 and a retraction spring pocket 14531 within the needle opening 14532. When the auto-injector 14002 is in its fourth configuration (see FIG. 130), the needle 14212 extends through the needle opening 14532. As shown in FIG. 110, a distal end 14354 of the retraction spring 14350 is retained within the retraction spring pocket 14531.

The distal portion 14529 of the base 14520 defines a proximally facing projection 14523 that defines a top opening 14523 and two side openings 14525. The top opening receives a distal end portion 14764 of the mixing safety lock 14760 (see FIG. 118). The two side openings 14525 receive the inwardly facing protrusions 14718 of the actuation safety lock 14710 (see FIG. 121). Accordingly, when the auto-injector is in its first configuration, the protrusions 14766 extending from the distal end portion 14764 of the mixing safety lock 14760 extend within the openings 14719 defined by the inwardly facing protrusions 14718 of the actuation safety lock 14710. This arrangement prevents the actuation safety lock 14710 from being removed when the mixing safety lock 14760 is in its initial position. Said another way, the mixing safety lock 14760 and the actuation safety lock 14710 are cooperatively arranged such that the actuation safety lock 14710 cannot be removed until after the mixing operation is complete.

The proximal portion 14521 of the base 14520 includes two opposing tapered surfaces 14524 (only one of the tapered surfaces is shown in FIG. 121) that define an opening 14522 configured to receive the corresponding tapered surfaces 14550 of the retaining rod 14540 when the base 14520 is moved proximally towards the housing 14110. When the projections 14548 of the retaining rod 14540 are received within the opening 14522, they are moved together, causing the distal end portion 14544 of the rod 14540 to be disengaged from the retention shoulder 14570.

As shown in FIG. 120, the actuation safety lock 14710 has a first end 14712 and a second end 14714. The second end 14714 of the actuation safety lock 14710 includes two extended portions 14716, each of which includes an inwardly facing protrusion 14718 defining an opening 14719. When the actuation safety lock 14710 is in its first (or locked) position, the extended portions 14716 extend around a portion of the base 14520 and/or housing 14110 to space the base 14520 apart from the distal end portion 14114 of the housing 14110. The ends 14721 of the extended portions 14716 are configured slightly wrap around a portion of the housing 14110, the base 14520 and/or the mounting screws 14174 to removably couple the actuation safety lock 14710 to the housing 14110 in its first position. Additionally, the inwardly facing protrusions 14718 extend within the side openings 14525 of the base 14520 to removably couple the actuation safety lock 14710 in its first position. The inwardly facing protrusions 14718 are at an acute angle with respect to the direction of motion of the actuation safety lock, as indicated by arrow GGG in FIG. 129, such that the inwardly facing protrusions 14718 provide resistance to, but do not prevent the removal of the actuation safety lock 14710.

The first end 14712 of the actuation safety lock 14710 includes a locking protrusion 14722 that extends inwardly. As shown in FIG. 124, when the actuation safety lock 14710 is in its first position, the locking protrusion 14722 extends within the opening 14554 between the projections 14548 of the retaining rod 14540 and the opening 14522 defined by the base 14520. In this manner, when the actuation safety lock 14710 is in its first position, the base 14520 cannot be moved proximally to allow the projections 14548 to be received within the opening 14522. The arrangement of the locking protrusion 14722 also prevents the projections 14548 of the retaining rod 14540 from being moved inwardly towards each other. Accordingly, when the actuation safety lock 14710 is in its first position, the auto-injector 14002 cannot be actuated.

The outer surface 14724 of the first end 14712 of the actuation safety lock 14710 includes a series of ridges 14726 to allow the user to more easily grip the actuation safety lock 14710. In some embodiments, the outer surface 14724 of the first end 14712 can include an indicia, such as, for example, a numeral, to instruct the user in operating the auto-injector 14002.

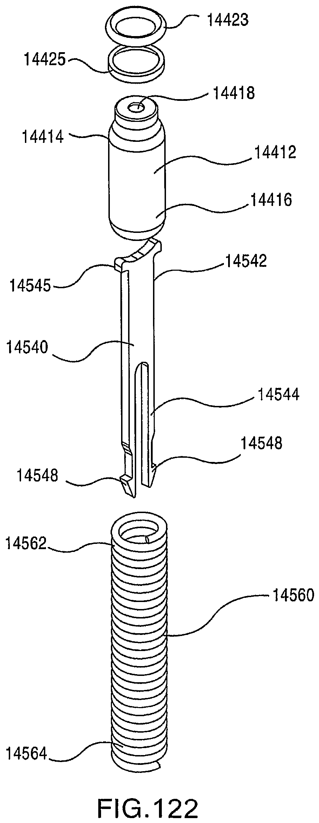

As described in more detail herein, upon actuation, the injection actuator 14510 is configured to move the compressed gas container 14412 into engagement with a puncturing element 14620 that is coupled to the proximal cover 14162. As shown in FIGS. 109 and 122, the compressed gas container 14412 is slidably disposed within the gas container opening 14124 of the housing 14110. The compressed gas container 14412 has a distal end portion 14416 and a proximal end portion 14414, and defines a longitudinal axis L_{E}. As described above, the distal end portion 14416 of the compressed gas container 14412 is engaged with the retention portion 14545 of the retention rod 14540.

The proximal end portion 14414 of the compressed gas container 14412 includes a frangible surface 14418. An o-ring 14423 and a spacer 14425 are disposed about the proximal end portion 14414 of the compressed gas container 14412 to hermetically seal the proximal end 14414 of the compressed gas container 14412 within the gas container opening 14124. This arrangement prevents pressurized gas from leaking around the compressed gas container 14412 to an area outside of the gas chamber 14120 after the frangible surface 14418 of the compressed gas container 14412 has been punctured.

The medicament injector 14210 includes a medicament container 14262, a needle 14212 and a movable member 14312 As described in more detail herein, the medicament container 14262 is movably disposed within the medicament injector opening 14190 defined by the housing 14110. The movable member 14312 is also movably disposed within the medicament injector opening 14190 defined by the housing 14110 and is movable relative to the medicament container 14262.

The medicament container 14262 defines a longitudinal axis L_{MED} that is non-coaxial with the longitudinal axis L_{E} of the compressed gas container 14412 and the longitudinal axis L_{MIX} of the mixing spring 14436. Accordingly, the medicament container 14262, the compressed gas container 14412 and the mixing actuator 14450 are arranged substantially parallel within the housing 14110 such that the housing has a substantially rectangular shape. Moreover, the non-coaxial relationship between the medicament container 14210, the compressed gas container 14412 and the mixing actuator 14450 allows the mixing actuator 14450 and/or the injection actuator 14510 to be actuated by manipulating a portion of the auto-injector 14002 disposed apart from the proximal end portion 14112 of the housing (e.g., the mixing actuator button 14468 disposed on the side portion 14195 of the housing and the base 14520 disposed at the distal end portion 14114 of the housing 14110).

As shown in FIG. 125 the medicament container 14262 includes a proximal end portion 14264 and a distal end portion 14266. The medicament container 14262 includes a first plunger 14284 and a second plunger 14282 disposed distally from the first plunger 14284. The first plunger 14284 and the second plunger 14282 are each movably disposed within the medicament container 14262. A frangible seal 14270 is fixedly disposed within the distal end portion 14266 of the medicament container 14262. The distal end portion 14266 of the medicament container 14262 defines a shoulder portion 14276 which engages a proximal end portion 14352 of the retraction spring 14350.

When the auto-injector 14002 is in the first configuration, as shown in FIG. 125, the first plunger 14284 is disposed in a first position within proximal end portion 14264 of the medicament container 14262. The second plunger 14282 is disposed in a second position distally from the first plunger 14284 such a first medicament containing portion 14283 having a volume V1 is defined between the first plunger 14284 and the second plunger 14282. Similarly, a second medicament containing portion 14285 having a volume V2 is defined between the second plunger 14282 and the frangible seal 14270. In some embodiments, the medicament container 14262 can include a first medicament 14268 (e.g., water) within the first medicament containing portion 14283 and a second medicament 14269 (e.g., a lyophilized powder) within the second medicament containing portion 14285.

The first plunger 14284 forms a fluid-tight seal when disposed within the medicament container 14262. Accordingly, when the first plunger 14284 moves within the medicament container 14262, the portions within the medicament container 14262 distally disposed from the first plunger 14284 (e.g., the first medicament containing portion 14283 and/or the second medicament containing portion 14285) remain fluidically isolated from the portions within the medicament container 14262 proximally disposed from the first plunger 14284.

The second plunger 14282 also forms a fluid-tight seal when stationary within the medicament container 14262. However, when the second plunger 14282 moves within the medicament container 14262, the flexible portions 14277 of the second plunger 14282 can deform thereby allowing the portions within the medicament container 14262 distally disposed from the first plunger 14284 (e.g., the second medicament containing portion 14285) to be in fluid communication with portions within the medicament container 14262 proximally disposed from the first plunger 14284 (e.g., the first medicament containing portion 14283).

The needle 14212 is coupled to the distal end portion 14266 of the medicament container 14262 such that a lumen 14217 defined by the needle 14212 is in fluid communication with a portion of the medicament container disposed distally from the frangible seal 14270. Accordingly, when the frangible seal 14270 is broken, the first medicament containing portion 12283 and/or the second medicament containing portion 12285 are in fluid communication with the lumen 14217 defined by the needle 14212.

As shown in FIGS. 126 and 127, the movable member 14312 includes a proximal end portion 14316 and a distal end portion 14318. The distal end portion 14318 of the movable member 14312 is disposed within the proximal portion 14264 of the medicament container 14262 such that the distal end portion 14318 engages the first plunger 14284. The movable member 14312 includes a side wall 14315 that extends longitudinally from the proximal end portion 14316 to the distal end portion 14318. The side wall 14315 defines a lumen 14319 and an opening 14317. As described above with reference to FIGS. 116 and 117, the plunger engagement portion 14388 of the medicament mixer 14360 is disposed through the opening 14317 and within the lumen 14319. The distal end portion 14318 of the movable member 14312 also defines an opening 14321 through which the plunger engagement portion 14388 is disposed.

The proximal end portion 14316 of the movable member 14312 includes a surface 14322 that, together with the interior surface 14166 of the proximal cover 14162 (see FIG. 114) and the surface 14122 defining the injector opening 14190 (see FIG. 111), defines a gas chamber 14120. Said another way, the surface 14322 defines a portion of a boundary of the gas chamber 14120.

The proximal end portion 14316 of the movable member 14312 also includes a seal 14314 that engages a portion the inner surface 14122 of the housing 14110 (see FIG. 130) to fluidically isolate the gas chamber 14120. Although the seal 14314 is shown as being an o-ring seal, in some embodiments, the seal need not be a separate component, but can rather be a portion of the proximal end portion 14316 of the movable member 14312.

As shown in FIGS. 126 and 127, the proximal end portion 14316 of the movable member 14312 defines an opening 14326 therethrough which is in fluid communication between the gas chamber 14120 and the interior of the housing 14110 outside the gas chamber 14120. The proximal end portion 14316 further defines two slots 14330 that receive a gas relief valve 14328, which can be, for example, a flexible rubber member. The gas relief valve 14328 is positioned within the slots 14330 and adjacent the opening 14326 to selectively allow fluid communication between the gas chamber 14120 and the area outside the gas chamber 14120 through the opening 14326.

As described in more detail herein, when the injection actuator 14510 is actuated, the movable member 14312 moves towards the distal end portion 14114 of the housing 14110, in response to a force produced by a pressurized gas on the surface 14322 of the movable member 14312. As a result, the medicament container 14262 is moved towards the distal end portion 14114 of the housing 14110, thereby exposing the needle 14212 from the housing 14110. The movable member 14312 then continues to move within the medicament container 14262 break the frangible seal 14270 and expel a medicament from the medicament container 14262 through the needle 14212.

As discussed above, the use and actuation of the auto-injector 14002 includes several discrete operations, as shown in FIGS. 110 and 128 - 132. Although FIGS. 110 and 128 - 132 show the same components of the auto-injector 14002, certain reference numerals are omitted in some of the figures for clarity. First, the mixing actuator 14450 is enabled by moving the safety cover 14452 proximally to expose the mixing actuator button 14468 (see FIG. 128). Second, the medicament mixer 14360 is actuated by pressing the mixing actuator button 14468 inward (see FIG. 128). When the medicament mixer 14360 is actuated, the force from the mixing spring 14436 causes the medicament mixer 14360 to move proximally within the housing 14110. Accordingly, the second plunger 14282 moves proximally within the medicament container 14262 to mix and/or combine the first medicament 14268 and the second medicament 14269.

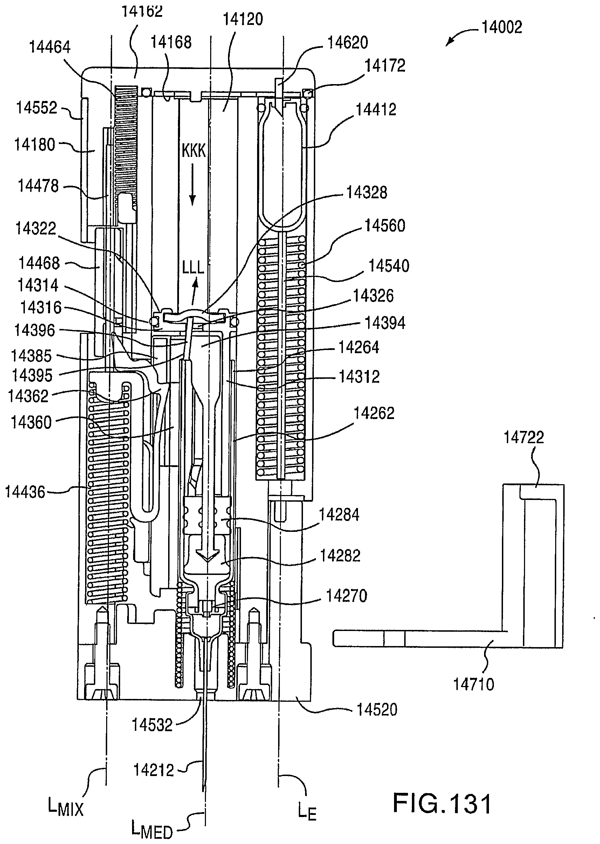

Upon completion of the mixing operation, the medicament injector 14210 is enabled by removing the safety lock 14710 (see FIG. 129). The medicament injector 14210 is then actuated by moving the base 14520 proximally towards the housing 14110. When the medicament injector 14210 is actuated, the compressed gas container 14412 is moved into engagement with the puncturing member 14620, which causes the pressurized gas to be released into the gas chamber 14120 (see FIG. 130). The pressurized gas produces a force that causes the movable member 14312 and the medicament container 14262 to move distally within the housing 14110 to extend the needle 14212 from distal end portion 14114 of the housing 14110 and the base 14520 (see FIG. 130). This operation can be referred to as the "needle insertion" operation. When the medicament container 14262 has completed its distal movement (i.e., the needle insertion operation is complete), the force from the movable member 14312 on the first plunger 14284 causes the frangible seal 14270 to break, thereby placing the second medicament containing portion 14285 in fluid communication with the needle 14212. Accordingly, the movable member 14312 moves within the medicament container 14262, thereby expelling the mixed medicament through the needle 14212 (see FIG. 131). This operation can be referred to as the "injection operation." Upon completion of the injection, the pressurized gas is released from the gas chamber 14120, thereby allowing the medicament container 14262 to be moved proximally within the housing (i.e., retracted, see FIG. 132). A detailed description of each of these operations is provided below.

FIG. 128 shows the auto-injector 14002 in a second configuration, in which the mixing actuator 14450 of the auto-injector 14002 has been actuated. Prior to actuation, the mixing actuator 14450 must first be enabled by moving the safety cover 14452 proximally to expose the mixing actuator button 14468. As described above, the elongated protrusions 14458 of the safety cover 14452 slide within the grooves 14197 (not shown in FIG. 128) such that safety cover 14452 moves in a direction parallel to the longitudinal axis L_{MIX}, between a first (i.e., distal) position and a second (i.e., proximal) position, as shown by the arrow AAA in FIG. 128. When the safety cover 14552 is in the second position, a portion of the slide track 14180 is received within the opening 14459 of the safety cover 14452.

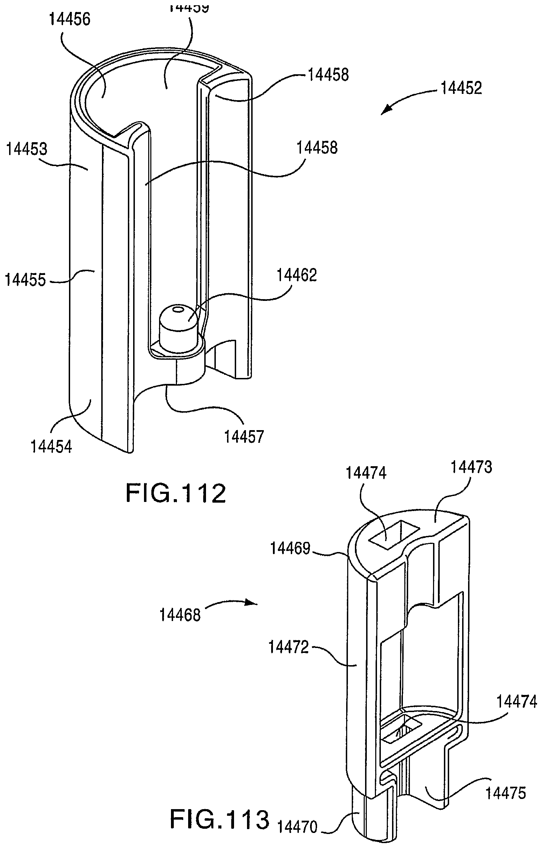

The medicament mixer 14360, is then actuated by pushing the mixing actuator button 14468 inwardly between a first position and a second position, as shown by the arrow BBB in FIG. 128. When the mixing actuator button 14468 is in its second position, its proximal end surface 14473 (see FIG. 113) engages the distal end surface 14457 of the safety cover 14452 (not shown in FIG. 128, see e.g. FIG. 112). Accordingly, the safety cover 14452 is prevented from moving distally (i.e., returning to its initial position) by the safety cover spring 14464 after the mixing actuator button 14468 has been pushed. This arrangement allows a user to know whether the auto-injector 14002 has been used via a quick visual examination of the position of the safety cover 14452.

When the mixing actuator button 14468 is moved between its first position and its second position, the retaining rod 14478 moves with the mixing actuator button 14468 from a first (i.e., initial) position to a second (i.e., intermediate) position. Because the distal end portion 14480 of the retaining rod 14478 remains in substantially the same position moving between its first position and its second position, the retaining rod 14478 rotates along an axis substantially normal to the longitudinal axis L_{MIX} of the mixing spring when the retaining rod moving between its first position and its second position.

As shown in FIG. 110, when the retaining rod 14478 is in the first position, the proximal end portion 14479 of the retaining rod 14478 engages the distal surface 14183 of the slide track 14180 such that the mixing spring 14436 is maintained in its compressed configuration. When the retaining rod 14478 is in the second position, the proximal end portion 14479 of the retaining rod 14478 is aligned with the groove 14188 defined in the slide track 14180 (see FIG. 114). Accordingly, the mixing spring 14436 moves from its compressed configuration to its expanded configuration along the longitudinal axis L_{MIX}, as shown by the arrow DDD in FIG. 128.

When the mixing spring 14436 moves from its compressed configuration (FIG. 110) to its expanded configuration (FIG. 128), the retaining rod 14478 moves from its second (i.e., intermediate) position to a third (i.e., actuated) position. When the retaining rod 14478 moves from its second to its third position, the proximal end portion 14479 of the retaining rod 14478 is received within the groove 14188 defined in the slide track 14180. Accordingly, the retaining rod 14478 moves proximally along the longitudinal axis L_{MIX}, as shown by the arrow EEE in FIG. 128.

Because the medicament mixer 14360 is coupled to the mixing spring 14436 via the spring clip 14362, when the mixing spring 14436 moves from its compressed configuration (FIG. 110) to its expanded configuration (FIG. 128), medicament mixer 14360 moves proximally the within the medicament injector opening 14190, as shown by arrow FFF in FIG. 128. Accordingly, the second plunger 14282 moves proximally within the medicament container 14262 from the second position (see FIG. 125) to a third position. As described above, when second plunger 14282 moves proximally within the medicament container 14262, the flexible portions 14277 of the second plunger 14282 deform to place the first medicament containing portion 14283 in fluid communication with the second medicament containing portion 14285. In this manner, the first medicament 14268 can be mixed with the second medicament 14269 to produce a mixture suitable for delivery via the auto-injector 14002.

When the second plunger 14282 moves proximally within the medicament container 14262 from the second position to the third position, the protrusion 14176 of the proximal cover 14162 (see FIG. 114) remains engaged with the gas relief valve 14328. This arrangement prevents the movable member 14312 (and therefore the first plunger 14284) from moving proximally within the housing 14110. Accordingly, the first plunger 14284 remains in the first position within the medicament container 14262 when the second plunger 14282 moves proximally within the medicament container 14262 from the second position to the third position. Said another way, the second plunger 14282 moves proximally within the medicament container 14262 from the second position to the third position relative to and independently from the movable member 14312 and/or the first plunger 14284.

Although FIG. 128 shows the protrusion 14176 of the proximal cover 14162 being engaged with the gas relief valve 14328 and/or the surface 14322 of the movable member 14312, in some embodiments, the protrusion 14176 can be slightly spaced apart from the gas relief valve 14328 and/or the surface 14322 of the movable member 14312. Such a slight space can result from normal deviations in manufacturing and assembly (i.e., deviations within the manufacturing tolerance of the components). Accordingly, in some embodiments, the first plunger 14284 can move slightly proximally within the medicament container 14262 when the second plunger 14282 moves proximally within the medicament container 14262 from the second position to the third position.

As shown in FIG. 128, when the auto-injector 14002 is in the second configuration, the volume V2' of the second medicament containing portion 14285 is greater than the volume V2 when the medicament container 14262 is in the first configuration (see FIG. 125). Moreover, the total volume of the medicament container 14262 when the auto-injector 14002 is in the first configuration (V1 + V2) is the same as the total volume of the medicament container 14262 when the auto-injector 14002 is in the second configuration (V1' + V2'). Although the volume V1' of the first medicament containing portion 14283 is shown as being greater than zero, substantially all of the first medicament 14268 and substantially all of the second medicament 14269 are contained within the second medicament containing portion 14285 when the auto-injector 14002 is in the second configuration.

Although the volume V1' of the first medicament containing portion 14283 is shown as being greater than zero, in some embodiments, the volume V1' of the first medicament containing portion 14283 can be substantially zero when the auto-injector 14002 is in the second configuration. Said another way, in some embodiments, the second plunger 14282 can engage the first plunger 14284 when the auto-injector 14002 is in the second configuration.

After the auto-injector 14002 is in the second configuration (FIG. 128), in some embodiments, the user can enhance the mixing, for example, by shaking the auto-injector 14002. Because the mixing process occurs at a substantially constant volume, the pressure within the first medicament containing portion 14283 and the second medicament containing portion 14285 remains substantially constant throughout the mixing process. Additionally, the medicament mixer 14360 is actuated independently from and using a separate energy storage member (e.g., the mixing spring 14436) from the medicament injector 14210. This arrangement can prevent premature breaking of the frangible seal 14270 which can result in the medicament being injected before the auto-injector 14002 is actuated. Moreover, because the mixing process occurs at a substantially constant volume, in some embodiments, the first medicament containing portion 14283 and/or the second medicament containing portion 14285 can be devoid of a gas (e.g., the first medicament 14268 and/or the second medicament 14269 can be stored in a vacuum).

Similarly, because the first plunger 14284 remains substantially in the first position within the medicament container 14262 when the second plunger 14282 moves proximally within the medicament container 14262 from the second position to the third position, the flow rate of the first medicament 14268 into the second medicament containing portion 14285 is dependent only on the velocity of the second plunger 14282 when moving between the second position and the third position. Accordingly, the flow rate of the first medicament 14268 can be adjusted by adjusting the velocity of the second plunger 14282 when moving between the second position and the third position. For example, in some embodiments, a high flow rate may be desired to enhance mixing (e.g., by creating a turbulent flow of first medicament 14268 into the second medicament containing portion 14285). The velocity of the second plunger 14282 can be adjusted, for example, by increasing the stiffness (i.e., the spring constant) of the mixing spring 14436.

The mixing safety lock 14766 (see FIG. 118) moves proximally within the housing along with the spring clip 14362 when the mixing spring 14436 moves from its compressed configuration (FIG. 110) to its expanded configuration (FIG. 128). Accordingly, when the auto-injector 14002 is in the second configuration (FIG. 128), the protrusions 14766 extending from the distal end portion 14764 of the mixing safety lock 14760 are moved outside of the openings 14719 defined by the inwardly facing protrusions 14718 of the actuation safety lock 14710 (see FIG. 120). In this manner, when the auto-injector 14002 is in the second configuration, the actuation safety lock 14710 can be removed.

During the mixing operation, when the spring clip 14362 moves proximally within the housing 14110 from a first (i.e., distal) position to a second (i.e., proximal) position. When the spring clip 14362 moves from its first position to its second position, the angled surface 14372 of the spring clip 14362 engages the surface 14194 of the housing 14110, thereby causing the inner portion 14367 to bend outwardly, as indicated by the arrow CCC in FIG. 128, when the spring clip 14362 moves proximally. Accordingly, when the spring clip 14362 is in its second position, as shown in FIG. 128, the engagement surface 14370 of the spring clip 14362 is disengaged from the engagement surface 14386 of the medicament mixer 14360, thereby decoupling the medicament mixer 14360 from the mixing spring 14436. The curved surface 14371 (see FIG. 119) of the clip 14362 improves the ease with which the medicament mixer 14360 is decoupled from the mixing spring 14436. Said another way, the curved surface 14371 helps to prevent the engagement surface 14370 of the spring clip 14362 from becoming bound with the engagement surface 14386 of the medicament mixer 14360 (e.g., by burrs, surface roughness or the like). In this manner, when the medicament mixer 14360 moves distally within the housing during the needle insertion and/or injection operations, the mixing spring 14436 is not compressed. Moreover, when the spring clip 14362 is in its second position, the point 14373 at the second end 14364 of the spring clip 14362 is received within the opening 14475 of the mixing actuator button 14468 (see FIG. 113).

FIG. 129 shows the auto-injector 14002 in a third configuration, in which the injection actuator 14510 of the auto-injector 14002 has been actuated. Before the injection actuator 14510 can be actuated, the actuation safety lock 14710 must be removed. As shown by the arrow GGG in FIG. 129, the actuation safety lock 14710 is removed by pulling it substantially normal to the longitudinal axis L_{E} of the compressed gas container 14412. When the actuation safety lock 14710 is removed, the locking protrusion 14722 (see FIG. 120) is removed from the area between the projections 14548 of the retaining rod 14540 and the opening 14522 defined by the base 14520.

After the actuation safety lock 14710 is removed, the base 14520 can be moved proximally, as shown by the arrow HHH in FIG. 129, to actuate the medicament injector 14210. When the base 14520 is moved proximally towards the housing 14110, the tapered surfaces 14550 of the projections 14548 of the retaining rod 14540 cooperate with the corresponding tapered surfaces 14524 of the base 14520 to move the extensions 14552 of the retaining rod 14540 inwardly towards each other. The inward motion of the extensions 14552 causes the engagement surfaces 14549 to become disengaged from the distal surface 14574 of the retention shoulder 14570 defined within the gas container opening 14124 defined by the housing 14110. Accordingly, the force from the spring 14560 moves the retaining rod 14540 proximally within the housing along the longitudinal axis L_{E} from a first position (FIG. 128) to a second position (FIG. 129).

Because the retaining rod 14540 is coupled to the compressed gas container 14412, when the rod 14540 is moved from its first (engaged) position to its second (actuated) position, the compressed gas container 14412 is moved proximally within the housing 14110 into engagement with the puncturing element 14620, as shown by the arrow III in FIG. 129. The puncturing element 14620 pierces the frangible surface 14418 at the proximal end portion 14414 of the compressed gas container 14412 thereby releasing a compressed gas into the gas chamber 14120 (see FIG. 130) to actuate the medicament injector 14210.

The pressurized gas released from the compressed gas container 14412 produces a force on the boundary of the gas chamber 14120, including the surface 14322 of the movable member 14312. This force causes the movable member 14312 and the medicament container 14262 move together distally within the injector opening 14190 of the housing 14110, as indicated by the arrow JJJ in FIG. 130, which shows the auto-injector 14002 in a fourth configuration. When in the fourth configuration, the needle 14212 is disposed through the opening 14532 defined by the base 14520 to an area outside of the auto-injector 14002. In this manner, the needle 14212 is automatically inserted into the patient (e.g., the needle insertion operation).

When the auto-injector 14002 is moving between the third configuration (FIG. 129) and the fourth configuration (FIG. 130), the frangible seal 14270 remains intact so that the needle 14212 remains fluidically isolated from the second medicament containing portion 14285 of the medicament container 14210. In this manner, the needle 14212 can be inserted into a patient as the auto-injector 14002 moves between its third configuration and its fourth configuration without injecting the medicament until after insertion is completed. Said another way, the needle insertion operation is distinct from the medicament injection operation.

When the auto-injector 14002 is moving between the third configuration (FIG. 129) and the fourth configuration (FIG. 130), a portion of the spring clip 14362 moves within the longitudinal opening 14385 of the spring engagement portion 14382 of the medicament mixer 14360 (see FIG. 116). In this manner, the medicament injector 14210 can move distally within the housing without compressing the mixing spring-14436.

During the needle insertion operation, the volume V2' of the second medicament containing portion 14285 and the volume V1' of the first medicament containing portion 14283 remain substantially constant. In this manner, the needle insertion operation is independent from the medicament injection operation. As shown in FIG. 130, when the needle insertion operation is completed, the retraction spring 14350 is fully compressed, preventing further distal movement of the medicament container 14262 within the housing 14110. Because the distal motion of the medicament container 14262 is opposed, the force exerted by the pressurized gas on the surface 14322 of the movable member 14312 increases until the frangible seal 14270 breaks, thereby placing the lumen 14217 of the needle 14212 is in fluid communication with the second medicament containing portion 14285 of the medicament container 14262.

Once the needle 14212 is in fluid communication with the medicament container 14262, the force from the pressurized gas causes the movable member 14312 to collectively move the first plunger 14284 and the second plunger 14282 within the medicament container 14262, as shown by arrow KKK in FIG. 131, thereby expelling the medicament through the needle 14212. The movable member 14312 moves a predetermined distance within the medicament container 14262, at which point the pin 14396 (see FIG. 115) contacts the proximal portion 14264 of the medicament container 14262. The continued distal movement of the movable member 14212 and the medicament mixer 14360 moves the pin 14396 proximally within the opening 14395, as shown by the arrow LLL in FIG. 131. In this manner, the pin 14396 actuates the gas release valve 14328 to allow the pressurized gas within the gas chamber 14120 to escape via the opening 14326 in the movable member 14312.

When the pressurized gas flows out of the gas chamber 14120, the pressure exerted on the surface 14322 of the movable member 14312 decreases. Accordingly, the force exerted by the retraction springs 14350 is sufficient to move the medicament injector 14210 proximally within the housing 14110, as shown by arrow MMM, into a sixth (or retracted) configuration as shown in FIG. 132. Because the medicament injector 14210 and the medicament mixer 14360 move proximally together, the pin 14396 remains engaged with the valve 14328 so that the opening 14326 remains in fluid communication with the gas chamber 14120 independent of the position of the movable member 14312. Such an arrangement ensures that all of the pressurized gas flows out of the gas chamber 14120, thereby ensuring that the medicament injector 14210 returns to the sixth configuration and does not oscillate between the sixth configuration and the fifth configuration, which could lead to the needle 14212 not being fully retracted into the housing 14110.

Although the auto-injector 14002 is shown and described as having six different configurations that are different from each other, in some embodiments, certain configuration of an auto-injector can be the same as another configuration. For example, in some embodiments, a "pre-actuation configuration can be the same as a "retracted" configuration. In other embodiments, any of the functions described above can be accomplished when an auto-injector is moved between any number of different configurations.

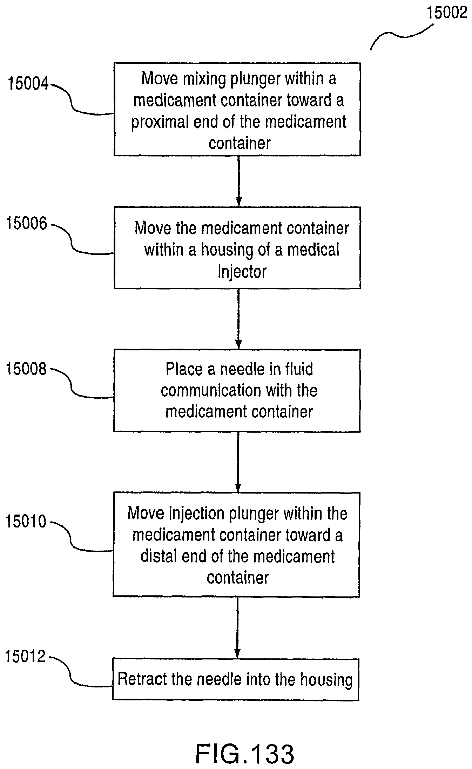

FIG. 133 is a flow chart of a method 15002 according to an embodiment of the invention. The method 15002 includes moving a mixing plunger within a medicament container toward a proximal end of the medicament container, 15004. As described above, the medicament container includes the mixing plunger and an injection plunger. The injection plunger is disposed at the proximal end of the medicament container. The mixing plunger is disposed within the medicament container between a distal end of the medicament container and the injection plunger. In this manner, the medicament container can define a first medicament containing portion between the injection plunger and the mixing plunger. Similarly, the medicament container can define a second medicament containing portion between the mixing plunger a distal end portion of the medicament container.

The injection plunger is then moved within the medicament container toward a distal end of the medicament container to expel a medicament contained within the medicament container, 15010.

In some embodiments, the first medicament containing portion and the second medicament containing portion are placed in fluid communication when the mixing plunger moves, thereby allowing a substance contained in the first medicament containing portion to be conveyed into the second medicament containing portion. For example, in some embodiments, the first medicament containing portion can include a liquid substance and the second medicament containing portion can include a solid substance. Accordingly, when the mixing plunger moves, the solid substance can be mixed and/or combined with the liquid substance to produce the medicament.

In some embodiments, the mixing plunger can be moved without moving the injection plunger. In this manner, as described above, the total volume of the first medicament containing portion and the second medicament containing portion remains constant when the mixing plunger is moved. Accordingly, a first medicament and a second medicament can be mixed at a substantially constant volume and/or a substantially constant pressure.

In some embodiments, the mixing plunger can be moved by actuating a first energy storage member, such as, for example, a spring, an electronic actuator, a magnetic actuator, a pneumatic actuator (e.g., a compressed gas container) and/or a hydraulic actuator. Similarly, the injection plunger can be moved by actuating a second energy storage member different than the first energy storage member.

In some embodiments, the method 15002 can optionally include moving the medicament container within a housing of a medical injector, 15006. In this manner, as described above, a needle can be disposed from a distal end of the housing. Similarly, in some embodiments, the method 15002 can optionally include placing the needle in fluid communication with the medicament container, 15008. In this manner, the mixing operation (e.g., operation 15004) and the insertion operation (e.g., operation 15006) can be done independently from the injection operation (15010).

In some embodiments, the method 15002 can optionally include retracting the needle into the housing, 15012.

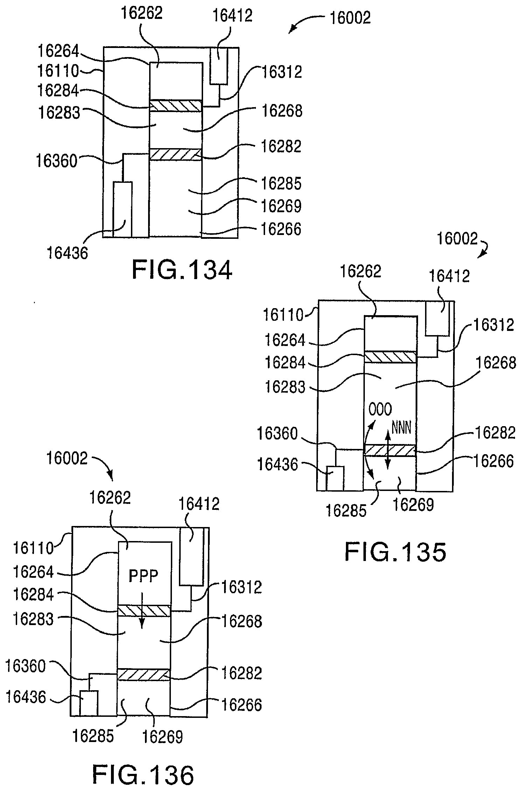

Although the auto-injectors are shown and described above as including a mixing plunger configured to move proximally within a medicament container, in some embodiments, an auto-injector can include a mixing plunger configured to move either proximally or distally within a medicament container. For example, FIGS. 134 - 136 are schematic illustrations of a medical device 16002 according to an embodiment of the invention in a first configuration, a second configuration and a third configuration, respectively. The medical device 16002 can be any suitable device for delivering a medicament into a body, such as for example, a syringe, a medical injector, an auto-injector or the like. The medical device 16002 includes a housing 16110 that contains a medicament container 16262, a first energy storage member 16412 and a second energy storage member 16436.

The medicament container 16262 has a proximal end portion 16264 and a distal end portion 16266. The medicament container 16262 includes a first plunger 16284 and a second plunger 16282. As shown in FIG. 134, when the medical device 16002 is in the first configuration, the first plunger 16284 is disposed in a first position within the medicament container 16262. The second plunger 16282 is disposed in a second position within the medicament container 16262. The second position is spaced apart from the first position such that a first medicament containing portion 16283 is defined between the first plunger 16284 and the second plunger 16282. Similarly, a second medicament containing portion 16285 is defined between the second plunger 16282 and the distal end portion 16266 of the medicament container 16262. As described above, in some embodiments, the first medicament containing portion 16283 can include a liquid medicament 16268, such as a water, and the second medicament containing portion 16285 can include a second medicament 16269, such as a lyophilized powder.

The first energy storage member 16412 is operatively coupled to the first plunger 16284 by a first movable member 16312. Although the first movable member 16312 is shown as being physically coupled to the first energy storage member 16412 and the first plunger 16284, in some embodiments, the first movable member 16312 can be electronically coupled to the first energy storage member 16412 and/or the first plunger 16284. Similarly, the second energy storage member 16436 is operatively coupled to the second plunger 16282 by a second movable member 16360. Although the second movable member 16360 is shown as being physically coupled to the second energy storage member 16436 and the second plunger 16282, in some embodiments, the second movable member 16360 can be electronically coupled to the second energy storage member 16436 and/or the second plunger 16282.

The first energy storage member 16412 and/or the second energy storage member 16436 can be any suitable energy storage member configured to produce a force when moved between a first configuration (FIG. 134) and a second configuration (FIGS. 135 and 136). The first energy storage member 16412 and/or the second energy storage member 16436 can be, for example, a mechanical energy storage member (e.g., a spring), an electrical energy storage member (e.g., a battery or a capacitor), a chemical energy storage member (e.g., a container containing two substances that can react to produce energy), a magnetic energy storage member or the like. In some embodiments, the first energy storage member 16412 can be of a different type than the second energy storage member 16436.

As shown in FIG. 135, when the medical device 16002 is in the second configuration, the second energy storage member 16436 is in a second configuration. Accordingly, the second energy storage member 16436 produces a force to move the second plunger 16282 within the medicament container 16262, as shown by the arrow NNN in FIG. 135. When the medical device 16002 is in the second configuration, the second plunger 16282 can be moved either proximally or distally within the medicament container.

In some embodiments, the first medicament containing portion 16283 can be fluidically isolated from the second medicament containing portion 16285 when the medical device 16002 is in the first configuration. The first medicament containing portion 16283 can be in fluid communication with the second medicament containing portion 16285 when the medical device 16002 is moving from the first configuration to the second configuration, as shown by the arrow OOO in FIG. 135. Accordingly, as described above, the medical device 16002 can combine and/or mix the first medicament 16268 with the second medicament 16269 contained in the second medicament containing portion 16285 to produce a mixture suitable for delivery via the medical device 16002.

As shown in FIG. 136, when the medical device 16002 is in the third configuration, the first energy storage member 16412 is in a second configuration. Accordingly, the first energy storage member 16412 produces a force to move the first plunger 16284 within the medicament container 16262, as shown by the arrow PPP in FIG. 136. As shown in FIG. 136, when the medical device 16002 is in the third configuration, the first plunger 16284 and the second plunger 16282 are collectively moved distally within the medicament container 16262. In this manner, the first medicament 16268 and the second medicament 16269 can be collectively expelled from the distal end portion 16266 of the medicament container 16262 (e.g., via a needle).

Because the auto-injector 16002 includes a first energy storage member 16412 and a second energy storage member 16436 different from the first energy storage member, the first plunger 16284 and the second plunger 16282 can be moved independently from each other. For example, in some embodiments, the second plunger 16282 can be repeatedly moved proximally and distally within the medicament container 16262 to mix the first medicament 16268 and the second medicament 16269. In other embodiments, the second plunger 16282 can move in a first direction (e.g., proximally) when the first plunger 16284 is moving in a second direction opposite the first direction (e.g., distally) to mix the first medicament 16268 and the second medicament 16269. This arrangement ensures that the mixing operation and the injection operations are distinct from each other.

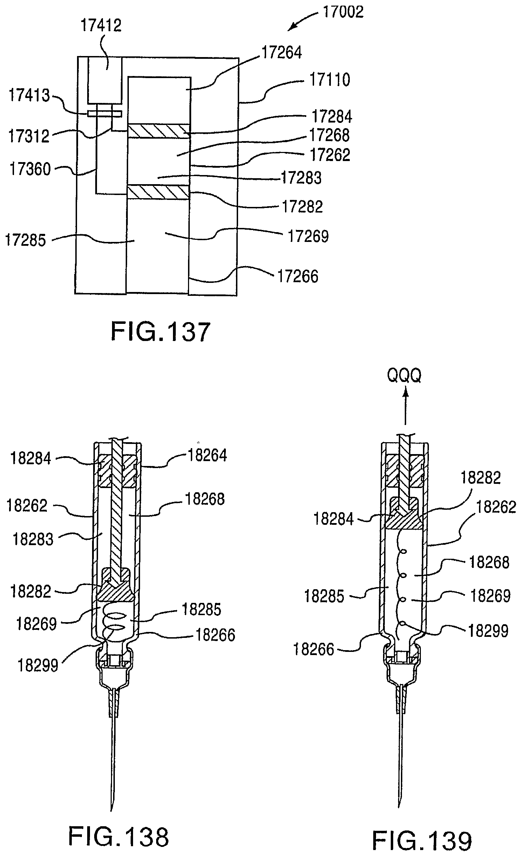

Although the auto-injectors are shown and described above as including different energy storage members, in some embodiments an auto-injector can include one energy storage member configured to independently move a mixing plunger and an injection plunger. For example, FIG. 137 is a schematic illustration of a medical device 17002 according to an embodiment of the invention. The medical device 17002 includes a housing 17110 that contains a medicament container 17262 and an energy storage member 17412, such as, for example, a compressed gas container.

The medicament container 17262 has a proximal end portion 17264 and a distal end portion 17266. The medicament container 17262 includes a first plunger 17284 and a second plunger 17282. As described above, when the medical device 17002 is in its initial configuration, the first plunger 17284 and the second plunger 17282 are arranged within the medicament container to define a first medicament containing portion 17283 and a second medicament containing portion 17285. As described above, in some embodiments, the first medicament containing portion 17283 can include a liquid medicament 17268 and the second medicament containing portion 17285 can include a solid medicament 17269 (e.g., a lyophilized powder).

The energy storage member 17412 is operatively coupled to the first plunger 17284 by a first movable member 17312. Similarly, the energy storage member 17412 is operatively coupled to the second plunger 17282 by a second movable member 17360. A switch 17413 is operatively disposed between the first movable member 17312, the second movable member 14360 and the energy storage member 17412. In use, the switch, which can be a valve, a mechanical linkage, an electronic switch or the like, selectively transmits the force produced by the energy storage member 17412 to the first movable member 17312 and the second movable member 14360. In this manner, the first plunger 17284 and the second plunger 17282 can be moved independently from each other by the energy storage member 17412.

For example, in some embodiments, the second plunger 17282 can be moved proximally within the medicament container 17262 to mix the first medicament 16268 and the second medicament 16269. When the second movable member 17360 reaches a predetermined point, the second movable member 17360 can actuate the switch 17413, thereby operatively disconnecting the energy storage member 17412 from the second movable member 17360 and operatively coupling the energy storage member 17412 to the first movable member 17312. As described above, this arrangement ensures that the mixing operation and the injection operations are distinct from each other.

While various embodiments of the invention have been described above, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above.

Although various embodiments have been described as having particular features and/or combinations of components, other embodiments are possible having a combination of any features and/or components from any of embodiments where appropriate. For example, in some embodiments, an auto-injector can include multiple medicament selectors and a medicament mixer. In this manner, a user can first select the type and/or amount of a first medicament and the type and/or amount of a second medicament to be mixed to produce an injectable medicament.

Certain components of the auto-injector 14002 are shown and described as being coupled together via protrusions and mating recesses. The protrusions and/or recesses can be disposed on any of the components to be coupled together and need not be limited to only a certain component. For example, the actuation safety lock 14710 is shown as defining two openings 14719 that receive corresponding protrusions 14766 extending from the distal end portion 14764 of the mixing safety lock 14760. In some embodiments, however, the protrusions can be disposed on the actuation safety lock and the mating openings can be defined by the mixing safety lock. In other embodiments, two or more components can be coupled together in any suitable way, which need not include protrusions and mating recesses. For example, in some embodiments, two or more components can be coupled together via mating shoulders, clips, adhesive and the like.

Similarly, although certain components of the auto-injector 14002 are shown and described as being constructed from multiple separate components, in some embodiments, such components can be monolithically constructed. For example, the proximal cover 14162 is shown and described as including a spring clip 14362 and a mixing actuator safety lock 14760 that are constructed separately and then coupled together. In other embodiments, a spring clip and a mixing actuator safety lock can be constructed monolithically.

Although the second plunger 14282 is shown and described as being coupled to the medicament mixer 14360 by a plunger coupling 14392 that is disposed within the second plunger 14282, in other embodiments the second plunger 14282 can be coupled to the medicament mixer 14360 by any suitable means. For example, in some embodiments, a second plunger can be coupled to a medicament mixer by an adhesive. In other embodiments, a second plunger can be pivotably coupled to a medicament mixer by a ball and socket joint. In yet other embodiments, a second plunger can be coupled to a medicament mixer by a plunger coupling that engages the distal surface of the second plunger.

Although the medicament containers shown and described above include two plunger and two medicament containing portions, in some embodiments, a medicament container can include more than two plungers and/or more than two medicament containing portions. For example, in some embodiments a medicament container can include a first medicament containing portion configured to contain a first medicament, a second medicament containing portion configured to store contain a second medicament and mixing portion configured to receive and mix the first medicament and the second medicament when the mixing actuator is actuated.

Although the rod 14540 is shown and described as being an elongated member that is released by being elastically deformed, in some embodiments, a rod can be of any suitable shape and in any suitable orientation within the housing. Moreover, in some embodiments, a rod can be released by being plastically deformed. For example, in some embodiments, a rod can be disposed along an axis that is offset from the longitudinal axis of the energy storage member. In some embodiments, the rod can be configured to break upon actuation.

Although the spring clip 14362 is shown and described above as being deformable to decouple the medicament mixer 14360 from mixing spring 14436 after the auto-injector 14002 is in the second configuration (FIG. 128), in other embodiments, the medicament mixer 14360 can be decoupled from the mixing spring 14436 in any suitable manner. For example, in some embodiments, the medicament mixer 14360 can be coupled to the mixing spring 14436 by a frangible clip configured to break when the auto-injector is in the second configuration.

Although the auto-injector 14002 is shown and described above without reference to a needle guard, in other embodiments an auto-injector can include any suitable needle guard. For example, in some embodiments, an auto-injector can include a rigid needle guard. In other embodiments, an auto-injector can include a flexible needle guard. In other embodiments, an auto-injector can include a needle guard that also functions as a mixing actuator safety cover (e.g., removal of the needle guard also enables the mixing actuator). Such needle guards can be made of any suitable material, such as, for example, polypropylene, rubber or any other elastomer.

Although the auto-injector 14002 is shown and described as including a puncturing element 14620 to puncture a portion of the compressed gas container 14412, in other embodiments any suitable gas release mechanism can be used. For example, in some embodiments, a gas release mechanism can include an actuator configured to actuate a valve that controls the flow of gas out of the compressed gas container. In some embodiments, a compressed gas container can include a spring loaded check ball and the gas release mechanism can include an actuator configured to engage and depress the check ball to release pressurized gas from the compressed gas container.

Although the auto-injector 14002 is shown and described as including a compressed gas cylinder 14412, in other embodiments an auto-injector can include any suitable energy storage member. For example, in some embodiments, an auto-injector can include a mechanical energy storage member, such as a spring, an electrical energy storage member, such as a battery or a capacitor, a chemical energy storage member, such as a container containing two substances that can react to produce energy, a magnetic energy storage member or the like. Similarly, although the auto-injector 14002 is shown and described as including a mixing spring 14436, in other embodiments an auto-injector can include any suitable energy storage member to move the medicament mixer.

Although the auto-injector 14002 has been shown and described having a housing 14110 having a substantially rectangular shape, in some embodiments, an auto-injector can have a housing having any shape. In some embodiments, for example, an auto-injector can have a substantially cylindrical shape. In other embodiments, for example, the auto-injector can have an irregular and/or asymmetrical shape.

Although the auto-injectors are shown and described above as including a mixing plunger that moves within a medicament container to combine and/or mix a first medicament and a second medicament, in some embodiments, the user can shake the auto-injector to enhance the mixing process. In other embodiments, an auto-injector can include an agitator to enhance the mixing process. For example, FIGS. 138 and 139 show a medicament container 18262 according to an embodiment of the invention in a first configuration and a second configuration, respectively. The medicament container 18262 includes a proximal end portion 18264 and a distal end portion 18266. The medicament container 18262 includes a first plunger 18284, a second plunger 18282 and an agitator 18299.

The second plunger 18282 is disposed distally from the first plunger 18284. The first plunger 18284 and the second plunger 18282 are each movably disposed within the medicament container 18262. The agitator, which can be, for example, a flexible wire, an elastic member or the like, is coupled to the second plunger 18282.

As shown in FIG. 138, when the medical device 18002 is in the first configuration, the first plunger 18284 is disposed in a first position within the medicament container 18262. The second plunger 18282 is disposed in a second position within the medicament container 18262. The second position is spaced apart from the first position such that a first medicament containing portion 18283 is defined between the first plunger 18284 and the second plunger 18282. Similarly, a second medicament containing portion 18285 is defined between the second plunger 18282 and the distal end portion 18266 of the medicament container 18262.

As described above, when the second plunger 18282 moves within the medicament container 18262, the first medicament containing portion 18283 is placed in fluid communication with the second medicament containing portion 18285. Accordingly, when the second plunger 18282 moves, the contents of the first medicament containing portion 18283 can be mixed with the contents of the second medicament containing portion 18285.

When the medicament container 18262 moves from the first configuration (FIG. 138) to the second configuration (FIG. 139), the agitator moves from its retracted position to an expanded position. In this manner, the agitator can enhance the mixing of the medicaments contained in the medicament container 18262.

Although the agitator 18299 is described as a flexible member, in other embodiments, an agitator can include any suitable mechanism for enhancing the mixing of medicaments within the medicament container. For example, in some embodiments, an agitator can include angled flow passages within the second plunger to produce a swirl effect when the first medicament containing portion is placed in fluid communication with the second medicament containing portion. In other embodiments, fluid communication between the first medicament containing portion and the second medicament containing portion can be established by a channel within a side wall of the medicament container. In such embodiments, the channel can be a spiral channel to produce a turbulent flow to enhance the mixing of medicaments within the medicament container.

Although the auto-injector 14002 is shown and described as mixing the medicaments contained therein at a substantially constant volume, which can allow the first medicament containing portion 14283 and/or the second medicament containing portion 14285 to be devoid of a gas, in other embodiments, the first medicament containing portion 14283 and/or the second medicament containing portion 14285 can contain a gas (e.g., air). In such embodiments, an auto-injector can include an air purge valve to purge the air contained within the medicament after the medicament is mixed, but before the medicament is injected. Such an air purge valve can be operatively coupled to the actuation safety lock and/or the medicament mixer.

Although the auto-injector 14002 is shown and described as being devoid of an electronic circuit system, in other embodiments, an auto-injector can include an electronic circuit system configured to output an electronic output. Such an electronic circuit system can be an electronic circuit system of the types shown and described in U.S. Patent Application Serial No. 11/671,025, entitled "Devices, Systems and Methods for Medicament Delivery," filed February 5, 2007. For example, in some embodiments, an auto-injector can include a electronic circuit system configured to sense the status of the medicaments contained therein (e.g., unmixed, partially mixed, fully mixed, or the like) and output an electronic output in response to such status. In other embodiments, an auto-injector can include an electronic circuit system configured to output an instruction associated with the mixing operation as described herein.

Although the medical device are shown and described above as being medicament delivery device including a medicament and/or a needle, in other embodiments, a medical device can be a simulated medicament delivery device. In some embodiments, for example, a simulated auto-injector can correspond to an actual auto-injector (e.g., auto-injector 14002) and can be used, for example, to train a user in the operation of the corresponding actual auto-injector.

In some embodiments, a simulated auto-injector can be devoid of a medicament and/or those components that cause the medicament to be delivered (e.g., a needle). In this manner, the simulated auto-injector can be used to train a user in the use of the actual medicament delivery device without exposing the user to a needle and/or a medicament. Moreover, the simulated auto-injector can have features to identify it as a training device to prevent a user from mistakenly believing that the simulated auto-injector can be used to deliver a medicament. Examples of such simulated auto-injectors are described in U.S. Patent Application Serial No. 11/679,331, entitled "Medical Injector Simulation Device," filed February 27, 2007.

## Claims

1. An apparatus, comprising:
a medicament delivery device (3002, 4002, 6002, 7002) configured to deliver a medicament, the medicament delivery device (3002, 4002, 6002, 7002) including:
a housing (3110, 4110, 6110, 7110);
an electronic circuit system (4900, 7920) coupled to the housing, the electronic circuit system (4900, 7920) configured to output an electronic signal when the electric circuit system (4900, 7920) is actuated, the electronic signal associated with a use of the medicament delivery device (3002, 4002, 6002, 7002), the electronic circuit system (4900, 7920) including a switch (4972, 7972) disposed on a printed circuit board (4922), the electronic circuit system (4900, 7920) configured to be actuated when the switch is moved from a first state to a second state;
**characterized in that** the apparatus further comprises:
a base (3520, 4520, 7520) coupled to a distal end portion (4114) of the housing, the base (3520, 4520, 7520) configured to be moved to actuate the medicament delivery device (3002, 4002, 6002, 7002); and
a lock member (3710, 4710, 6710, 7710) movably coupled to the housing, the lock member (3710, 4710, 6710, 7710) configured to limit movement of the base (3520, 4520, 7520) when in a first position, the lock member (3710, 4710, 6710, 7710) configured to engage a portion (4926) of the printed circuit board (4922) to move the switch from the first state to the second state when the lock member (3710, 4710, 6710, 7710) is moved from the first position to a second position.

2. The apparatus of claim 1, wherein:
the medicament delivery device (3002, 4002, 6002, 7002) includes an energy storage member (2410, 3412) configured to produce a force to deliver the medicament from within a medicament container (3262, 7262) when the medicament delivery device (3002, 4002, 6002, 7002) is actuated; and
the base (3520, 4520, 7520) is configured to engage a rod (3540) to actuate the energy storage member when the base (3520, 4520, 7520) is moved.

3. The apparatus of claim 2, wherein the energy storage member is a non-electronic mechanism configured to produce the force.

4. The apparatus of claim 3, wherein the non-electronic mechanism (2410, 3412) is a compressed gas container.

5. The apparatus of claim 2, wherein the medicament includes epinephrine.

6. The apparatus of any of claims 1-5, wherein the portion of the printed circuit board (4922) defines an opening (4928A, 4928B) adjacent the switch, the opening configured to receive a protrusion (4730) of the lock member (3710, 4710, 6710, 7710).

7. The apparatus of any of claims 1-5, wherein:
the switch includes an electrical conductor (4934) disposed on the printed circuit board (4922); and
the protrusion of the lock member (3710, 4710, 6710, 7710) is configured move substantially parallel to a surface of the printed circuit board (4922) to tear the portion of the printed circuit board (4922), thereby severing the electrical conductor.

8. The apparatus of any of claims 1-5, wherein:
the portion of the printed circuit board (4922)defines an opening (4928A, 4928B) adjacent the switch, the opening configured to receive the protrusion of the lock member (3710, 4710, 6710, 7710);
the protrusion of the lock member (3710, 4710, 6710, 7710) is configured move relative to the printed circuit board (4922) to produce a tear in the portion of the printed circuit board (4922), thereby severing a portion of the switch; and
the opening is configured to propagate the tear in a predetermined direction.

9. The apparatus of any of claims 1-5, wherein:
the portion of the printed circuit board (4922) defines an opening (4928A, 4928B) adjacent the switch, the opening configured to receive the protrusion of the lock member (3710, 4710, 6710, 7710); and
a boundary (4930) of the opening has a discontinuous shape.

10. The apparatus of any of claims 1-5, wherein the switch is a microswitch mounted to the portion of the printed circuit board (4922).

11. The apparatus of any of claims 1-5, wherein:
the electronic circuit system (4900, 7920) includes a speaker (4956); and
the electronic signal produces an audible output via the speaker, the audible output of the being recorded speech output.

12. The apparatus of any of claims 1-5, wherein the electronic circuit system (4900, 7920) includes a network communication interface configured to transmit a wireless signal to a remote computing device.

13. The apparatus of any of claims 1-5, wherein:
the electronic signal is a first electronic signal;
the base (3520, 4520, 7520) is configured to actuate the electronic circuit system (4900, 7920) such that the electronic circuit system (4900, 7920) outputs a second electronic signal when the base (3520, 4520, 7520) is moved from a first base (3520, 4520, 7520) position to a second base (3520, 4520, 7520) position.

14. The apparatus of claim 2, wherein:
a portion (3722) of the lock member (3710, 4710, 6710, 7710) is configured to engage the rod (3540) to limit movement of the rod (3540) when the lock member (3710, 4710, 6710, 7710) is in the first position, the portion of the lock member (3710, 4710, 6710, 7710) spaced apart from the rod (3540) when the lock member is in the second position.

15. The apparatus of claims 1-5, wherein the lock member (3710, 4710, 6710, 7710) includes any one of a removable cap, a protective barrier, a safety tab or a needle guard.

16. The apparatus of any of claims 1-5, wherein:
the electronic signal is a first electronic signal;
the switch is a first switch (4972A);
the portion of the printed circuit board (4922) is a first portion;
the electronic circuit system (4900, 7920) including a second switch (4972B) disposed on the printed circuit board (4922), the electronic circuit system (4900, 7920) configured to output a second electronic signal when the second switch is moved from a first state to a second state; and
a portion (4538) of the base (3520, 4520, 7520) is configured to engage a second portion of the printed circuit board (4922) to move the second switch from its first state to its second state when the base (3520, 4520, 7520) is moved from a first base (3520, 4520, 7520) position to a second base (3520, 4520, 7520) position.

17. The apparatus of any of claims 1-5, further comprising:
an isolation member (4860) configured to move relative to the electronic circuit system (4900, 7920), the isolation member electrically isolating the electronic circuit system (4900, 7920) from a power source (4962) when the isolation member is in a first isolation position, the electronic circuit system (4900, 7920) electrically coupled to the power source when the isolation member is in a second isolation position.

18. The apparatus of claim 17, wherein a portion of the isolation member is in contact with a portion of the power source when the isolation member is in the first isolation position, the isolation member disposed apart from the portion of the power source when the isolation member is in the second isolation position.

19. The apparatus of any of claims 1-5, wherein the electronic output causes a first audible output, the apparatus further comprising:
a cover member (3810, 4810) movably coupled to the housing, an isolation portion (4860) of the cover member in contact with the electronic circuit system (4900, 7920) when the cover member is in a first cover position, an outer portion (3840, 4840) of the cover member disposed about a portion of the base (3520, 4520, 7520) when the cover member is in the first cover position, the isolation portion spaced apart from the electronic circuit system (4900, 7920) and the outer portion spaced apart from the base (3520, 4520, 7520) when the cover member is in a second cover position, the isolation portion configured to actuate the electronic circuit system (4900, 7920) when the cover member is moved from the first cover position to the second cover position,
the electronic circuit system (4900, 7920) configured to produce a second audible output when the electronic circuit system (4900, 7920) is actuated by the isolation portion of the cover member.

20. The apparatus of claim 19, wherein:
the electronic circuit system (4900, 7920) includes a processor and a power source; and
the isolation portion of the cover member is disposed between a terminal of the power source an electrical contact when the cover member is in the first cover position, the first portion being spaced apart from the terminal of the power source to electrically couple the processor to the power source when the cover member is in the second cover position.

21. The apparatus of claims 1-5, wherein the portion of the lock member (3710, 4710, 6710, 7710) includes a protrusion (4730) configured to tear the portion of the printed circuit board (4922) when the lock member (3710, 4710, 6710, 7710) is moved from the first position to the second position.

22. The apparatus of any of claims 1-5, wherein the switch is configured to be irreversibly moved from the first state to the second state.

23. The apparatus of any of claims 1-5, wherein the medicament delivery device (3002, 4002, 6002, 7002) is any one of a pen injector, an auto-injector, an inhaler or a transdermal delivery device.

## Patentansprüche

1. Einrichtung, umfassend:
eine Medikamentenabgabevorrichtung (3002, 4002, 6002, 7002), die konfiguriert ist, um ein Medikament abzugeben, wobei die Medikamentenabgabevorrichtung (3002, 4002, 6002, 7002) enthält:
ein Gehäuse (3110, 4110, 6110, 7110);
ein elektronisches Schaltungssystem (4900, 7920), das an das Gehäuse gekoppelt ist, wobei das elektronische Schaltungssystem (4900, 7920) konfiguriert ist, um ein elektronisches Signal auszugeben, wenn das elektronische Schaltungssystem (4900, 7290) betätigt wird, wobei das elektronische Signal einer Verwendung der Medikamentenabgabevorrichtung (3002, 4002, 6002, 7002) zugeordnet ist, wobei das elektronische Schaltungssystem (4900, 7920) einen Schalter (4972, 7972) enthält, der auf einer Leiterplatte (4922) angeordnet ist, wobei das elektronische Schaltungssystem (4900, 7920) konfiguriert ist, um betätigt zu werden, wenn der Schalter aus einem ersten Zustand in einen zweiten Zustand bewegt wird;
**dadurch gekennzeichnet, dass** die Einrichtung ferner umfasst:
eine Basis (3520, 4520, 7520), die an einen distalen Endabschnitt (4114) des Gehäuses gekoppelt ist, wobei die Basis (3520, 4520, 7520) konfiguriert ist, um bewegt zu werden, um die Medikamentenabgabevorrichtung (3002, 4002, 6002, 7002) zu betätigen; und
ein Sperrelement (3710, 4710, 6710, 7710), das beweglich an das Gehäuse gekoppelt ist, wobei das Sperrelement (3710, 4710, 6710, 7710) konfiguriert ist, um die Bewegung der Basis (3520, 4520, 7520) zu begrenzen, wenn in einer ersten Position, wobei das Sperrelement (3710, 4710, 6710, 7710) konfiguriert ist, um einen Abschnitt (4926) der Leiterplatte (4922) in Eingriff zu nehmen, um den Schalter aus dem ersten Zustand in den zweiten Zustand zu bewegen, wenn das Sperrelement (3710, 4710, 6710, 7710) von der ersten Position in eine zweite Position bewegt wird.

2. Einrichtung nach Anspruch 1, wobei:
die Medikamentenabgabevorrichtung (3002, 4002, 6002, 7002) ein Energiespeicherelement (2410, 3412) enthält, das konfiguriert ist, um eine Kraft zu erzeugen, die das Medikament aus einem Medikamentenbehälter (3262, 7262) abgeben kann, wenn die Medikamentenabgabevorrichtung (3002, 4002, 6002, 7002) betätigt wird; und
die Basis (3520, 4520, 7520) konfiguriert ist, um einen Stab (3540) in Eingriff zu nehmen, um das Energiespeicherelement zu betätigen, wenn die Basis (3520, 4520, 7520) bewegt wird.

3. Einrichtung nach Anspruch 2, wobei das Energiespeicherelement ein nichtelektronischer Mechanismus ist, der konfiguriert ist, um die Kraft zu erzeugen.

4. Einrichtung nach Anspruch 3, wobei der nichtelektronische Mechanismus (2410, 3412) ein komprimierter Gasbehälter ist.

5. Einrichtung nach Anspruch 2, wobei das Medikament Epinephrin enthält.

6. Einrichtung nach einem der Ansprüche 1 bis 5, wobei der Abschnitt der Leiterplatte (4922) eine Öffnung (4928A, 4928B) neben dem Schalter definiert, wobei die Öffnung konfiguriert ist, um einen Vorsprung (4730) des Sperrelementes (3710, 4710, 6710, 7710) aufzunehmen.

7. Einrichtung nach einem der Ansprüche 1 bis 5, wobei:
der Schalter einen elektrischen Leiter (4934) enthält, der auf der Leiterplatte (4922) angeordnet ist; und
der Vorsprung des Sperrelementes (3710, 4710, 6710, 7710) konfiguriert ist, um sich im Wesentlichen parallel zu einer Oberfläche der Leiterplatte (4922) zu bewegen, um den Abschnitt der Leiterplatte (4922) abzureißen, wobei dadurch der elektrische Leiter getrennt wird.

8. Einrichtung nach einem der Ansprüche 1 bis 5, wobei:
der Abschnitt der Leiterplatte (4922) eine Öffnung (4928A, 4928B) neben dem Schalter definiert, wobei die Öffnung konfiguriert ist, um den Vorsprung des Sperrelements (3710, 4710, 6710, 7710) aufzunehmen;
der Vorsprung des Sperrelementes (3710, 4710, 6710, 7710) konfiguriert ist, um sich relativ zu der Leiterplatte (4922) zu bewegen, um einen Riss in dem Abschnitt der Leiterplatte (4922) zu erzeugen, wobei dadurch ein Abschnitt des Schalters getrennt wird; und
die Öffnung konfiguriert ist, um den Riss in einer vorbestimmten Richtung auszubreiten.

9. Einrichtung nach einem der Ansprüche 1 bis 5, wobei:
der Abschnitt der Leiterplatte (4922) eine Öffnung (4928A, 4928B) neben dem Schalter definiert, wobei die Öffnung konfiguriert ist, um den Vorsprung des Sperrelementes (3710, 4710, 6710, 7710) aufzunehmen; und
eine Begrenzung (4930) der Öffnung eine unterbrochene Form aufweist.

10. Einrichtung nach einem der Ansprüche 1 bis 5, wobei der Schalter ein Mikroschalter ist, der an dem Abschnitt der Leiterplatte (4922) angebracht ist.

11. Einrichtung nach einem der Ansprüche 1 bis 5, wobei:
das elektronische Schaltungssystem (4900, 7920) einen Lautsprecher (4956) enthält; und
das elektronische Signal eine hörbare Ausgabe über den Lautsprecher erzeugt, wobei die hörbare Ausgabe des eine aufgenommene Sprachausgabe ist.

12. Einrichtung nach einem der Ansprüche 1 bis 5, wobei das elektronische Schaltungssystem (4900, 7920) eine Netzwerkkommunikationsschnittstelle enthält, die konfiguriert ist, um ein drahtloses Signal an eine entfernte Rechenvorrichtung zu übertragen.

13. Einrichtung nach einem der Ansprüche 1 bis 5, wobei:
das elektronische Signal ein erstes elektronisches Signal ist;
die Basis (3520, 4520, 7520) konfiguriert ist, um das elektronische Schaltungssystem (4900, 7920) derart zu betätigen, dass das elektronische Schaltungssystem (4900, 7920) ein zweites elektronisches Signal ausgibt, wenn die Basis (3520, 4520, 7520) von einer ersten Basis- (3520, 4520, 7520) Position zu einer zweiten Basis- (3520, 4520, 7520) Position bewegt wird.

14. Einrichtung nach Anspruch 2, wobei:
ein Abschnitt (3722) des Sperrelementes (3710, 4710, 6710, 7710) konfiguriert ist, um den Stab (3540) in Eingriff zu nehmen, um eine Bewegung des Stabs (3540) zu begrenzen, wenn das Sperrelement (3710, 4710, 6710, 7710) in der ersten Position ist, wobei der Abschnitt des Sperrelementes (3710, 4710, 6710, 7710) von dem Stab (3540) weg beabstandet ist, wenn das Sperrelement in der zweiten Position ist.

15. Einrichtung nach Anspruch 1 bis 5, wobei das Sperrelement (3710, 4710, 6710, 7710) eine beliebige einer entfernbaren Kappe, einer Schutzbarriere, einer Sicherheitslasche und/oder eines Nadelschutzes enthält.

16. Einrichtung nach einem der Ansprüche 1 bis 5, wobei:
das elektronische Signal ein erstes elektronisches Signal ist;
der Schalter ein erster Schalter (4972A) ist;
der Abschnitt der Leiterplatte (4922) ein erster Abschnitt ist;
das elektronische Schaltungssystem (4900, 7920) einen zweiten Schalter (4972B) enthält, der auf der Leiterplatte (4922) angeordnet ist, wobei das elektronische Schaltungssystem (4900, 7920) konfiguriert ist, um ein zweites elektronisches Signal auszugeben, wenn der zweite Schalter aus einem ersten Zustand in einen zweiten Zustand bewegt wird; und
ein Abschnitt (4538) der Basis (3520, 4520, 7520) konfiguriert ist, um einen zweiten Abschnitt der Leiterplatte (4922) in Eingriff zu nehmen, um den zweiten Schalter aus seinem ersten Zustand in seinen zweiten Zustand zu bewegen, wenn die Basis (3520, 4520, 7520) von einer ersten Basis- (3520, 4520, 7520) Position zu einer zweiten Basis- (3520, 4520, 7520) Position bewegt wird.

17. Einrichtung nach einem der Ansprüche 1 bis 5, ferner umfassend:
ein Isolationselement (4860), das konfiguriert ist, um sich relativ zu dem elektronischen Schaltungssystem (4900, 7920) zu bewegen, wobei das Isolationselement das elektronische Schaltungssystem (4900, 7920) elektrisch von einer Stromquelle (4962) isoliert, wenn das Isolationselement in einer ersten Isolationsposition ist, wobei das elektronische Schaltungssystem (4900, 7920) mit der Stromquelle elektrisch gekoppelt ist, wenn das Isolationselement in einer zweiten Isolationsposition ist.

18. Einrichtung nach Anspruch 17, wobei ein Abschnitt des Isolationselementes mit einem Abschnitt der Stromquelle in Berührung steht, wenn das Isolationselement in der ersten Isolationsposition ist, wobei das Isolationselement von dem Abschnitt der Stromquelle weg angeordnet ist, wenn das Isolationselement in der zweiten Isolationsposition ist.

19. Einrichtung nach einem der Ansprüche 1 bis 5, wobei die elektronische Ausgabe eine erste hörbare Ausgabe bewirkt, wobei die Einrichtung ferner umfasst:
ein Abdeckungselement (3810, 4810), das beweglich mit dem Gehäuse gekoppelt ist, wobei ein Isolationsabschnitt (4860) des Abdeckungselementes mit dem elektronischen Schaltungssystem (4900, 7920) in Berührung steht, wenn das Abdeckungselement in einer ersten Abdeckungsposition ist, wobei ein äußerer Abschnitt (3840, 4840) des Abdeckungselementes um einen Abschnitt der Basis (3520, 4520, 7520) angeordnet ist, wenn das Abdeckungselement in der ersten Abdeckungsposition ist, wobei der Isolationsabschnitt von dem elektronischen Schaltungssystem (4900, 7920) weg beabstandet ist und der äußere Abschnitt von der Basis (3520, 4520, 7520) weg beabstandet ist, wenn das Abdeckungselement in einer zweiten Abdeckungsposition ist, wobei der Isolationsabschnitt konfiguriert ist, um das elektronische Schaltungssystem (4900, 7920) zu betätigen, wenn das Abdeckungselement von der ersten Abdeckungsposition in die zweite Abdeckungsposition bewegt wird,
wobei das elektronische Schaltungssystem (4900, 7920) konfiguriert ist, um eine zweite hörbare Ausgabe zu erzeugen, wenn das elektronische Schaltungssystem (4900, 7920) durch den Isolationsabschnitt des Abdeckungselementes betätigt wird.

20. Einrichtung nach Anspruch 19, wobei:
das elektronische Schaltungssystem (4900, 7920) einen Prozessor und eine Stromquelle enthält; und
der Isolationsabschnitt des Abdeckungselementes zwischen einer Klemme der Stromquelle ein elektrischer Kontakt angeordnet ist, wenn das Abdeckungselement in der ersten Abdeckungsposition ist, wobei der erste Abschnitt von der Klemme der Stromquelle weg beabstandet ist, um den Prozessor elektrisch mit der Stromquelle zu koppeln, wenn das Abdeckungselement in der zweiten Abdeckungsposition ist.

21. Einrichtung nach Anspruch 1 bis 5, wobei der Abschnitt des Sperrelementes (3710, 4710, 6710, 7710) einen Vorsprung (4730) enthält, der konfiguriert ist, um den Abschnitt der Leiterplatte (4922) abzureißen, wenn das Sperrelement (3710, 4710, 6710, 7710) von der ersten Position in die zweite Position bewegt wird.

22. Einrichtung nach einem der Ansprüche 1 bis 5, wobei der Schalter konfiguriert ist, um unabänderlich aus dem ersten Zustand in den zweiten Zustand bewegt zu werden.

23. Einrichtung nach einem der Ansprüche 1 bis 5, wobei die Medikamentenabgabevorrichtung (3002, 4002, 6002, 7002) ein beliebiger eines Stiftinj ektors, eines Autoinj ektors, eines Inhalators oder einer transdermalen Abgabevorrichtung ist.

## Revendications

1. Un appareil qui se compose des éléments suivants :
un dispositif pour l'administration d'un médicament (3002, 4002, 6002, 7002) configuré pour administrer un médicament et ce dispositif pour l'administration d'un médicament (3002, 4002, 6002, 7002) se compose :
d'un coffret (3110, 4110, 6110, 7110)
d'un système à circuit électronique (4900, 7920) raccordé au coffret, et ce système à circuit électronique (4900, 7920) est configuré pour produire un signal électronique lorsque le système à circuit électronique (4900, 7920) est activé, et ce signal électronique est associé à l'utilisation du dispositif d'administration d'un médicament (3002, 4002, 6002, 7002), et ce système à circuit électronique (4900, 7920) comporte un contacteur (4972, 7972) implanté sur un carte à circuits imprimés (4922), et ce système à circuit électronique (4900, 7920) est configuré pour une activation lorsque ce contacteur passe d'un premier état à un deuxième état
et cet appareil se **caractérise par le fait qu'**il comporte les éléments suivants :
un socle (3520, 4520, 7520) raccordé à une partie à l'extrémité distale (4114) du coffret, et ce socle (3520, 4520, 7520) est configuré pour se déplacer afin d'activer le dispositif d'administration d'un médicament (3002, 4002, 6002, 7002) et
un élément de verrouillage (3710, 4710, 6710, 7710) qui peut se déplacer afin de se raccorder au coffret, et cet élément de verrouillage (3710, 4710, 6710, 7710) est configuré pour limiter le déplacement du socle (3520, 4520, 7520) lorsqu'il est à la première position, et cet élément de verrouillage (3710, 4710, 6710, 7710) est configuré pour mettre en prise une partie (4926) de la carte à circuits imprimés (4922) afin de faire passer le contacteur du premier état au deuxième état lorsque l'élément de verrouillage (3710, 4710, 6710, 7710) passe de la première position à une deuxième position.

2. L'appareil que décrit la revendication 1, si ce n'est que :
le dispositif d'administration d'un médicament (3002, 4002, 6002, 7002) comporte un élément de stockage d'énergie (2410, 3412) configuré pour produire une force chargée d'administrer le médicament depuis l'intérieur d'un récipient contenant des médicaments (3262, 7262) lors de l'activation du dispositif d'administration d'un médicament (3002, 4002, 6002, 7002), et
le socle (3520, 4520, 7520) est configuré pour mettre en prise une tige (3540) chargée d'activer l'élément de stockage d'énergie lors du déplacement du socle (3520, 4520, 7520).

3. L'appareil que décrit la revendication 2, si ce n'est que l'élément de stockage d'énergie est un mécanisme non-électronique configuré pour produire cette force.

4. L'appareil que décrit la revendication 3, si ce n'est que le mécanisme non-électronique (2410, 3412) est un récipient contenant un gaz comprimé.

5. L'appareil que décrit la revendication 2, si ce n'est que ce médicament contient de l'épinéphrine.

6. L'appareil que décrit l'une ou l'autre des revendications 1 à 5, si ce n'est que la partie contenant la carte à circuits imprimés (4922) définit une ouverture (4928A, 4928B) à proximité immédiate du contacteur, et cette ouverture est configurée pour recevoir une partie saillante (4730) de l'élément de verrouillage (3710, 4710, 6710, 7710).

7. L'appareil que décrit l'une ou l'autre des revendications 1 à 5, si ce n'est que :
le contacteur comporte un conducteur électrique (4934) implanté sur la carte à circuits imprimés (4922) et
la partie saillante de l'élément de verrouillage (3710, 4710, 6710, 7710) est configurée pour se déplacer essentiellement parallèlement à une surface de la carte à circuits imprimés (4922) afin de provoquer le détachement de la carte à circuits imprimés (4922) et de couper ainsi le conducteur électrique.

8. L'appareil que décrit l'une ou l'autre des revendications 1 à 5, si ce n'est que :
la partie contenant la carte à circuits imprimés (4922) définit une ouverture (4928A, 4928B) à proximité immédiate du contacteur, et cette ouverture est configurée pour recevoir la partie saillante de l'élément de verrouillage (3710, 4710, 6710, 7710) et
la partie saillante de l'élément de verrouillage (3710, 4710, 6710, 7710) est configurée pour se déplacer par rapport à la carte à circuits imprimée (4922) afin de provoquer le détachement de la partie contenant la carte à circuits imprimés (4922) et de couper ainsi une partie du contacteur, et
cette ouverture est configurée pour propager ce détachement dans un sens prédéterminé.

9. L'appareil que décrit l'une ou l'autre des revendications 1 à 5, si ce n'est que :
la partie contenant la carte à circuits imprimés (4922) définit une ouverture (4928A, 4928B) à proximité immédiate du contacteur, et cette ouverture est configurée pour recevoir la partie saillante de l'élément de verrouillage (3710, 4710, 6710, 7710), et
une limite (4930) de cette ouverture a un profil discontinu.

10. L'appareil que décrit l'une ou l'autre des revendications 1 à 5, si ce n'est que le contacteur est un microrupteur implanté sur la partie contenant la carte à circuits imprimés (4922).

11. L'appareil que décrit l'une ou l'autre des revendications 1 à 5, si ce n'est que :
le système à circuit électronique (4900, 7920) comporte une enceinte haut-parleur (4956) et
que le signal électronique produit une sortie sonore en provenance de cette enceinte haut-parleur, et cette sortie sonore est un message sonore enregistré.

12. L'appareil que décrit l'une ou l'autre des revendications 1 à 5, si ce n'est que le système à circuit électronique (4900, 7920) comporte une interface de communication en réseau configurée pour transmettre un signal sans fil à un périphérique informatique à distance.

13. L'appareil que décrit l'une ou l'autre des revendications 1 à 5, si ce n'est que :
Le signal électronique est un premier signal électronique
le socle (3520, 4520, 7520) est configuré pour activer le système à circuit électronique (4900, 7920) de manière à ce que le système à circuit électronique (4900, 7920) produise un deuxième signal électronique lorsque le socle (3520, 4520, 7520) passe d'une première position du socle (3520, 4520, 7520) à une deuxième position du socle (3520, 4520, 7520).

14. L'appareil que décrit la revendication 2, si ce n'est que :
une partie (3722) de l'élément de verrouillage (3710, 4710, 6710, 7710) est configurée pour se mettre en prise avec la tige (3540) afin de limiter le déplacement de la tige (3540) lorsque l'élément de verrouillage (3710, 4710, 6710, 7710) occupe la première position, et la partie occupée par cet élément de verrouillage (3710, 4710, 6710, 7710) est à l'écart de cette tige (3540) lorsque cet élément de verrouillage occupe la deuxième position.

15. L'appareil que décrit l'une ou l'autre des revendications 1 à 5, si ce n'est que l'élément de verrouillage (3710, 4710, 6710, 7710) comporte l'un des éléments suivants : un chapeau amovible, une barrière de protection, une languette de sécurité ou un protège-aiguille.

16. L'appareil que décrit l'une ou l'autre des revendications 1 à 5, si ce n'est que :
le signal électronique est un premier signal électronique
le contacteur est un premier contacteur (4972A)
la partie contenant la carte à circuits imprimés (4922) est une première partie
le système à circuit électronique (4900, 7920) comporte un deuxième contacteur (4972B) implanté sur la carte à circuits imprimés (4922) et ce système à circuit électronique (4900, 7920) est configuré pour produire un deuxième signal électronique lorsque le deuxième contacteur passe d'un premier état à un deuxième état, et
une partie (4538) du socle (3520, 4520, 7520) est configurée pour se mettre en prise avec une deuxième partie de la carte à circuits imprimés (4922) afin de faire passer le deuxième contacteur de son premier état à son deuxième état lorsque le socle (3520, 4520, 7520) passe d'une première position de socle (3520, 4520, 75220) à une deuxième position de socle (3520, 4520, 7520).

17. L'appareil que décrit l'une ou l'autre des revendications 1 à 5, si ce n'est qu'il comporte, en outre :
un élément isolant (4860) configuré pour effecteur un déplacement par rapport au système à circuit électronique (4900, 7920), et cet élément isolant assure l'isolation électrique du système à circuit électronique (4900, 7920) par rapport à une source d'alimentation électrique (4962) lorsque cet élément isolant occupe une première position d'isolement, et ce système à circuit électronique (4900, 7920) est raccordé, sur le plan électrique, à cette source d'alimentation électrique lorsque cet élément isolant occupe une deuxième position d'isolement.

18. L'appareil que décrit la revendication 17, si ce n'est qu'une partie de l'élément isolant est au contact d'une partie de la source d'alimentation électrique lorsque cet élément isolant occupe la première position d'isolement, et cet élément isolant se situe à l'écart de cette partie de la source d'alimentation électrique lorsque cet élément isolant occupe la deuxième position d'isolement.

19. L'appareil que décrit l'une ou l'autre des revendications 1 à 5, si ce n'est que la sortie électronique provoque une première sortie sonore et que cet appareil comporte, en outre :
un couvercle (3810, 4810) mobile et rattaché au coffret, et une partie isolante (4860) de ce couvercle est au contact de ce système à circuit électronique (4900, 7920) lorsque ce couvercle occupe une première position de couvercle, une partie externe (3840, 4840) de ce couvercle est implantée autour d'une partie du socle (3520, 4520, 7520) lorsque ce couvercle occupe la première position du couvercle, la partie isolante se situe à l'écart du système à circuit électronique (4900, 7920) et la partie externe se trouve à l'écart du socle (3520, 4520, 7520) lorsque ce couvercle occupe la deuxième position de couvercle, la partie isolante est configurée pour activer le système à circuit électronique (4900, 7920) lorsque le couvercle passe de la première position du couvercle à la deuxième position du couvercle
le système à circuit électronique (4900, 7920), configuré pour produire une deuxième sortie sonore lorsque le système à circuit électronique (4900, 7920) est activé par la partie isolante du couvercle.

20. L'appareil que décrit la revendication 19, si ce n'est que :
le système à circuit électronique (4900, 7920) comporte un processeur et une source d'alimentation électrique et que
la partie isolante du couvercle vient s'intercaler entre une borne de la source d'alimentation électrique et un contact électrique lorsque le couvercle occupe la première position du couvercle, et cette première partie est à l'écart de cette borne de la source d'alimentation électrique pour raccorder, sur le plan électrique, le processeur à la source d'alimentation électrique lorsque ce couvercle occupe la deuxième position du couvercle.

21. L'appareil que décrit l'une ou l'autre des revendications 1 à 5, si ce n'est que la partie occupée par l'élément de verrouillage (3710, 4710, 6710, 7710) comporte une partie saillante (4730) configurée pour détacher la parie contenant la carte à circuits imprimés (4922) lorsque l'élément de verrouillage (3710, 4710, 6710, 7710) passe de la première position à la deuxième position.

22. L'appareil que décrit l'une ou l'autre des revendications 1 à 5, si ce n'est que le contacteur est configuré pour passer, de manière irréversible, du premier état au deuxième état.

23. L'appareil que décrit l'une ou l'autre des revendications 1 à 5, si ce n'est que le dispositif pour l'administration d'un médicament (3002, 4002, 6002, 7002) est l'une des possibilités suivantes : un stylo injecteur, un auto-injecteur, un inhalateur ou un dispositif d'administration transdermique.
